# EUROPEAN PATENT SPECIFICATION

(11) **EP 0 885 299 B1**
(45) Date of publication and mention of the grant of the patent: **26.10.2005**
(21) Application number: 97901961.9
(22) Date of filing: 07.01.1997
(51) Int. Cl.: C12N 15/12, C07K 14/715, C07K 16/46, C07K 19/00, C07K 16/28, C12N 15/62, A61K 39/395, C12N 5/10, C12N 15/85, G01N 33/577, G01N 33/68

(54) **OB RECEPTOR AND LIGANDS**
OB-REZEPTOR UND LIGANDEN
RECEPTEUR ET LIGANDS OB

(30) Priority: 08.01.1996 US 585005; 20.06.1996 US 667197
(43) Date of publication of application: 23.12.1998
(60) Divisional application: 05018563.6
(73) Proprietor: GENENTECH, INC., South San Francisco, CA 94080-4990 (US)
(72) Inventor: BENNETT, Brian, Thousand Oaks CA 91360 (US); CARTER, Paul, J., San Francisco, CA 94116 (US); CHIANG, Nancy, Y., San Francisco, CA 94116 (US); KIM, Kyung, Jin, Los Altos, CA 94024 (US); MATTHEWS, William, Woodside, CA 94062 (US); RODRIGUES, Maria, L., South San Francisco, CA 94080 (US)
(74) Representative: Kremer, Simon Mark
(86) International application number: PCT/US1997/000325
(87) International publication number: WO 1997/025425

(56) References cited:
- WO-A-91/01743
- WO-A-94/05332
- WO-A-96/08510
- US-A- 5 378 808
- CELL, vol. 83, no. 7, 29 December 1995, pages 1263-1271, XP000602068 TARTAGLIA L A ET AL: "IDENTIFICATION AND EXPRESSION CLONING OF A LEPTIN RECEPTOR, OB-R" cited in the application
- PROCEEDINGS OF THE NATIONAL ACADEMY OF SCIENCE USA, vol. 88, 1991, pages 10535-10539, XP002029642 ASHKENAZI ET AL.: "Protection against endotoxic shock by a tumor necrosis factor receptor immunoadhesin" cited in the application
- SCIENCE, vol. 271, 5 January 1996, page 29 XP002029643 BARINAGA M: "Obesity: Leptin receptor weighs in"
- CURRENT BIOLOGY, vol. 6, no. 9, 1 September 1996, pages 1170-1180, XP000673008 BENNET ET AL.: "A role for Leptin and its cognate receptor in hematopoiesis"

### SEQUENCE LISTING

(1) GENERAL INFORMATION:
   (i) APPLICANT: GENENTECH, INC.
   (ii) TITLE OF INVENTION: WSX RECEPTOR AND LIGANDS
   (iii) NUMBER OF SEQUENCES: 51
   (iv) CORRESPONDENCE ADDRESS:
      (A) ADDRESSEE: Genentech, Inc.
      (B) STREET: 460 Point San Bruno Blvd
      (C) CITY: South San Francisco
      (D) STATE: California
      (E) COUNTRY: USA
      (F) ZIP: 94080
   (v) COMPUTER READABLE FORM:
      (A) MEDIUM TYPE: 3.5 inch, 1.44 Mb floppy disk
      (B) COMPUTER: IBM PC compatible
      (C) OPERATING SYSTEM: PC-DOS/MS-DOS
      (D) SOFTWARE: WinPatin (Genentech)
   (vi) CURRENT APPLICATION DATA:
      (A) APPLICATION NUMBER:
      (B) FILING DATE:
      (C) CLASSIFICATION:
   (vii) PRIOR APPLICATION DATA:
      (A) APPLICATION NUMBER: 08/667197
      (B) FILING DATE: 06/20/96
   (vii) PRIOR APPLICATION DATA:
      (A) APPLICATION NUMBER: 08/585005
      (B) FILING DATE: 01/08/96
   (viii) ATTORNEY/AGENT INFORMATION:
      (A) NAME: Lee, Wendy M.
      (B) REGISTRATION NUMBER: 40,378
      (C) REFERENCE/DOCKET NUMBER: P0986P2PCT
   (ix) TELECOMMUNICATION INFORMATION:
      (A) TELEPHONE: 415/225-1994
      (B) TELEFAX: 415/952-9881
      (C) TELEX: 910/371-7168
(2) INFORMATION FOR SEQ ID NO:1:
   (i) SEQUENCE CHARACTERISTICS:
      (A) LENGTH: 4102 base pairs
      (B) TYPE: Nucleic Acid
      (C) STRANDEDNESS: Double
      (D) TOPOLOGY: Linear
   (xi) SEQUENCE DESCRIPTION: SEQ ID NO:1:
   GAATTCTCGA GTCGACGGCG GGCGTTAAAG CTCTCGTGGC ATTATCCTTC 50
   AGTGGGGCTA TTGGACTGAC TTTTCTTATG CTGGGATGTG CCTTAGAGGA 100
   TTATGGGTGT ACTTCTCTGA AGTAAGATGA TTTGTCAAAA ATTCTGTGTG 150
   GTTTTGTTAC ATTGGGAATT TATTTATGTG ATAACTGCGT TTAACTTGTC 200
   ATATCCAATT ACTCCTTGGA GATTTAAGTT GTCTTGCATG CCACCAAATT 250
   CAACCTATGA CTACTTCCTT TTGCCTGCTG GACTCTCAAA GAATACTTCA 300
   AATTCGAATG GACATTATGA GACAGCTGTT GAACCTAAGT TTAATTCAAG 350
   TGGTACTCAC TTTTCTAACT TATCCAAAAC AACTTTCCAC TGTTGCTTTC 400
   GGAGTGAGCA AGATAGAAAC TGCTCCTTAT GTGCAGACAA CATTGAAGGA 450
   AAGACATTTG TTTCAACAGT AAATTCTTTA GTTTTTCAAC AAATAGATGC 500
   AAACTGGAAC ATACAGTGCT GGCTAAAAGG AGACTTAAAA TTATTCATCT 550
   GTTATGTGGA GTCATTATTT AAGAATCTAT TCAGGAATTA TAACTATAAG 600
   GTCCATCTTT TATATGTTCT GCCTGAAGTG TTAGAAGATT CACCTCTGGT 650
   TCCCCAAAAA GGCAGTTTTC AGATGGTTCA CTGCAATTGC AGTGTTCATG 700
   AATGTTGTGA ATGTCTTGTG CCTGTGCCAA CAGCCAAACT CAACGACACT 750
   CTCCTTATGT GTTTGAAAAT CACATCTGGT GGAGTAATTT TCCAGTCACC 800
   TCTAATGTCA GTTCAGCCCA TAAATATGGT GAAGCCTGAT CCACCATTAG 850
   GTTTGCATAT GGAAATCACA GATGATGGTA ATTTAAAGAT TTCTTGGTCC 900
   AGCCCACCAT TGGTACCATT TCCACTTCAA TATCAAGTGA AATATTCAGA 950
   GAATTCTACA ACAGTTATCA GAGAAGCTGA CAAGATTGTC TCAGCTACAT 1000
   CCCTGCTAGT AGACAGTATA CTTCCTGGGT CTTCGTATGA GGTTCAGGTG 1050
   AGGGGCAAGA GACTGGATGG CCCAGGAATC TGGAGTGACT GGAGTACTCC 1100
   TCGTGTCTTT ACCACACAAG ATGTCATATA CTTTCCACCT AAAATTCTGA 1150
   CAAGTGTTGG GTCTAATGTT TCTTTTCACT GCATCTATAA GAAGGAAAAC 1200
   AAGATTGTTC CCTCAAAAGA GATTGTTTGG TGGATGAATT TAGCTGAGAA 1250
   AATTCCTCAA AGCCAGTATG ATGTTGTGAG TGATCATGTT AGCAAAGTTA 1300
   CTTTTTTCAA TCTGAATGAA ACCAAACCTC GAGGAAAGTT TACCTATGAT 1350
   GCAGTGTACT GCTGCAATGA ACATGAATGC CATCATCGCT ATGCTGAATT 1400
   ATATGTGATT GATGTCAATA TCAATATCTC ATGTGAAACT GATGGGTACT 1450
   TAACTAAAAT GACTTGCAGA TGGTCAACCA GTACAATCCA GTCACTTGCG 1500
   GAAAGCACTT TGCAATTGAG GTATCATAGG AGCAGCCTTT ACTGTTCTGA 1550
   TATTCCATCT ATTCATCCCA TATCTGAGCC CAAAGATTGC TATTTGCAGA 1600
   GTGATGGTTT TTATGAATGC ATTTTCCAGC CAATCTTCCT ATTATCTGGC 1650
   TACACAATGT GGATTAGGAT CAATCACTCT CTAGGTTCAC TTGACTCTCC 1700
   ACCAACATGT GTCCTTCCTG ATTCTGTGGT GAAGCCACTG CCTCCATCCA 1750
   GTGTGAAAGC AGAAATTACT ATAAACATTG GATTATTGAA AATATCTTGG 1800
   GAAAAGCCAG TCTTTCCAGA GAATAACCTT CAATTCCAGA TTCGCTATGG 1850
   TTTAAGTGGA AAAGAAGTAC AATGGAAGAT GTATGAGGTT TATGATGCAA 1900
   AATCAAAATC TGTCAGTCTC CCAGTTCCAG ACTTGTGTGC AGTCTATGCT 1950
   GTTCAGGTGC GCTGTAAGAG GCTAGATGGA CTGGGATATT GGAGTAATTG 2000
   GAGCAATCCA GCCTACACAG TTGTCATGGA TATAAAAGTT CCTATGAGAG 2050
   GACCTGAATT TTGGAGAATA ATTAATGGAG ATACTATGAA AAAGGAGAAA 2100
   AATGTCACTT TACTTTGGAA GCCCCTGATG AAAAATGACT CATTGTGCAG 2150
   TGTTCAGAGA TATGTGATAA ACCATCATAC TTCCTGCAAT GGAACATGGT 2200
   CAGAAGATGT GGGAAATCAC ACGAAATTCA CTTTCCTGTG GACAGAGCAA 2250
   GCACATACTG TTACGGTTCT GGCCATCAAT TCAATTGGTG CTTCTGTTGC 2300
   AAATTTTAAT TTAACCTTTT CATGGCCTAT GAGCAAAGTA AATATCGTGC 2350
   AGTCACTCAG TGCTTATCCT TTAAACAGCA GTTGTGTGAT TGTTTCCTGG 2400
   ATACTATCAC CCAGTGATTA CAAGCTAATG TATTTTATTA TTGAGTGGAA 2450
   AAATCTTAAT GAAGATGGTG AAATAAAATG GCTTAGAATC TCTTCATCTG 2500
   TTAAGAAGTA TTATATCCAT GATCATTTTA TCCCCATTGA GAAGTACCAG 2550
   TTCAGTCTTT ACCCAATATT TATGGAAGGA GTGGGAAAAC CAAAGATAAT 2600
   TAATAGTTTC ACTCAAGATG ATATTGAAAA ACACCAGAGT GATGCAGGTT 2650
   TATATGTAAT TGTGCCAGTA ATTATTTCCT CTTCCATCTT ATTGCTTGGA 2700
   ACATTATTAA TATCACACCA AAGAATGAAA AAGCTATTTT GGGAAGATGT 2750
   TCCGAACCCC AAGAATTGTT CCTGGGCACA AGGACTTAAT TTTCAGAAGC 2800
   CAGAAACGTT TGAGCATCTT TTTATCAAGC ATACAGCATC AGTGACATGT 2850
   GGTCCTCTTC TTTTGGAGCC TGAAACAATT TCAGAAGATA TCAGTGTTGA 2900
   TACATCATGG AAAAATAAAG ATGAGATGAT GCCAACAACT GTGGTCTCTC 2950
   TACTTTCAAC AACAGATCTT GAAAAGGGTT CTGTTTGTAT TAGTGACCAG 3000
   TTCAACAGTG TTAACTTCTC TGAGGCTGAG GGTACTGAGG TAACCTATGA 3050
   GGACGAAAGC CAGAGACAAC CCTTTGTTAA ATACGCCACG CTGATCAGCA 3100
   ACTCTAAACC AAGTGAAACT GGTGAAGAAC AAGGGCTTAT AAATAGTTCA 3150
   GTCACCAAGT GCTTCTCTAG CAAAAATTCT CCGTTGAAGG ATTCTTTCTC 3200
   TAATAGCTCA TGGGAGATAG AGGCCCAGGC ATTTTTTATA TTATCAGATC 3250
   AGCATCCCAA CATAATTTCA CCACACCTCA CATTCTCAGA AGGATTGGAT 3300
   GAACTTTTGA AATTGGAGGG AAATTTCCCT GAAGAAAATA ATGATAAAAA 3350
   GTCTATCTAT TATTTAGGGG TCACCTCAAT CAAAAAGAGA GAGAGTGGTG 3400
   TGCTTTTGAC TGACAAGTCA AGGGTATCGT GCCCATTCCC AGCCCCCTGT 3450
   TTATTCACGG ACATCAGAGT TCTCCAGGAC AGTTGCTCAC ACTTTGTAGA 3500
   AAATAATATC AACTTAGGAA CTTCTAGTAA GAAGACTTTT GCATCTTACA 3550
   TGCCTCAATT CCAAACTTGT TCTACTCAGA CTCATAAGAT CATGGAAAAC 3600
   AAGATGTGTG ACCTAACTGT GTAATTTCAC TGAAGAAACC TTCAGATTTG 3650
   TGTTATAATG GGTAATATAA AGTGTAATAG ATTATAGTTG TGGGTGGGAG 3700
   AGAGAAAAGA AACCAGAGTC AAATTTGAAA ATAATTGTTC CAAATGAATG 3750
   TTGTCTGTTT GTTCTCTCTT AGTAACATAG ACAAAAAATT TGAGAAAGCC 3800
   TTCATAAGCC TACCAATGTA GACACGCTCT TCTATTTTAT TCCCAAGCTC 3850
   TAGTGGGAAG GTCCCTTGTT TCCAGCTAGA AATAAGCCCA ACAGACACCA 3900
   TCTTTTGTGA GATGTAATTG TTTTTTCAGA GGGCGTGTTG TTTTACCTCA 3950
   AGTTTTTGTT TTGTACCAAC ACACACACAC ACACACATTC TTAACACATG 4000
   TCCTTGTGTG TTTTGAGAGT ATATTATGTA TTTATATTTT GTGCTATCAG 4050
   ACTGTAGGAT TTGAAGTAGG ACTTTCCTAA ATGTTTAAGA TAAACAGAAT 4100
   TC 4102
(2) INFORMATION FOR SEQ ID NO:2:
   (i) SEQUENCE CHARACTERISTICS:
      (A) LENGTH: 1165 amino acids
      (B) TYPE: Amino Acid
      (D) TOPOLOGY: Linear
   (xi) SEQUENCE DESCRIPTION: SEQ ID NO:2:
(2) INFORMATION FOR SEQ ID NO:3:
   (i) SEQUENCE CHARACTERISTICS:
      (A) LENGTH: 896 amino acids
      (B) TYPE: Amino Acid
      (D) TOPOLOGY: Linear
   (xi) SEQUENCE DESCRIPTION: SEQ ID NO:3:
   Met Ile Cys Gln Lys Phe Cys Val Val Leu Leu His Trp Glu Phe
   1 5 10 15
   Ile Tyr Val Ile Thr Ala Phe Asn Leu Ser Tyr Pro Ile Thr Pro
   20 25 30
   Trp Arg Phe Lys Leu Ser Cys Met Pro Pro Asn Ser Thr Tyr Asp
   35 40 45
   Tyr Phe Leu Leu Pro Ala Gly Leu Ser Lys Asn Thr Ser Asn Ser
   50 55 60
   Asn Gly His Tyr Glu Thr Ala Val Glu Pro Lys Phe Asn Ser Ser
   65 70 75
   Gly Thr His Phe Ser Asn Leu Ser Lys Thr Thr Phe His Cys Cys
   80 85 90
   Phe Arg Ser Glu Gln Asp Arg Asn Cys Ser Leu Cys Ala Asp Asn
   95 100 105
   Ile Glu Gly Lys Thr Phe Val Ser Thr Val Asn Ser Leu Val Phe
   110 115 120
   Gln Gln Ile Asp Ala Asn Trp Asn Ile Gln Cys Trp Leu Lys Gly
   125 130 135
   Asp Leu Lys Leu Phe Ile Cys Tyr Val Glu Ser Leu Phe Lys Asn
   140 145 150
   Leu Phe Arg Asn Tyr Asn Tyr Lys Val His Leu Leu Tyr Val Leu
   155 160 165
   Pro Glu Val Leu Glu Asp Ser Pro Leu Val Pro Gln Lys Gly Ser
   170 175 180
   Phe Gln Met Val His Cys Asn Cys Ser Val His Glu Cys Cys Glu
   185 190 195
   Cys Leu Val Pro Val Pro Thr Ala Lys Leu Asn Asp Thr Leu Leu
   200 205 210
   Met Cys Leu Lys Ile Thr Ser Gly Gly Val Ile Phe Gln Ser Pro
   215 220 225
   Leu Met Ser Val Gln Pro Ile Asn Met Val Lys Pro Asp Pro Pro
   230 235 240
   Leu Gly Leu His Met Glu Ile Thr Asp Asp Gly Asn Leu Lys Ile
   245 250 255
   Ser Trp Ser Ser Pro Pro Leu Val Pro Phe Pro Leu Gln Tyr Gln
   260 265 270
   Val Lys Tyr Ser Glu Asn Ser Thr Thr Val Ile Arg Glu Ala Asp
   275 280 285
   Lys Ile Val Ser Ala Thr Ser Leu Leu Val Asp Ser Ile Leu Pro
   290 295 300
   Gly Ser Ser Tyr Glu Val Gln Val Arg Gly Lys Arg Leu Asp Gly
   305 310 315
   Pro Gly Ile Trp Ser Asp Trp Ser Thr Pro Arg Val Phe Thr Thr
   320 325 330
   Gln Asp Val Ile Tyr Phe Pro Pro Lys Ile Leu Thr Ser Val Gly
   335 340 345
   Ser Asn Val Ser Phe His Cys Ile Tyr Lys Lys Glu Asn Lys Ile
   350 355 360
   Val Pro Ser Lys Glu Ile Val Trp Trp Met Asn Leu Ala Glu Lys
   365 370 375
   Ile Pro Gln Ser Gln Tyr Asp Val Val Ser Asp His Val Ser Lys
   380 385 390
   Val Thr Phe Phe Asn Leu Asn Glu Thr Lys Pro Arg Gly Lys Phe
   395 400 405
   Thr Tyr Asp Ala Val Tyr Cys Cys Asn Glu His Glu Cys His His
   410 415 420
   Arg Tyr Ala Glu Leu Tyr Val Ile Asp Val Asn Ile Asn Ile Ser
   425 430 435
   Cys Glu Thr Asp Gly Tyr Leu Thr Lys Met Thr Cys Arg Trp Ser
   440 445 450
   Thr Ser Thr Ile Gln Ser Leu Ala Glu Ser Thr Leu Gln Leu Arg
   455 460 465
   Tyr His Arg Ser Ser Leu Tyr Cys Ser Asp Ile Pro Ser Ile His
   470 475 480
   Pro Ile Ser Glu Pro Lys Asp Cys Tyr Leu Gln Ser Asp Gly Phe
   485 490 495
   Tyr Glu Cys Ile Phe Gln Pro Ile Phe Leu Leu Ser Gly Tyr Thr
   500 505 510
   Met Trp Ile Arg Ile Asn His Ser Leu Gly Ser Leu Asp Ser Pro
   515 520 525
   Pro Thr Cys Val Leu Pro Asp Ser Val Val Lys Pro Leu Pro Pro
   530 535 540
   Ser Ser Val Lys Ala Glu Ile Thr Ile Asn Ile Gly Leu Leu Lys
   545 550 555
   Ile Ser Trp Glu Lys Pro Val Phe Pro Glu Asn Asn Leu Gln Phe
   560 565 570
   Gln Ile Arg Tyr Gly Leu Ser Gly Lys Glu Val Gln Trp Lys Met
   575 580 585
   Tyr Glu Val Tyr Asp Ala Lys Ser Lys Ser Val Ser Leu Pro Val
   590 595 600
   Pro Asp Leu Cys Ala Val Tyr Ala Val Gln Val Arg Cys Lys Arg
   605 610 615
   Leu Asp Gly Leu Gly Tyr Trp Ser Asn Trp Ser Asn Pro Ala Tyr
   620 625 630
   Thr Val Val Met Asp Ile Lys Val Pro Met Arg Gly Pro Glu Phe
   635 640 645
   Trp Arg Ile Ile Asn Gly Asp Thr Met Lys Lys Glu Lys Asn Val
   650 655 660
   Thr Leu Leu Trp Lys Pro Leu Met Lys Asn Asp Ser Leu Cys Ser
   665 670 675
   Val Gln Arg Tyr Val Ile Asn His His Thr Ser Cys Asn Gly Thr
   680 685 690
   Trp Ser Glu Asp Val Gly Asn His Thr Lys Phe Thr Phe Leu Trp
   695 700 705
   Thr Glu Gln Ala His Thr Val Thr Val Leu Ala Ile Asn Ser Ile
   710 715 720
   Gly Ala Ser Val Ala Asn Phe Asn Leu Thr Phe Ser Trp Pro Met
   725 730 735
   Ser Lys Val Asn Ile Val Gln Ser Leu Ser Ala Tyr Pro Leu Asn
   740 745 750
   Ser Ser Cys Val Ile Val Ser Trp Ile Leu Ser Pro Ser Asp Tyr
   755 760 765
   Lys Leu Met Tyr Phe Ile Ile Glu Trp Lys Asn Leu Asn Glu Asp
   770 775 780
   Gly Glu Ile Lys Trp Leu Arg Ile Ser Ser Ser Val Lys Lys Tyr
   785 790 795
   Tyr Ile His Asp His Phe Ile Pro Ile Glu Lys Tyr Gln Phe Ser
   800 805 810
   Leu Tyr Pro Ile Phe Met Glu Gly Val Gly Lys Pro Lys Ile Ile
   815 820 825
   Asn Ser Phe Thr Gln Asp Asp Ile Glu Lys His Gln Ser Asp Ala
   830 835 840
   Gly Leu Tyr Val Ile Val Pro Val Ile Ile Ser Ser Ser Ile Leu
   845 850 855
   Leu Leu Gly Thr Leu Leu Ile Ser His Gln Arg Met Lys Lys Leu
   860 865 870
   Phe Trp Glu Asp Val Pro Asn Pro Lys Asn Cys Ser Trp Ala Gln
   875 880 885
   Gly Leu Asn Phe Gln Lys Arg Thr Asp Ile Leu
   890 895 896
(2) INFORMATION FOR SEQ ID NO:4:
   (i) SEQUENCE CHARACTERISTICS:
      (A) LENGTH: 923 amino acids
      (B) TYPE: Amino Acid
      (D) TOPOLOGY: Linear
   (xi) SEQUENCE DESCRIPTION: SEQ ID NO:4:
   Met Ile Cys Gln Lys Phe Cys Val Val Leu Leu His Trp Glu Phe
   1 5 10 15
   Ile Tyr Val Ile Thr Ala Phe Asn Leu Ser Tyr Pro Ile Thr Pro
   20 25 30
   Trp Arg Phe Lys Leu Ser Cys Met Pro Pro Asn Ser Thr Tyr Asp
   35 40 45
   Tyr Phe Leu Leu Pro Ala Gly Leu Ser Lys Asn Thr Ser Asn Ser
   50 55 60
   Asn Gly His Tyr Glu Thr Ala Val Glu Pro Lys Phe Asn Ser Ser
   65 70 75
   Gly Thr His Phe Ser Asn Leu Ser Lys Thr Thr Phe His Cys Cys
   80 85 90
   Phe Arg Ser Glu Gln Asp Arg Asn Cys Ser Leu Cys Ala Asp Asn
   95 100 105
   Ile Glu Gly Lys Thr Phe Val Ser Thr Val Asn Ser Leu Val Phe
   110 115 120
   Gln Gln Ile Asp Ala Asn Trp Asn Ile Gln Cys Trp Leu Lys Gly
   125 130 135
   Asp Leu Lys Leu Phe Ile Cys Tyr Val Glu Ser Leu Phe Lys Asn
   140 145 150
   Leu Phe Arg Asn Tyr Asn Tyr Lys Val His Leu Leu Tyr Val Leu
   155 160 165
   Pro Glu Val Leu Glu Asp Ser Pro Leu Val Pro Gln Lys Gly Ser
   170 175 180
   Phe Gln Met Val His Cys Asn Cys Ser Val His Glu Cys Cys Glu
   185 190 195
   Cys Leu Val Pro Val Pro Thr Ala Lys Leu Asn Asp Thr Leu Leu
   200 205 210
   Met Cys Leu Lys Ile Thr Ser Gly Gly Val Ile Phe Gln Ser Pro
   215 220 225
   Leu Met Ser Val Gln Pro Ile Asn Met Val Lys Pro Asp Pro Pro
   230 235 240
   Leu Gly Leu His Met Glu Ile Thr Asp Asp Gly Asn Leu Lys Ile
   245 250 255
   Ser Trp Ser Ser Pro Pro Leu Val Pro Phe Pro Leu Gln Tyr Gln
   260 265 270
   Val Lys Tyr Ser Glu Asn Ser Thr Thr Val Ile Arg Glu Ala Asp
   275 280 285
   Lys Ile Val Ser Ala Thr Ser Leu Leu Val Asp Ser Ile Leu Pro
   290 295 300
   Gly Ser Ser Tyr Glu Val Gln Val Arg Gly Lys Arg Leu Asp Gly
   305 310 315
   Pro Gly Ile Trp Ser Asp Trp Ser Thr Pro Arg Val Phe Thr Thr
   320 325 330
   Gln Asp Val Ile Tyr Phe Pro Pro Lys Ile Leu Thr Ser Val Gly
   335 340 345
   Ser Asn Val Ser Phe His Cys Ile Tyr Lys Lys Glu Asn Lys Ile
   350 355 360
   Val Pro Ser Lys Glu Ile Val Trp Trp Met Asn Leu Ala Glu Lys
   365 370 375
   Ile Pro Gln Ser Gln Tyr Asp Val Val Ser Asp His Val Ser Lys
   380 385 390
   Val Thr Phe Phe Asn Leu Asn Glu Thr Lys Pro Arg Gly Lys Phe
   395 400 405
   Thr Tyr Asp Ala Val Tyr Cys Cys Asn Glu His Glu Cys His His
   410 415 420
   Arg Tyr Ala Glu Leu Tyr Val Ile Asp Val Asn Ile Asn Ile Ser
   425 430 435
   Cys Glu Thr Asp Gly Tyr Leu Thr Lys Met Thr Cys Arg Trp Ser
   440 445 450
   Thr Ser Thr Ile Gln Ser Leu Ala Glu Ser Thr Leu Gln Leu Arg
   455 460 465
   Tyr His Arg Ser Ser Leu Tyr Cys Ser Asp Ile Pro Ser Ile His
   470 475 480
   Pro Ile Ser Glu Pro Lys Asp Cys Tyr Leu Gln Ser Asp Gly Phe
   485 490 495
   Tyr Glu Cys Ile Phe Gln Pro Ile Phe Leu Leu Ser Gly Tyr Thr
   500 505 510
   Met Trp Ile Arg Ile Asn His Ser Leu Gly Ser Leu Asp Ser Pro
   515 520 525
   Pro Thr Cys Val Leu Pro Asp Ser Val Val Lys Pro Leu Pro Pro
   530 535 540
   Ser Ser Val Lys Ala Glu Ile Thr Ile Asn Ile Gly Leu Leu Lys
   545 550 555
   Ile Ser Trp Glu Lys Pro Val Phe Pro Glu Asn Asn Leu Gln Phe
   560 565 570
   Gln Ile Arg Tyr Gly Leu Ser Gly Lys Glu Val Gln Trp Lys Met
   575 580 585
   Tyr Glu Val Tyr Asp Ala Lys Ser Lys Ser Val Ser Leu Pro Val
   590 595 600
   Pro Asp Leu Cys Ala Val Tyr Ala Val Gln Val Arg Cys Lys Arg
   605 610 615
   Leu Asp Gly Leu Gly Tyr Trp Ser Asn Trp Ser Asn Pro Ala Tyr
   620 625 630
   Thr Val Val Met Asp Ile Lys Val Pro Met Arg Gly Pro Glu Phe
   635 640 645
   Trp Arg Ile Ile Asn Gly Asp Thr Met Lys Lys Glu Lys Asn Val
   650 655 660
   Thr Leu Leu Trp Lys Pro Leu Met Lys Asn Asp Ser Leu Cys Ser
   665 670 675
   Val Gln Arg Tyr Val Ile Asn His His Thr Ser Cys Asn Gly Thr
   680 685 690
   Trp Ser Glu Asp Val Gly Asn His Thr Lys Phe Thr Phe Leu Trp
   695 700 705
   Thr Glu Gln Ala His Thr Val Thr Val Leu Ala Ile Asn Ser Ile
   710 715 720
   Gly Ala Ser Val Ala Asn Phe Asn Leu Thr Phe Ser Trp Pro Met
   725 730 735
   Ser Lys Val Asn Ile Val Gln Ser Leu Ser Ala Tyr Pro Leu Asn
   740 745 750
   Ser Ser Cys Val Ile Val Ser Trp Ile Leu Ser Pro Ser Asp Tyr
   755 760 765
   Lys Leu Met Tyr Phe Ile Ile Glu Trp Lys Asn Leu Asn Glu Asp
   770 775 780
   Gly Glu Ile Lys Trp Leu Arg Ile Ser Ser Ser Val Lys Lys Tyr
   785 790 795
   Tyr Ile His Asp His Phe Ile Pro Ile Glu Lys Tyr Gln Phe Ser
   800 805 810
   Leu Tyr Pro Ile Phe Met Glu Gly Val Gly Lys Pro Lys Ile Ile
                   815                 820                 825
   Asn Ser Phe Thr Gln Asp Asp Ile Glu Lys His Gln Ser Asp Ala
                   830                 835                 840
   Gly Leu Tyr Val Ile Val Pro Val Ile Ile Ser Ser Ser Ile Leu
                   845                 850                 855
   Leu Leu Gly Thr Leu Leu Ile Ser His Gln Arg Met Lys Lys Leu
                   860                 865                 870
   Phe Trp Glu Asp Val Pro Asn Pro Lys Asn Cys Ser Trp Ala Gln
                   875                 880                 885
   Gly Leu Asn Phe Gln Lys Met Phe Arg Thr Pro Arg Ile Val Pro
                   890                 895                 900
   Gly His Lys Asp Leu Ile Phe Arg Arg Cys Leu Lys Ala Ala Cys
                   905                 910                 915
   Ser Leu Arg Val Ile Thr Thr Pro
                   920         923
(2) INFORMATION FOR SEQ ID NO:5:
   (i) SEQUENCE CHARACTERISTICS:
      (A) LENGTH: 3004 base pairs
      (B) TYPE: Nucleic Acid
      (C) STRANDEDNESS: Single
      (D) TOPOLOGY: Linear
   (xi) SEQUENCE DESCRIPTION: SEQ ID NO:5:
   GAATTCCGGG TTAAAGCTCT CGTGGCATTA TCCTTCAGTG GGGCTATTGG 50
   ACTGACTTTT CTTATGCTGG GATGTGCCTT AGAGGATTAT GGATTTGCCA 100
   GTTCACCCTG ACCATCTTGA AAATAAGTTA TCTCTGATCT CTGTCTGTAT 150
   GTTACTTCTC TCCCCTCACC AATGGAGAAC AAATGTGGGC AAAGTGTACT 200
   TCTCTGAAGT AAGATGATTT GTCAAAAATT CTGTGTGGTT TTGTTACATT 250
   GGGAATTTAT TTATGTGATA ACTGCGTTTA ACTTGTCATA TCCAATTACT 300
   CCTTGGAGAT TTAAGTTGTC TTGCATGCCA CCAAATTCAA CCTATGACTA 350
   CTTCCTTTTG CCTGCTGGAC TCTCAAAGAA TACTTCAAAT TCGAATGGAC 400
   ATTATGAGAC AGCTGTTGAA CCTAAGTTTA ATTCAAGTGG TACTCACTTT 450
   TCTAACTTAT CCAAAACAAC TTTCCACTGT TGCTTTCGGA GTGAGCAAGA 500
   TAGAAACTGC TCCTTATGTG CAGACAACAT TGAAGGAAAG ACATTTGTTT 550
   CNACAGTAAA TTCTTTAGTT TTTCAACAAA TAGATGCAAA CTGGAACATA 600
   CAGTGCTGGC TAAAAGGAGA CTTAAAATTA TTCATCTGTT ATGTGGAGTC 650
   ATTATTTAAG AATCTATTCA GGAATTATAA CTATAAGGTC CATCTTTTAT 700
   ATGTTCTGCC TGAAGTGTTA GAAGATTCAC CTCTGGTTCC CCAAAAAGGC 750
   AGTTTTCAGA TGGTTCACTG CAATTGCAGT GTTCATGAAT GTTGTGAATG 800
   TCTTGTGCCT GTGCCAACAG CCAAACTCAA CGACACTCTC CTTATGTGTT 850
   TGAAAATCAC ATCTGGTGGA GTAATTTTCC AGTCACCTCT AATGTCAGTT 900
   CAGCCCATAA ATATGGTGAA GCCTGATCCA CCATTAGGTT TGCATATGGA 950
   AATCACAGAT GATGGTAATT TAAAGATTTC TTGGTCCAGC CCACCATTGG 1000
   TACCATTTCC ACTTCAATAT CAAGTGAAAT ATTCAGAGAA TTCTACAACA 1050
   GTTATCAGAG AAGCTGACAA GATTGTCTCA GCTACATCCC TGCTAGTAGA 1100
   CAGTATACTT CCTGGGTCTT CGTATGAGGT TCAGGTGAGG GGCAAGAGAC 1150
   TGGATGGCCC AGGAATCTGG AGTGACTGGA GTACTCCTCG TGTCTTTACC 1200
   ACACAAGATG TCATATACTT TCCACCTAAA ATTCTGACAA GTGTTGGGTC 1250
   TAATGTTTCT TTTCACTGCA TCTATAAGAA GGAAAACAAG ATTGTTCCCT 1300
   CAAAAGAGAT TGTTTGGTGG ATGAATTTAG CTGAGAAAAT TCCTCAAAGC 1350
   CAGTATGATG TTGTGAGTGA TCATGTTAGC AAAGTTACTT TTTTCAATCT 1400
   GAATGAAACC AAACCTCGAG GAAAGTTTAC CTATGATGCA GTGTACTGCT 1450
   GCAATGAACA TGAATGCCAT CATCGCTATG CTGAATTATA TGTGATTGAT 1500
   GTCAATATCA ATATCTCATG TGAAACTGAT GGGTACTTAA CTAAAATGAC 1550
   TTGCAGATGG TCAACCAGTA CAATCCAGTC ACTTGCGGAA AGCACTTTGC 1600
   AATTGAGGTA TCATAGGAGC AGCCTTTACT GTTCTGATAT TCCATCTATT 1650
   CATCCCATAT CTGAGCCCAA AGATTGCTAT TTGCAGAGTG ATGGTTTTTA 1700
   TGAATGCATT TTCCAGCCAA TCTTCCTATT ATCTGGCTAC ACAATGTGGA 1750
   TTAGGATCAA TCACTCTCTA GGTTCACTTG ACTCTCCACC AACATGTGTC 1800
   CTTCCTGATT CTGTGGTGAA GCCACTGCCT CCATCCAGTG TGAAAGCAGA 1850
   AATTACTATA AACATTGGAT TATTGAAAAT ATCTTGGGAA AAGCCAGTCT 1900
   TTCCAGAGAA TAACCTTCAA TTCCAGATTC GCTATGGTTT AAGTGGAAAA 1950
   GAAGTACAAT GGAAGATGTA TGAGGTTTAT GATGCAAAAT CAAAATCTGT 2000
   CAGTCTCCCA GTTCCAGACT TGTGTGCAGT CTATGCTGTT CAGGTGCGCT 2050
   GTAAGAGGCT AGATGGACTG GGATATTGGA GTAATTGGAG CAATCCAGCC 2100
   TACACAGTTG TCATGGATAT AAAAGTTCCT ATGAGAGGAC CTGAATTTTG 2150
   GAGAATAATT AATGGAGATA CTATGAAAAA GGAGAAAAAT GTCACTTTAC 2200
   TTTGGAAGCC CCTGATGAAA AATGACTCAT TGTGCAGTGT TCAGAGATAT 2250
   GTGATAAACC ATCATACTTC CTGCAATGGA ACATGGTCAG AAGATGTGGG 2300
   AAATCACACG AAATTCACTT TCCTGTGGAC AGAGCAAGCA CATACTGTTA 2350
   CGGTTCTGGC CATCAATTCA ATTGGTGCTT CTGTTGCAAA TTTTAATTTA 2400
   ACCTTTTCAT GGCCTATGAG CAAAGTAAAT ATCGTGCAGT CACTCAGTGC 2450
   TTATCCTTTA AACAGCAGTT GTGTGATTGT TTCCTGGATA CTATCACCCA 2500
   GTGATTACAA GCTAATGTAT TTTATTATTG AGTGGAAAAA TCTTAATGAA 2550
   GATGGTGAAA TAAAATGGCT TAGAATCTCT TCATCTGTTA AGAAGTATTA 2600
   TATCCATGAT CATTTTATCC CCATTGAGAA GTACCAGTTC AGTCTTTACC 2650
   CAATATTTAT GGAAGGAGTG GGAAAACCAA AGATAATTAA TAGTTTCACT 2700
   CAAGATGATA TTGAAAAACA CCAGAGTGAT GCAGGTTTAT ATGTAATTGT 2750
   GCCAGTAATT ATTTCCTCTT CCATCTTATT GCTTGGAACA TTATTAATAT 2800
   CACACCAAAG AATGAAAAAG CTATTTTGGG AAGATGTTCC GAACCCCAAG 2850
   AATTGTTCCT GGGCACAAGG ACTTAATTTT CAGAAGAGAA CGGACATTCT 2900
   TTGAAGTCTA ATCATGATCA CTACAGATGA ACCCAATGTG CCAACTTCCC 2950
   AACAGTCTAT AGAGTATTAG AAGATTTTTA CATTTTGAAG AAGGGCCGGA 3000
   ATTC 3004
(2) INFORMATION FOR SEQ ID NO:6:
   (i) SEQUENCE CHARACTERISTICS:
      (A) LENGTH: 3102 base pairs
      (B) TYPE: Nucleic Acid
      (C) STRANDEDNESS: Single
      (D) TOPOLOGY: Linear
   (xi) SEQUENCE DESCRIPTION: SEQ ID NO:6:
   GAATTCTCGA GTCGACGGCG GGCGTTAAAG CTCTCGTGGC ATTATCCTTC 50
   AGTGGGGCTA TTGGACTGAC TTTTCTTATG CTGGGATGTG CCTTAGAGGA 100
   TTATGGGTGT ACTTCTCTGA AGTAAGATGA TTTGTCAAAA ATTCTGTGTG 150
   GTTTTGTTAC ATTGGGAATT TATTTATGTG ATAACTGCGT TTAACTTGTC 200
   ATATCCAATT ACTCCTTGGA GATTTAAGTT GTCTTGCATG CCACCAAATT 250
   CAACCTATGA CTACTTCCTT TTGCCTGCTG GACTCTCAAA GAATACTTCA 300
   AATTCGAATG GACATTATGA GACAGCTGTT GAACCTAAGT TTAATTCAAG 350
   TGGTACTCAC TTTTCTAACT TATCCAAAAC AACTTTCCAC TGTTGCTTTC 400
   GGAGTGAGCA AGATAGAAAC TGCTCCTTAT GTGCAGACAA CATTGAAGGA 450
   AAGACATTTG TTTCAACAGT AAATTCTTTA GTTTTTCAAC AAATAGATGC 500
   AAACTGGAAC ATACAGTGCT GGCTAAAAGG AGACTTAAAA TTATTCATCT 550
   GTTATGTGGA GTCATTATTT AAGAATCTAT TCAGGAATTA TAACTATAAG 600
   GTCCATCTTT TATATGTTCT GCCTGAAGTG TTAGAAGATT CACCTCTGGT 650
   TCCCCAAAAA GGCAGTTTTC AGATGGTTCA CTGCAATTGC AGTGTTCATG 700
   AATGTTGTGA ATGTCTTGTG CCTGTGCCAA CAGCCAAACT CAACGACACT 750
   CTCCTTATGT GTTTGAAAAT CACATCTGGT GGAGTAATTT TCCAGTCACC 800
   TCTAATGTCA GTTCAGCCCA TAAATATGGT GAAGCCTGAT CCACCATTAG 850
   GTTTGCATAT GGAAATCACA GATGATGGTA ATTTAAAGAT TTCTTGGTCC 900
   AGCCCACCAT TGGTACCATT TCCACTTCAA TATCAAGTGA AATATTCAGA 950
   GAATTCTACA ACAGTTATCA GAGAAGCTGA CAAGATTGTC TCAGCTACAT 1000
   CCCTGCTAGT AGACAGTATA CTTCCTGGGT CTTCGTATGA GGTTCAGGTG 1050
   AGGGGCAAGA GACTGGATGG CCCAGGAATC TGGAGTGACT GGAGTACTCC 1100
   TCGTGTCTTT ACCACACAAG ATGTCATATA CTTTCCACCT AAAATTCTGA 1150
   CAAGTGTTGG GTCTAATGTT TCTTTTCACT GCATCTATAA GAAGGAAAAC 1200
   AAGATTGTTC CCTCAAAAGA GATTGTTTGG TGGATGAATT TAGCTGAGAA 1250
   AATTCCTCAA AGCCAGTATG ATGTTGTGAG TGATCATGTT AGCAAAGTTA 1300
   CTTTTTTCAA TCTGAATGAA ACCAAACCTC GAGGAAAGTT TACCTATGAT 1350
   GCAGTGTACT GCTGCAATGA ACATGAATGC CATCATCGCT ATGCTGAATT 1400
   ATATGTGATT GATGTCAATA TCAATATCTC ATGTGAAACT GATGGGTACT 1450
   TAACTAAAAT GACTTGCAGA TGGTCAACCA GTACAATCCA GTCACTTGCG 1500
   GAAAGCACTT TGCAATTGAG GTATCATAGG AGCAGCCTTT ACTGTTCTGA 1550
   TATTCCATCT ATTCATCCCA TATCTGAGCC CAAAGATTGC TATTTGCAGA 1600
   GTGATGGTTT TTATGAATGC ATTTTCCAGC CAATCTTCCT ATTATCTGGC 1650
   TACACAATGT GGATTAGGAT CAATCACTCT CTAGGTTCAC TTGACTCTCC 1700
   ACCAACATGT GTCCTTCCTG ATTCTGTGGT GAAGCCACTG CCTCCATCCA 1750
   GTGTGAAAGC AGAAATTACT ATAAACATTG GATTATTGAA AATATCTTGG 1800
   GAAAAGCCAG TCTTTCCAGA GAATAACCTT CAATTCCAGA TTCGCTATGG 1850
   TTTAAGTGGA AAAGAAGTAC AATGGAAGAT GTATGAGGTT TATGATGCAA 1900
   AATCAAAATC TGTCAGTCTC CCAGTTCCAG ACTTGTGTGC AGTCTATGCT 1950
   GTTCAGGTGC GCTGTAAGAG GCTAGATGGA CTGGGATATT GGAGTAATTG 2000
   GAGCAATCCA GCCTACACAG TTGTCATGGA TATAAAAGTT CCTATGAGAG 2050
   GACCTGAATT TTGGAGAATA ATTAATGGAG ATACTATGAA AAAGGAGAAA 2100
   AATGTCACTT TACTTTGGAA GCCCCTGATG AAAAATGACT CATTGTGCAG 2150
   TGTTCAGAGA TATGTGATAA ACCATCATAC TTCCTGCAAT GGAACATGGT 2200
   CAGAAGATGT GGGAAATCAC ACGAAATTCA CTTTCCTGTG GACAGAGCAA 2250
   GCACATACTG TTACGGTTCT GGCCATCAAT TCAATTGGTG CTTCTGTTGC 2300
   AAATTTTAAT TTAACCTTTT CATGGCCTAT GAGCAAAGTA AATATCGTGC 2350
   AGTCACTCAG TGCTTATCCT TTAAACAGCA GTTGTGTGAT TGTTTCCTGG 2400
   ATACTATCAC CCAGTGATTA CAAGCTAATG TATTTTATTA TTGAGTGGAA 2450
   AAATCTTAAT GAAGATGGTG AAATAAAATG GCTTAGAATC TCTTCATCTG 2500
   TTAAGAAGTA TTATATCCAT GATCATTTTA TCCCCATTGA GAAGTACCAG 2550
   TTCAGTCTTT ACCCAATATT TATGGAAGGA GTGGGAAAAC CAAAGATAAT 2600
   TAATAGTTTC ACTCAAGATG ATATTGAAAA ACACCAGAGT GATGCAGGTT 2650
   TATATGTAAT TGTGCCAGTA ATTATTTCCT CTTCCATCTT ATTGCTTGGA 2700
   ACATTATTAA TATCACACCA AAGAATGAAA AAGCTATTTT GGGAAGATGT 2750
   TCCGAACCCC AAGAATTGTT CCTGGGCACA AGGACTTAAT TTTCAGAAGA 2800
   TGTTCCGAAC CCCAAGAATT GTTCCTGGGC ACAAGGACTT AATTTTCAGA 2850
   AGATGCTTGA AGGCAGCATG TTCGTTAAGA GTCATCACCA CTCCCTAATC 2900
   TCAAGTACCC AGGGACACAA ACACTGCGGA AGGCCACAGG GTCCTCTGCA 2950
   TAGGAAAACC AGAGACCTTT GTTCACTTGT TTATCTGCTG ACCCTCCCTC 3000
   CACTATTGTC CTATGACCCT GCCAAATCCC CCTCTGTGAG AAACACCCAA 3050
   GAATGATCAA TAAAAAAAAA AAAAAAAAAA AAAAAAGTCG ACTCGAGAAT 3100
   TC 3102
(2) INFORMATION FOR SEQ ID NO:7:
   (i) SEQUENCE CHARACTERISTICS:
      (A) LENGTH: 783 amino acids
      (B) TYPE: Amino Acid
      (D) TOPOLOGY: Linear
   (xi) SEQUENCE DESCRIPTION: SEQ ID NO:7:
   Met Met Cys Gln Lys Phe Tyr Val Val Leu Leu His Trp Glu Phe
   1 5 10 15
   Leu Tyr Val Ile Ala Ala Leu Asn Leu Ala Tyr Pro Ile Ser Pro
   20 25 30
   Trp Lys Phe Lys Leu Phe Cys Gly Pro Pro Asn Thr Thr Asp Asp
   35 40 45
   Ser Phe Leu Ser Pro Ala Gly Ala Pro Asn Asn Ala Ser Ala Leu
   50 55 60
   Lys Gly Ala Ser Glu Ala Ile Val Glu Ala Lys Phe Asn Ser Ser
   65 70 75
   Gly Ile Tyr Val Pro Glu Leu Ser Lys Thr Val Phe His Cys Cys
   80 85 90
   Phe Gly Asn Glu Gln Gly Gln Asn Cys Ser Ala Leu Thr Asp Asn
   95 100 105
   Thr Glu Gly Lys Thr Leu Ala Ser Val Val Lys Ala Ser Val Phe
   110 115 120
   Arg Gln Leu Gly Val Asn Trp Asp Ile Glu Cys Trp Met Lys Gly
   125 130 135
   Asp Leu Thr Leu Phe Ile Cys His Met Glu Pro Leu Pro Lys Asn
   140 145 150
   Pro Phe Lys Asn Tyr Asp Ser Lys Val His Leu Leu Tyr Asp Leu
   155 160 165
   Pro Glu Val Ile Asp Asp Ser Pro Leu Pro Pro Leu Lys Asp Ser
   170 175 180
   Phe Gln Thr Val Gln Cys Asn Cys Ser Leu Arg Gly Cys Glu Cys
   185 190 195
   His Val Pro Val Pro Arg Ala Lys Leu Asn Tyr Ala Leu Leu Met
   200 205 210
   Tyr Leu Glu Ile Thr Ser Ala Gly Val Ser Phe Gln Ser Pro Leu
   215 220 225
   Met Ser Leu Gln Pro Met Leu Val Val Lys Pro Asp Pro Pro Leu
   230 235 240
   Gly Leu His Met Glu Val Thr Asp Asp Gly Asn Leu Lys Ile Ser
   245 250 255
   Trp Asp Ser Gln Thr Met Ala Pro Phe Pro Leu Gln Tyr Gln Val
   260 265 270
   Lys Tyr Leu Glu Asn Ser Thr Ile Val Arg Glu Ala Ala Glu Ile
   275 280 285
   Val Ser Ala Thr Ser Leu Leu Val Asp Ser Val Leu Pro Gly Ser
   290 295 300
   Ser Tyr Glu Val Gln Val Arg Ser Lys Arg Leu Asp Gly Ser Gly
   305 310 315
   Val Trp Ser Asp Trp Ser Ser Pro Gln Val Phe Thr Thr Gln Asp
   320 325 330
   Val Val Tyr Phe Pro Pro Lys Ile Leu Thr Ser Val Gly Ser Asn
   335 340 345
   Ala Ser Phe His Cys Ile Tyr Lys Asn Glu Asn Gln Ile Val Ser
   350 355 360
   Ser Lys Gln Ile Val Trp Trp Arg Asn Leu Ala Glu Lys Ile Pro
   365 370 375
   Glu Ile Gln Tyr Ser Ile Val Ser Asp Arg Val Ser Lys Val Thr
   380 385 390
   Phe Ser Asn Leu Lys Ala Thr Arg Pro Arg Gly Lys Phe Thr Tyr
   395 400 405
   Asp Ala Val Tyr Cys Cys Asn Glu Gln Ala Cys His His Arg Tyr
   410 415 420
   Ala Glu Leu Tyr Val Ile Asp Val Asn Ile Asn Ile Ser Cys Glu
   425 430 435
   Thr Asp Gly Tyr Leu Thr Lys Met Thr Cys Arg Trp Ser Pro Ser
   440 445 450
   Thr Ile Gln Ser Leu Val Gly Ser Thr Val Gln Leu Arg Tyr His
   455 460 465
   Arg Cys Ser Leu Tyr Cys Pro Asp Ser Pro Ser Ile His Pro Thr
   470 475 480
   Ser Glu Pro Lys Thr Ala Ser Tyr Arg Glu Thr Ala Phe Met Asn
   485 490 495
   Val Phe Ser Ser Gln Ser Phe Tyr Tyr Leu Ala Ile Gln Cys Gly
   500 505 510
   Phe Arg Ile Asn His Ser Leu Gly Ser Leu Asp Ser Pro Pro Thr
   515 520 525
   Cys Val Leu Pro Asp Ser Val Val Lys Pro Leu Pro Pro Ser Asn
   530 535 540
   Val Lys Ala Glu Ile Thr Val Asn Thr Gly Leu Leu Lys Val Ser
   545 550 555
   Trp Glu Lys Pro Val Phe Pro Glu Asn Asn Leu Gln Phe Gln Ile
   560 565 570
   Arg Tyr Gly Leu Ser Gly Lys Glu Ile Gln Trp Lys Thr His Glu
   575 580 585
   Val Phe Asp Ala Lys Ser Lys Ser Ala Ser Leu Leu Val Ser Asp
   590 595 600
   Leu Cys Ala Val Tyr Val Val Gln Val Arg Cys Arg Arg Leu Asp
   605 610 615
   Gly Leu Gly Tyr Trp Ser Asn Trp Ser Ser Pro Ala Tyr Thr Leu
   620 625 630
   Val Met Asp Val Lys Val Pro Met Arg Gly Pro Glu Phe Trp Arg
   635 640 645
   Lys Met Asp Gly Asp Val Thr Lys Lys Glu Arg Asn Val Thr Leu
   650 655 660
   Leu Trp Lys Pro Leu Thr Lys Asn Asp Ser Leu Cys Ser Val Arg
   665 670 675
   Arg Tyr Val Val Lys His Arg Thr Ala His Asn Gly Thr Trp Ser
   680 685 690
   Glu Asp Val Gly Asn Arg Thr Asn Leu Thr Phe Leu Trp Thr Glu
   695 700 705
   Pro Ala His Thr Val Thr Val Leu Ala Val Asn Ser Leu Gly Ala
   710 715 720
   Ser Leu Val Asn Phe Asn Leu Thr Phe Ser Trp Pro Met Ser Lys
   725 730 735
   Val Ser Ala Val Glu Ser Leu Ser Ala Tyr Pro Leu Ser Ser Ser
   740 745 750
   Cys Val Ile Leu Ser Trp Thr Leu Ser Pro Asp Asp Tyr Ser Leu
   755 760 765
   Leu Tyr Leu Val Ile Glu Trp Lys Ile Leu Asn Glu Asp Asp Gly
   770 775 780
   Met Lys Trp
   783
(2) INFORMATION FOR SEQ ID NO:8:
   (i) SEQUENCE CHARACTERISTICS:
      (A) LENGTH: 2868 base pairs
      (B) TYPE: Nucleic Acid
      (C) STRANDEDNESS: Single
      (D) TOPOLOGY: Linear
   (xi) SEQUENCE DESCRIPTION: SEQ ID NO:8:
   GGGCCCCCCC TCGAAGTCGA CGGTATCGAT AAGCTTGATA TCGAATTCCG 50
   GCCGGGACAC AGGTGGGACA CTCTTTTAGT CCTCAATCCC TGGCGCGAGG 100
   CCACCCAAGG CAACGCAGGA CGCAGGGCGT TTGGGGACCA GGCAGCAGAC 150
   TGGGGCGGTA CCTGCGGAGA GCCACGCAAC TTCTCCAGGC CTCTGACTAC 200
   TTTGGAAACT GCCCGGGGCT GCGACATCAA CCCCTTAAGT CCCGGAGGCG 250
   GAAAGAGGGT GGGTTGGTTT GAAAGACACA AGGAAGAAAA ATGTGCTGTG 300
   GGGCGGGTTA AGTTTCCCAC CCTCTTCCCC CTTCCCGAGC AAATTAGAAA 350
   CAAAACAAAT AGAAAAGCCA GCCCTCCGGC CAACCAAAGC CCCAAGCGGA 400
   GCCCCAAGCG GAGCCCCAGC CGGAGCACTC CTTTAAAAGG ATTTGCAGCG 450
   GTGAGGAAAA AACCAGACCC GACCGAGGAA TCGTTCTGCA AATCCAGGTG 500
   TACACCTCTG AAGAAAGATG ATGTGTCAGA AATTCTATGT GGTTTTGTTA 550
   CACTGGGAAT TTCTTTATGT GATAGCTGCA CTTAACCTGG CATATCCAAT 600
   CTCTCCCTGG AAATTTAAGT TGTTTTGTGG ACCACCGAAC ACAACCGATG 650
   ACTCCTTTCT CTCACCTGCT GGAGCCCCAA ACAATGCCTC GGCTTTGAAG 700
   GGGGCTTCTG AAGCAATTGT TGAAGCTAAA TTTAATTCAA GTGGTATCTA 750
   CGTTCCTGAG TTATCCAAAA CAGTCTTCCA CTGTTGCTTT GGGAATGAGC 800
   AAGGTCAAAA CTGCTCTGCA CTCACAGACA ACACTGAAGG GAAGACACTG 850
   GCTTCAGTAG TGAAGGCTTC AGTTTTTCGC CAGCTAGGTG TAAACTGGGA 900
   CATAGAGTGC TGGATGAAAG GGGACTTGAC ATTATTCATC TGTCATATGG 950
   AGCCATTACC TAAGAACCCC TTCAAGAATT ATGACTCTAA GGTCCATCTT 1000
   TTATATGATC TGCCTGAAGT CATAGATGAT TCGCCTCTGC CCCCACTGAA 1050
   AGACAGCTTT CAGACTGTCC AATGCAACTG CAGTCTTCGG GGATGTGAAT 1100
   GTCATGTGCC AGTACCCAGA GCCAAACTCA ACTACGCTCT TCTGATGTAT 1150
   TTGGAAATCA CATCTGCCGG TGTGAGTTTT CAGTCACCTC TGATGTCACT 1200
   GCAGCCCATG CTTGTTGTGA AACCCGATCC ACCCTTAGGT TTGCATATGG 1250
   AAGTCACAGA TGATGGTAAT TTAAAGATTT CTTGGGACAG CCAAACAATG 1300
   GCACCATTTC CGCTTCAATA TCAGGTGAAA TATTTAGAGA ATTCTACAAT 1350
   TGTAAGAGAG GCTGCTGAAA TTGTCTCAGC TACATCTCTG CTGGTAGACA 1400
   GTGTGCTTCC TGGATCTTCA TATGAGGTCC AGGTGAGGAG CAAGAGACTG 1450
   GATGGTTCAG GAGTCTGGAG TGACTGGAGT TCACCTCAAG TCTTTACCAC 1500
   ACAAGATGTT GTGTATTTTC CACCCAAAAT TCTGACTAGT GTTGGATCGA 1550
   ATGCTTCCTT TCATTGCATC TACAAAAACG AAAACCAGAT TGTCTCCTCA 1600
   AAACAGATAG TTTGGTGGAG GAATCTAGCT GAGAAAATCC CTGAGATACA 1650
   GTACAGCATT GTGAGTGACC GAGTTAGCAA AGTTACCTTC TCCAACCTGA 1700
   AAGCCACCAG ACCTCGAGGG AAGTTTACCT ATGACGCAGT GTACTGCTGC 1750
   AATGAGCAGG CGTGCCATCA CCGCTATGCT GAATTATACG TGATCGATGT 1800
   CAATATCAAT ATATCATGTG AAACTGACGG GTACTTAACT AAAATGACTT 1850
   GCAGATGGTC ACCCAGCACA ATCCAATCAC TAGTGGGAAG CACTGTGCAG 1900
   CTGAGGTATC ACAGGTGCAG CCTGTATTGT CCTGATAGTC CATCTATTCA 1950
   TCCTACGTCT GAGCCCAAAA CTGCGTCTTA CAGAGAGACG GCTTTTATGA 2000
   ATGTGTTTTC CAGCCAATCT TTCTATTATC TGGCTATACA ATGTGGATTC 2050
   AGGATCAACC ATTCTTTAGG TTCACTTGAC TCGCCACCAA CGTGTGTCCT 2100
   TCCTGACTCC GTAGTAAAAC CACTACCTCC ATCTAACGTA AAAGCAGAGA 2150
   TTACTGTAAA CACTGGATTA TTGAAAGTAT CTTGGGAAAA GCCAGTCTTT 2200
   CCGGAGAATA ACCTTCAATT CCAGATTCGA TATGGCTTAA GTGGAAAAGA 2250
   AATACAATGG AAGACACATG AGGTATTCGA TGCAAAGTCA AAGTCTGCCA 2300
   GCCTGCTGGT GTCAGACCTC TGTGCAGTCT ATGTGGTCCA GGTTCGCTGC 2350
   CGGCGGTTGG ATGGACTAGG ATATTGGAGT AATTGGAGCA GTCCAGCCTA 2400
   TACGCTTGTC ATGGATGTAA AAGTTCCTAT GAGAGGGCCT GAATTTTGGA 2450
   GAAAAATGGA TGGGGACGTT ACTAAAAAGG AGAGAAATGT CACCTTGCTT 2500
   TGGAAGCCCC TGACGAAAAA TGACTCACTG TGTAGTGTGA GGAGGTACGT 2550
   GGTGAAGCAT CGTACTGCCC ACAATGGGAC GTGGTCAGAA GATGTGGGAA 2600
   ATCGGACCAA TCTCACTTTC CTGTGGACAG AACCAGCGCA CACTGTTACA 2650
   GTTCTGGCTG TCAATTCCCT CGGCGCTTCC CTTGTGAATT TTAACCTTAC 2700
   CTTCTCATGG CCCATGAGTA AAGTGAGTGC TGTGGAGTCA CTCAGTGCTT 2750
   ATCCCCTGAG CAGCAGCTGT GTCATCCTTT CCTGGACACT GTCACCTGAT 2800
   GATTATAGTC TGTTATATCT GGTTATTGAA TGGAAGATCC TTAATGAAGA 2850
   TGATGGAATG AAGTGGCT 2868
(2) INFORMATION FOR SEQ ID NO:9:
   (i) SEQUENCE CHARACTERISTICS:
      (A) LENGTH: 18 base pairs
      (B) TYPE: Nucleic Acid
      (C) STRANDEDNESS: Single
      (D) TOPOLOGY: Linear
   (xi) SEQUENCE DESCRIPTION: SEQ ID NO:9:
   GGGTTAAGTT TCCCACCC 18
(2) INFORMATION FOR SEQ ID NO:10:
   (i) SEQUENCE CHARACTERISTICS:
      (A) LENGTH: 18 base pairs
      (B) TYPE: Nucleic Acid
      (C) STRANDEDNESS: Single
      (D) TOPOLOGY: Linear
   (xi) SEQUENCE DESCRIPTION: SEQ ID NO:10:
   GGGTGGGAAA CTTAACCC 18
(2) INFORMATION FOR SEQ ID NO:11:
   (i) SEQUENCE CHARACTERISTICS:
      (A) LENGTH: 18 base pairs
      (B) TYPE: Nucleic Acid
      (C) STRANDEDNESS: Single
      (D) TOPOLOGY: Linear
   (xi) SEQUENCE DESCRIPTION: SEQ ID NO:11:
   AGGATACAGT GGGATCCC 18
(2) INFORMATION FOR SEQ ID NO:12:
   (i) SEQUENCE CHARACTERISTICS:
      (A) LENGTH: 18 base pairs
      (B) TYPE: Nucleic Acid
      (C) STRANDEDNESS: Single
      (D) TOPOLOGY: Linear
   (xi) SEQUENCE DESCRIPTION: SEQ ID NO:12:
   GCCCGAGCAC TCCTTTAA 18
(2) INFORMATION FOR SEQ ID NO:13:
   (i) SEQUENCE CHARACTERISTICS:
      (A) LENGTH: 18 base pairs
      (B) TYPE: Nucleic Acid
      (C) STRANDEDNESS: Single
      (D) TOPOLOGY: Linear
   (xi) SEQUENCE DESCRIPTION: SEQ ID NO:13:
   TTAAAGGAGT GCTCCCGC 18
(2) INFORMATION FOR SEQ ID NO:14:
   (i) SEQUENCE CHARACTERISTICS:
      (A) LENGTH: 18 base pairs
      (B) TYPE: Nucleic Acid
      (C) STRANDEDNESS: Single
      (D) TOPOLOGY: Linear
   (xi) SEQUENCE DESCRIPTION: SEQ ID NO:14:
   GAGCGGCCCT GTTAGATA 18
(2) INFORMATION FOR SEQ ID NO:15:
   (i) SEQUENCE CHARACTERISTICS:
      (A) LENGTH: 18 base pairs
      (B) TYPE: Nucleic Acid
      (C) STRANDEDNESS: Single
      (D) TOPOLOGY: Linear
   (xi) SEQUENCE DESCRIPTION: SEQ ID NO:15:
   GTATACACCT CTGAAGAA 18
(2) INFORMATION FOR SEQ ID NO:16:
   (i) SEQUENCE CHARACTERISTICS:
      (A) LENGTH: 18 base pairs
      (B) TYPE: Nucleic Acid
      (C) STRANDEDNESS: Single
      (D) TOPOLOGY: Linear
   (xi) SEQUENCE DESCRIPTION: SEQ ID NO:16:
   TTCTTCAGAG GTGTACAC 18
(2) INFORMATION FOR SEQ ID NO:17:
   (i) SEQUENCE CHARACTERISTICS:
      (A) LENGTH: 18 base pairs
      (B) TYPE: Nucleic Acid
      (C) STRANDEDNESS: Single
      (D) TOPOLOGY: Linear
   (xi) SEQUENCE DESCRIPTION: SEQ ID NO:17:
   ATGCGAGGCT ACTTCTAT 18
(2) INFORMATION FOR SEQ ID NO:18:
   (i) SEQUENCE CHARACTERISTICS:
      (A) LENGTH: 18 base pairs
      (B) TYPE: Nucleic Acid
      (C) STRANDEDNESS: Single
      (D) TOPOLOGY: Linear
   (xi) SEQUENCE DESCRIPTION: SEQ ID NO:18:
   CTCTCCCTGG AAATTTAA 18
(2) INFORMATION FOR SEQ ID NO:19:
   (i) SEQUENCE CHARACTERISTICS:
      (A) LENGTH: 18 base pairs
      (B) TYPE: Nucleic Acid
      (C) STRANDEDNESS: Single
      (D) TOPOLOGY: Linear
   (xi) SEQUENCE DESCRIPTION: SEQ ID NO:19:
   TTAAATTTCC AGGGAGAG 18
(2) INFORMATION FOR SEQ ID NO:20:
   (i) SEQUENCE CHARACTERISTICS:
      (A) LENGTH: 18 base pairs
      (B) TYPE: Nucleic Acid
      (C) STRANDEDNESS: Single
      (D) TOPOLOGY: Linear
   (xi) SEQUENCE DESCRIPTION: SEQ ID NO:20:
   ATTTGAAGGA GTTAAGCC 18
(2) INFORMATION FOR SEQ ID NO:21:
   (i) SEQUENCE CHARACTERISTICS:
      (A) LENGTH: 18 base pairs
      (B) TYPE: Nucleic Acid
      (C) STRANDEDNESS: Single
      (D) TOPOLOGY: Linear
   (xi) SEQUENCE DESCRIPTION: SEQ ID NO:21:
   AATTTAATTC AAGTGGTA 18
(2) INFORMATION FOR SEQ ID NO:22:
   (i) SEQUENCE CHARACTERISTICS:
      (A) LENGTH: 18 base pairs
      (B) TYPE: Nucleic Acid
      (C) STRANDEDNESS: Single
      (D) TOPOLOGY: Linear
   (xi) SEQUENCE DESCRIPTION: SEQ ID NO:22:
   TACCAGTTGA ATTAAATT 18
(2) INFORMATION FOR SEQ ID NO:23:
   (i) SEQUENCE CHARACTERISTICS:
      (A) LENGTH: 18 base pairs
      (B) TYPE: Nucleic Acid
      (C) STRANDEDNESS: Single
      (D) TOPOLOGY: Linear
   (xi) SEQUENCE DESCRIPTION: SEQ ID NO:23:
   GTATCACTTC ATAATATA 18
(2) INFORMATION FOR SEQ ID NO:24:
   (i) SEQUENCE CHARACTERISTICS:
      (A) LENGTH: 18 base pairs
      (B) TYPE: Nucleic Acid
      (C) STRANDEDNESS: Single
      (D) TOPOLOGY: Linear
   (xi) SEQUENCE DESCRIPTION: SEQ ID NO:24:
   GATGGTCAGG GTGAACTG 18
(2) INFORMATION FOR SEQ ID NO:25:
   (i) SEQUENCE CHARACTERISTICS:
      (A) LENGTH: 18 base pairs
      (B) TYPE: Nucleic Acid
      (C) STRANDEDNESS: Single
      (D) TOPOLOGY: Linear
   (xi) SEQUENCE DESCRIPTION: SEQ ID NO:25:
   CAGTTCACCC TGACCATC 18
(2) INFORMATION FOR SEQ ID NO:26:
   (i) SEQUENCE CHARACTERISTICS:
      (A) LENGTH: 18 base pairs
      (B) TYPE: Nucleic Acid
      (C) STRANDEDNESS: Single
      (D) TOPOLOGY: Linear
   (xi) SEQUENCE DESCRIPTION: SEQ ID NO:26:
   GAGGCGAATG TGCGGATT 18
(2) INFORMATION FOR SEQ ID NO:27:
   (i) SEQUENCE CHARACTERISTICS:
      (A) LENGTH: 18 base pairs
      (B) TYPE: Nucleic Acid
      (C) STRANDEDNESS: Single
      (D) TOPOLOGY: Linear
   (xi) SEQUENCE DESCRIPTION: SEQ ID NO:27:
   CTTAAATCTC CAAGGAGT 18
(2) INFORMATION FOR SEQ ID NO:28:
   (i) SEQUENCE CHARACTERISTICS:
      (A) LENGTH: 18 base pairs
      (B) TYPE: Nucleic Acid
      (C) STRANDEDNESS: Single
      (D) TOPOLOGY: Linear
   (xi) SEQUENCE DESCRIPTION: SEQ ID NO:28:
   ACTCCTTGGA GATTTAAG 18
(2) INFORMATION FOR SEQ ID NO:29:
   (i) SEQUENCE CHARACTERISTICS:
      (A) LENGTH: 18 base pairs
      (B) TYPE: Nucleic Acid
      (C) STRANDEDNESS: Single
      (D) TOPOLOGY: Linear
   (xi) SEQUENCE DESCRIPTION: SEQ ID NO:29:
   AAGTCTTAAG CCAGACTT 18
(2) INFORMATION FOR SEQ ID NO:30:
   (i) SEQUENCE CHARACTERISTICS:
      (A) LENGTH: 18 base pairs
      (B) TYPE: Nucleic Acid
      (C) STRANDEDNESS: Single
      (D) TOPOLOGY: Linear
   (xi) SEQUENCE DESCRIPTION: SEQ ID NO:30:
   TCTAAGGCAC ATCCCAGC 18
(2) INFORMATION FOR SEQ ID NO:31:
   (i) SEQUENCE CHARACTERISTICS:
      (A) LENGTH: 18 base pairs
      (B) TYPE: Nucleic Acid
      (C) STRANDEDNESS: single
      (D) TOPOLOGY: Linear
   (xi) SEQUENCE DESCRIPTION: SEQ ID NO:31:
   GCTGGGATGT GCCTTAGA 18
(2) INFORMATION FOR SEQ ID NO:32:
   (i) SEQUENCE CHARACTERISTICS:
      (A) LENGTH: 18 base pairs
      (B) TYPE: Nucleic Acid
      (C) STRANDEDNESS: Single
      (D) TOPOLOGY: Linear
   (xi) SEQUENCE DESCRIPTION: SEQ ID NO:32:
   CGCAATGAAT TGACCCCC 18
(2) INFORMATION FOR SEQ ID NO:33:
   (i) SEQUENCE CHARACTERISTICS:
      (A) LENGTH: 18 base pairs
      (B) TYPE: Nucleic Acid
      (C) STRANDEDNESS: Single
      (D) TOPOLOGY: Linear
   (xi) SEQUENCE DESCRIPTION: SEQ ID NO:33:
   TACTTCAGAG AAGTACAC 18
(2) INFORMATION FOR SEQ ID NO:34:
   (i) SEQUENCE CHARACTERISTICS:
      (A) LENGTH: 18 base pairs
      (B) TYPE: Nucleic Acid
      (C) STRANDEDNESS: Single
      (D) TOPOLOGY: Linear
   (xi) SEQUENCE DESCRIPTION: SEQ ID NO:34:
   GTGTACTTCT CTGAAGTA 18
(2) INFORMATION FOR SEQ ID NO:35:
   (i) SEQUENCE CHARACTERISTICS:
      (A) LENGTH: 18 base pairs
      (B) TYPE: Nucleic Acid
      (C) STRANDEDNESS: Single
      (D) TOPOLOGY: Linear
   (xi) SEQUENCE DESCRIPTION: SEQ ID NO:35:
   GAATCACGGT AACTATCA 18
(2) INFORMATION FOR SEQ ID NO:36:
   (i) SEQUENCE CHARACTERISTICS:
      (A) LENGTH: 18 base pairs
      (B) TYPE: Nucleic Acid
      (C) STRANDEDNESS: Single
      (D) TOPOLOGY: Linear
   (xi) SEQUENCE DESCRIPTION: SEQ ID NO:36:
   CAGCTGTCTC ATAATGTC 18
(2) INFORMATION FOR SEQ ID NO:37:
   (i) SEQUENCE CHARACTERISTICS:
      (A) LENGTH: 18 base pairs
      (B) TYPE: Nucleic Acid
      (C) STRANDEDNESS: Single
      (D) TOPOLOGY: Linear
   (xi) SEQUENCE DESCRIPTION: SEQ ID NO:37:
   GACATTATGA GACAGCTG 18
(2) INFORMATION FOR SEQ ID NO:38:
   (i) SEQUENCE CHARACTERISTICS:
      (A) LENGTH: 18 base pairs
      (B) TYPE: Nucleic Acid
      (C) STRANDEDNESS: Single
      (D) TOPOLOGY: Linear
   (xi) SEQUENCE DESCRIPTION: SEQ ID NO:38:
   TTCGTCAAGC CATCTGAT 18
(2) INFORMATION FOR SEQ ID NO:39:
   (i) SEQUENCE CHARACTERISTICS:
      (A) LENGTH: 8 amino acids
      (B) TYPE: Amino Acid
      (D) TOPOLOGY: Linear
   (xi) SEQUENCE DESCRIPTION: SEQ ID NO:39:
   His Gln Asn Leu Ser Asp Gly Lys
    1               5           8
(2) INFORMATION FOR SEQ ID NO:40:
   (i) SEQUENCE CHARACTERISTICS:
      (A) LENGTH: 8 amino acids
      (B) TYPE: Amino Acid
      (D) TOPOLOGY: Linear
   (xi) SEQUENCE DESCRIPTION: SEQ ID NO:40:
   His Gln Asn Ile Ser Asp Gly Lys
    1               5           8
(2) INFORMATION FOR SEQ ID NO:41:
   (i) SEQUENCE CHARACTERISTICS:
      (A) LENGTH: 7 amino acids
      (B) TYPE: Amino Acid
      (D) TOPOLOGY: Linear
   (xi) SEQUENCE DESCRIPTION: SEQ ID NO:41:
   His Gln Ser Leu Gly Thr Gln
    1               5       7
(2) INFORMATION FOR SEQ ID NO:42:
   (i) SEQUENCE CHARACTERISTICS:
      (A) LENGTH: 8 amino acids
      (B) TYPE: Amino Acid
      (D) TOPOLOGY: Linear
   (xi) SEQUENCE DESCRIPTION: SEQ ID NO:42:
   Val Ile Ser Ser His Leu Gly Gln
    1               5           8
(2) INFORMATION FOR SEQ ID NO:43:
   (i) SEQUENCE CHARACTERISTICS:
      (A) LENGTH: 11 amino acids
      (B) TYPE: Amino Acid
      (D) TOPOLOGY: Linear
   (xi) SEQUENCE DESCRIPTION: SEQ ID NO:43:
   Pro Lys Asn Ser Ser Met Ile Ser Asn Thr Pro
    1               5                   10  11
(2) INFORMATION FOR SEQ ID NO:44:
   (i) SEQUENCE CHARACTERISTICS:
      (A) LENGTH: 10 amino acids
      (B) TYPE: Amino Acid
      (D) TOPOLOGY: Linear
   (xi) SEQUENCE DESCRIPTION: SEQ ID NO:44:
   Asp Lys Thr His Thr Cys Pro Pro Cys Pro
    1               5                   10
(2) INFORMATION FOR SEQ ID NO:45:
   (i) SEQUENCE CHARACTERISTICS:
      (A) LENGTH: 51 base pairs
      (B) TYPE: Nucleic Acid
      (C) STRANDEDNESS: Single
      (D) TOPOLOGY: Linear
   (xi) SEQUENCE DESCRIPTION: SEQ ID NO:45:
   GTCAGTCTCC CAGTTCCAGA CTTGTGTGCA GTCTATGCTG TTCAGGTGCG 50
   C 51
(2) INFORMATION FOR SEQ ID NO:46:
   (i) SEQUENCE CHARACTERISTICS:
      (A) LENGTH: 7127 base pairs
      (B) TYPE: Nucleic Acid
      (C) STRANDEDNESS: Double
      (D) TOPOLOGY: Linear
   (xi) SEQUENCE DESCRIPTION: SEQ ID NO:46:
   TTCGAGCTCG CCCGACATTG ATTATTGACT AGTTATTAAT AGTAATCAAT 50
   TACGGGGTCA TTAGTTCATA GCCCATATAT GGAGTTCCGC GTTACATAAC 100
   TTACGGTAAA TGGCCCGCCT GGCTGACCGC CCAACGACCC CCGCCCATTG 150
   ACGTCAATAA TGACGTATGT TCCCATAGTA ACGCCAATAG GGACTTTCCA 200
   TTGACGTCAA TGGGTGGAGT ATTTACGGTA AACTGCCCAC TTGGCAGTAC 250
   ATCAAGTGTA TCATATGCCA AGTACGCCCC CTATTGACGT CAATGACGGT 300
   AAATGGCCCG CCTGGCATTA TGCCCAGTAC ATGACCTTAT GGGACTTTCC 350
   TACTTGGCAG TACATCTACG TATTAGTCAT CGCTATTACC ATGGTGATGC 400
   GGTTTTGGCA GTACATCAAT GGGCGTGGAT AGCGGTTTGA CTCACGGGGA 450
   TTTCCAAGTC TCCACCCCAT TGACGTCAAT GGGAGTTTGT TTTGGCACCA 500
   AAATCAACGG GACTTTCCAA AATGTCGTAA CAACTCCGCC CCATTGACGC 550
   AAATGGGCGG TAGGCGTGTA CGGTGGGAGG TCTATATAAG CAGAGCTCGT 600
   TTAGTGAACC GTCAGATCGC CTGGAGACGC CATCCACGCT GTTTTGACCT 650
   CCATAGAAGA CACCGGGACC GATCCAGCCT CCGCGGCCGG GAACGGTGCA 700
   TTGGAACGCG GATTCCCCGT GCCAAGAGTG ACGTAAGTAC CGCCTATAGA 750
   GTCTATAGGC CCACCCCCTT GGCTTCGTTA GAACGCGGCT ACAATTAATA 800
   CATAACCTTA TGTATCATAC ACATACGATT TAGGTGACAC TATAGAATAA 850
   CATCCACTTT GCCTTTCTCT CCACAGGTGT CCACTCCCAG GTCCAACTGC 900
   ACCTCGGTTC TATCGATATG CATTGGGGAA CCCTGTGCGG ATTCTTGTGG 950
   CTTTGGCCCT ATCTTTTCTA TGTCCAAGCT GTGCCCATCC AAAAAGTCCA 1000
   AGATGACACC AAAACCCTCA TCAAGACAAT TGTCACCAGG ATCAATGACA 1050
   TTTCACACAC GCAGTCAGTC TCCTCCAAAC AGAAAGTCAC CGGTTTGGAC 1100
   TTCATTCCTG GGCTCCACCC CATCCTGACC TTATCCAAGA TGGACCAGAC 1150
   ACTGGCAGTC TACCAACAGA TCCTCACCAG TATGCCTTCC AGAAACGTGA 1200
   TCCAAATATC CAACGACCTG GAGAACCTCC GGGATCTTCT TCACGTGCTG 1250
   GCCTTCTCTA AGAGCTGCCA CTTGCCCTGG GCCAGTGGCC TGGAGACCTT 1300
   GGACAGCCTG GGGGGTGTCC TGGAAGCTTC AGGCTACTCC ACAGAGGTGG 1350
   TGGCCCTGAG CAGGCTGCAG GGGTCTCTGC AGGACATGCT GTGGCAGCTG 1400
   GACCTCAGCC CTGGGTGCGG GGTCACCGAC AAAACTCACA CATGCCCACC 1450
   GTGCCCAGCA CCTGAACTCC TGGGGGGACC GTCAGTCTTC CTCTTCCCCC 1500
   CAAAACCCAA GGACACCCTC ATGATCTCCC GGACCCCTGA GGTCACATGC 1550
   GTGGTGGTGG ACGTGAGCCA CGAAGACCCT GAGGTCAAGT TCAACTGGTA 1600
   CGTGGACGGC GTGGAGGTGC ATAATGCCAA GACAAAGCCG CGGGAGGAGC 1650
   AGTACAACAG CACGTACCGT GTGGTCAGCG TCCTCACCGT CCTGCACCAG 1700
   GACTGGCTGA ATGGCAAGGA GTACAAGTGC AAGGTCTCCA ACAAAGCCCT 1750
   CCCAGCCCCC ATCGAGAAAA CCATCTCCAA AGCCAAAGGG CAGCCCCGAG 1800
   AACCACAGGT GTACACCCTG CCCCCATCCC GGGAAGAGAT GACCAAGAAC 1850
   CAGGTCAGCC TGACCTGCCT GGTCAAAGGC TTCTATCCCA GCGACATCGC 1900
   CGTGGAGTGG GAGAGCAATG GGCAGCCGGA GAACAACTAC AAGACCACGC 1950
   CTCCCGTGCT GGACTCCGAC GGCTCCTTCT TCCTCTACAG CAAGCTCACC 2000
   GTGGACAAGA GCAGGTGGCA GCAGGGGAAC GTCTTCTCAT GCTCCGTGAT 2050
   GCATGAGGCT CTGCACAACC ACTACACGCA GAAGAGCCTC TCCCTGTCTC 2100
   CGGGTAAATG AGTGCGACGG CCCTAGAGTC GACCTGCAGA AGCTTCTAGA 2150
   GTCGACCTGC AGAAGCTTGG CCGCCATGGC CCAACTTGTT TATTGCAGCT 2200
   TATAATGGTT ACAAATAAAG CAATAGCATC ACAAATTTCA CAAATAAAGC 2250
   ATTTTTTTCA CTGCATTCTA GTTGTGGTTT GTCCAAACTC ATCAATGTAT 2300
   CTTATCATGT CTGGATCGAT CGGGAATTAA TTCGGCGCAG CACCATGGCC 2350
   TGAAATAACC TCTGAAAGAG GAACTTGGTT AGGTACCTTC TGAGGCGGAA 2400
   AGAACCAGCT GTGGAATGTG TGTCAGTTAG GGTGTGGAAA GTCCCCAGGC 2450
   TCCCCAGCAG GCAGAAGTAT GCAAAGCATG CATCTCAATT AGTCAGCAAC 2500
   CAGGTGTGGA AAGTCCCCAG GCTCCCCAGC AGGCAGAAGT ATGCAAAGCA 2550
   TGCATCTCAA TTAGTCAGCA ACCATAGTCC CGCCCCTAAC TCCGCCCATC 2600
   CCGCCCCTAA CTCCGCCCAG TTCCGCCCAT TCTCCGCCCC ATGGCTGACT 2650
   AATTTTTTTT ATTTATGCAG AGGCCGAGGC CGCCTCGGCC TCTGAGCTAT 2700
   TCCAGAAGTA GTGAGGAGGC TTTTTTGGAG GCCTAGGCTT TTGCAAAAAG 2750
   CTGTTAATTC GAACACGCAG ATGCAGTCGG GGCGGCGCGG TCCCAGGTCC 2800
   ACTTCGCATA TTAAGGTGAC GCGTGTGGCC TCGAACACCG AGCGACCCTG 2850
   CAGCGACCCG CTTAACAGCG TCAACAGCGT GCCGCAGATC TGATCAAGAG 2900
   ACAGGATGAG GATCGTTTCG CATGATTGAA CAAGATGGAT TGCACGCAGG 2950
   TTCTCCGGCC GCTTGGGTGG AGAGGCTATT CGGCTATGAC TGGGCACAAC 3000
   AGACAATCGG CTGCTCTGAT GCCGCCGTGT TCCGGCTGTC AGCGCAGGGG 3050
   CGCCCGGTTC TTTTTGTCAA GACCGACCTG TCCGGTGCCC TGAATGAACT 3100
   GCAGGACGAG GCAGCGCGGC TATCGTGGCT GGCCACGACG GGCGTTCCTT 3150
   GCGCAGCTGT GCTCGACGTT GTCACTGAAG CGGGAAGGGA CTGGCTGCTA 3200
   TTGGGCGAAG TGCCGGGGCA GGATCTCCTG TCATCTCACC TTGCTCCTGC 3250
   CGAGAAAGTA TCCATCATGG CTGATGCAAT GCGGCGGCTG CATACGCTTG 3300
   ATCCGGCTAC CTGCCCATTC GACCACCAAG CGAAACATCG CATCGAGCGA 3350
   GCACGTACTC GGATGGAAGC CGGTCTTGTC GATCAGGATG ATCTGGACGA 3400
   AGAGCATCAG GGGCTCGCGC CAGCCGAACT GTTCGCCAGG CTCAAGGCGC 3450
   GCATGCCCGA CGGCGAGGAT CTCGTCGTGA CCCATGGCGA TGCCTGCTTG 3500
   CCGAATATCA TGGTGGAAAA TGGCCGCTTT TCTGGATTCA TCGACTGTGG 3550
   CCGGCTGGGT GTGGCGGACC GCTATCAGGA CATAGCGTTG GCTACCCGTG 3600
   ATATTGCTGA AGAGCTTGGC GGCGAATGGG CTGACCGCTT CCTCGTGCTT 3650
   TACGGTATCG CCGCTCCCGA TTCGCAGCGC ATCGCCTTCT ATCGCCTTCT 3700
   TGACGAGTTC TTCTGAGCGG GACTCTGGGG TTCGAAATGA CCGACCAAGC 3750
   GACGCCCAAC CTGCCATCAC GAGATTTCGA TTCCACCGCC GCCTTCTATG 3800
   AAAGGTTGGG CTTCGGAATC GTTTTCCGGG ACGCCGGCTG GATGATCCTC 3850
   CAGCGCGGGG ATCTCATGCT GGAGTTCTTC GCCCACCCCG GGAGATGGGG 3900
   GAGGCTAACT GAAACACGGA AGGAGACAAT ACCGGAAGGA ACCCGCGCTA 3950
   TGACGGCAAT AAAAAGACAG AATAAAACGC ACGGGTGTTG GGTCGTTTGT 4000
   TCATAAACGC GGGGTTCGGT CCCAGGGCTG GCACTCTGTC GATACCCCAC 4050
   CGAGACCCCA TTGGGGCCAA TACGCCCGCG TTTCTTCCTT TTCCCCACCC 4100
   CAACCCCCAA GTTCGGGTGA AGGCCCAGGG CTCGCAGCCA ACGTCGGGGC 4150
   GGCAAGCCCG CCATAGCCAC GGGCCCCGTG GGTTAGGGAC GGGGTCCCCC 4200
   ATGGGGAATG GTTTATGGTT CGTGGGGGTT ATTCTTTTGG GCGTTGCGTG 4250
   GGGTCAGGTC CACGACTGGA CTGAGCAGAC AGACCCATGG TTTTTGGATG 4300
   GCCTGGGCAT GGACCGCATG TACTGGCGCG ACACGAACAC CGGGCGTCTG 4350
   TGGCTGCCAA ACACCCCCGA CCCCCAAAAA CCACCGCGCG GATTTCTGGC 4400
   GCCGCCGGAC GAACTAAACC TGACTACGGC ATCTCTGCCC CTTCTTCGCT 4450
   GGTACGAGGA GCGCTTTTGT TTTGTATTGG TCACCACGGC CGAGTTTCCG 4500
   CGGGACCCCG GCCAGGGCAC CTGTCCTACG AGTTGCATGA TAAAGAAGAC 4550
   AGTCATAAGT GCGGCGACGA TAGTCATGCC CCGCGCCCAC CGGAAGGAGC 4600
   TGACTGGGTT GAAGGCTCTC AAGGGCATCG GTCGAGCGGC CGCATCAAAG 4650
   CAACCATAGT ACGCGCCCTG TAGCGGCGCA TTAAGCGCGG CGGGTGTGGT 4700
   GGTTACGCGC AGCGTGACCG CTACACTTGC CAGCGCCCTA GCGCCCGCTC 4750
   CTTTCGCTTT CTTCCCTTCC TTTCTCGCCA CGTTCGCCGG CTTTCCCCGT 4800
   CAAGCTCTAA ATCGGGGGCT CCCTTTAGGG TTCCGATTTA GTGCTTTACG 4850
   GCACCTCGAC CCCAAAAAAC TTGATTTGGG TGATGGTTCA CGTAGTGGGC 4900
   CATCGCCCTG ATAGACGGTT TTTCGCCCTT TGACGTTGGA GTCCACGTTC 4950
   TTTAATAGTG GACTCTTGTT CCAAACTGGA ACAACACTCA ACCCTATCTC 5000
   GGGCTATTCT TTTGATTTAT AAGGGATTTT GCCGATTTCG GCCTATTGGT 5050
   TAAAAAATGA GCTGATTTAA CAAAAATTTA ACGCGAATTT TAACAAAATA 5100
   TTAACGTTTA CAATTTTATG GTGCAGGCCT CGTGATACGC CTATTTTTAT 5150
   AGGTTAATGT CATGATAATA ATGGTTTCTT AGACGTCAGG TGGCACTTTT 5200
   CGGGGAAATG TGCGCGGAAC CCCTATTTGT TTATTTTTCT AAATACATTC 5250
   AAATATGTAT CCGCTCATGA GACAATAACC CTGATAAATG CTTCAATAAT 5300
   ATTGAAAAAG GAAGAGTATG AGTATTCAAC ATTTCCGTGT CGCCCTTATT 5350
   CCCTTTTTTG CGGCATTTTG CCTTCCTGTT TTTGCTCACC CAGAAACGCT 5400
   GGTGAAAGTA AAAGATGCTG AAGATCAGTT GGGTGCACGA GTGGGTTACA 5450
   TCGAACTGGA TCTCAACAGC GGTAAGATCC TTGAGAGTTT TCGCCCCGAA 5500
   GAACGTTTTC CAATGATGAG CACTTTTAAA GTTCTGCTAT GTGGCGCGGT 5550
   ATTATCCCGT GATGACGCCG GGCAAGAGCA ACTCGGTCGC CGCATACACT 5600
   ATTCTCAGAA TGACTTGGTT GAGTACTCAC CAGTCACAGA AAAGCATCTT 5650
   ACGGATGGCA TGACAGTAAG AGAATTATGC AGTGCTGCCA TAACCATGAG 5700
   TGATAACACT GCGGCCAACT TACTTCTGAC AACGATCGGA GGACCGAAGG 5750
   AGCTAACCGC TTTTTTGCAC AACATGGGGG ATCATGTAAC TCGCCTTGAT 5800
   CGTTGGGAAC CGGAGCTGAA TGAAGCCATA CCAAACGACG AGCGTGACAC 5850
   CACGATGCCA GCAGCAATGG CAACAACGTT GCGCAAACTA TTAACTGGCG 5900
   AACTACTTAC TCTAGCTTCC CGGCAACAAT TAATAGACTG GATGGAGGCG 5950
   GATAAAGTTG CAGGACCACT TCTGCGCTCG GCCCTTCCGG CTGGCTGGTT 6000
   TATTGCTGAT AAATCTGGAG CCGGTGAGCG TGGGTCTCGC GGTATCATTG 6050
   CAGCACTGGG GCCAGATGGT AAGCCCTCCC GTATCGTAGT TATCTACACG 6100
   ACGGGGAGTC AGGCAACTAT GGATGAACGA AATAGACAGA TCGCTGAGAT 6150
   AGGTGCCTCA CTGATTAAGC ATTGGTAACT GTCAGACCAA GTTTACTCAT 6200
   ATATACTTTA GATTGATTTA AAACTTCATT TTTAATTTAA AAGGATCTAG 6250
   GTGAAGATCC TTTTTGATAA TCTCATGACC AAAATCCCTT AACGTGAGTT 6300
   TTCGTTCCAC TGAGCGTCAG ACCCCGTAGA AAAGATCAAA GGATCTTCTT 6350
   GAGATCCTTT TTTTCTGCGC GTAATCTGCT GCTTGCAAAC AAAAAAACCA 6400
   CCGCTACCAG CGGTGGTTTG TTTGCCGGAT CAAGAGCTAC CAACTCTTTT 6450
   TCCGAAGGTA ACTGGCTTCA GCAGAGCGCA GATACCAAAT ACTGTCCTTC 6500
   TAGTGTAGCC GTAGTTAGGC CACCACTTCA AGAACTCTGT AGCACCGCCT 6550
   ACATACCTCG CTCTGCTAAT CCTGTTACCA GTGGCTGCTG CCAGTGGCGA 6600
   TAAGTCGTGT CTTACCGGGT TGGACTCAAG ACGATAGTTA CCGGATAAGG 6650
   CGCAGCGGTC GGGCTGAACG GGGGGTTCGT GCACACAGCC CAGCTTGGAG 6700
   CGAACGACCT ACACCGAACT GAGATACCTA CAGCGTGAGC ATTGAGAAAG 6750
   CGCCACGCTT CCCGAAGGGA GAAAGGCGGA CAGGTATCCG GTAAGCGGCA 6800
   GGGTCGGAAC AGGAGAGCGC ACGAGGGAGC TTCCAGGGGG AAACGCCTGG 6850
   TATCTTTATA GTCCTGTCGG GTTTCGCCAC CTCTGACTTG AGCGTCGATT 6900
   TTTGTGATGC TCGTCAGGGG GGCGGAGCCT ATGGAAAAAC GCCAGCTGGC 6950
   ACGACAGGTT TCCCGACTGG AAAGCGGGCA GTGAGCGCAA CGCAATTAAT 7000
   GTGAGTTACC TCACTCATTA GGCACCCCAG GCTTTACACT TTATGCTTCC 7050
   GGCTCGTATG TTGTGTGGAA TTGTGAGCGG ATAACAATTT CACACAGGAA 7100
   ACAGCTATGA CCATGATTAC GAATTAA 7127
(2) INFORMATION FOR SEQ ID NO:47:
   (i) SEQUENCE CHARACTERISTICS:
      (A) LENGTH: 397 amino acids
      (B) TYPE: Amino Acid
      (D) TOPOLOGY: Linear
   (xi) SEQUENCE DESCRIPTION: SEQ ID NO:47:
   Met His Trp Gly Thr Leu Cys Gly Phe Leu Trp Leu Trp Pro Tyr
   1 5 10 15
   Leu Phe Tyr Val Gln Ala Val Pro Ile Gln Lys Val Gln Asp Asp
   20 25 30
   Thr Lys Thr Leu Ile Lys Thr Ile Val Thr Arg Ile Asn Asp Ile
   35 40 45
   Ser His Thr Gln Ser Val Ser Ser Lys Gln Lys Val Thr Gly Leu
   50 55 60
   Asp Phe Ile Pro Gly Leu His Pro Ile Leu Thr Leu Ser Lys Met
   65 70 75
   Asp Gln Thr Leu Ala Val Tyr Gln Gln Ile Leu Thr Ser Met Pro
   80 85 90
   Ser Arg Asn Val Ile Gln Ile Ser Asn Asp Leu Glu Asn Leu Arg
   95 100 105
   Asp Leu Leu His Val Leu Ala Phe Ser Lys Ser Cys His Leu Pro
   110 115 120
   Trp Ala Ser Gly Leu Glu Thr Leu Asp Ser Leu Gly Gly Val Leu
   125 130 135
   Glu Ala Ser Gly Tyr Ser Thr Glu Val Val Ala Leu Ser Arg Leu
   140 145 150
   Gln Gly Ser Leu Gln Asp Met Leu Trp Gln Leu Asp Leu Ser Pro
   155 160 165
   Gly Cys Gly Val Thr Asp Lys Thr His Thr Cys Pro Pro Cys Pro
   170 175 180
   Ala Pro Glu Leu Leu Gly Gly Pro Ser Val Phe Leu Phe Pro Pro
   185 190 195
   Lys Pro Lys Asp Thr Leu Met Ile Ser Arg Thr Pro Glu Val Thr
   200 205 210
   Cys Val Val Val Asp Val Ser His Glu Asp Pro Glu Val Lys Phe
   215 220 225
   Asn Trp Tyr Val Asp Gly Val Glu Val His Asn Ala Lys Thr Lys
   230 235 240
   Pro Arg Glu Glu Gln Tyr Asn Ser Thr Tyr Arg Val Val Ser Val
   245 250 255
   Leu Thr Val Leu His Gln Asp Trp Leu Asn Gly Lys Glu Tyr Lys
   260 265 270
   Cys Lys Val Ser Asn Lys Ala Leu Pro Ala Pro Ile Glu Lys Thr
   275 280 285
   Ile Ser Lys Ala Lys Gly Gln Pro Arg Glu Pro Gln Val Tyr Thr
   290 295 300
   Leu Pro Pro Ser Arg Glu Glu Met Thr Lys Asn Gln Val Ser Leu
   305 310 315
   Thr Cys Leu Val Lys Gly Phe Tyr Pro Ser Asp Ile Ala Val Glu
   320 325 330
   Trp Glu Ser Asn Gly Gln Pro Glu Asn Asn Tyr Lys Thr Thr Pro
   335 340 345
   Pro Val Leu Asp Ser Asp Gly Ser Phe Phe Leu Tyr Ser Lys Leu
   350 355 360
   Thr Val Asp Lys Ser Arg Trp Gln Gln Gly Asn Val Phe Ser Cys
   365 370 375
   Ser Val Met His Glu Ala Leu His Asn His Tyr Thr Gln Lys Ser
   380 385 390
   Leu Ser Leu Ser Pro Gly Lys
   395 397
(2) INFORMATION FOR SEQ ID NO:48:
   (i) SEQUENCE CHARACTERISTICS:
      (A) LENGTH: 249 amino acids
      (B) TYPE: Amino Acid
      (D) TOPOLOGY: Linear
   (xi) SEQUENCE DESCRIPTION: SEQ ID NO:48:
   Glu Val Gln Leu Val Gln Ser Gly Ala Glu Val Lys Lys Pro Gly
   1 5 10 15
   Ala Ser Val Lys Val Ser Cys Lys Ala Ser Gly Tyr Thr Phe Thr
   20 25 30
   Gly Tyr Tyr Met Tyr Trp Val Arg Gln Ala Pro Gly Gln Gly Leu
   35 40 45
   Glu Trp Met Gly Trp Ile Asn Pro Asn Ser Gly Gly Thr Asn Tyr
   50 55 60
   Ala Gln Lys Phe Gln Gly Arg Val Thr Met Thr Arg Asp Thr Ser
   65 70 75
   Ile Gly Thr Ala Tyr Met Glu Leu Ser Arg Leu Ser Ser Asp Asp
   80 85 90
   Thr Ala Val Tyr Tyr Cys Ala Arg Asp Arg Tyr Tyr Gly Ser Ser
   95 100 105
   Ala Tyr His Arg Gly Ser Tyr Tyr Met Asp Val Trp Gly Arg Gly
   110 115 120
   Thr Leu Val Thr Val Ser Ser Gly Gly Gly Gly Thr Gly Gly Gly
   125 130 135
   Gly Ser Gly Gly Gly Gly Ser Ser Glu Leu Thr Gln Asp Pro Ala
                   140                 145                 150
   Val Ser Val Ala Leu Gly Gln Thr Val Arg Ile Thr Cys Gln Gly
                   155                 160                 165
   Asp Ser Leu Arg Ser Tyr Tyr Ala Ser Trp Tyr Gln Gln Lys Pro
                   170                 175                 180
   Gly Gln Ala Pro Val Leu Val Ile Tyr Gly Lys Asn Asn Arg Pro
                   185                 190                 195
   Ser Gly Ile Pro Asp Arg Phe Ser Gly Ser Ser Ser Gly Asn Thr
                   200                 205                 210
   Ala Ser Leu Thr Ile Thr Gly Ala Gln Ala Glu Asp Glu Ala Asp
                   215                 220                 225
   Tyr Tyr Cys Asn Ser Arg Asp Ser Ser Gly Asn His Val Val Phe
                   230                 235                 240
   Gly Gly Gly Thr Lys Leu Thr Val Leu
                   245             249
(2) INFORMATION FOR SEQ ID NO:49:
   (i) SEQUENCE CHARACTERISTICS:
      (A) LENGTH: 250 amino acids
      (B) TYPE: Amino Acid
      (D) TOPOLOGY: Linear
   (xi) SEQUENCE DESCRIPTION: SEQ ID NO:49:
   Glu Val Gln Leu Val Gln Ser Gly Ala Glu Val Lys Lys Pro Gly
   1 5 10 15
   Glu Ser Leu Lys Ile Ser Cys Gln Gly Ser Gly Phe Thr Phe Ser
   20 25 30
   Ser Tyr Lys Met Asn Trp Val Arg Gln Ala Pro Gly Lys Gly Leu
   35 40 45
   Glu Trp Met Gly Gly Ile Ile Pro Ile Phe Gly Thr Ala Asn Tyr
   50 55 60
   Ala Gln Lys Phe Gln Gly Arg Val Thr Ile Thr Ala Asp Glu Ser
   65 70 75
   Thr Ser Thr Ala Tyr Met Glu Leu Ser Ser Leu Arg Ser Glu Asp
   80 85 90
   Thr Ala Val Tyr Tyr Cys Ala Arg Asp Arg Val Val Val Pro Ala
   95 100 105
   Thr Ser Leu Arg Gly Gly Met Asp Val Trp Gly Gln Gly Thr Thr
   110 115 120
   Val Thr Val Ser Ser Gly Gly Gly Gly Ser Gly Gly Gly Gly Ser
   125 130 135
   Gly Gly Gly Gly Ser Gln Ser Val Leu Thr Gln Pro Ala Ser Val
   140 145 150
   Ser Gly Ser Pro Gly Gln Ser Ile Thr Ile Ser Cys Thr Gly Thr
   155 160 165
   Ser Ser Asp Val Gly Gly Tyr Asn Tyr Val Ser Trp Tyr Gln Gln
   170 175 180
   His Pro Gly Lys Ala Pro Lys Leu Met Ile Tyr Glu Gly Ser Lys
   185 190 195
   Arg Pro Ser Gly Val Ser Asn Arg Phe Ser Gly Ser Lys Ser Gly
   200 205 210
   Ser Thr Ala Ser Leu Thr Ile Ser Gly Leu Gln Ala Glu Asp Glu
   215 220 225
   Ala Asp Tyr Tyr Cys Ser Ser Tyr Thr Thr Arg Ser Thr Arg Val
   230 235 240
   Phe Gly Gly Gly Thr Lys Leu Thr Val Leu
   245 250
(2) INFORMATION FOR SEQ ID NO:50:
   (i) SEQUENCE CHARACTERISTICS:
      (A) LENGTH: 241 amino acids
      (B) TYPE: Amino Acid
      (D) TOPOLOGY: Linear
   (xi) SEQUENCE DESCRIPTION: SEQ ID NO:50:
   Gln Val Arg Leu Gln Gln Ser Gly Gly Gly Leu Val Gln Pro Gly
   1 5 10 15
   Arg Ser Leu Arg Leu Ser Cys Ala Ala Ser Gly Phe Thr Phe Asp
   20 25 30
   Asp Tyr Ala Met His Trp Val Arg Gln Ala Pro Gly Lys Gly Leu
   35 40 45
   Glu Trp Val Ser Gly Met Thr Trp Asn Ser Gly Ser Ile Gly Tyr
   50 55 60
   Ala Asp Ser Val Lys Gly Arg Phe Thr Ile Ser Arg Asp Asn Ala
   65 70 75
   Lys Asn Ser Leu Tyr Leu Gln Met Asn Ser Leu Arg Ala Glu Asp
   80 85 90
   Thr Ala Val Tyr Tyr Cys Ala Arg Glu Pro His Asn Thr Asp Ala
   95 100 105
   Phe Asp Ile Trp Gly Arg Gly Thr Leu Val Thr Val Ser Ser Gly
   110 115 120
   Gly Gly Gly Pro Gly Gly Gly Gly Ser Gly Gly Gly Gly Ser Asp
   125 130 135
   Val Val Met Thr Gln Ser Pro Ser Phe Leu Ser Ala Phe Val Gly
   140 145 150
   Asp Thr Ile Thr Ile Thr Cys Arg Ala Ser Gln Gly Ile Tyr Asn
   155 160 165
   Tyr Leu Ala Trp Tyr Gln Gln Lys Pro Gly Lys Ala Pro Lys Leu
   170 175 180
   Leu Ile Tyr Ala Ala Ser Thr Leu Gln Ser Gly Val Pro Ser Arg
   185 190 195
   Phe Ser Gly Ser Gly Ser Gly Thr Glu Phe Thr Leu Thr Ile Ser
   200 205 210
   Ser Leu Gln Pro Glu Asp Phe Gly Thr Tyr Tyr Cys Gln Gln Leu
   215 220 225
   Ile Ser Tyr Pro Leu Thr Phe Gly Gly Gly Thr Lys Val Glu Ile
   230 235 240
   Lys
   241
(2) INFORMATION FOR SEQ ID NO:51:
   (i) SEQUENCE CHARACTERISTICS:
      (A) LENGTH: 894 amino acids
      (B) TYPE: Amino Acid
      (D) TOPOLOGY: Linear
   (xi) SEQUENCE DESCRIPTION: SEQ ID NO:51:
   Met Met Cys Gln Lys Phe Tyr Val Val Leu Leu His Trp Glu Phe
   1 5 10 15
   Leu Tyr Val Ile Ala Ala Leu Asn Leu Ala Tyr Pro Ile Ser Pro
   20 25 30
   Trp Lys Phe Lys Leu Phe Cys Gly Pro Pro Asn Thr Thr Asp Asp
   35 40 45
   Ser Phe Leu Ser Pro Ala Gly Ala Pro Asn Asn Ala Ser Ala Leu
   50 55 60
   Lys Gly Ala Ser Glu Ala Ile Val Glu Ala Lys Phe Asn Ser Ser
   65 70 75
   Gly Ile Tyr Val Pro Glu Leu Ser Lys Thr Val Phe His Cys Cys
   80 85 90
   Phe Gly Asn Glu Gln Gly Gln Asn Cys Ser Ala Leu Thr Asp Asn
   95 100 105
   Thr Glu Gly Lys Thr Leu Ala Ser Val Val Lys Ala Ser Val Phe
   110 115 120
   Arg Gln Leu Gly Val Asn Trp Asp Ile Glu Cys Trp Met Lys Gly
   125 130 135
   Asp Leu Thr Leu Phe Ile Cys His Met Glu Pro Leu Pro Lys Asn
   140 145 150
   Pro Phe Lys Asn Tyr Asp Ser Lys Val His Leu Leu Tyr Asp Leu
   155 160 165
   Pro Glu Val Ile Asp Asp Ser Pro Leu Pro Pro Leu Lys Asp Ser
   170 175 180
   Phe Gln Thr Val Gln Cys Asn Cys Ser Leu Arg Gly Cys Glu Cys
   185 190 195
   His Val Pro Val Pro Arg Ala Lys Leu Asn Tyr Ala Leu Leu Met
   200 205 210
   Tyr Leu Glu Ile Thr Ser Ala Gly Val Ser Phe Gln Ser Pro Leu
   215 220 225
   Met Ser Leu Gln Pro Met Leu Val Val Lys Pro Asp Pro Pro Leu
   230 235 240
   Gly Leu His Met Glu Val Thr Asp Asp Gly Asn Leu Lys Ile Ser
   245 250 255
   Trp Asp Ser Gln Thr Met Ala Pro Phe Pro Leu Gln Tyr Gln Val
   260 265 270
   Lys Tyr Leu Glu Asn Ser Thr Ile Val Arg Glu Ala Ala Glu Ile
   275 280 285
   Val Ser Ala Thr Ser Leu Leu Val Asp Ser Val Leu Pro Gly Ser
   290 295 300
   Ser Tyr Glu Val Gln Val Arg Ser Lys Arg Leu Asp Gly Ser Gly
   305 310 315
   Val Trp Ser Asp Trp Ser Ser Pro Gln Val Phe Thr Thr Gln Asp
   320 325 330
   Val Val Tyr Phe Pro Pro Lys Ile Leu Thr Ser Val Gly Ser Asn
   335 340 345
   Ala Ser Phe His Cys Ile Tyr Lys Asn Glu Asn Gln Ile Ile Ser
   350 355 360
   Ser Lys Gln Ile Val Trp Trp Arg Asn Leu Ala Glu Lys Ile Pro
   365 370 375
   Glu Ile Gln Tyr Ser Ile Val Ser Asp Arg Val Ser Lys Val Thr
   380 385 390
   Phe Ser Asn Leu Lys Ala Thr Arg Pro Arg Gly Lys Phe Thr Tyr
   395 400 405
   Asp Ala Val Tyr Cys Cys Asn Glu Gln Ala Cys His His Arg Tyr
   410 415 420
   Ala Glu Leu Tyr Val Ile Asp Val Asn Ile Asn Ile Ser Cys Glu
   425 430 435
   Thr Asp Gly Tyr Leu Thr Lys Met Thr Cys Arg Trp Ser Pro Ser
   440 445 450
   Thr Ile Gln Ser Leu Val Gly Ser Thr Val Gln Leu Arg Tyr His
   455 460 465
   Arg Arg Ser Leu Tyr Cys Pro Asp Ser Pro Ser Ile His Pro Thr
   470 475 480
   Ser Glu Pro Lys Asn Cys Val Leu Gln Arg Asp Gly Phe Tyr Glu
   485 490 495
   Cys Val Phe Gln Pro Ile Phe Leu Leu Ser Gly Tyr Thr Met Trp
   500 505 510
   Ile Arg Ile Asn His Ser Leu Gly Ser Leu Asp Ser Pro Pro Thr
   515 520 525
   Cys Val Leu Pro Asp Ser Val Val Lys Pro Leu Pro Pro Ser Asn
   530 535 540
   Val Lys Ala Glu Ile Thr Val Asn Thr Gly Leu Leu Lys Val Ser
   545 550 555
   Trp Glu Lys Pro Val Phe Pro Glu Asn Asn Leu Gln Phe Gln Ile
   560 565 570
   Arg Tyr Gly Leu Ser Gly Lys Glu Ile Gln Trp Lys Thr His Glu
   575 580 585
   Val Phe Asp Ala Lys Ser Lys Ser Ala Ser Leu Leu Val Ser Asp
   590 595 600
   Leu Cys Ala Val Tyr Val Val Gln Val Arg Cys Arg Arg Leu Asp
   605 610 615
   Gly Leu Gly Tyr Trp Ser Asn Trp Ser Ser Pro Ala Tyr Thr Leu
   620 625 630
   Val Met Asp Val Lys Val Pro Met Arg Gly Pro Glu Phe Trp Arg
   635 640 645
   Lys Met Asp Gly Asp Val Thr Lys Lys Glu Arg Asn Val Thr Leu
   650 655 660
   Leu Trp Lys Pro Leu Thr Lys Asn Asp Ser Leu Cys Ser Val Arg
   665 670 675
   Arg Tyr Val Val Lys His Arg Thr Ala His Asn Gly Thr Trp Ser
   680 685 690
   Glu Asp Val Gly Asn Arg Thr Asn Leu Thr Phe Leu Trp Thr Glu
   695 700 705
   Pro Ala His Thr Val Thr Val Leu Ala Val Asn Ser Leu Gly Ala
   710 715 720
   Ser Leu Val Asn Phe Asn Leu Thr Phe Ser Trp Pro Met Ser Lys
   725 730 735
   Val Ser Ala Val Glu Ser Leu Ser Ala Tyr Pro Leu Ser Ser Ser
   740 745 750
   Cys Val Ile Leu Ser Trp Thr Leu Ser Pro Asp Asp Tyr Ser Leu
   755 760 765
   Leu Tyr Leu Val Ile Glu Trp Lys Ile Leu Asn Glu Asp Asp Gly
                   770                 775                 780
   Met Lys Trp Leu Arg Ile Pro Ser Asn Val Lys Lys Phe Tyr Ile
                   785                 790                 795
   His Asp Asn Phe Ile Pro Ile Glu Lys Tyr Gln Phe Ser Leu Tyr
                   800                 805                 810
   Pro Val Phe Met Glu Gly Val Gly Lys Pro Lys Ile Ile Asn Gly
                   815                 820                 825
   Phe Thr Lys Asp Ala Ile Asp Lys Gln Gln Asn Asp Ala Gly Leu
                   830                 835                 840
   Tyr Val Ile Val Pro Ile Ile Ile Ser Ser Cys Val Leu Leu Leu
                   845                 850                 855
   Gly Thr Leu Leu Ile Ser His Gln Arg Met Lys Lys Leu Phe Trp
                   860                 865                 870
   Asp Asp Val Pro Asn Pro Lys Asn Cys Ser Trp Ala Gln Gly Leu
                   875                 880                 885
   Asn Phe Gln Lys Arg Thr Asp Thr Leu
                   890             894

## Description

### BACKGROUND OF THE INVENTION

### Field of the Invention

The present invention pertains generally to the OB receptor and ligands and uses for these molecules.

### Description of Related Art

### A. HEMATOPOIESIS

The process of blood cell formation whereby red and white blood cells are replaced through the division of cells located in the bone marrow is called hematopoiesis. For a review of hematopoiesis see Dexter and Spooncer (*Ann. Rev. Cell Biol*. 3:423-441 (1987)).

There are many different types of blood cells which belong to distinct cell lineages. Along each lineage, there are cells at different stages of maturation. Mature blood cells are specialized for different functions. For example, erythrocytes are involved in O₂ and CO₂ transport; T and B lymphocytes are involved in cell and antibody mediated immune responses, respectively; platelets are required for blood clotting; and the granulocytes and macrophages act as general scavengers and accessory cells. Granulocytes can be further divided into basophils, eosinophils, neutrophils and mast cells.

Each of the various blood cell types arises from pluripotent or totipotent stem cells which are able to undergo self-renewal or give rise to progenitor cells or Colony Forming Units (CFU) that yield a more limited array of cell types. As stem cells progressively lose their ability to self-renew, they become increasingly lineage restricted. It has been shown that stem cells can develop into multipotent cells (called "CFC-Mix" by Dexter and Spooncer, *supra*). Some of the CFC-Mix cells can undergo renewal whereas others lead to lineage-restricted progenitors which eventually develop into mature myeloid cells (*e*.*g*., neutrophils, megakaryocytes, macrophages and basophils). Similarly, pluripotent stem cells are able to give rise to PreB and PreT lymphoid cell lineages which differentiate into mature B and T lymphocytes, respectively. Progenitors are defined by their progeny, e.g., granulocyte/macrophage colony-forming progenitor cells (GM-CFU) differentiate into neutrophils or macrophages; primitive erythroid burst-forming units (BFU-E) differentiate into erythroid colony-forming units (CFU-E) which give rise to mature erythrocytes. Similarly, the Meg-CFU, Eos-CFU and Bas-CFU progenitors are able to differentiate into megakaryocytes, eosinophils and basophils, respectively.

Hematopoietic growth factors (reviewed in Andrea, *NEJM* 330(12):839-846 (1994)) have been shown to enhance growth and/or differentiation of blood cells via activation of receptors present on the surface of blood progenitor cells of the bone marrow. While some of these growth factors stimulate proliferation of restricted lineages of blood cells, others enhance proliferation of multiple lineages of blood cells. For example, erythropoietin (EPO) supports the proliferation of erythroid cells, whereas interleukin-3 (IL-3) induces proliferation of erythroid and myeloid lineages and is therefore considered a multi-lineage factor.

In recent years, several hematopoietic growth factor receptors have been isolated. Due to their low abundance and their existence in both high-affinity and low-affinity forms, biochemical characterization of these receptors has been hampered.

Cytokine receptors frequently assemble into multi-subunit complexes. Sometimes, the α subunit of this complex is involved in binding the cognate growth factor and the β-subunit may contain an ability to transduce a signal to the cell. These receptors have been assigned to three subfamilies depending on the complexes formed. Subfamily 1 includes the receptors for erythropoietin (EPO), granulocyte colony-stimulating factor (G-CSF), interleukin-4 (IL-4), interleukin-7 (IL-7), growth hormone (GH) and prolactin (PRL). Ligand binding to receptors belonging to this subfamily is thought to result in homodimerization of the receptor. Subfamily 2 includes receptors for IL-3, granulocyte-macrophage colony-stimulating factor (GM-CSF), interleukin-5 (IL-5), interleukin-6 (IL-6), leukemia inhibitory factor (LIF), oncostatin M (OSM) and ciliary neurotrophic factor (CNTF). Subfamily 2 receptors are heterodimers having an α-subunit for ligand binding and β-subunit (either the shared β-subunit of the IL-3, GM-CSF and IL-5 receptors or the gp130 subunit of the IL-6, LIF, OSM and CNTF receptors) for signal transduction. Subfamily 3 contains only the interleukin-2 (IL-2) receptor. The β and γ subunits of the IL-2 receptor complex are cytokine-receptor polypeptides which associate with the α-subunit of the unrelated Tac antigen.

### B. OBESITY

Obesity is the most common nutritional disorder which, according to recent epidemiologic studies, affects about one third of all Americans 20 years of age or older. Kuczmarski *et al., J. Am. Med Assoc.* 272:205-11 (1994). Obesity is responsible for a variety of serious health problems, including cardiovascular disorders, type II diabetes, insulin-resistance, hypertension, hyperdriglyceridemia, dyslipoproteinemia, and some forms of cancer. Pi-Sunyer, F., *Anns. Int. Med* 119: 655-60 (1993); Colfitz, G., *Am. J. Clin. Nutr*. 55:503S-507S (1992). A single-gene mutation (the obesity or "*ob*" mutation) has been shown to result in obesity and type II diabetes in mice. Friedman, *Genomics* 11:1054-1062 (1991).

Zhang *et al., Nature* 372:425-431 (1994) have recently reported the cloning and sequencing of the mouse *ob* gene and its human homologue, and suggested that the *ob* gene product, leptin or OB protein, may function as part of a signalling pathway from adipose tissue that acts to regulate the size of the body fat depot. Parabiosis experiments performed more than 20 years ago predicted that the genetically obese mouse containing two mutant copies of the *ob* gene (*ob*/*ob* mouse) does not produce a satiety factor which regulates its food intake, while the diabetic (*db*/*db*) mouse produces but does not respond to a satiety factor. Coleman and Hummal, *Am. J. Physiol*. 217:1298-1304 (1969); Coleman, *Diabetol* 9:294-98 (1973). Recent reports by three independent research teams have demonstrated that daily injections of recombinant OB protein inhibit food intake and reduce body weight and fat in grossly obese *ob*/*ob* mice but not in *db*/*db* mice (Pelleymounter *et al., Science* 269:540-43 (1995); Halaas *et al., Science* 269:543-46 (1995); Campfield *et al., Science* 269: 546-49 (1995)), suggesting that the OB protein is such a satiety factor as proposed in early cross-circulation studies.

Researchers suggest that at least one OB receptor is localized in the brain. The identification and expression cloning of a leptin receptor (OB-R) was reported by Tartaglia *et al. Cell* 83:1263-71 (1995). Various isoforms of a OB receptor are described by Cioffi *et al. Nature* 2:585-89 (1996). See, also, WO 96/08510.

The mouse *db* gene has recently been cloned (Lee *et al. Nature* 379:632 (1996) and Chen *et al. Cell* 84:491-495 (1996)). Previous data had suggested that the *db* gene encoded the receptor for the obese (*ob*) gene product, leptin (Coleman *et al., Diebetologia* 9:294-8 (1973) and Coleman *et al., Diebetologia* 14:141-8 (1978)). It has been very recently confirmed that the *db*/*db* mouse results from a truncated splice variant of the OB receptor which likely renders the receptor defective in signal transduction (Lee *et al., Nature* 379:632 (1996) and Chen *et al., Cell* 84: 491-495 (1996)).

### SUMMARY OF THE INVENTION

The invention herein is concerned with the OB cytokine receptor, also referred to herein as the WSX receptor.

The invention provides in one aspect methods for identifying a molecule which binds to and/or activates the WSX receptor, according to claims 16 or 17. This is useful for discovering molecules (such as peptides, antibodies, and small molecules) which are agonists of the WSX receptor. Such methods generally involve exposing an immobilized WSX receptor to a molecule suspected of binding thereto and determining binding of the molecule to the immobilized WSX receptor and/or evaluating whether or not the molecule activates the WSX receptor. In order to identify such WSX ligands, the WSX receptor may be expressed on the surface of a cell and used to screen libraries of synthetic compounds and naturally occurring compounds (*e.g*.,endogenous sources of such naturally occurring compounds, such as serum).

In further embodiments, the invention provides antibodies that specifically bind to the WSX receptor. Preferred antibodies are monoclonal antibodies which are non-immunogenic in a human and bind to an epitope in the extracellular domain of the receptor. Preferred antibodies bind the WSX receptor with an affinity of at least about 10⁶ L/mole, more preferably 10⁷ L/mole.

Antibodies which bind to the WSX receptor may optionally be fused to a heterologous polypeptide and the antibody or fusion thereof may be used to isolate and purify WSX receptor from a source of the receptor. Based on the observation herein that CD34+ cells possess WSX receptor, use of WSX antibodies for identification and/or enrichment of stem cell populations (in a similar manner to that in which CD34 antibodies are presently used) is envisaged.

For certain applications, it is desirable to have an agonist antibody which can be screened for as described herein. Such agonist antibodies are useful for activating the WSX receptor for *in vitro* uses whereby enhancement of proliferation and/or differentiation of a cell comprising the receptor is desired. Furthermore, these antibodies may be used to treat conditions in which an effective amount of WSX receptor activation leads to a therapeutic benefit in the mammal treated therewith. For example, the agonist antibody can be used to enhance proliferation and/or differentiation of a cell comprising the WSX receptor according to claim 12. In particular, agonist antibodies and other WSX ligands may be used to stimulate proliferation of stem cells/progenitor cells either *in vitro* or *in vivo*.

For therapeutic applications it is desirable to prepare a composition comprising the agonist antibody and a physiologically acceptable carrier. Optionally, such a composition may further comprise one or more cytokines.

In addition to the above, disclosed herein are isolated nucleic acid molecules, expression vectors and host cells encoding the WSX receptor which can be used in the recombinant production of WSX receptor as described herein. The isolated nucleic acid molecules and vectors are also useful for gene therapy applications to treat patients with WSX receptor defects and/or to increase responsiveness of cells to WSX ligand.

This application also relates to agonist antibodies which specifically bind to the WSX receptor and mimic one or more biological activities of naturally occurring WSX ligand, OB protein. Preferred antibodies are those with a strong binding affinity for human WSX receptor (*e*.*g*. having a Kd of no more than about 1 x 10⁸M; and preferably no more than about 1 x 10⁹M). In preferred embodiments, the agonist antibody binds to both human and murine WSX receptor.

Antibodies with defined agonistic activity in a bioassay, the KIRA ELISA, are disclosed herein. Preferred antibodies have an IC50 in the KIRA ELISA of about 0.5µg/ml or less, preferably about 0.2µg/ml or less, and most preferably about 0.1µg/ml or less.

The agonist antibodies of interest herein may have one or more of the biological characteristics of antibody 2D7, 1G4, 1E11 or 1C11 (see Example 13) or clones 3, 4, or 17 (see Example 14). For example, the antibody may bind to the epitope bound by any one of these antibodies, and/or may have some or all of the hypervariable region residues of these antibodies.

The agonist antibody may be one which decreases body weight and/or fat-depot weight and/or food intake in an obese mammal (*e.g.* in an *ob*/*ob* mouse). The preferred agonist antibody is one which exerts an adipose-reducing effect in an obese mammal (*e.g*. an *ob*/*ob* mouse) which is in excess of that induced by a reduction in food intake (Levin *et al. Proc. Natl. Acad. Sci. USA* 93:1726-1730 (1996)).

The agonist antibody may also have the property of inducing differentiation and/or proliferation and/or survival of hematopoietic progenitor cells. For example, the agonist antibody may induce lymphopoiesis, erythropoiesis and/or myelopoiesis.

The invention discloses herein a composition comprising the agonist antibody and a physiologically acceptable carrier. The composition for therapeutic use is sterile and may be lyophilized. For use in hematopoiesis, for example, the composition may further comprise a cytokine.

In another aspect, the invention provides a method for activating the WSX receptor which comprises exposing the WSX receptor to an amount of an agonist anti-WSX receptor antibody which is effective for activating the WSX receptor. In particular, the invention provides a method for enhancing proliferation and/or differentiation of a cell which expresses the WSX receptor at its cell surface comprising exposing the cell to an amount of exogenous agonist anti-WSX receptor antibody which is effective for enhancing proliferation and/or differentiation of the cell.

In another aspect, the present invention pertains to the discovery herein that WSX ligands, such as obesity (OB) protein, play a role in hematopoiesis via signalling through the WSX receptor. The role of the WSX receptor-ligand signalling pathway appears to be at the level of the early hematopoietic precursor as is evident by the ability of OB protein to simulate myelopoiesis, erythropoiesis (*e.g*. splenic erythropoiesis) and most dramatically, lymphopoiesis. Accordingly, WSX ligands can be used to stimulate proliferation and/or differentiation and/or survival of hematopoietic progenitor cells *in vitro* .

Thus, the invention provides a method for stimulating proliferation and/or differentiation of a cell which expresses the WSX receptor (especially the WSX receptor variant 13.2, which is demonstrated herein to have the capacity to transmit a proliferative signal) at its cell surface comprising the step of contacting the WSX receptor with an amount of WSX ligand which is effective for stimulating proliferation and/or OB protein differentiation of the cell. In prefered embodiments, the cell which is exposed to the WSX ligand is a hematopoeitic precursor, *e.g*. a CD34+ cell. The WSX ligand may be an agonist antibody which binds to the WSX receptor. The method contemplated herein may lead to an increase in the proliferation and/or differentiation of lymphoid, myeloid and/or erythroid blood cell lineages and encompasses both *in vitro* and *in vivo* methods. For *in vitro* uses, the cell possessing the WSX receptor may be present in cell culture. As to *in vivo* methods, the cell may be present in a mammal, especially a human (*e.g*. one who is suffering from decreased blood levels and who could benefit from an increase in various blood cells). Potential patients include those who have undergone chemo- or radiation therapy, or bone marrow transplantation therapy. Thus, the invention provides a method for repopulating blood cells (*e.g*. erythroid, myeloid and/or lymphoid blood cells) in a mammal comprising administering to the mammal a therapeutically effective amount of a WSX ligand.

Mammals which may benefit from an enhancement of lymphopoiesis include those predisposed to, or suffering from, any ony or more of the following exemplary conditions: lymphocytopenia; lymphorrhea; lymphostasis; immunodeficiency (*e.g*. HIV and AIDS); infections (including, for example, opportunistic infections and tuberculosis (TB)); lupus; and other disorders characterized by lymphocyte deficiency. An effective amount of the WSX ligand can be used in a method of immunopotentiation or to improve immune function in a mammal.

On the other hand, WSX receptor or WSX ligand antagonists (such as WSX receptor ECD or immunoadhesin, and WSX receptor or OB protein neutralizing antibodies) may be used in the treatment of those disorders wherein unacceptable lymphocyte levels are present in the mammal, particularly where this is caused by excessive activation of the WSX receptor. Examples of conditions in which administration of such an antagonist may be beneficial include: neoplastic disorders (such as Hodkin's disease; lymphosarcoma; lymphoblastoma; lymphocytic leukemia; and lymphoma) and lymphocytosis.

Diseases or disorders in which an increase in erythropoiesis may be beneficial include, but are not limited to: erythrocytopenia; erthrodegenerative disorders; erythroblastopenia; leukoerythroblastosis; erythroclasis; thalassemia; and anemia (*e*.*g*. hemolytic anemia, such as acquired, autoimmune, or microangiopathic hemolytic anemia; aplastic anemia; congenital anemia, *e*.*g*., congenital dyserythropoietic anemia, congenital hemolytic anemia or congenital hypoplastic anemia; dyshemopoietic anemia; Faconi's anemia; genetic anemia; hemorrhagic anemia; hyperchromic or hypochromic anemia; nutritional, hypoferric, or iron deficiency anemia; hypoplastic anemia; infectious anemia; lead anemia; local anemia; macrocytic or microcytic anemia; malignant or pernicious anemia; megaloblastic anemia; molecular anemia; normocytic anemia; physiologic anemia; traumatic or posthemorrhagic anemia; refractory anemia; radiation anemia; sickle cell anemia; splenic anemia; and toxic anemia).

An increase in myelopoiesis may be beneficial in any of the above-mentioned diseases or disorders as well as the following exemplary conditions: myelofibrosis; thrombocytopenia; hypoplasia; disseminated intravascular coagulation (DIC); immune (autoimmune) thrombocytopenic purpura (ITP); HIV induced ITP; myelodysplasia; thrombocytotic diseases and thrombocytosis.

The method may further involve the step of exposing hematopoeitic cells (whether they be in cell culture or in a mammal) to one or more other cytokines (*e.g*. lineage-specific cytokines) and this may lead to a synergistic enhancement of the proliferation and/or differentiation of the cells. Exemplary cytokines include thrombopoietin (TPO); erythropoietin (EPO); macrophage-colony stimulating factor (M-CSF); granulocyte-macrophage-CSF (GM-CSF); granulocyte-CSF (G-CSF); interleukin-1 (IL-1); IL-1α; IL-2; IL-3; IL-4; IL-5; IL-6; IL-7; IL-8; IL-9; IL-11; IL10; IL-12; leukemia inhibitory factor (LIF) or kit ligand (KL). In this embodiment, exposure to the cytokine may proceed, occur simultaneously with, or follow, exposure to the WSX ligand. Preferably, the WSX ligand and one or more further cytokines are administered simultaneously to the patient (where the method is an *in vivo* one) and, optionally, are combined to form a pharmaceutical composition.

For use in the above methods, there is described herein an article of manufacture, comprising: a container; a label on the container; and a composition comprising an active agent within the container; wherein the composition is effective for enhancing proliferation and/or differentiation of cells comprising the WSX receptor in a mammal, the label on the container indicates that the composition can be used for enhancing proliferation and/or differentiation of those cells and the active agent in the composition is a WSX ligand. Optionally, the article ofmanufacture includes one or more futher containers which hold further cytokine(s) in a packaged combination with the container holding the WSX ligand.

In another embodiment, an effective amount of the WSX ligand may be used to improve engraftment in bone marrow transplantation or to stimulate mobilization of hematopoietic stem cells in a mammal prior to harvesting hematopoietic progenitors from the peripheral blood thereof.

### BRIEF DESCRIPTION OF THE DRAWINGS

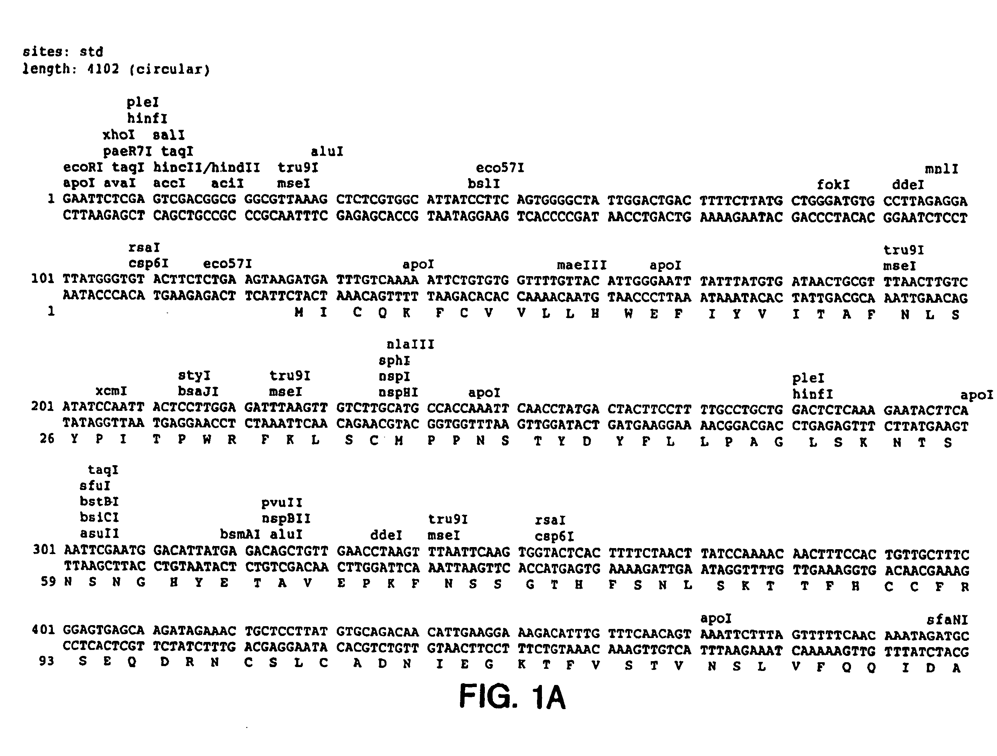

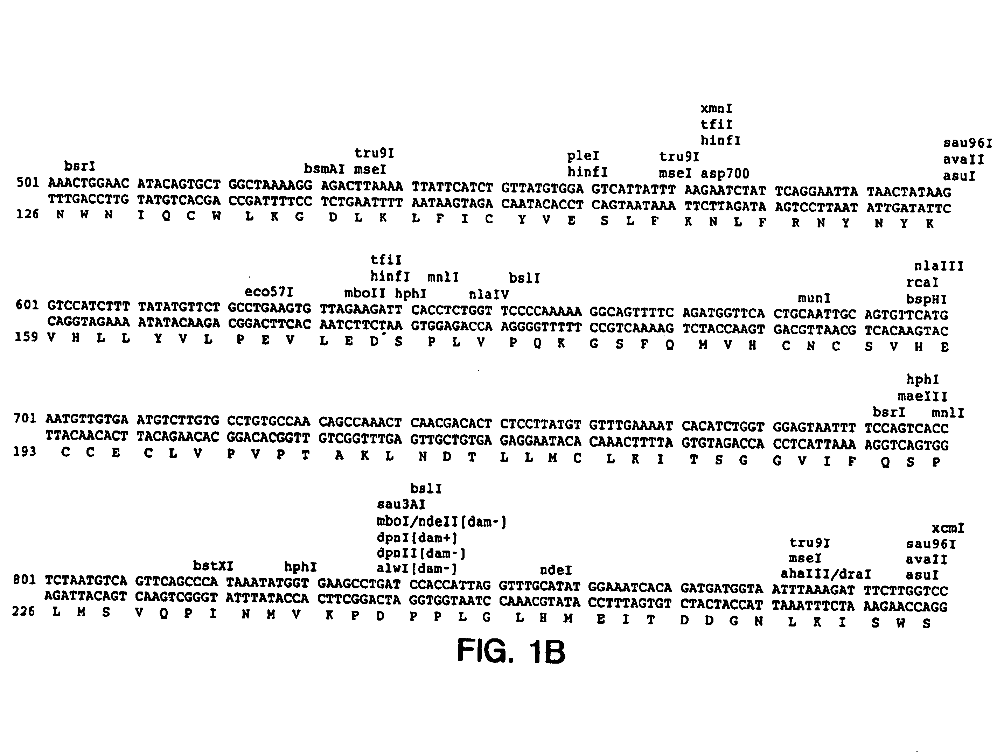

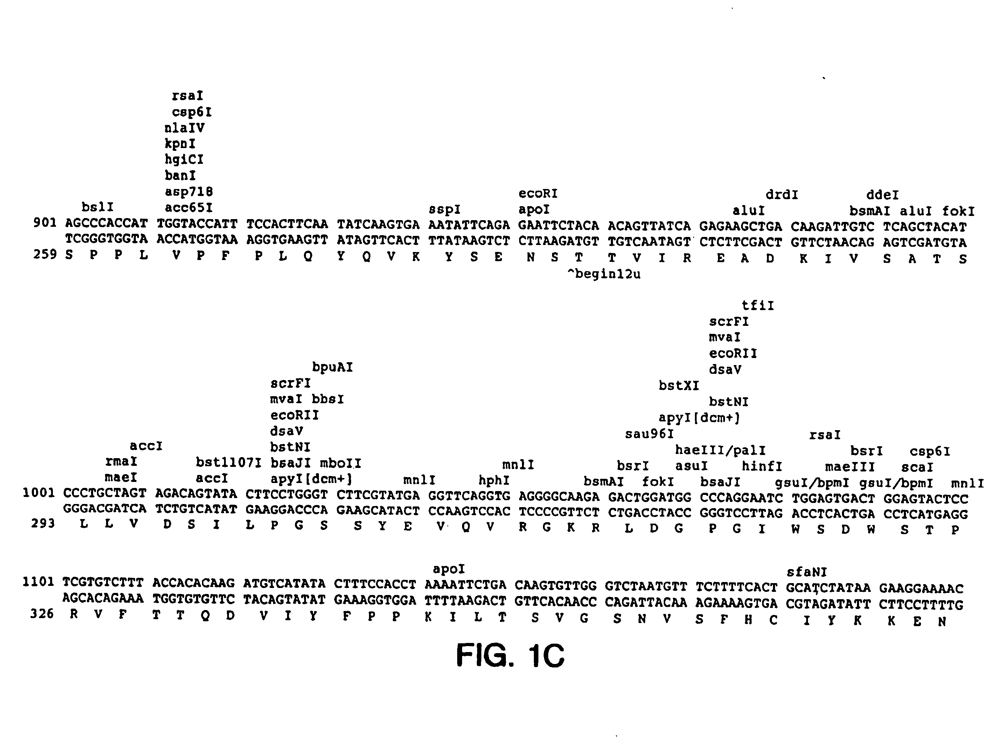

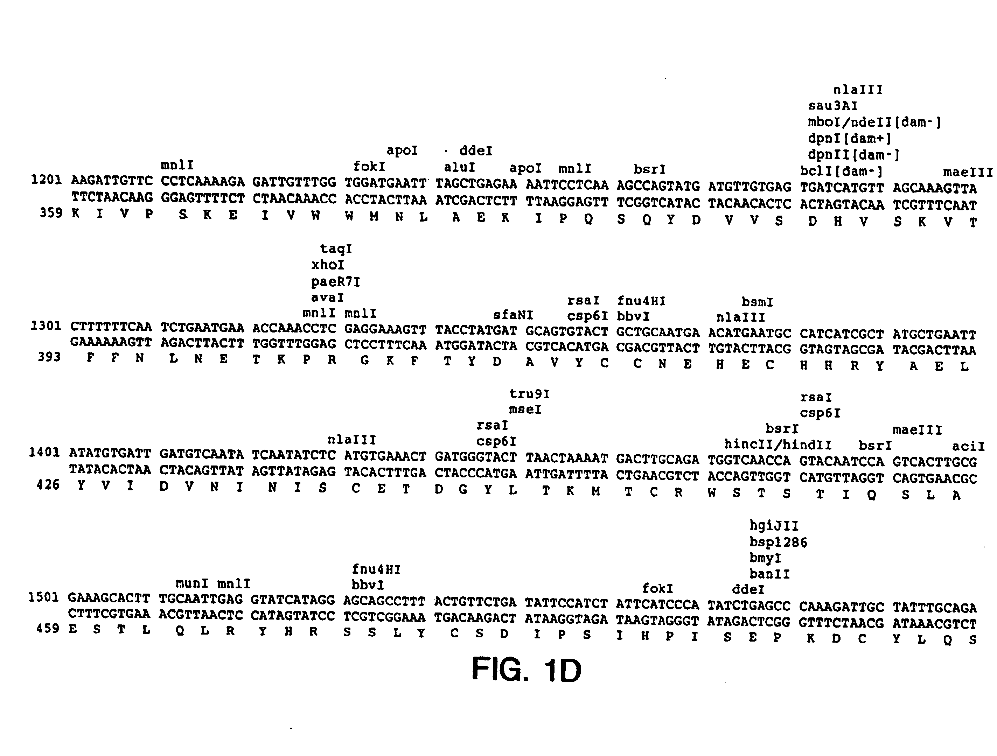

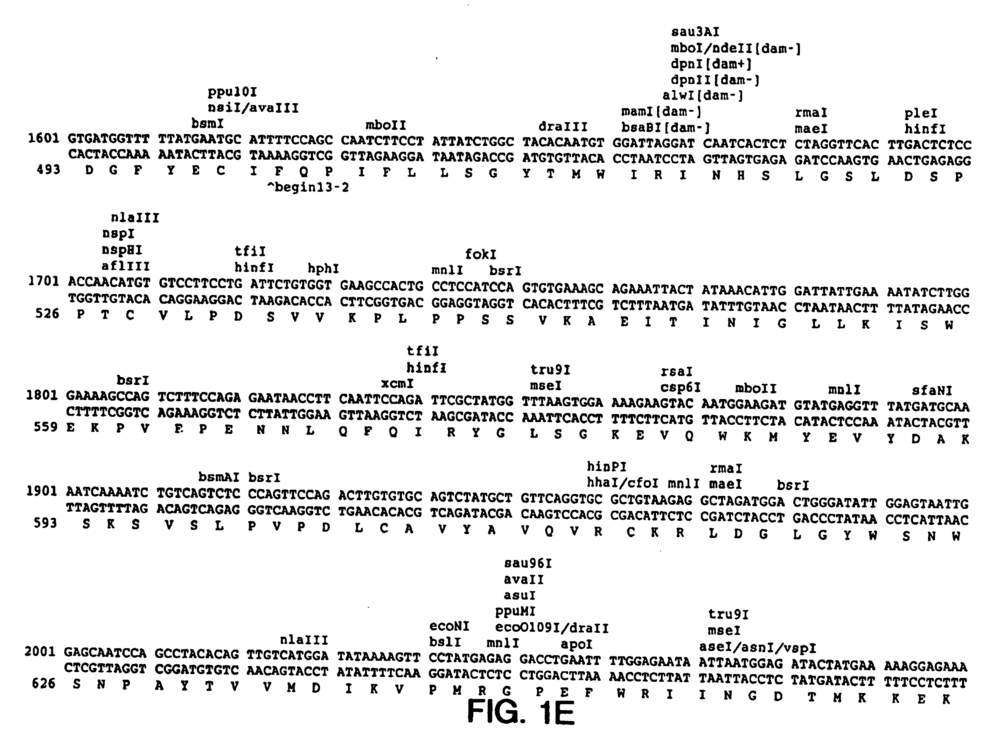

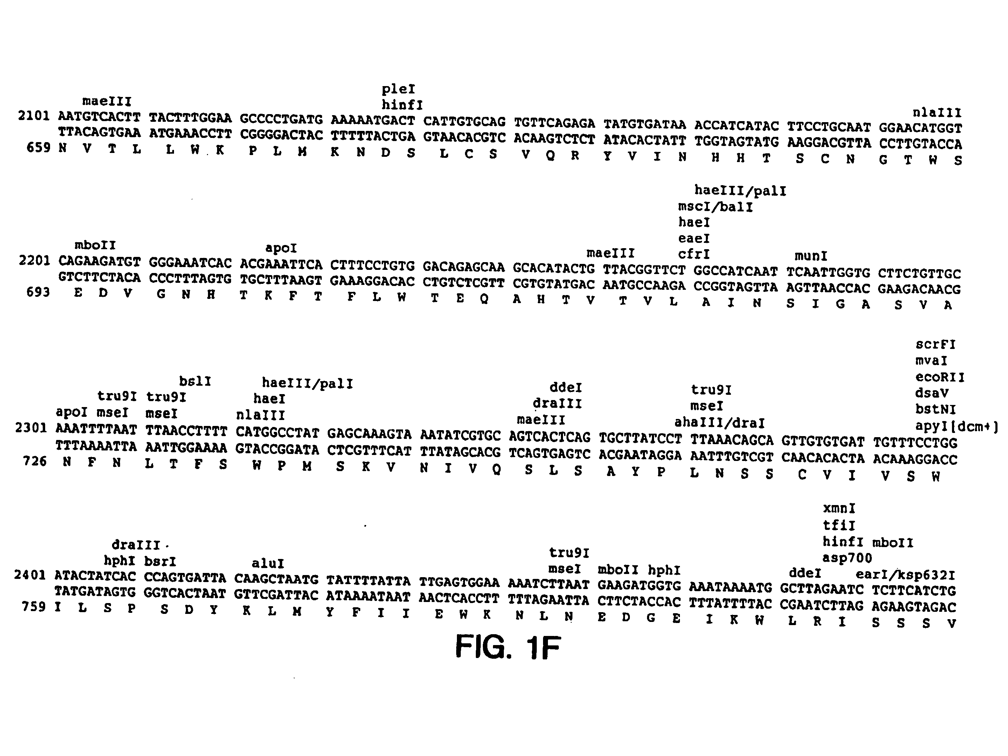

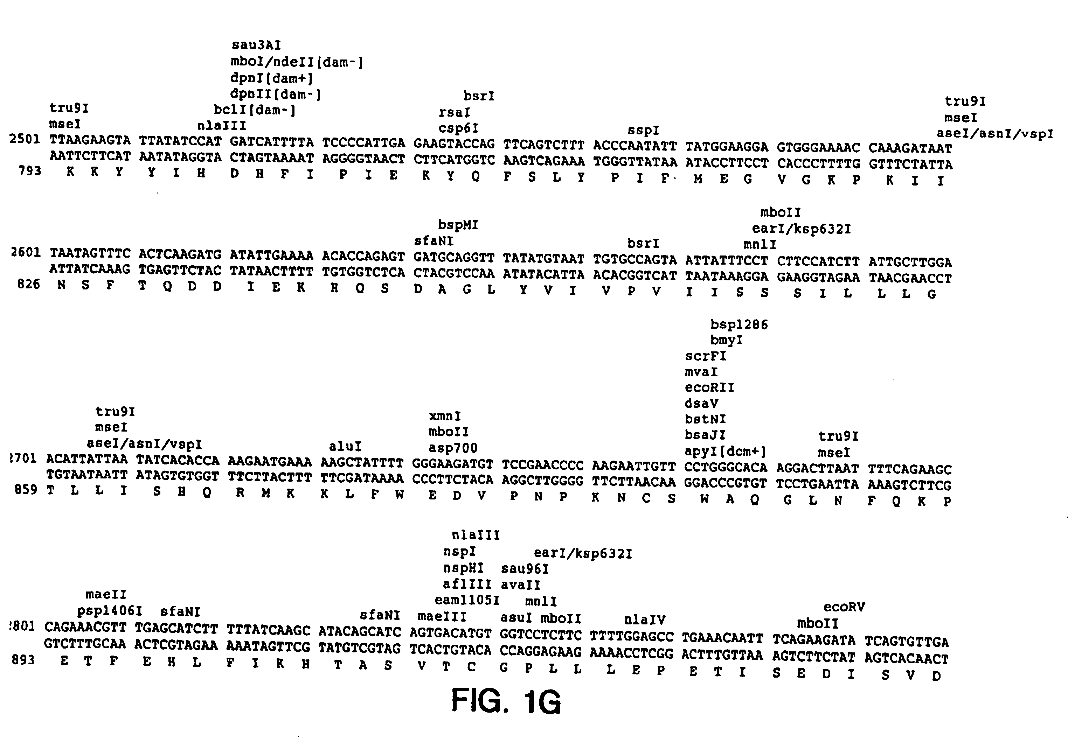

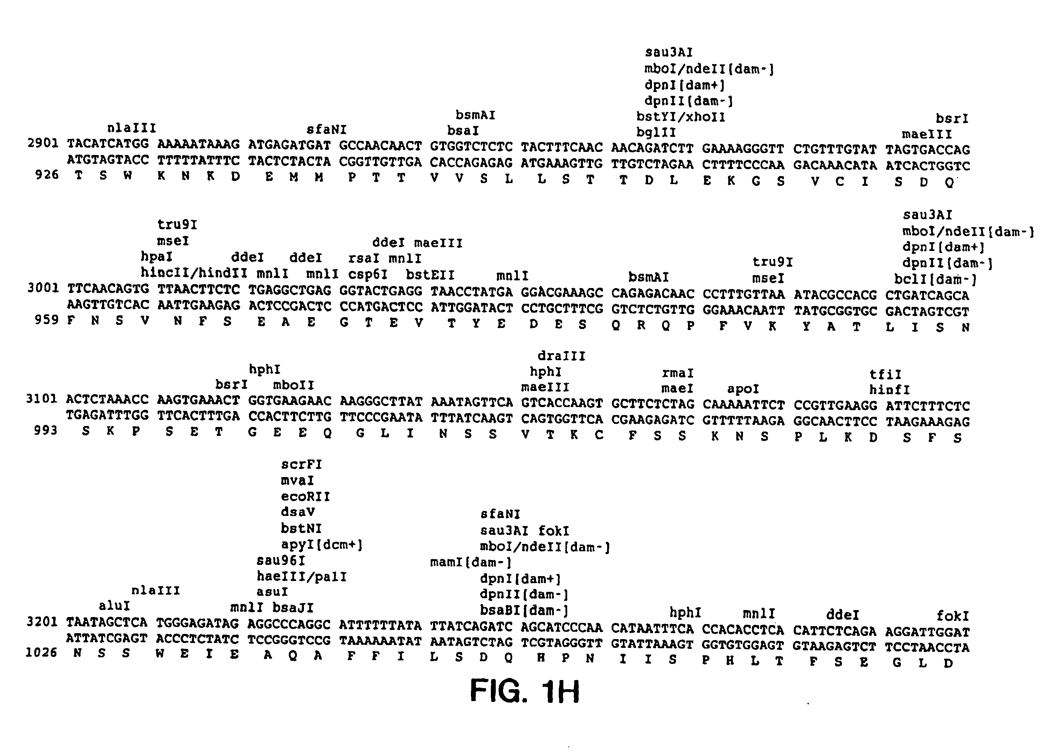

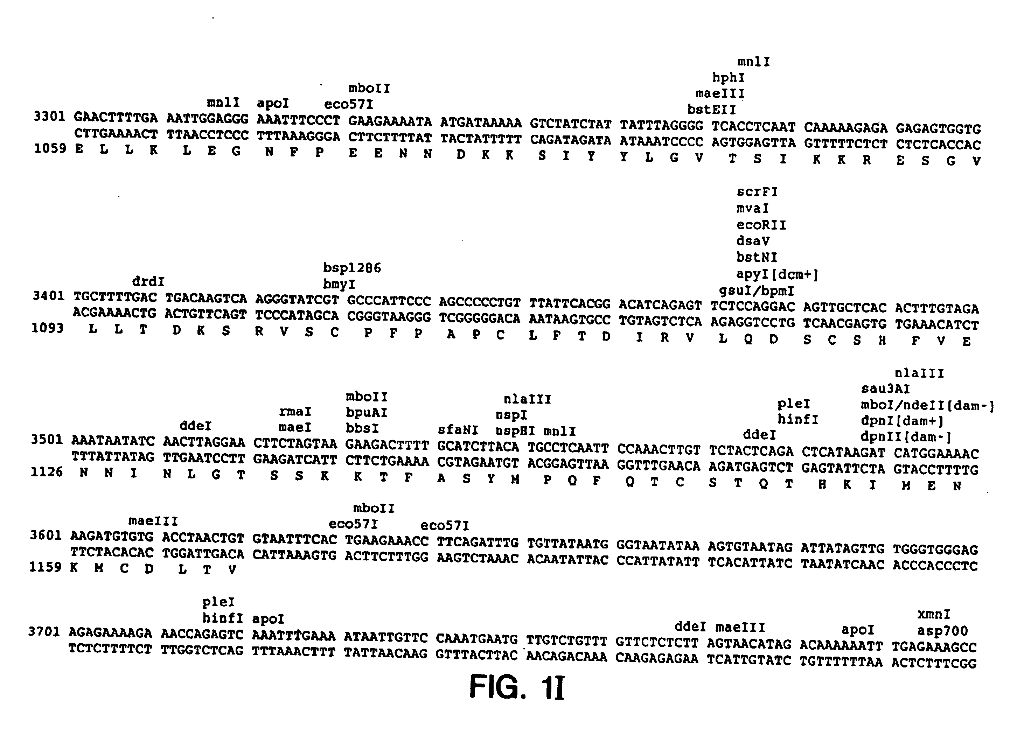

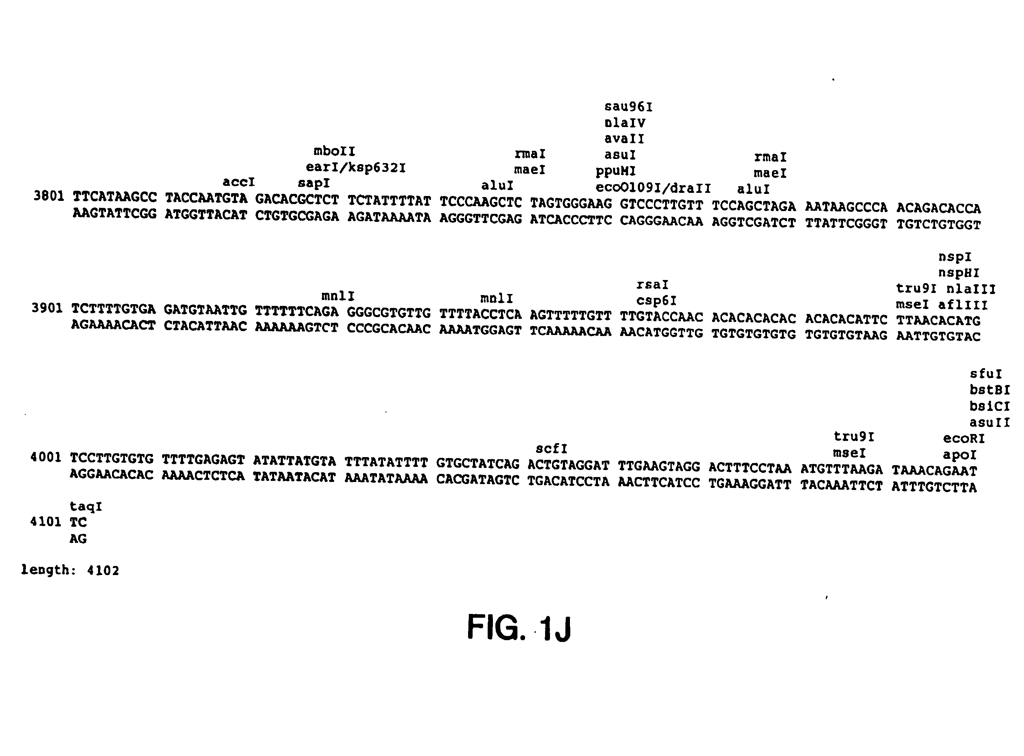

Figs. 1A-H together depict the double stranded nucleotide (SEQ ID NO:1) and deduced amino acid sequence (SEQ ID NO:2) encoding full length human WSX receptor variant 13.2. Nucleotides are numbered at the beginning of the sense strand. Amino acid residues are numbered at the beginning of the amino acid sequence. Restriction enzyme sites are depicted above the nucleotide sequence.

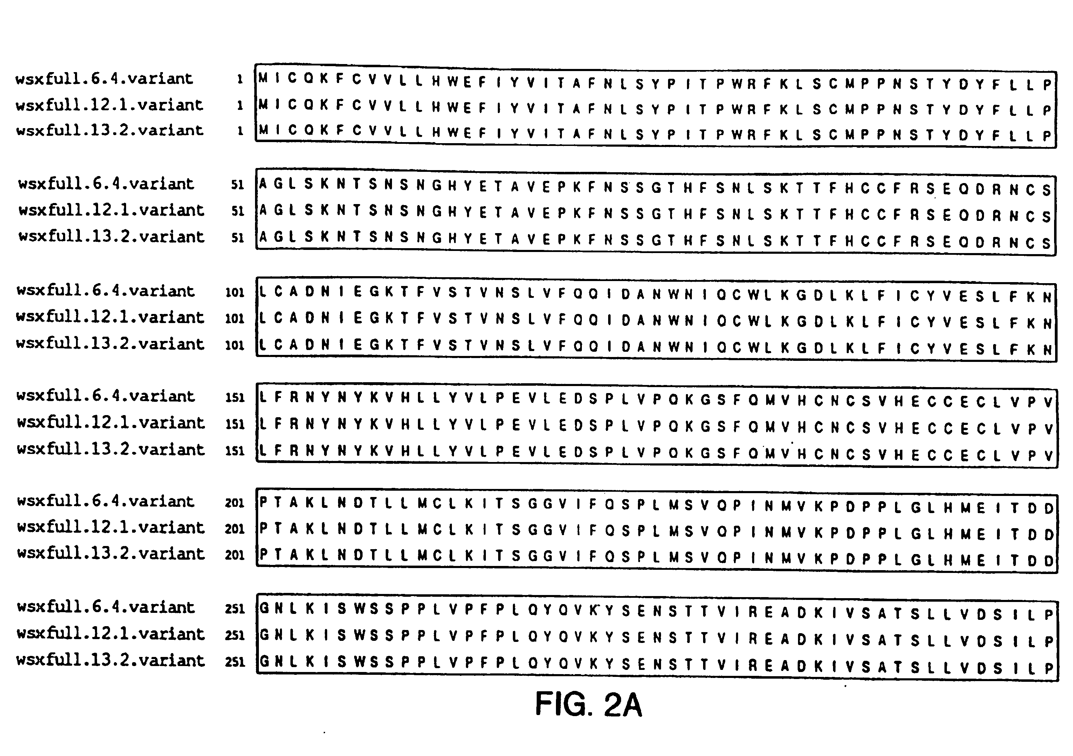

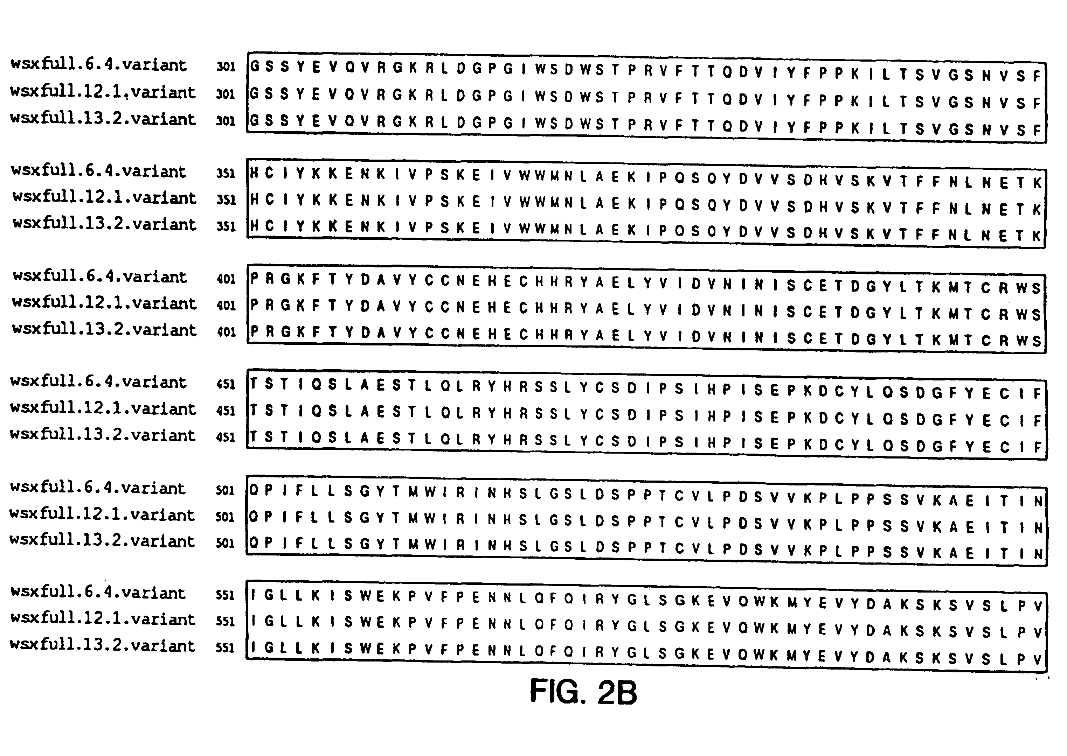

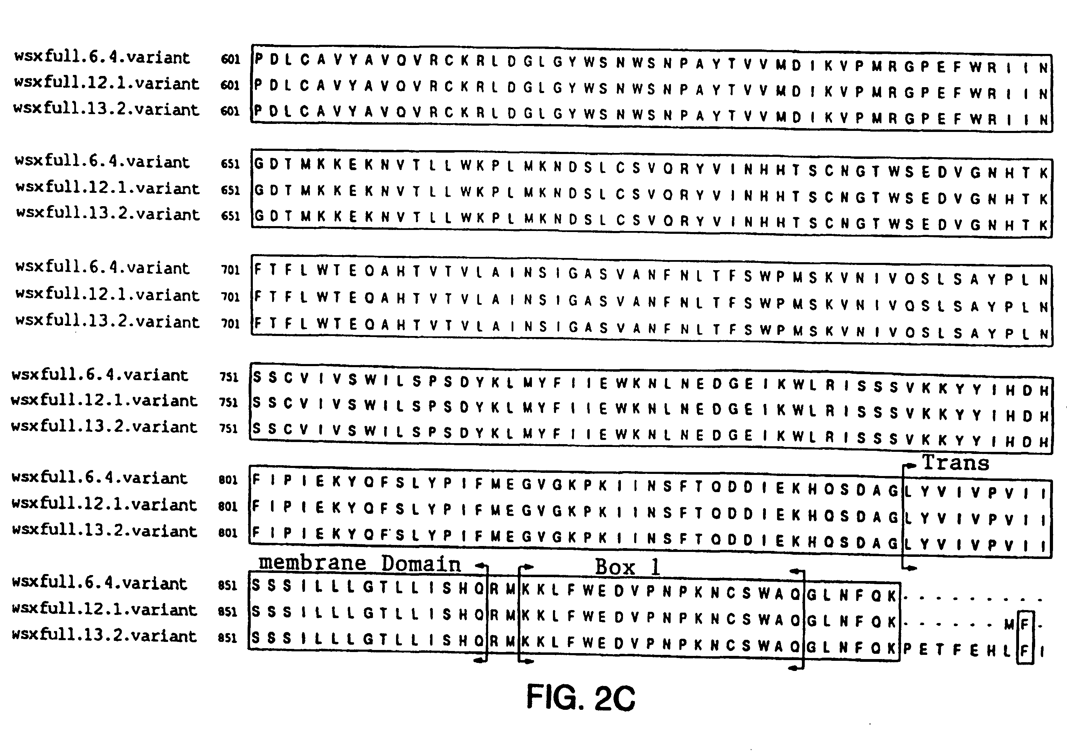

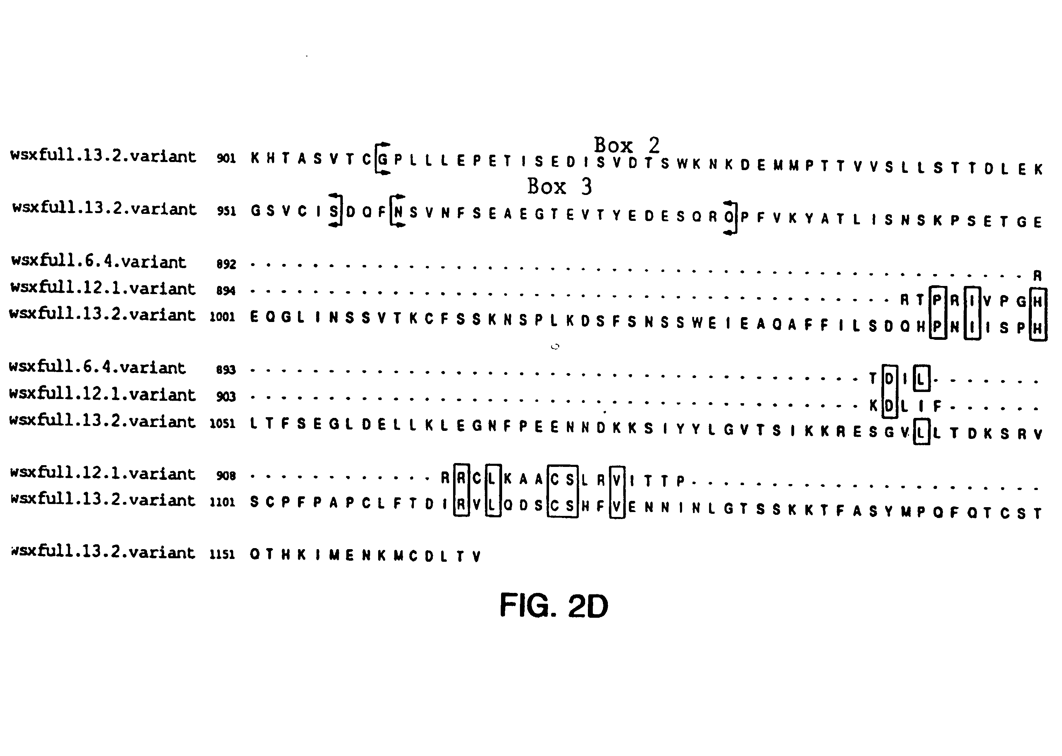

Figs. 2A-B together depict an amino acid sequence alignment of full length human WSX receptor variants 6.4 (SEQ ID NO:3), 12.1 (SEQ ID NO:4) and 13.2, respectively. Homologous residues are boxed. WSX receptor variants 6.4, 12.1 and 13.2 are native sequence human WSX receptor variants which, without being bound to any one theory, appear to be generated by alternate splicing of WSX receptor mRNA. The putative signal peptide, transmembrane, Box 1, Box 2, and Box 3 domains are indicated. The extracellular and cytoplasmic domains are amino- and carboxy-terminal, respectively, to the transmembrane domain. The Box 1-3 domains shown correspond to the box 1-3 motifs described in Baumann *et al., Mol. Cell. Biol*. 14(1):138-146 (1994).

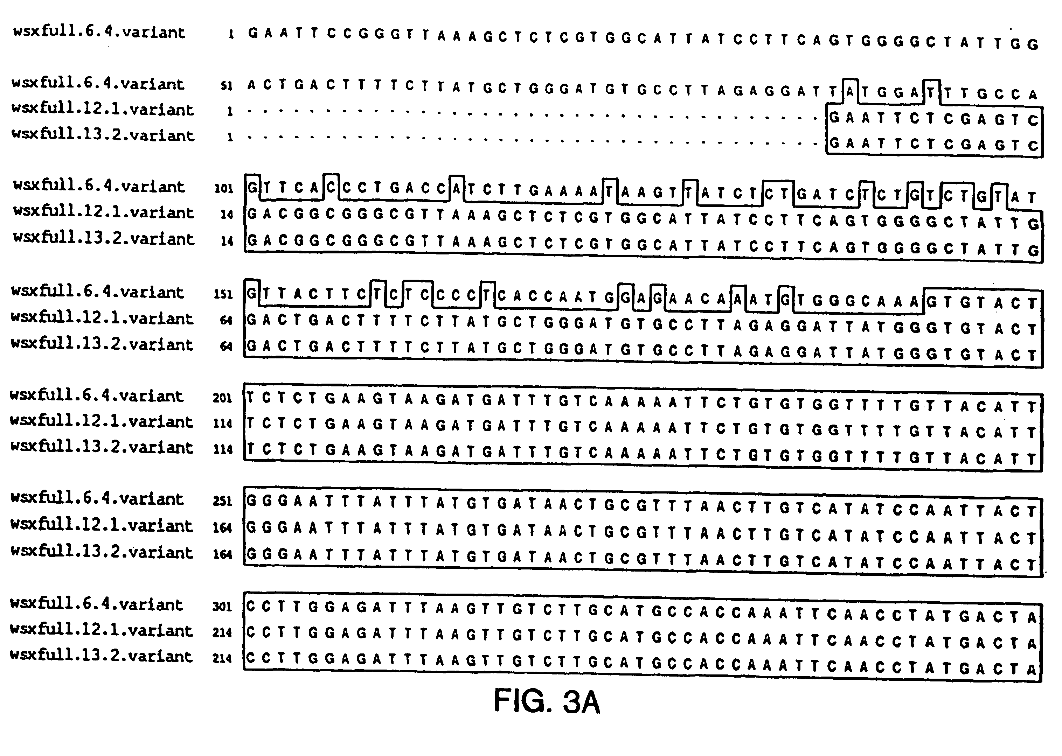

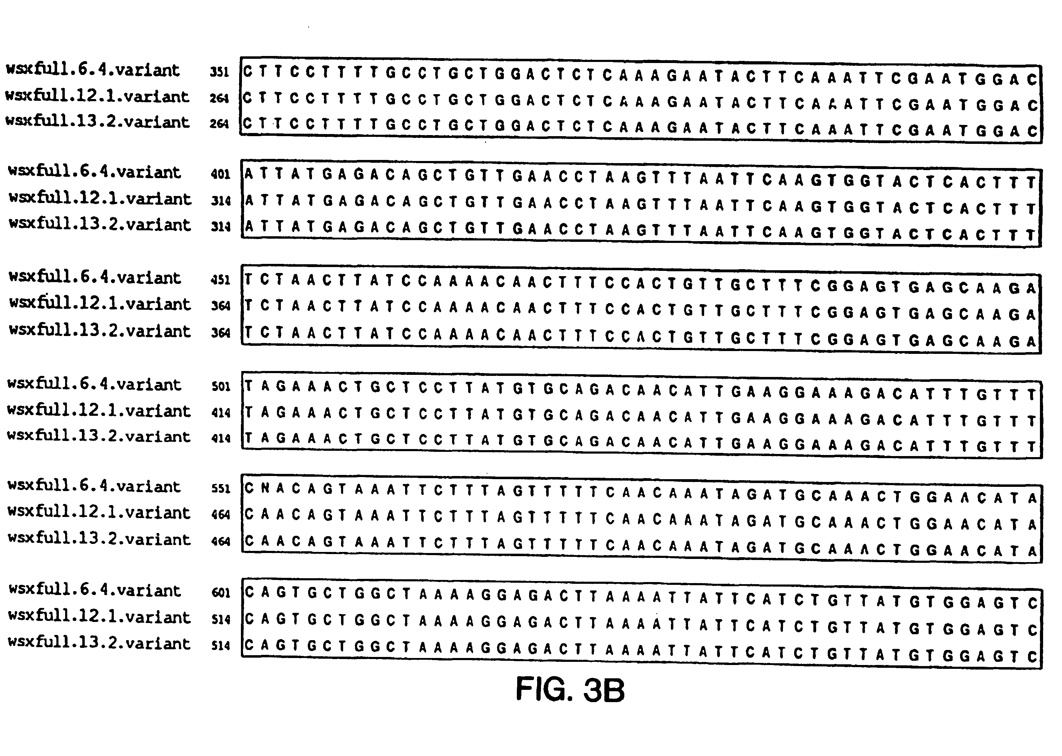

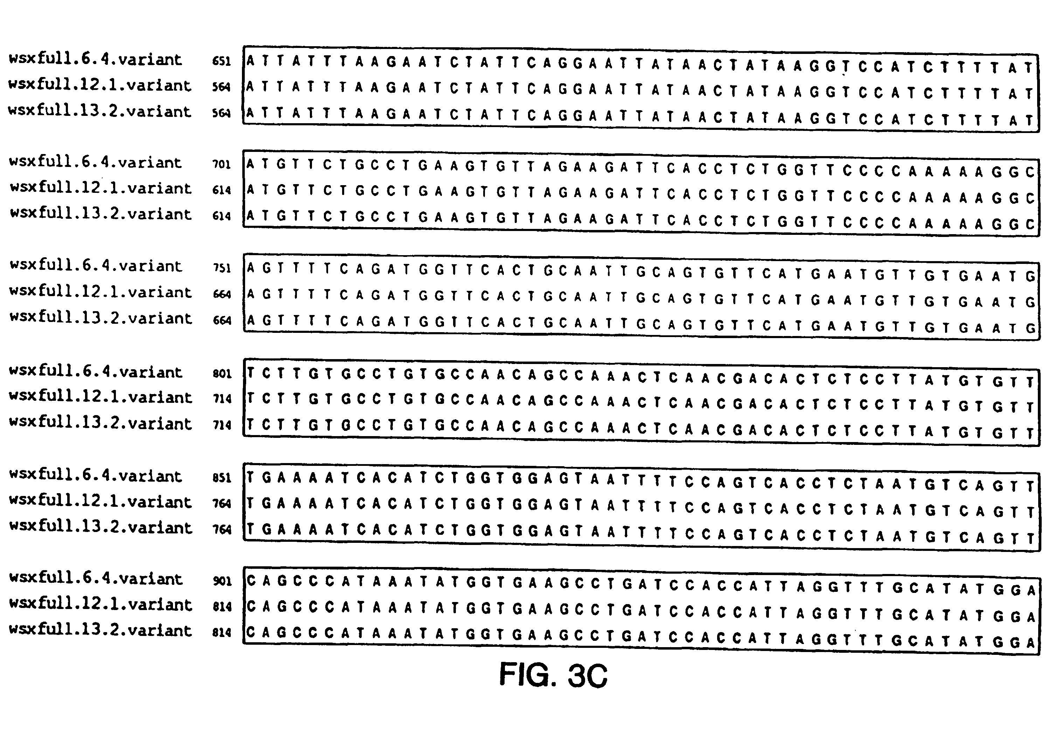

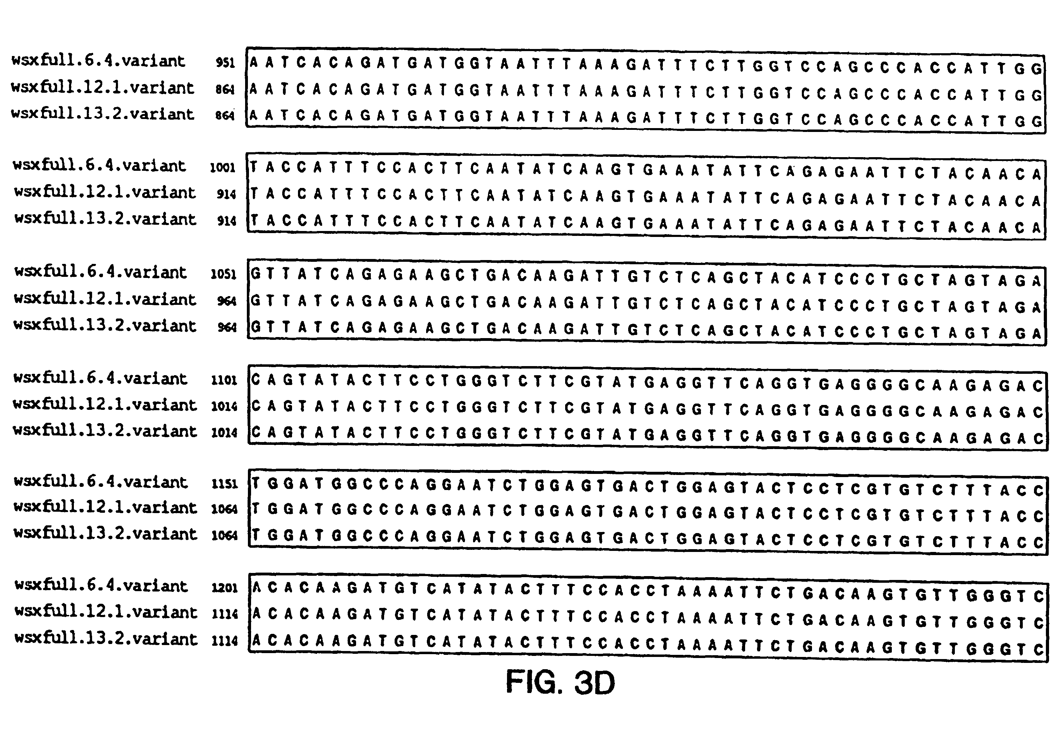

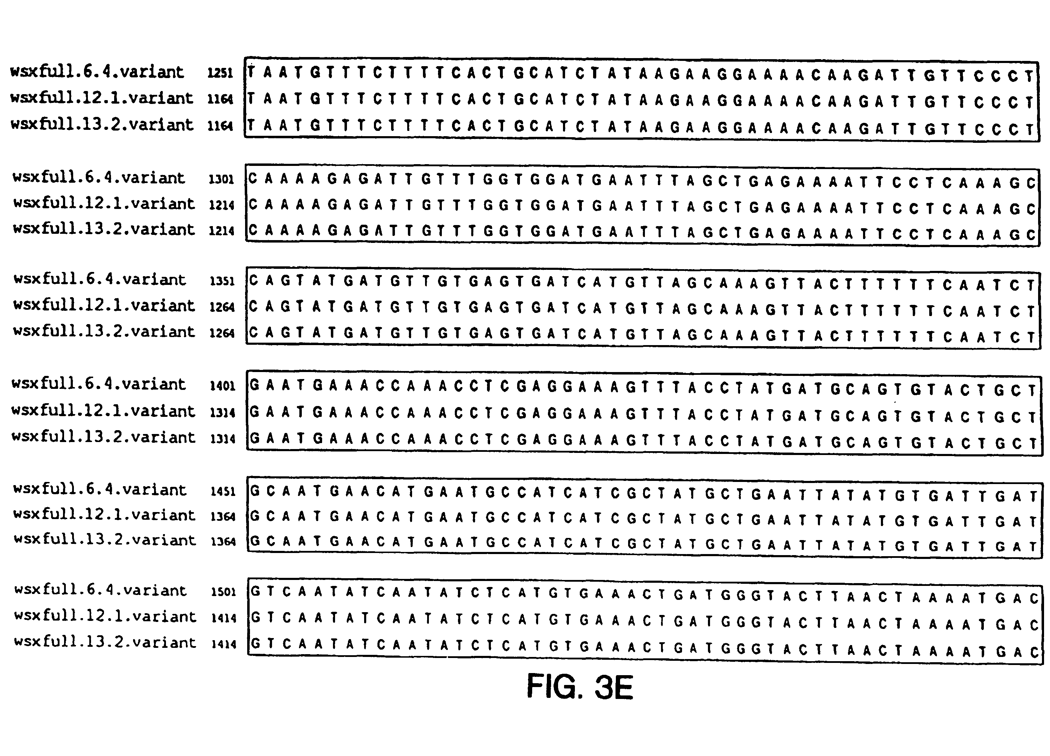

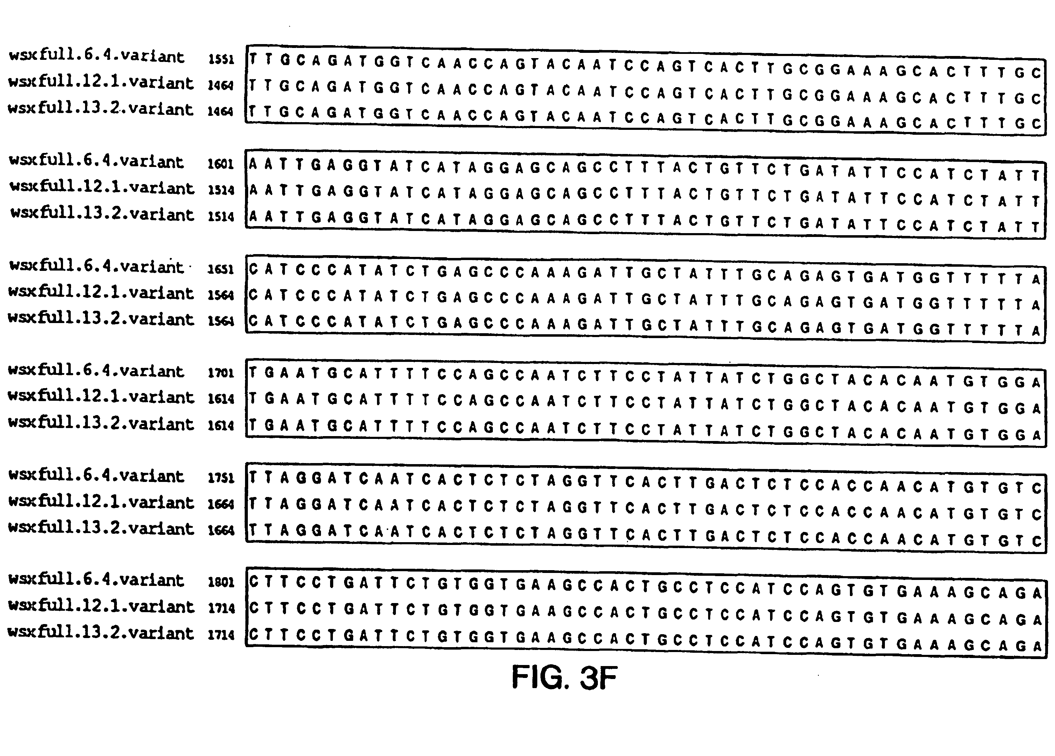

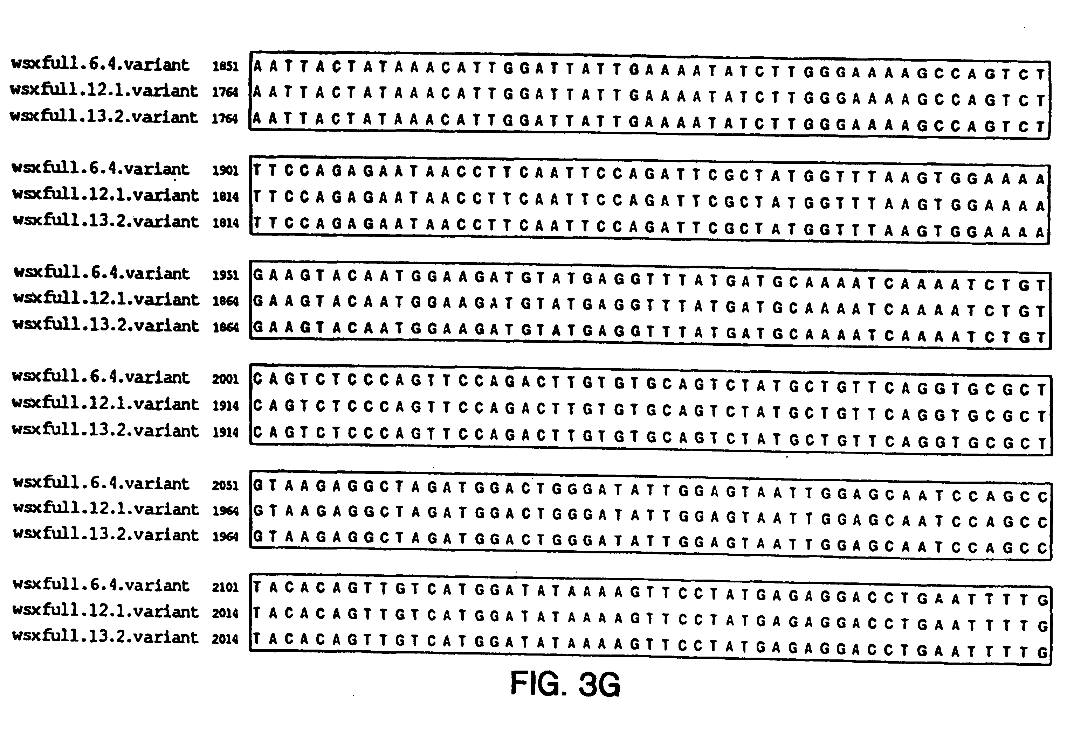

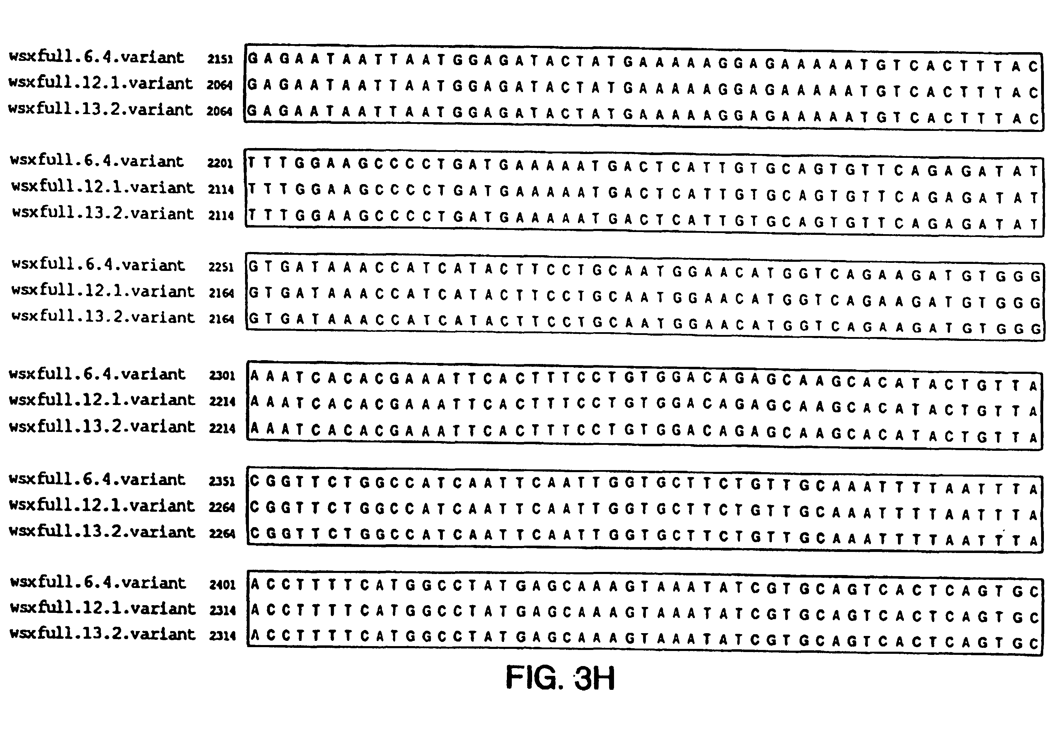

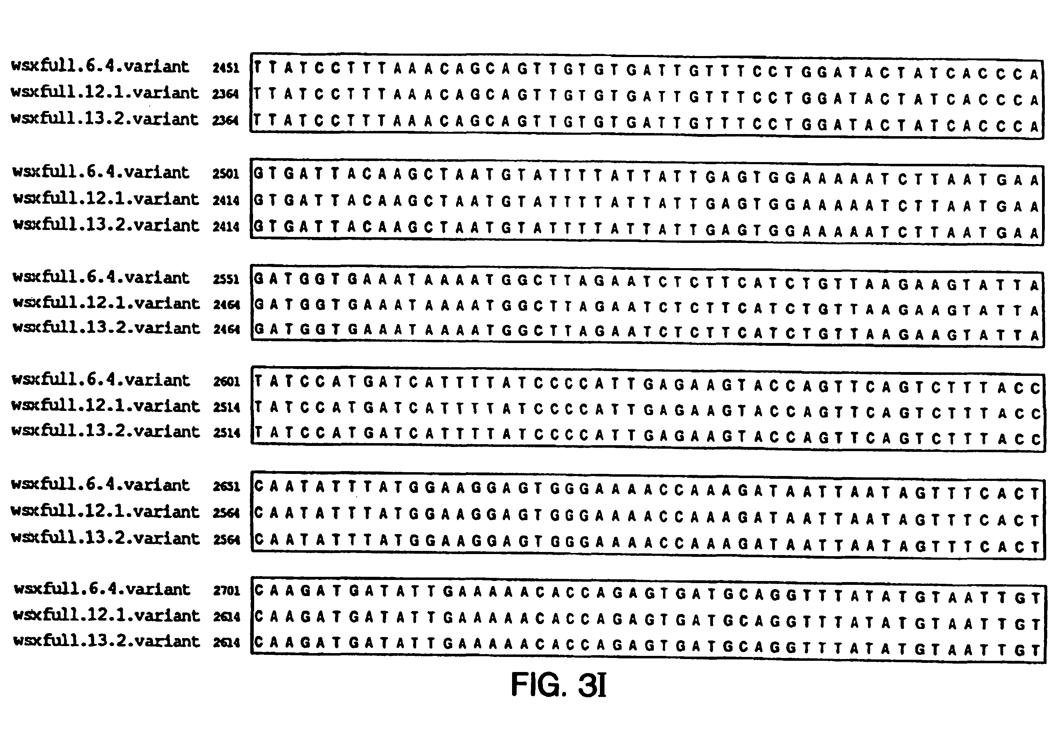

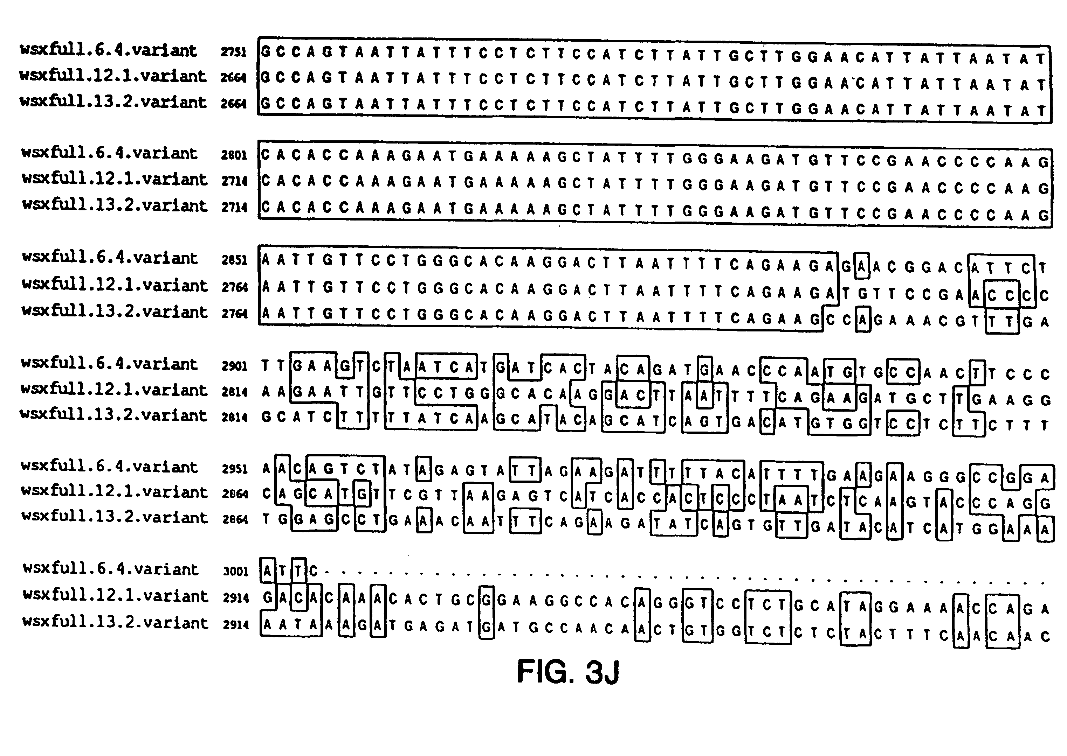

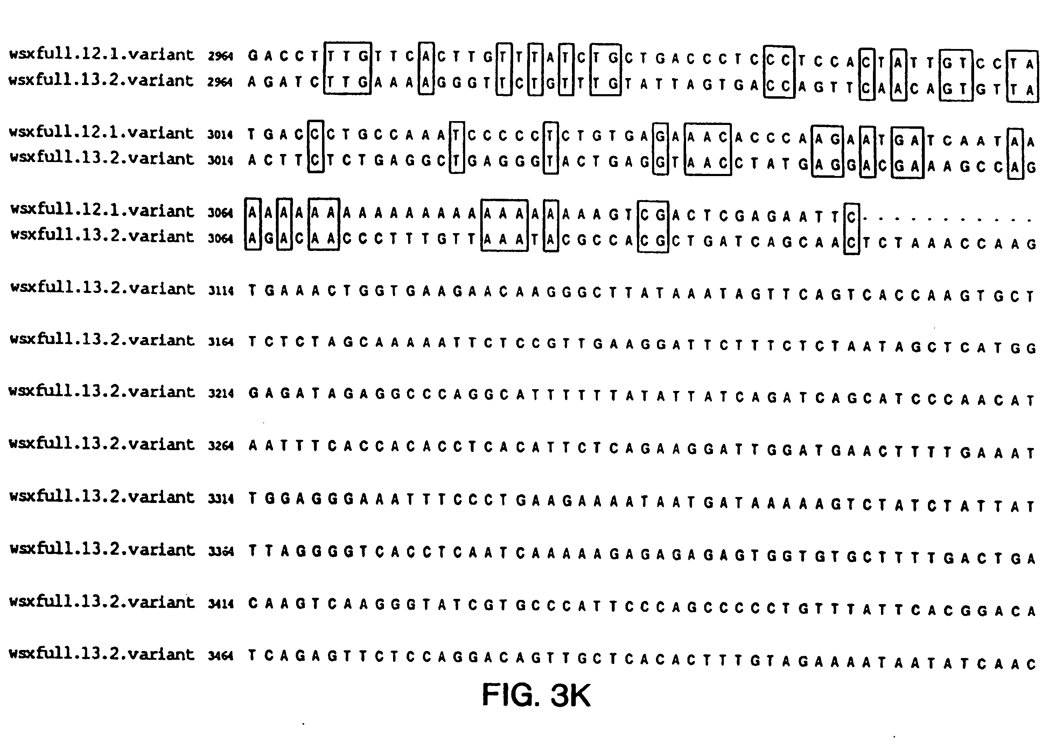

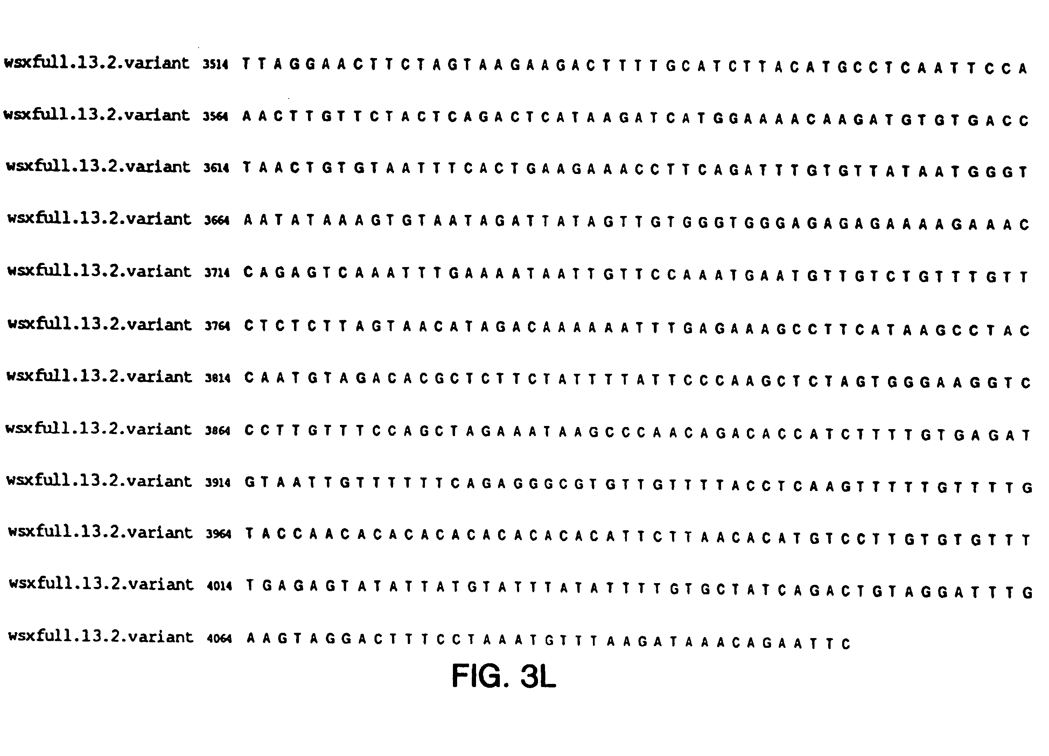

Figs. 3A-E together depict an alignment of the nucleotide sequences encoding human WSX receptor variants 6.4 (SEQ ID NO:5), 12.1 (SEQ ID NO:6) and 13.2, respectively.

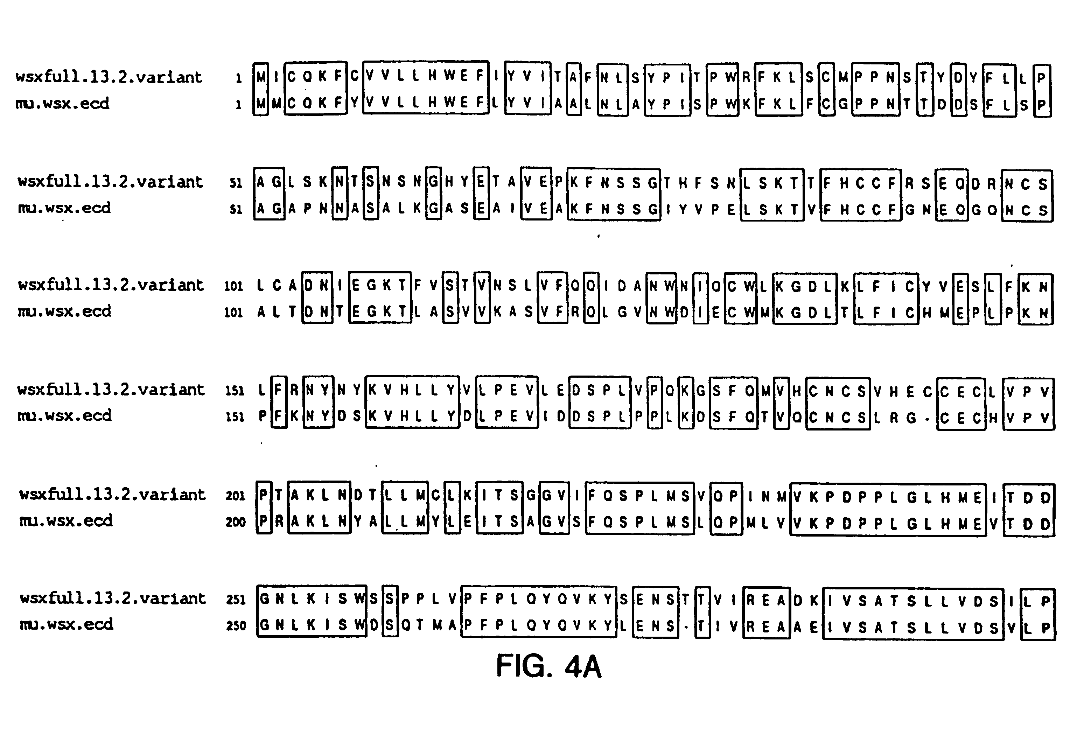

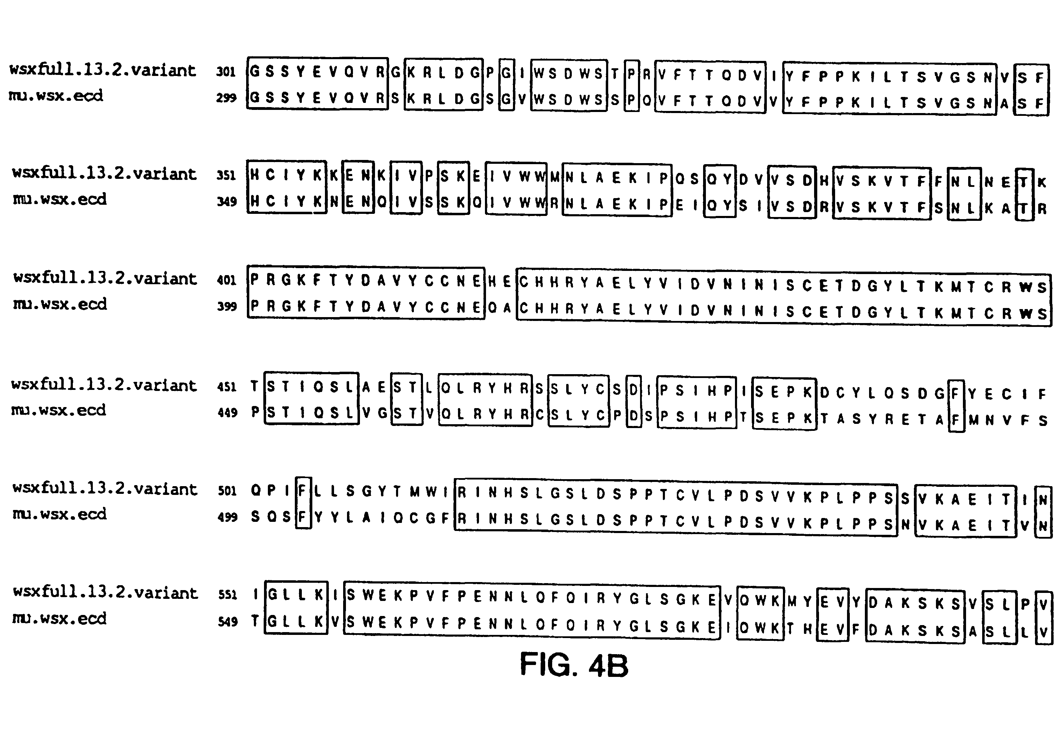

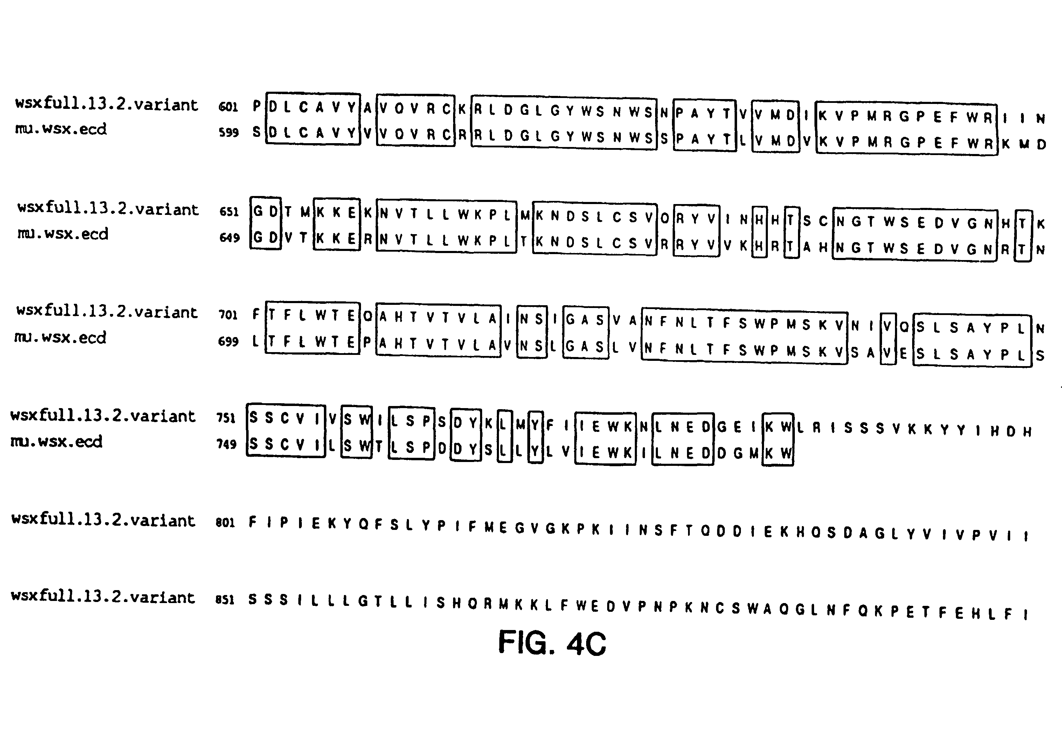

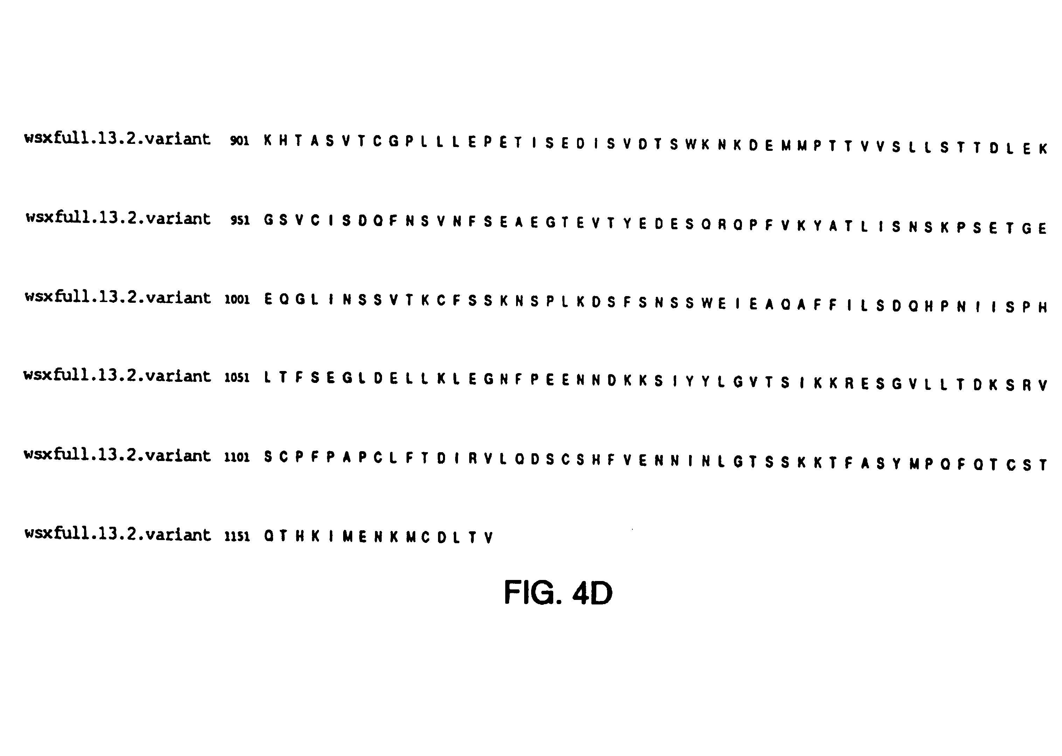

Figs. 4A-B depict an alignment of the full length human WSX receptor variant 13.2 amino acid sequence (top) with that of partial murine WSX receptor extracellular domain sequence (bottom) (SEQ ID NO:7) obtained as described in Example 7. The putative murine signal peptide is marked with an arrow.

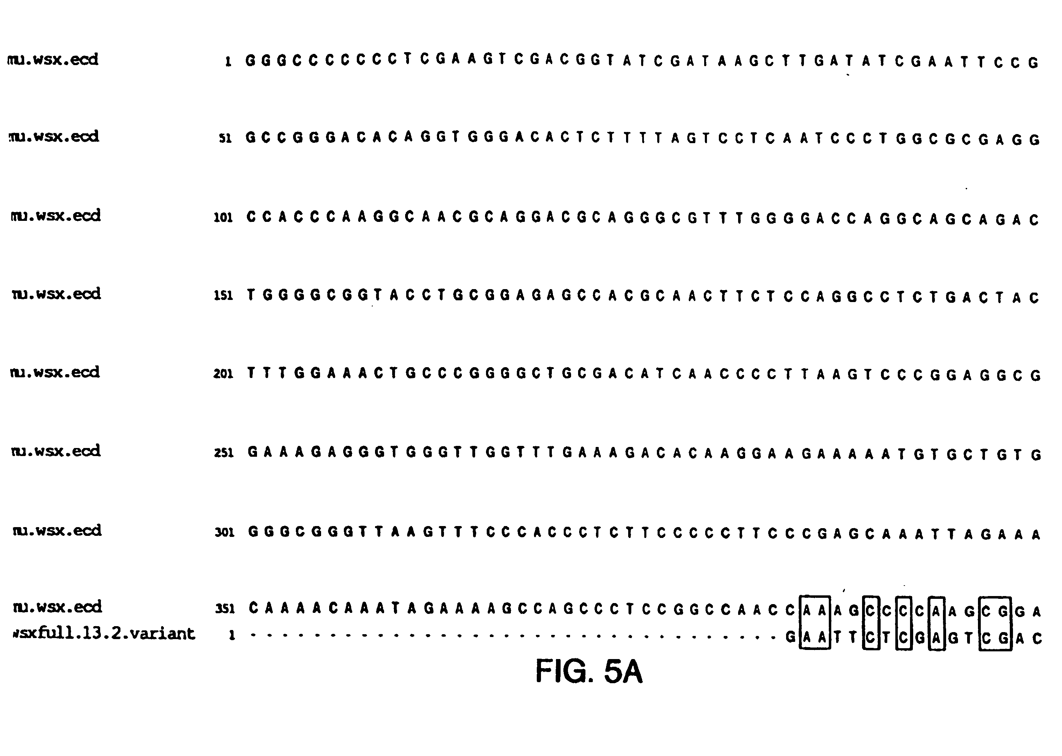

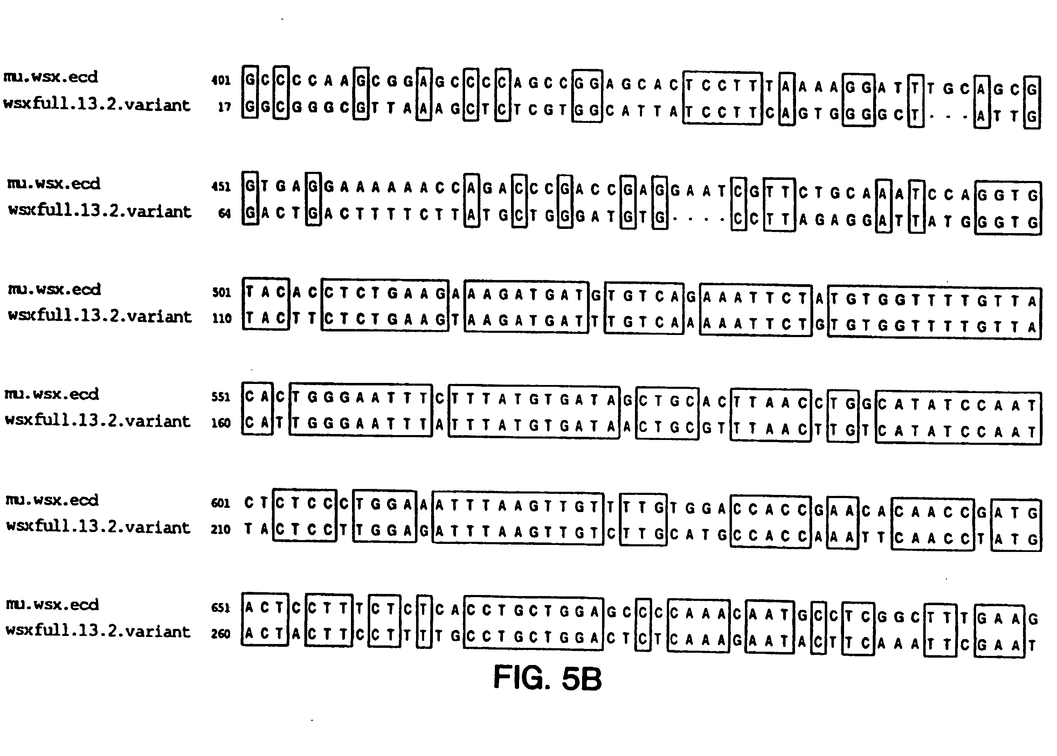

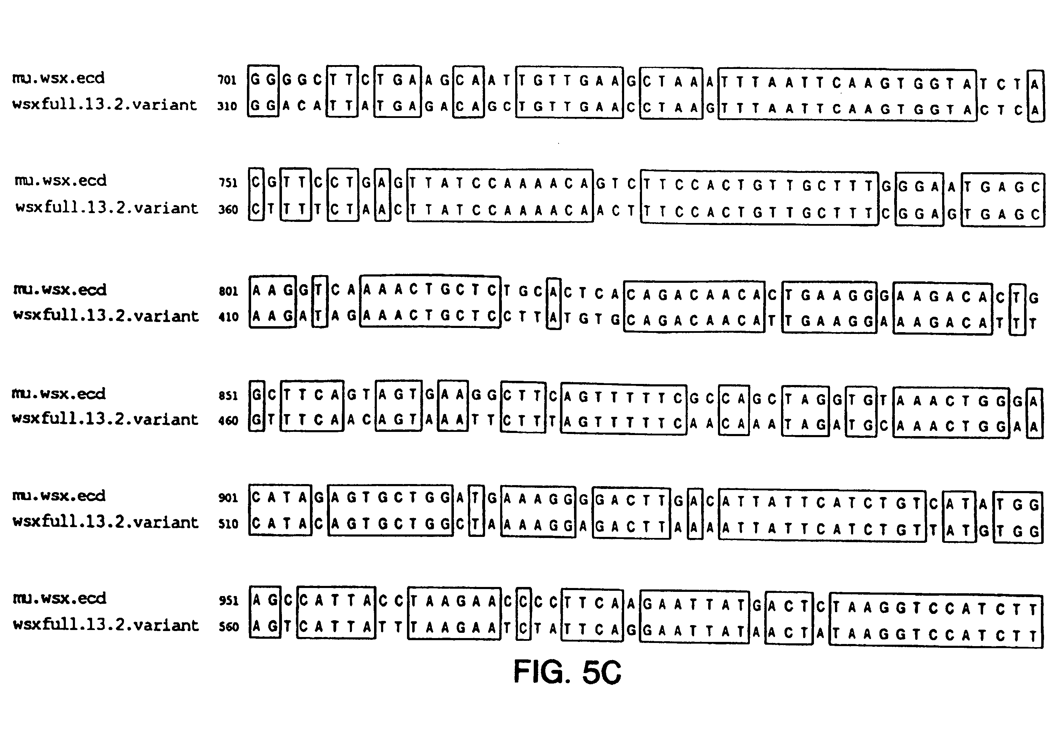

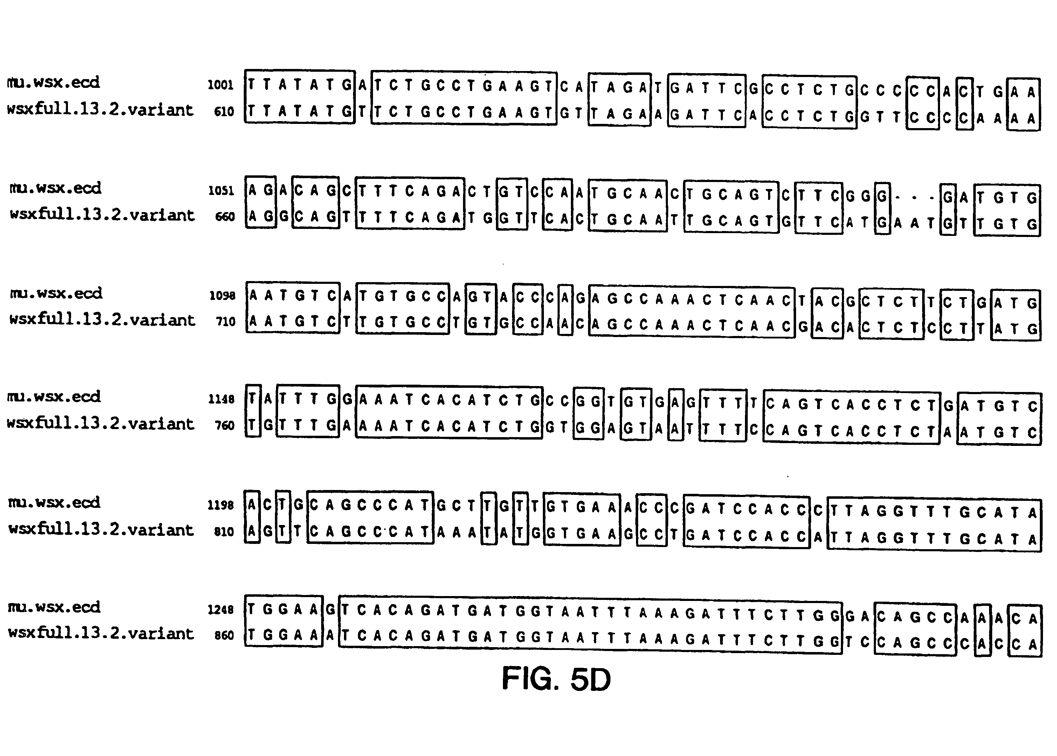

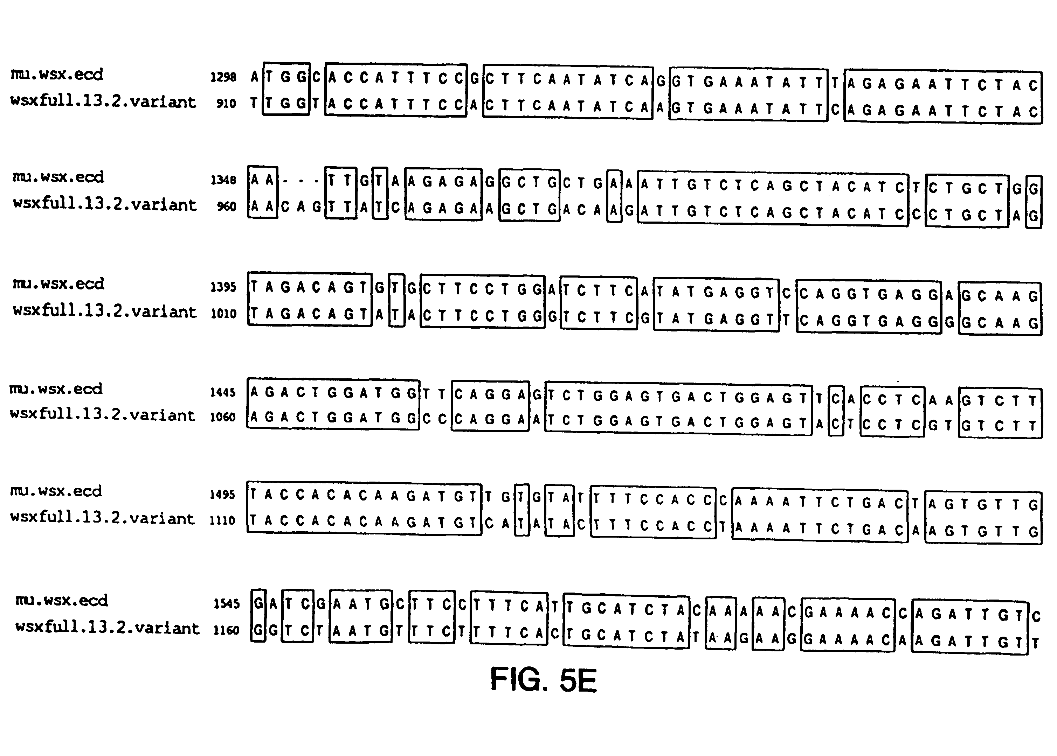

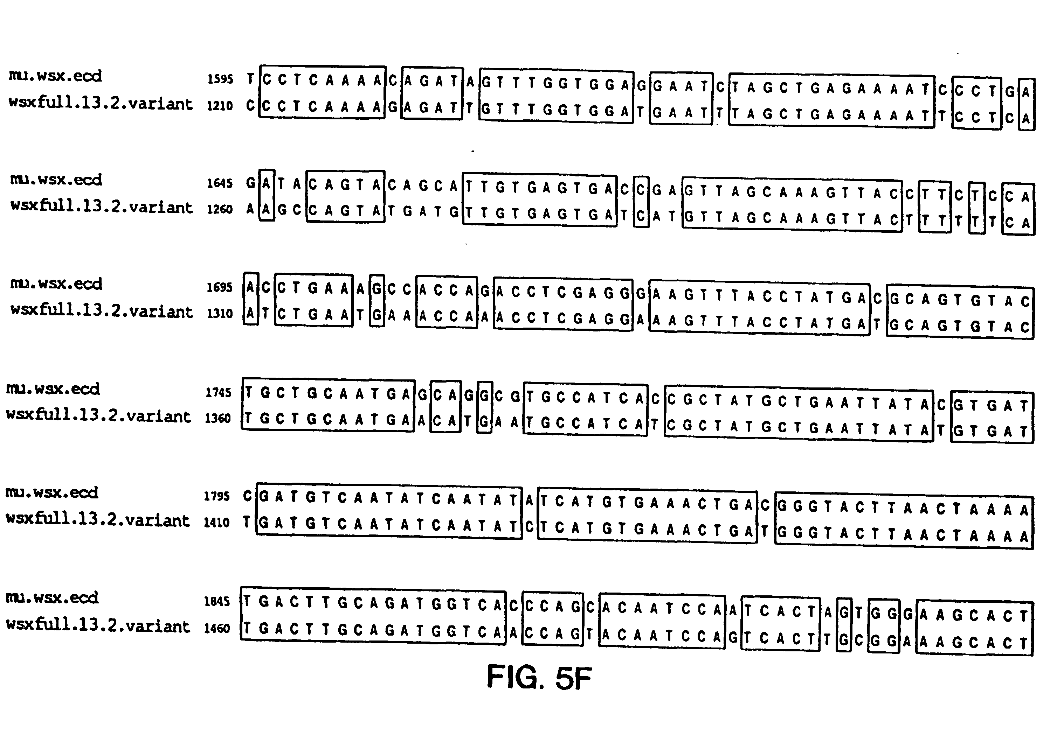

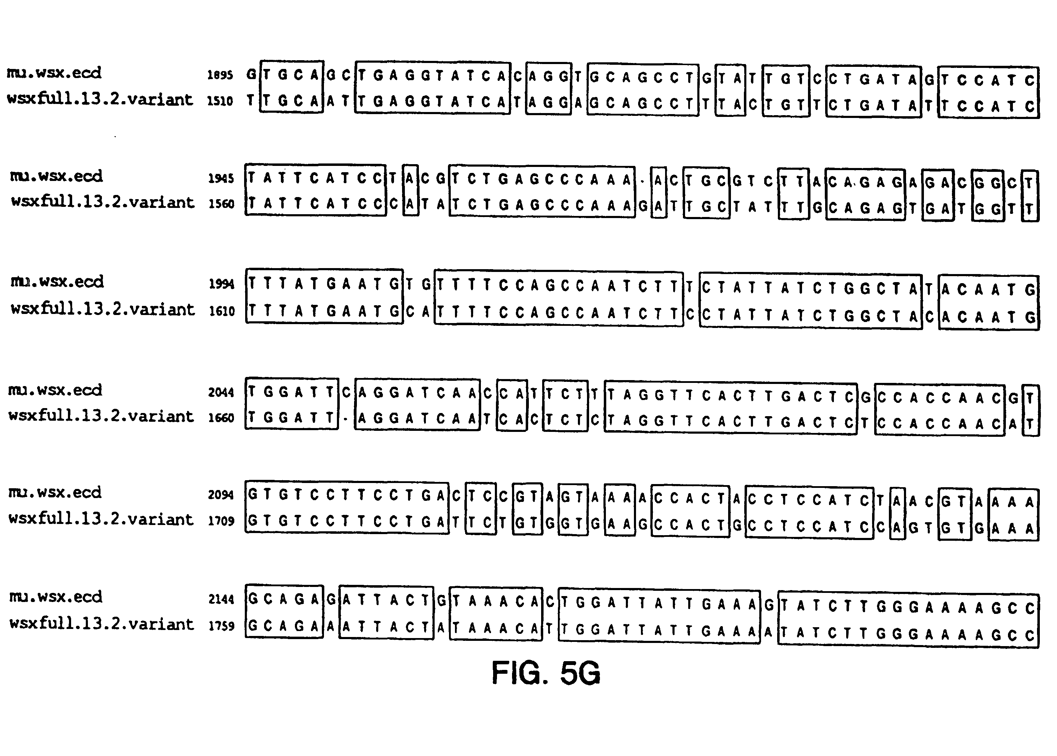

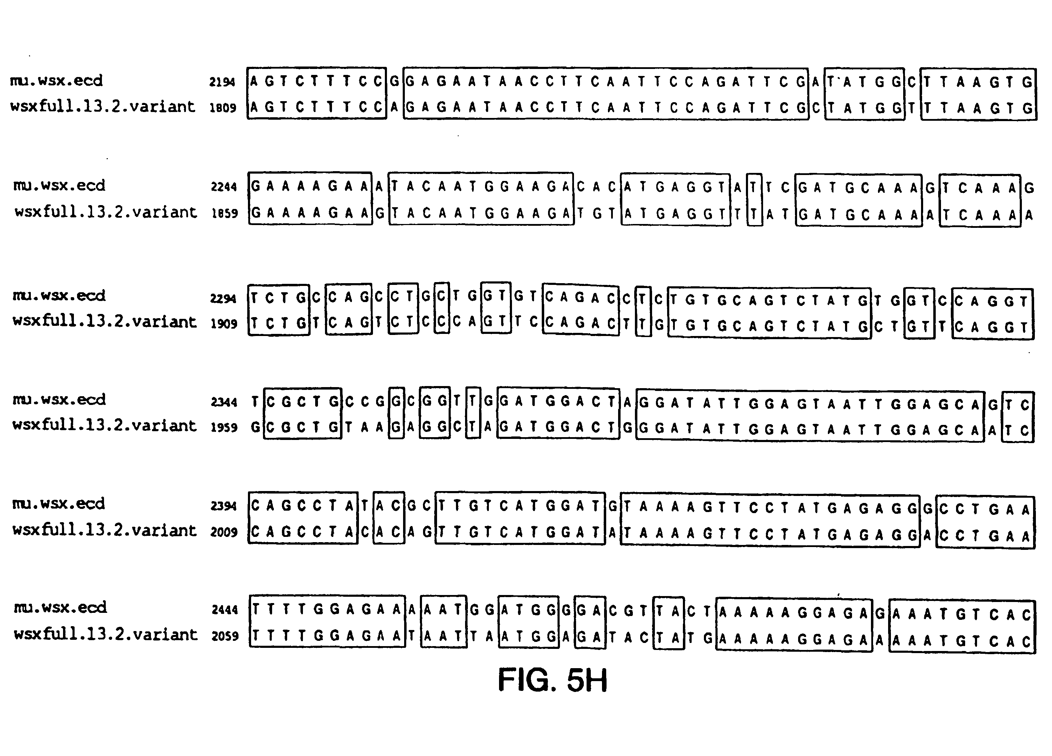

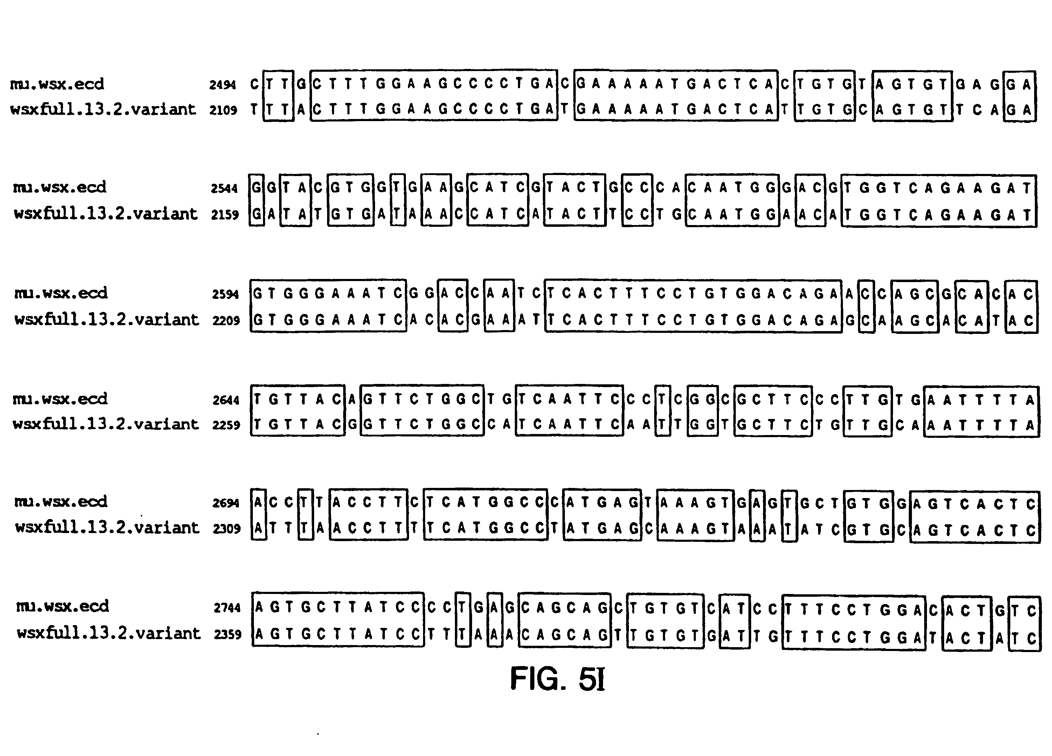

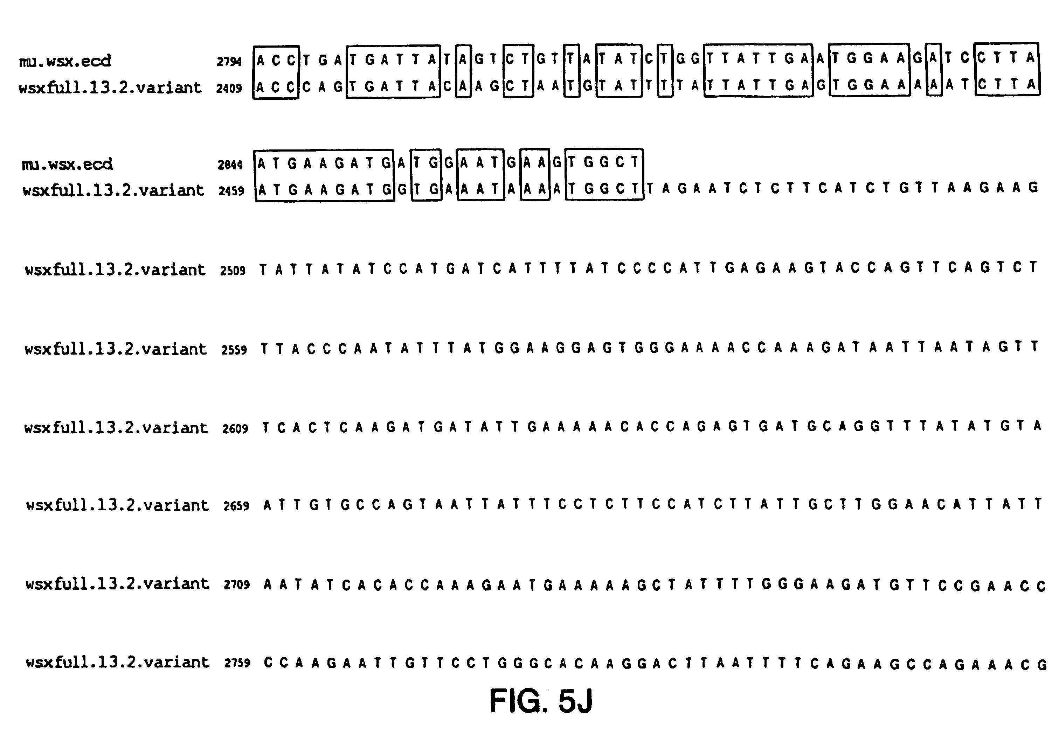

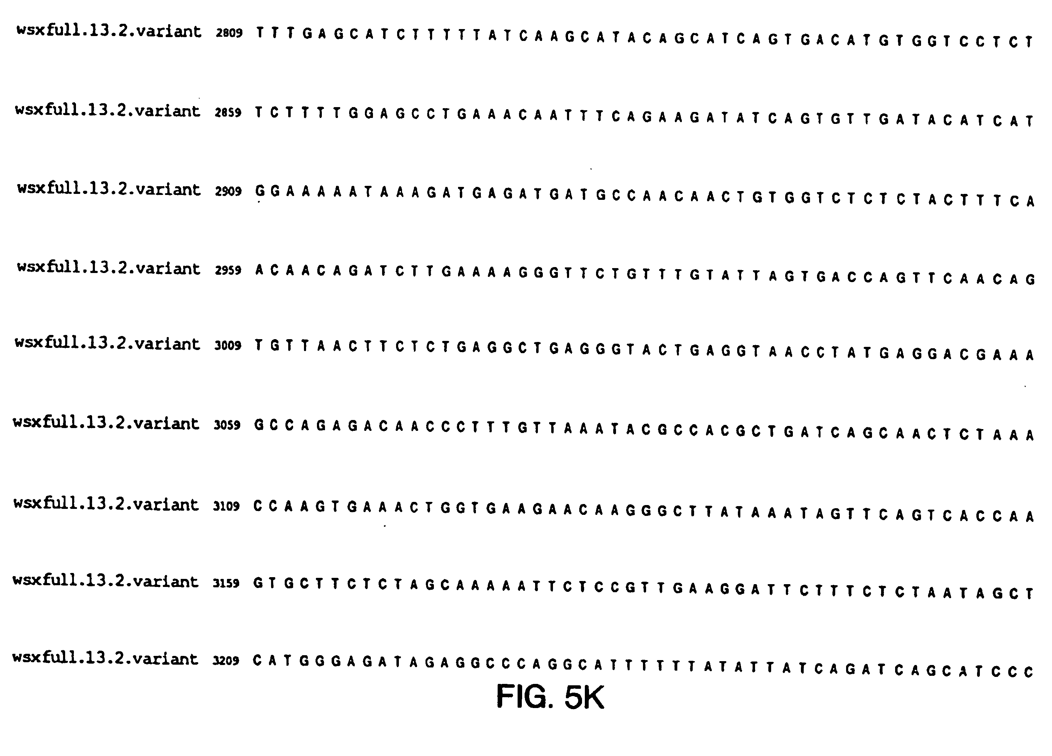

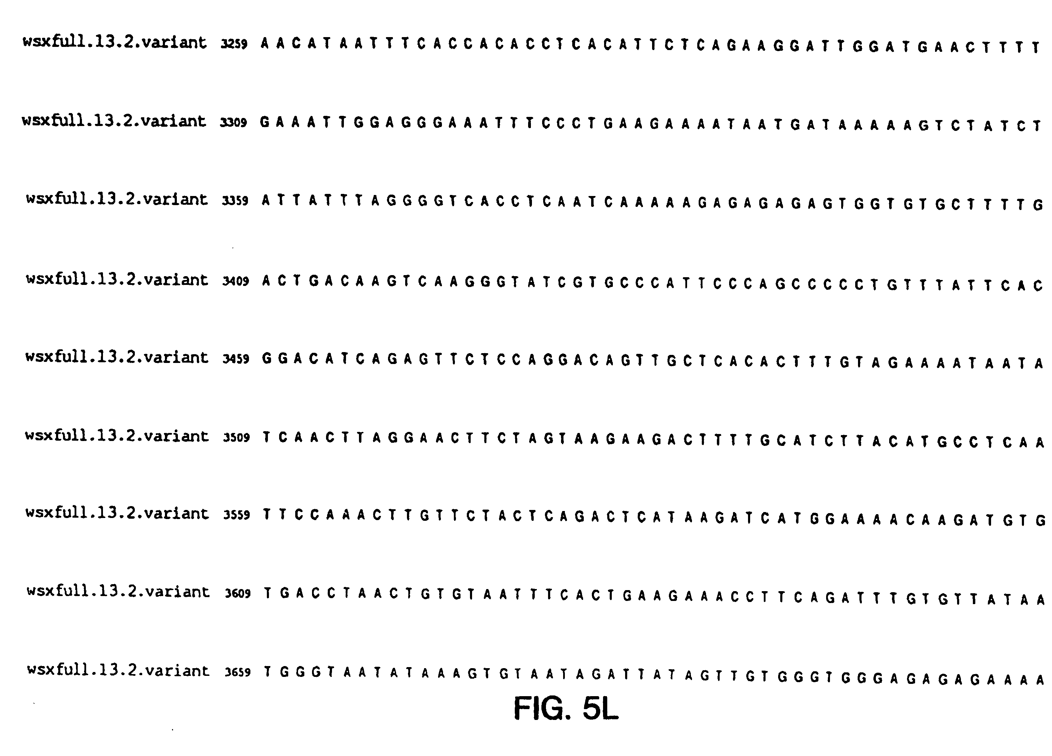

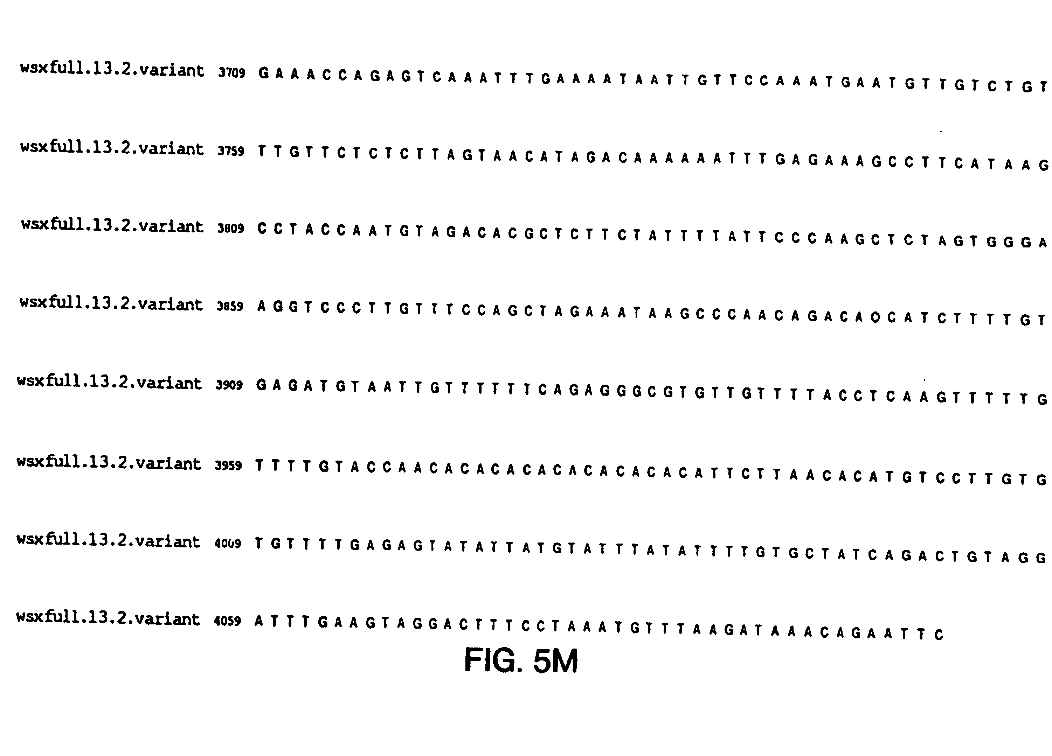

Figs. 5A-F represent an alignment of the nucleotide sequences encoding human WSX receptor variant 13.2 (bottom) and partial murine WSX receptor extracellular domain (top) (SEQ ID NO:8), respectively.

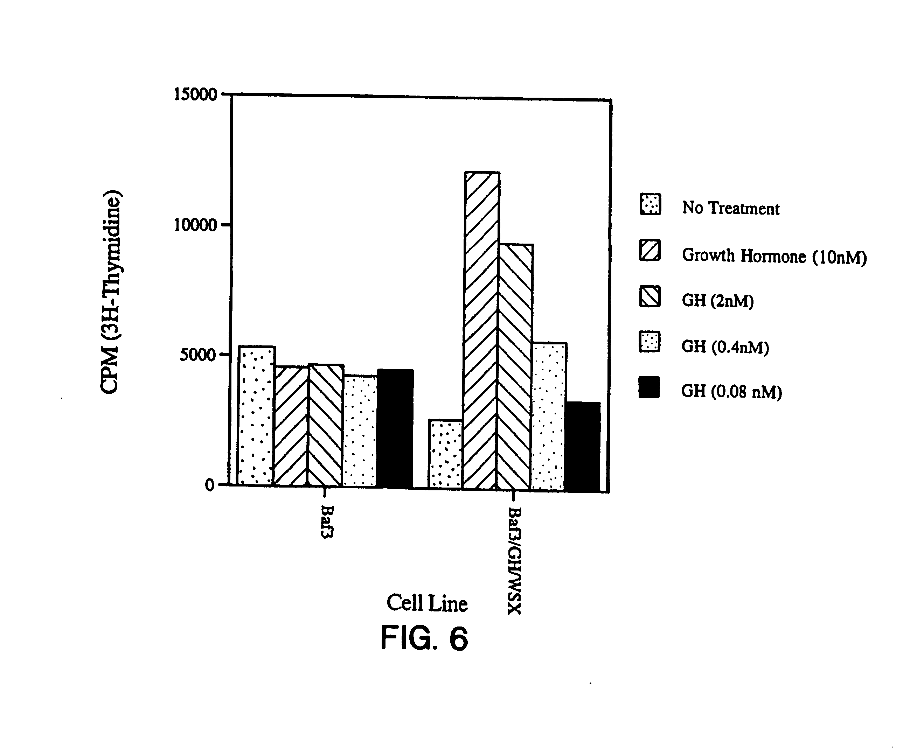

Fig. 6 is a bar graph depicting results of the thymidine incorporation assay described in Example 5. ³H-thymidine incorporation (counts per minute, CPM) in parental Baf3 cells or Baf3 cells electroporated with GH/WSX variant 13.2 chimera in the presence of varying concentrations of human growth hormone (GH) is shown.

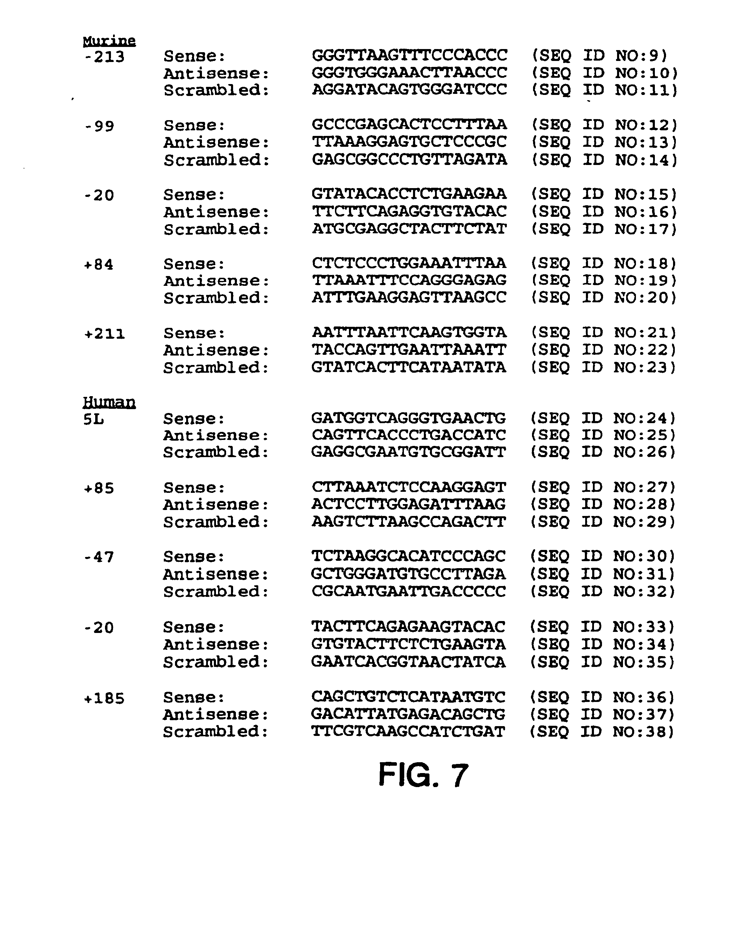

Fig. 7 shows the human and murine oligonucleotides (SEQ ID NOS:9-38, respectively) used for the antisense experiment described in Example 8.

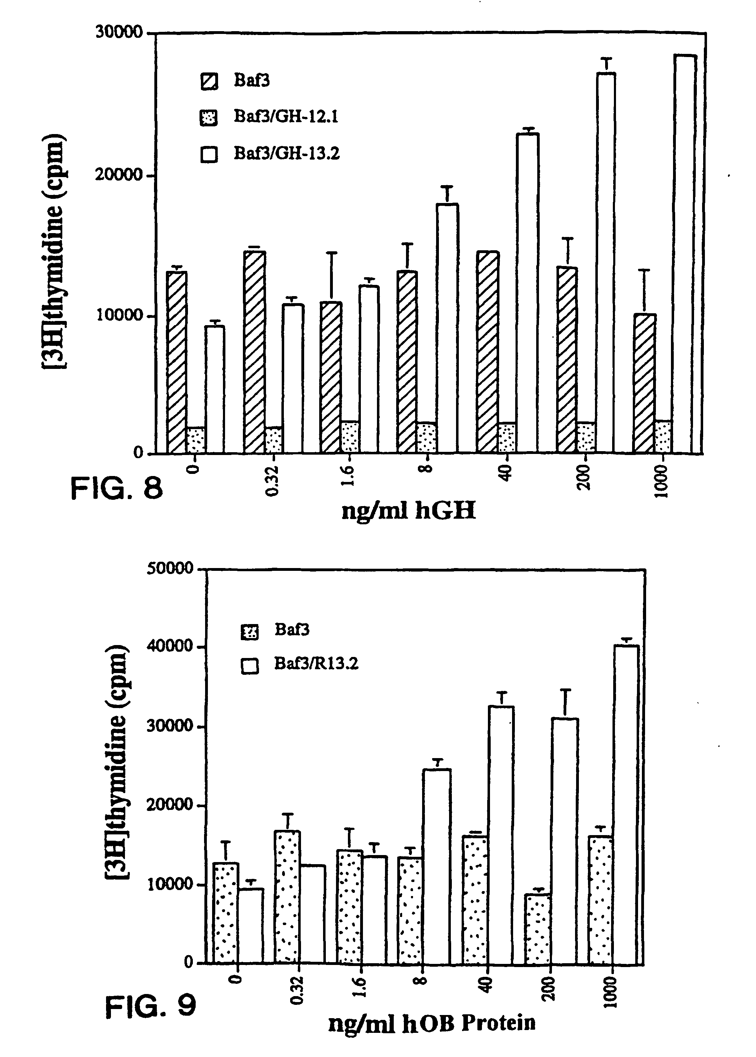

Figs. 8 and 9 show thymidine incorporation assays in Baf-3 cells. For these assays, cells were deprived of IL-3 for 16-18 hours (in RPMI 1640 supplemented with 10% fetal calf serum (FCS)). Cells were washed in serum free RPMI 1640 and plated at 50,000 cells per well in 0.2 mls of serum free RPM1 1640 supplemented with the indicated concentration of human GH or human OB protein. Cells were stimulated for 24 hours and thymidine incorporation was determined as described (Zeigler *et al. Blood* 84:2422-2430 (1994)). Assays were performed in triplicate and the results were confirmed in three independent experiments.
In Fig. 8, GH receptor-WSX receptor variant 12.1 or 13.2 chimeric proteins were expressed in Baf-3 cells as described in Example 5. These transfected cells and the parental Baf-3 line were stimulated with hGH and the incorporation of titrated thymidine determined.
In Fig. 9, Baf-3 cells were stably transfected with WSX receptor variant 13.2. Thymidine incorporation was then determined in these cell lines following stimulation with human OB protein.

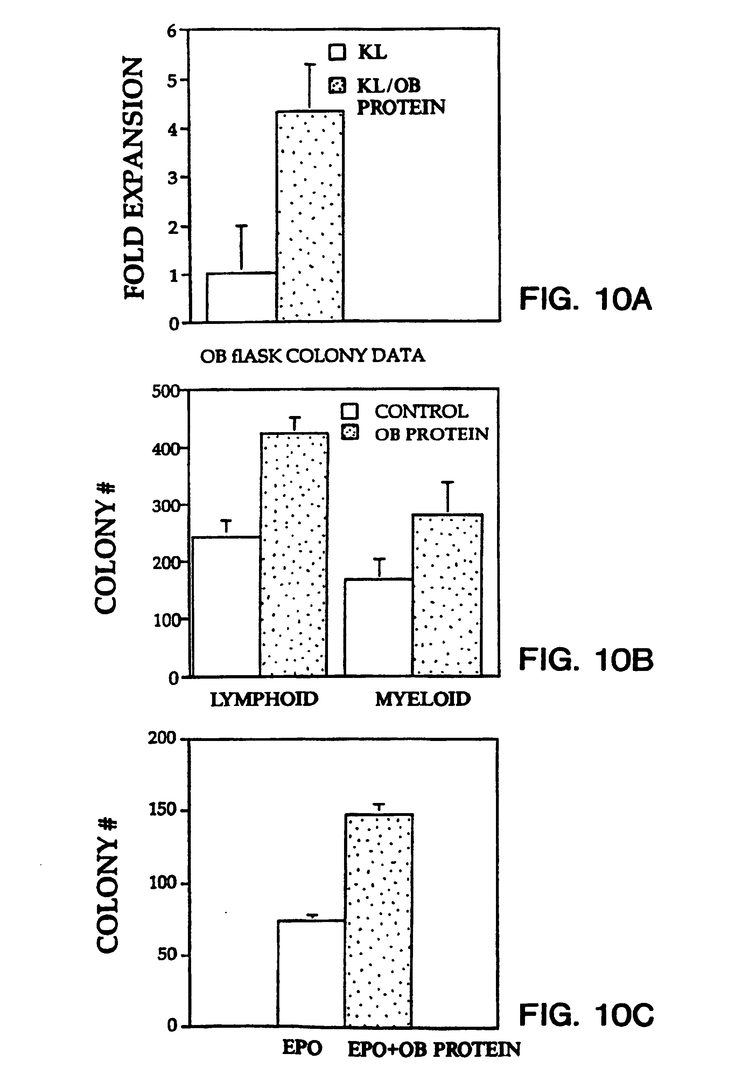

In Figs. 10A-C, murine fetal liver AA4⁺Sca⁺Kit⁺ (flASK) stem cells were cultured in suspension culture or methylcellulose. In Fig. 10A, flASK cells were cultured in suspension culture containing serum with kit ligand (KL) or kit ligand and OB protein. Cell counts and cytospin analyses were performed 7 days later. In Fig. 10B, flASK cells were seeded into methylcellulose under either myeloid or lymphoid conditions as described in Example 10. Colony counts were performed 14 days later. For colonies produced under lymphoid conditions, FACS analysis demonstrated the vast majority of cells to be B220 positive. In Fig. 10C, flASK cells were seeded into methylcellulose containing kit ligand. To this base media, erythropoietin (EPO) or erythropoietin and OB protein were then added. The resultant colonies were counted 14 days later. FACS analysis demonstrated approximately 95% of these colonies to be TER 119 positive. All assays were performed in triplicate and confirmed in at least three independent experiments.
Fig. 11 illustrates methylcellulose assays to determine the colony forming potential of *db*/*db, ob*/*ob* and the corresponding wild-type marrow. 100,000 bone marrow cells were seeded into methylcellulose and the resultant colonies counted after 14 days. Assays were performed using both myeloid and lymphoid conditions. Assays were performed in triplicate and the experiments were repeated a minimum of 3 times.
Figs. 12A-B show bone marrow cellular profiles in wild-type misty gray homozygotes, misty gray/*db* heterozygotes, and homozygote *db*/*db* mice. Overall cellularity in the *db*/*db* marrow was unchanged compared to controls. Fig. 12A shows cellular profiles determined using anti-B220, anti-CD43, and anti-TER119 antibodies. Fig. 12B shows cellular profiles of the spleens from the above groups.
Figs. 13A-C are an analysis of peripheral blood in *db*/*db* homozygotes, *db*/*db* misty gray heterozygotes and misty gray homozygotes. 40 microliters of peripheral blood was taken via orbital bleed and analyzed on a Serrono Baker system 9018. All areas described by the boxes represent the mean ± one standard deviation of the two parameters.
Fig. 14 is a comparison of peripheral lymphocyte counts and blood glucose level. Five groups of animals, misty-gray, misty-gray/db, *db*/*db,* interferon α-transgenic, and glucokinase transgenic heterozygote mice (gLKa) were sampled via retro-orbital bleed. Blood glucose levels in these mice were determined. All areas described by the boxes represent the mean ± standard deviation of the two parameters.

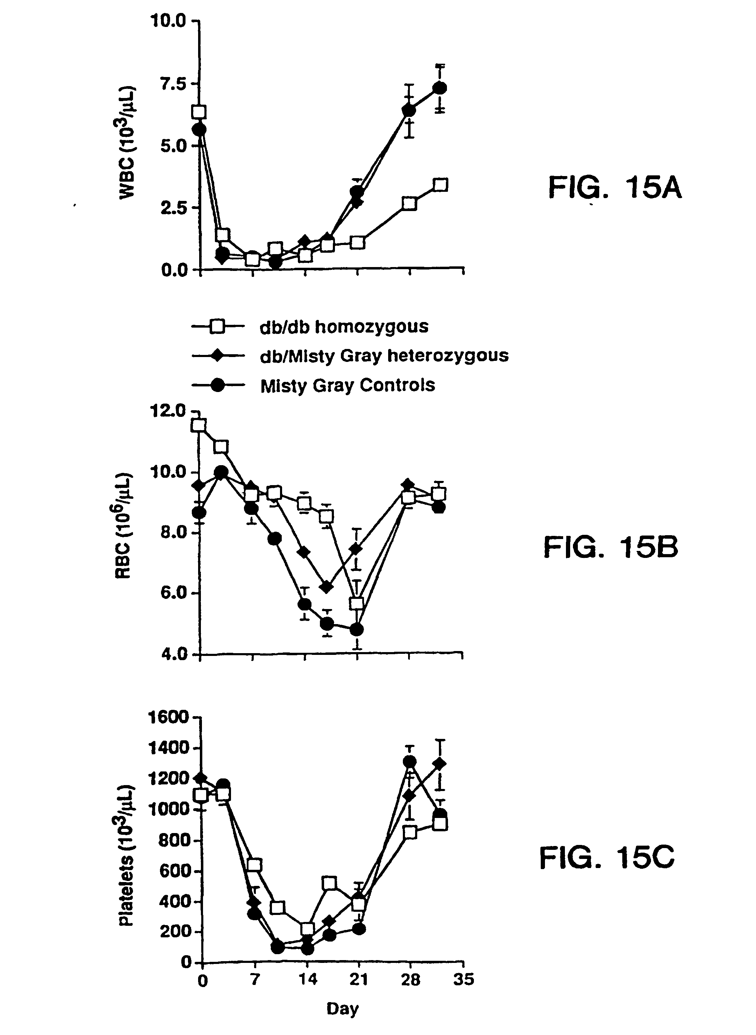

In Figs. 15A-C, misty gray homozygotes, db/misty gray heterozygotes, and homozygous *db*/*db* mice were subjected to sub-lethal irradiation and the recovery kinetics of the peripheral blood was determined via retro-orbital bleeds.
Figs. 16A-16Q together show the nucleotide sequence (SEQ ID NO:46) and the amino acid sequence (SEQ ID NO: 47) of the human OB-immunoglobulin chimera in the plasmid described in of Example 11.

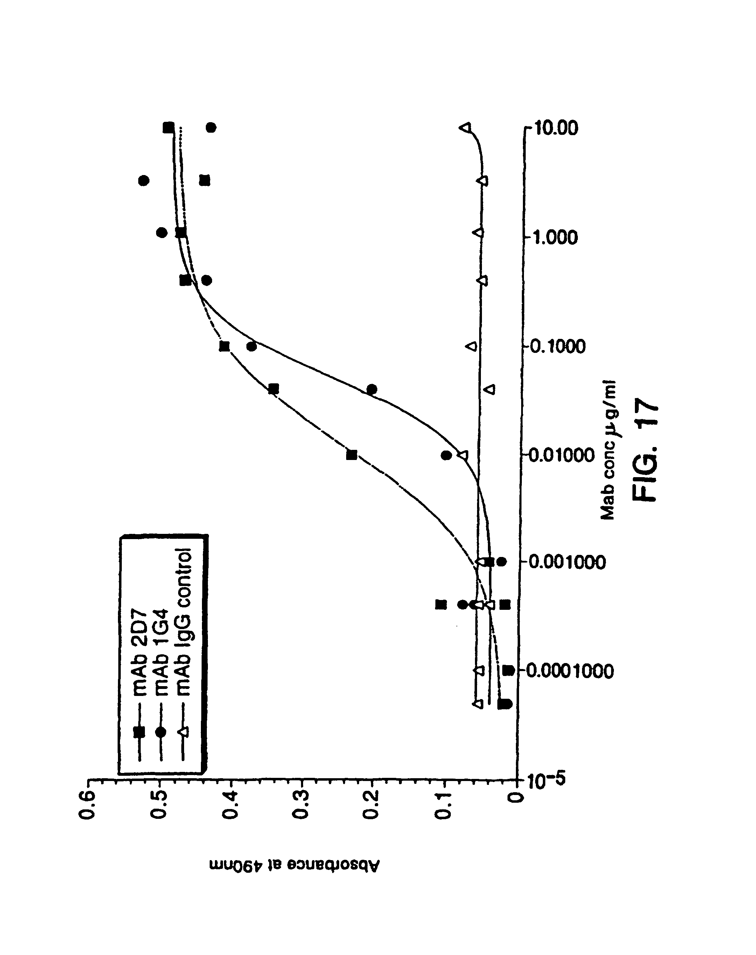

Fig. 17 shows binding of anti-WSX receptor agonist antibodies to human WSX receptor. The anti-WSX receptor agonist antibodies (2D7 and 1G4) produced as described in Example 13 and an IgG isotope control were evaluated for their ability to bind to human WSX receptor by capture ELISA.

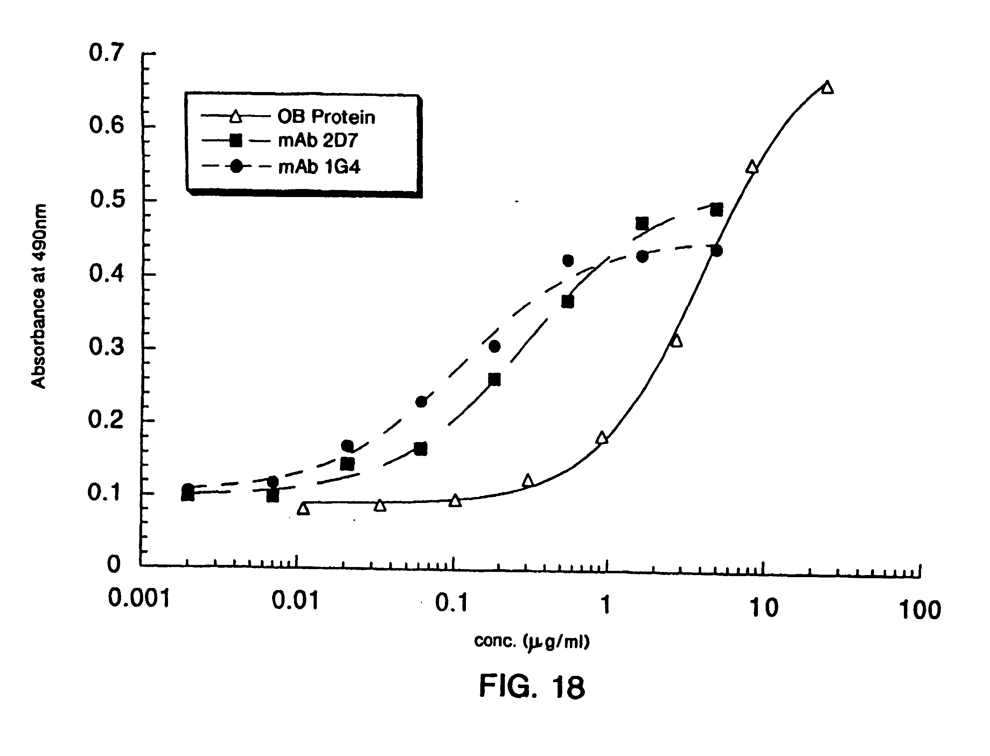

Fig. 18 shows the activity of mAbs 2D7 and 1G4 as well as OB protein in the KIRA ELISA (see Example 13). Absorbance at 490nm versus concentration of antibody or ligand in this assay is shown.

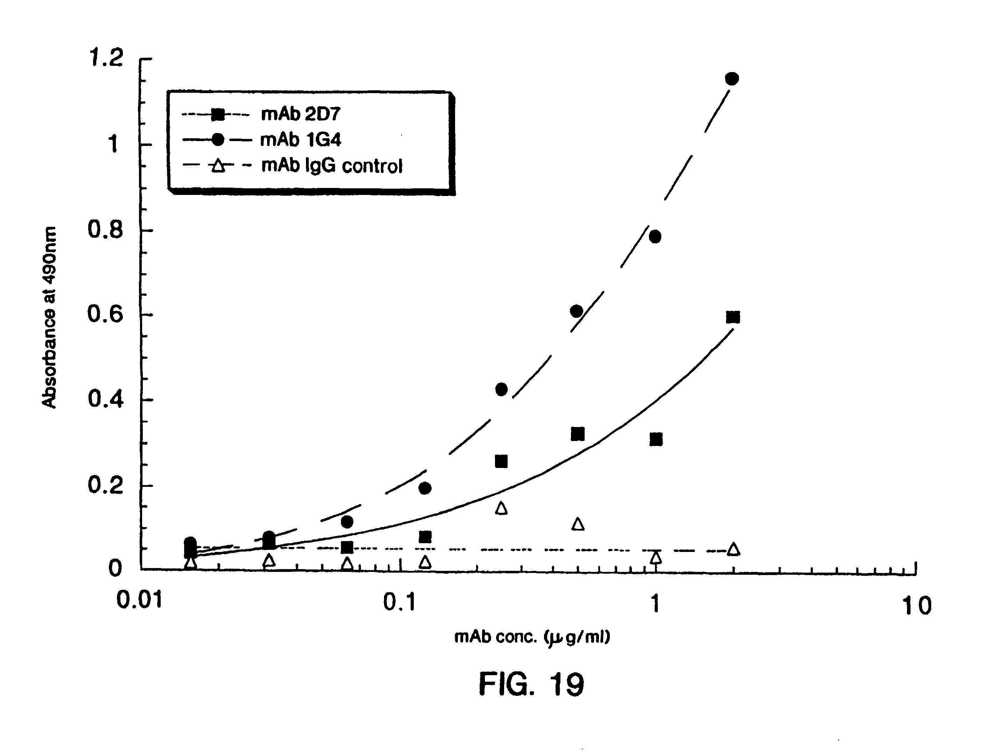

Fig. 19 depicts binding of anti-WSX receptor agonist antibodies to murine WSX receptor. The anti-WSX receptor agonist antibodies (2D7 and 1G4) and an IgG isotope control were evaluated for their ability to bind to murine WSX receptor by capture ELISA.

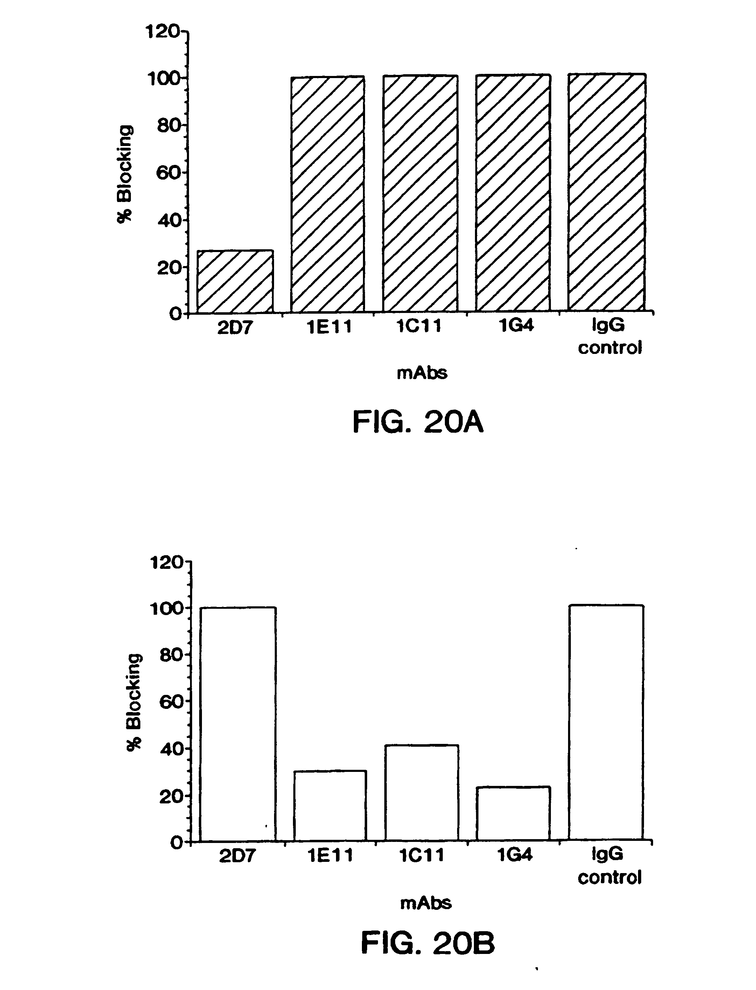

Figs. 20A-B show the results of epitope mapping of the agonist anti-WSX receptor antibodies produced as described in Example 13. Fig. 20A shows blocking ability of anti-WSX receptor antibodies on Epitope A using biotinylated 2D7. Fig. 20B shows blocking ability of anti-WSX receptor antibodies on Epitope B using biotinylated 1C11. Based on the competitive binding ELISA, 2D7 bound a different epitope from 1E11, 1C11 and 1G4.
Fig. 21 depicts an alignment of the amino acid sequences of full length human WSX receptor variant 6.4 (hWSXR) (SEQ ID NO:3) and murine WSX receptor (mWSXR) (SEQ ID NO:51).
Fig. 22 is a standard curve for human OB protein in the KIRA ELISA, which illustrates schematically inside the graph WSX receptor KIRA ELISA panning with scFv phage as described in Example 14.
Fig. 23 shows the activity of clone # 3, #4 and # 17 scFv phage from Example 14 and anti-HER2 scFv phage control in the KIRA ELISA. Absorbance versus phage titer is shown.
Fig. 24 shows the activity of clone # 3, #4 and # 17 scFv from Example 14, anti-HER2 scFv control (Her2 clone) and OB protein in the KIRA ELISA. Absorbance versus antibody concentration is shown.
Fig. 25 aligns the amino acid sequences of agonist antibody clone #3 (3.scFv) (SEQ ID NO:48), clone #4 (4.scFv) (SEQ ID NO:49) and clone #17 (17.scFv) (SEQ ID NO:50) obtained as described in Example 14. Complementarity determining region (CDR) residues according to Kabat *et al., Sequences of Proteins of Immunological Interest*. 5th Ed. Public Health Service, National Institutes of Health, Bethesda, MD. (1991) are underlined and hypervariable loop residues (Chothia *et al., Nature* 342:8767 (1989)) are in italics.

### DETAILED DESCRIPTION OF THE PREFERRED EMBODIMENTS

### I. Definitions

In describing the present invention, the following terms will be employed, and are intended to be defined as indicated below.

The terms "WSX receptor" or "WSX receptor polypeptide" when used herein encompass native sequence WSX receptor, WSX receptor variants; WSX extracellular domain; and chimeric WSX receptor (each of which is defined herein). Optionally, the WSX receptor is not associated with native glycosylation. "Native glycosylation" refers to the carbohydrate moieties which are covalently attached to WSX receptor when it is produced in the mammalian cell from which it is derived in nature. Accordingly, human WSX receptor produced in a non-human cell is an example of a WSX receptor which is "not associated with native glycosylation". Sometimes, the WSX receptor is unglycosylated (*e.g*.,as a result of being produced recombinantly in a prokaryote).

"WSX ligand" is a molecule which binds to and activates native sequence WSX receptor (especially WSX receptor variant 13.2). The ability of a molecule to bind to WSX receptor can be determined by the ability of a putative WSX ligand to bind to WSX receptor immunoadhesin (see Example 2) coated on an assay plate, for example. The thymidine incorporation assay provides a means for screening for WSX ligands which activate the WSX receptor. Exemplary WSX ligands include anti-WSX receptor agonist antibodies and OB protein (*e.g*., described in Zhang *et al. Nature* 372:425-431 (1994)).

The terms "OB protein" and "OB" are used interchangeably herein and refer to native sequence OB proteins (also known as "leptins") and their functional derivatives.

A "native sequence" polypeptide is one which has the same amino acid sequence as a polypeptide (*e.g*., WSX receptor or OB protein) derived from nature. Such native sequence polypeptides can be isolated from nature or can be produced by recombinant or synthetic means. Thus, a native sequence polypeptide can have the amino acid sequence of naturally occurring human polypeptide, murine polypeptide, or polypeptide from any other mammalian species.

The term "native sequence WSX receptor" specifically encompasses naturally-occurring truncated forms of the WSX receptor, naturally-occurring variant forms (*e*.*g*., alternatively spliced forms such as human WSX receptor variants 6.4, 12.1 and 13.2 described herein) and naturally-occurring allelic variants of the WSX receptor. The preferred native sequence WSX receptor is a mature native sequence human WSX receptor, such as human WSX receptor variant 6.4, human WSX receptor variant 12.1 or human WSX receptor variant 13.2 (each shown in Figs. 2A-B). Most preferred is mature human WSX receptor variant 13.2.

The term "native sequence OB protein" includes those OB proteins from any animal species (*e.g*. human, murine, rabbit, cat, cow, sheep, chicken, porcine, equine, etc.) as occurring in nature. The definition specifically includes variants with or without a glutamine at amino acid position 49, using the amino acid numbering of Zhang *et al., supra*. The term "native sequence OB protein" includes the native proteins with or without the initiating N-terminal methionine (Met), and with or without the native signal sequence, either in monomeric or in dimeric form. The native sequence human and murine OB proteins known in the art are 167 amino acids long, contain two conserved cysteines, and have the features of a secreted protein. The protein is largely hydrophilic, and the predicted signal sequence cleavage site is at position 21, using the amino acid numbering of Zhang *et al., supra*. The overall sequence homology of the human and murine sequences is about 84%. The two proteins show a more extensive identity in the N-terminal region of the mature protein, with only four conservative and three non-conservative substitutions among the residues between the signal sequence cleavage site and the conserved Cys at position 117. The molecular weight of OB protein is about 16 kD in a monomeric form.

The "WSX receptor extracellular domain" (ECD) is a form of the WSX receptor which is essentially free of the transmembrane and cytoplasmic domains of WSX receptor, *i*.*e*., has less than 1% of such domains, preferably 0.5 to 0% of such domains, and more preferably 0.1 to 0% of such domains. Ordinarily, the WSX receptor ECD will have an amino acid sequence having at least about 95% amino acid sequence identity with the amino acid sequence of the ECD of WSX receptor indicated in Figs. 2A-B for human WSX receptor variants 6.4, 12.1 and 13.2, preferably at least about 98%, more preferably at least about 99% amino acid sequence identity, and thus includes WSX receptor variants as defined below.

A "variant" polypeptide means a biologically active polypeptide as defined below having less than 100% sequence identity with a native sequence polypeptide (*e*.*g*., WSX receptor having the deduced amino acid sequence shown in Figs. 1A-H for human WSX receptor variant 13.2). Such variants include polypeptides wherein one or more amino acid residues are added at the N- or C-terminus of, or within, the native sequence; from about one to thirty amino acid residues are deleted, and optionally substituted by one or more amino acid residues; and derivatives of the above polypeptides, wherein an amino acid residue has been covalently modified so that the resulting product has a non-naturally occurring amino acid. Ordinarily, a biologically active WSX receptor variant will have an amino acid sequence having at least about 90% amino acid sequence identity with human WSX receptor variant 13.2 shown in Figs. 1A-H, preferably at least about 95%, more preferably at least about 99%. Ordinarily, a biologically active OB protein variant will have an amino acid sequence having at least about 90% amino acid sequence identity with a native sequence OB protein, preferably at least about 95%, more preferably at least about 99%.

A "chimeric" OB protein or WSX receptor is a polypeptide comprising OB protein or full-length WSX receptor or one or more domains thereof (*e*.*g*.,the extracellular domain of the WSX receptor) fused or bonded to heterologous polypeptide. The chimeric WSX receptor will generally share at least one biological property in common with human WSX receptor variant 13.2. The chimeric OB protein will generally share at least one biological property in common with a native sequence OB protein. Examples of chimeric polypeptides include immunoadhesins and epitope tagged polyeptides.

The term "WSX immunoadhesin" is used interchangeably with the expression "WSX receptor-immunoglobulin chimera" and refers to a chimeric molecule that combines a portion of the WSX receptor (generally the extracellular domain thereof) with an immunoglobulin sequence. Likewise, an "OB protein immunoadhesin" or "OB-immunoglobulin chimera" refers to a chimeric molecule which combines OB protein (or a portion thereof) with an immunoglobulin sequence. The immunoglobulin sequence preferably, but not necessarily, is an immunoglobulin constant domain. The immunoglobulin moiety in the chimeras of the present invention may be obtained from IgG1, IgG2, IgG3 or IgG4 subtypes, IgA, IgE, IgD or IgM, but preferably IgGI or IgG3.

The term "epitope tagged" when used herein refers to a chimeric polypeptide comprising WSX receptor or OB protein fused to a "tag polypeptide". The tag polypeptide has enough residues to provide an epitope against which an antibody thereagainst can be made, yet is short enough such that it does not interfere with biological activity of the WSX receptor or OB protein. The tag polypeptide preferably also is fairly unique so that the antibody thereagainst does not substantially cross-react with other epitopes. Suitable tag polypeptides generally have at least six amino acid residues and usually between about 8-50 amino acid residues (preferably between about 9-30 residues).

"Isolated" WSX receptor (or OB protein) means WSX receptor (or OB protein) that has been purified from a WSX receptor (or OB protein) source or has been prepared by recombinant or synthetic methods and is sufficiently free of other peptides or proteins (1) to obtain at least 15 and preferably 20 amino acid residues of the N-terminal or of an internal amino acid sequence by using a spinning cup sequenator or the best commercially available amino acid sequenator marketed or as modified by published methods as of the filing date of this application, or (2) to homogeneity by SDS-PAGE under non-reducing or reducing conditions using Coomassie blue or, preferably, silver stain. Homogeneity here means less than about 5% contamination with other source proteins.

"Essentially pure" protein means a composition comprising at least about 90% by weight of the protein, based on total weight of the composition, preferably at least about 95% by weight. "Essentially homogeneous" protein means a composition comprising at least about 99% by weight of protein, based on total weight of the composition.

"Biological property" when used in conjunction with either "WSX receptor" or "isolated WSX receptor" means having an effector or antigenic function or activity that is directly or indirectly caused or performed by native sequence WSX receptor (whether in its native or denatured conformation). Effector functions include ligand binding; and enhancement of survival, differentiation and/or proliferation of cells (especially proliferation of cells). However, effector functions do not include possession of an epitope or antigenic site that is capable of cross-reacting with antibodies raised against native sequence WSX receptor.

"Biological property" when used in conjunction with either "OB protein" or "isolated OB protein" means having an effector function that is directly or indirectly caused or performed by native sequence OB protein. Effector functions of native sequence OB protein include WSX receptor binding and activation; and enhancement of differentiation and/or proliferation of cells expressing this receptor (as determined in the thymidine incorporation assay, for example). A "biologically active" OB protein is one which possesses a biological property of native sequence OB protein.

A "functional derivative" of a native sequence OB protein is a compound having a qualitative biological property in common with a native sequence OB protein. "Functional derivatives" include, but are not limited to, fragments of native sequence OB proteins and derivatives of native sequence OB proteins and their fragments, provided that they have a biological activity in common with a corresponding native sequence OB protein. The term "derivative" encompasses both amino acid sequence variants of OB protein and covalent modifications thereof.

The phrase "long half-life" as used in connection with OB derivatives, concerns OB derivatives having a longer plasma half-life and/or slower clearance than a corresponding native sequence OB protein. The long half-life derivatives preferably will have a half-life at least about 1.5-times longer than a native OB protein; more preferably at least about 2-times longer than a native OB protein, more preferably at least about 3-time longer than a native OB protein. The native OB protein preferably is that of the individual to be treated.

An "antigenic function" means possession of an epitope or antigenic site that is capable of cross-reacting with antibodies raised against native sequence WSX receptor. The principal antigenic function of a WSX receptor is that it binds with an affinity of at least about 10⁶ L/mole to an antibody raised against native sequence WSX receptor. Ordinarily, the polypeptide binds with an affinity of at least about 10⁷ L/mole. The antibodies used to define "antigenic function" are rabbit polyclonal antibodies raised by formulating the WSX receptor in Freund's complete adjuvant, subcutaneously injecting the formulation, and boosting the immune response by intraperitoneal injection of the formulation until the titer of the anti-WSX receptor or antibody plateaus.

"Biologically active" when used in conjunction with either "WSX receptor" or "isolated WSX receptor" means a WSX receptor polypeptide that exhibits or shares an effector function of native sequence WSX receptor and that may (but need not) in addition possess an antigenic function. A principal effector function of the WSX receptor is its ability to induce proliferation of CD34+ human umbilical cord blood cells in the colony assay described in Example 8.

"Antigenically active" WSX receptor is defined as a polypeptide that possesses an antigenic function of WSX receptor and that may (but need not) in addition possess an effector function.

"Percent amino acid sequence identity" is defined herein as the percentage of amino acid residues in the candidate sequence that are identical with the residues in the native sequence, after aligning the sequences and introducing gaps, if necessary, to achieve the maximum percent sequence identity, and not considering any conservative substitutions as part of the sequence identity. None of N-terminal, C-terminal, or internal extensions, deletions, or insertions into the candidate sequence shall be construed as affecting sequence identity or homology.

A "thymidine incorporation assay" can be used to screen for molecules which activate the WSX receptor. In order to perform this assay, IL-3 dependent Baf3 cells (Palacios *et al., Cell*, 41:727-734 (1985)) are stably transfected with full length native sequence WSX receptor as described in Example 4. The WSX receptor/Baf3 cells so generated are starved of IL-3 for, *e.g*., 24 hours in a humidified incubator at 37°C in 5%CO₂ and air. Following IL-3 starvation, the cells are plated out in 96 well culture dishes with, or without, a test sample containing a potential agonist (such test samples are optionally diluted) and cultured for 24 hours in a cell culture incubator. 20µl of serum free RPMI media containing 1µCi of ³H thymidine is added to each well for the last 6-8 hours. The cells are then harvested in 96 well filter plates and washed with water. The filters are then counted using a Packard Top Count Microplate Scintillation Counter, for example. Agonists are expected to induce a statistically significant increase (to a P value of 0.05) in ³H uptake, relative to control. Preferred agonists leads to an increase in ³H uptake which is at least two fold of that of the control.

An "isolated" WSX receptor nucleic acid molecule is a nucleic acid molecule that is identified and separated from at least one contaminant nucleic acid molecule with which it is ordinarily associated in the natural source of the WSX receptor nucleic acid. An isolated WSX receptor nucleic acid molecule is other than in the form or setting in which it is found in nature. Isolated WSX receptor nucleic acid molecules therefore are distinguished from the WSX receptor nucleic acid molecule as it exists in natural cells. However, an isolated WSX receptor nucleic acid molecule includes WSX receptor nucleic acid molecules contained in cells that ordinarily express WSX receptor where, for example, the nucleic acid molecule is in a chromosomal location different from that of natural cells.

The expression "control sequences" refers to DNA sequences necessary for the expression of an operably linked coding sequence in a particular host organism. The control sequences that are suitable for prokaryotes, for example, include a promoter, optionally an operator sequence, a ribosome binding site, and possibly, other as yet poorly understood sequences. Eukaryotic cells are known to utilize promoters, polyadenylation signals, and enhancers.

Nucleic acid is "operably linked" when it is placed into a functional relationship with another nucleic acid sequence. For example, DNA for a presequence or secretory leader is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous, and, in the case of a secretory leader, contiguous and in reading phase. However, enhancers do not have to be contiguous. Linking is accomplished by ligation at convenient restriction sites. If such sites do not exist, the synthetic oligonucleotide adaptors or linkers are used in accordance with conventional practice.

As used herein, the expressions "cell," "cell line," and "cell culture" are used interchangeably and all such designations include progeny. Thus, the words "transformants" and "transformed cells" include the primary subject cell and cultures derived therefrom without regard for the number of transfers. It is also understood that all progeny may not be precisely identical in DNA content, due to deliberate or inadvertent mutations. Mutant progeny that have the same function or biological activity as screened for in the originally transformed cell are included. Where distinct designations are intended, it will be clear from the context.

The term "antibody" is used in the broadest sense and specifically covers monoclonal antibodies, antibody compositions with polyepitopic specificity, bispecific antibodies, diabodies, and single-chain molecules, as well as antibody fragments (*e.g*., Fab, F(ab')₂, and Fv), so long as they exhibit the desired biological activity.

The term "monoclonal antibody" as used herein refers to an antibody obtained from a population of substantially homogeneous antibodies, *i.e*., the individual antibodies comprising the population are identical except for possible naturally occurring mutations that may be present in minor amounts. Monoclonal antibodies are highly specific, being directed against a single antigenic site. Furthermore, in contrast to conventional (polyclonal) antibody preparations which typically include different antibodies directed against different determinants (epitopes), each monoclonal antibody is directed against a single determinant on the antigen. In addition to their specificity, the monoclonal antibodies are advantageous in that they are synthesized by the hybridoma culture, uncontaminated by other immunoglobulins. The modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by the hybridoma method first described by Kohler *et al., Nature* 256:495 (1975), or may be made by recombinant DNA methods (see, *e.g.,* U.S. Patent No. 4,816,567 (Cabilly *et al*.)). The "monoclonal antibodies" may also be isolated from phage antibody libraries using the techniques described in Clackson *et al., Nature* 352:624-628 (1991) and Marks *et al., J. Mol. Biol.* 222:581-597 (1991), for example.

The monoclonal antibodies herein specifically include "chimeric" antibodies (immunoglobulins) in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (Cabilly *et al., supra*; Morrison *et al., Proc. Natl. Acad Sci. USA*, 81:6851-6855 (1984)).

"Humanized" forms of non-human (*e.g.,* murine) antibodies are chimeric immunoglobulins, immunoglobulin chains or fragments thereof (such as Fv, Fab, Fab', F(ab')₂ or other antigen-binding subsequences of antibodies) which contain minimal sequence derived from non-human immunoglobulin. For the most part, humanized antibodies are human immunoglobulins (recipient antibody) in which residues from a complementary-determining region (CDR) of the recipient are replaced by residues from a CDR of a non-human species (donor antibody) such as mouse, rat or rabbit having the desired specificity, affinity, and capacity. In some instances, Fv framework region (FR) residues of the human immunoglobulin are replaced by corresponding non-human residues. Furthermore, humanized antibodies may comprise residues which are found neither in the recipient antibody nor in the imported CDR or framework sequences. These modifications are made to further refine and optimize antibody performance. In general, the humanized antibody will comprise substantially all of at least one, and typically two, variable domains, in which all or substantially all of the CDR regions correspond to those of a non-human immunoglobulin and all or substantially all of the FR regions are those of a human immunoglobulin sequence. The humanized antibody optimally also will comprise at least a portion of an immunoglobulin constant region (Fc), typically that of a human immunoglobulin. For further details, see Jones *et al., Nature* 321:522-525 (1986); Reichmann *et al., Nature* 332:323-329 (1988); and Presta, *Curr. Op. Struct. Biol*. 2:593-596 (1992). The humanized antibody includes a Primatized™ antibody wherein the antigen-binding region of the antibody is derived from an antibody produced by immunizing macaque monkeys with the antigen of interest.

The term "hypervariable region" when used herein refers to the amino acid residues of an antibody which are responsible for antigen-binding. The hypervariable region comprises amino acid residues from a "complementarity determining region" or "CDR" (*i*.*e*. residues 24-34 (L1), 50-56 (L2) and 89-97 (L3) in the light chain variable domain and 31-35 (H1), 50-65 (H2) and 95-102 (H3) in the heavy chain variable domain; Kabat *et al., Sequences ofProteins of Immunological Interest*, 5th Ed. Public Health Service, National Institutes of Health, Bethesda, MD. (1991)) and/or those residues from a "hypervariable loop" (*i*.*e*. residues 26-32 (L1), 50-52 (L2) and 91-96 (L3) in the light chain variable domain and 26-32 (H1), 53-55 (H2) and 96-101 (H3) in the heavy chain variable domain; Chothia and Lesk *J. Mol. Biol.* 196:901-917 (1987)). "Framework" or "FR" residues are those variable domain residues other than the hypervariable region residues as herein defined.

"Non-immunogenic in a human" means that upon contacting the polypeptide of interest in a physiologically acceptable carrier and in a therapeutically effective amount with the appropriate tissue of a human, no state of sensitivity or resistance to the polypeptide of interest is demonstrable upon the second administration of the polypeptide of interest after an appropriate latent period (*e.g*., 8 to 14 days).

By "agonist antibody" is meant an antibody which is able to activate native sequence WSX receptor. The agonist antibody of particular interest herein is one which mimics one or more (*e.g*. all) of the biological properties of naturally occurring WSX ligand, OB protein. In preferred embodiments, the agonist antibody has a quantitative biological property of OB protein which is within about two orders of magnitude, and preferably within about one order of magnitude, that of OB protein. The agonist antibody may bind to and activate WSX receptor and thereby stimulate proliferation and/or differentiation and/or maturation and/or survival of a cell which expresses the WSX receptor (*e.g*. WSX receptor variant 13.2). In this embodiment of the invention, the agonist antibody may be one which enhances proliferation and/or differentiation of a hematopoietic progenitor cell which expresses the WSX receptor at its cell surface; enhances proliferation and/or differentiation of lymphoid blood cell lineages; enhances proliferation and/or differentiation of myeloid blood cell lineages; and/or enhances proliferation and/or differentiation of erythroid blood cell lineages. The agonist antibody may display agonist activity upon binding to a chimeric receptor comprising the WSX receptor extracellular domain in the KIRA ELISA. The agonist antibody may stimulate ³H uptake in the thymidine incorporation assay using a signaling WSX receptor (see above); decrease body weight and/or fat-depot weight and/or food intake in an obese mammal (*e.g*. in the *ob*/*ob* mouse); effect Ca²⁺ influx in adipocytes; and/or activate downstream signaling molecules of OB protein.

A "neutralizing antibody" is one which is able to block or significantly reduce an effector function of native sequence WSX receptor or OB protein. For example, a neutralizing antibody may inhibit or reduce WSX receptor activation by a WSX ligand as determined in the thymidine incorporation assay or in a KIRA ELISA.

The term "cytotoxic agent" as used herein refers to a substance that inhibits or prevents the function of cells and/or causes destruction of cells. The term is intended to include radioactive isotopes (*e.g*., I¹³¹, I¹²⁵, Y⁹⁰ and Re¹⁸⁶), chemotherapeutic agents, and toxins such as enzymatically active toxins of bacterial, fungal, plant or animal origin, or fragments thereof.

A "chemotherapeutic agent" is a chemical compound useful in the treatment of cancer. Examples of chemotherapeutic agents include Adriamycin, Doxorubicin, 5-Fluorouracil, Cytosine arabinoside ("Ara-C"), Cyclophosphamide, Thiotepa, Taxotere (docetaxel), Busulfan, Cytoxin, Taxol, Methotrexate, Cisplatin, Melphalan, Vinblastine, Bleomycin, Etoposide, Ifosfamide, Mitomycin C, Mitoxantrone, Vincreistine, Vinorelbine, Carboplatin, Teniposide, Daunomycin, Carminomycin, Aminopterin, Dactinomycin, Mitomycins, Esperamicins (see U.S. Pat. No. 4,675,187), Melphalan and other related nitrogen mustards.

The term "prodrug" as used in this application refers to a precursor or derivative form of a pharmaceutically active substance that is less cytotoxic to tumor cells compared to the parent drug and is capable of being enzymatically activated or converted into the more active parent form. See, *e*.*g*., Wilman, "Prodrugs in Cancer Chemotherapy" *Biochemical Society Transactions*, 14, pp. 375-382, 615th Meeting Belfast (1986) and Stella *et al*., "Prodrugs: A Chemical Approach to Targeted Drug Delivery," *Directed Drug Delivery*, Borchardt *el al*., (ed.), pp. 247-267, Humana Press (1985). The prodrugs of this invention include, but are not limited to, phosphate-containing prodrugs, thiophosphate-containing prodrugs, sulfate-containing prodrugs, peptide-containing prodrugs, D-amino acid-modified prodrugs, glycosylated prodrugs, β-lactam-containing prodrugs, optionally substituted phenoxyacetamide-containing prodrugs or optionally substituted phenylacetamide-containing prodrugs, 5-fluorocytosine and other 5-fluorouridine prodrugs which can be converted into the more active cytotoxic free drug. Examples of cytotoxic drugs that can be derivatized into a prodrug form for use in this invention include, but are not limited to, those chemotherapeutic agents described above.

An "antagonist" of the WSX receptor and/or OB protein is a molecule which prevents, or interferes with, binding and/or activation of the WSX receptor or OB protein. Such molecules can be screened for their ability to competitively inhibit WSX receptor activation by OB protein in the thymidine incorporation assay disclosed herein, for example. Examples of such molecules include: WSX receptor ECD; WSX receptor immunoadhesin; neutralizing antibodies against WSX receptor or OB protein; small molecule and peptide antagonists; and antisense nucleotides against the WSX receptor or *ob* gene.

The phrase "enhancing proliferation of a cell" encompasses the step of increasing the extent of growth and/or reproduction of the cell relative to an untreated cell either *in vitro* or *in vivo.* An increase in cell proliferation in cell culture can be detected by counting the number of cells before and after exposure to a molecule of interest. The extent of proliferation can be quantified via microscopic examination of the degree of confluency. Cell proliferation can also be quantified using the thymidine incorporation assay described herein.

By "enhancing differentiation of a cell" is meant the act of increasing the extent of the acquisition or possession of one or more characteristics or functions which differ from that of the original cell (*i*.*e*. cell specialization). This can be detected by screening for a change in the phenotype of the cell (*e*.*g*.,identifying morphological changes in the cell).

A "hematopoietic progenitor cell" or "primitive hematopoietic cell" is one which is able to differentiate to form a more committed or mature blood cell type.

"Lymphoid blood cell lineages" are those hematopoietic precursor cells which are able to differentiate to form lymphocytes (B-cells or T-cells). Likewise, "lymphopoeisis" is the formation of lymphocytes.

"Erythroid blood cell lineages" are those hematopoietic precursor cells which are able to differentiate to form erythrocytes (red blood cells) and "erythropoeisis" is the formation of erythrocytes.

The phrase "myeloid blood cell lineages", for the purposes herein, encompasses all hematopoietic precursor cells, other than lymphoid and erythroid blood cell lineages as defined above, and "myelopoiesis" involves the formation of blood cells (other than lymphocytes and erythrocytes).

A "CD34+ cell population" is enriched for hematopoietic stem cells. A CD34+ cell population can be obtained from umbilical cord blood or bone marrow, for example. Human umbilical cord blood CD34+ cells can be selected for using immunomagnetic beads sold by Miltenyi (California), following the manufacturer's directions.

"Physiologically acceptable" carriers, excipients, or stabilizers are ones which are nontoxic to the cell or mammal being exposed thereto at the dosages and concentrations employed. Often the physiologically acceptable carrier is an aqueous pH buffered solution. Examples of physiologically acceptable carriers include buffers such as phosphate, citrate, and other organic acids; antioxidants including ascorbic acid; low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, arginine or lysine; monosaccharides, disaccharides, and other carbohydrates including glucose, mannose, or dextrins; chelating agents such as EDTA; sugar alcohols such as mannitol or sorbitol; salt-forming counterions such as sodium; and/or nonionic surfactants such as Tween, Pluronics or polyethylene glycol (PEG).

As used herein, the term "salvage receptor binding epitope" refers to an epitope of the Fc region of an IgG molecule (*e.g*., IgG1, IgG2, IgG3, and IgG4) that is responsible for increasing the *in vivo* serum half-life of the IgG molecule. Exemplary salvage receptor binding epitope sequences include HQNLSDGK (SEQ ID NO:39); HQNISDGK (SEQ ID NO:40); HQSLGTQ (SEQ ID NO:41); VISSHLGQ (SEQ ID NO:42); and PKNSSMISNTP (SEQ ID NO:43).

The term "cytokine" is a generic term for proteins released by one cell population which act on another cell as intercellular mediators. Examples of such cytokines are lymphokines, monokines, and traditional polypeptide hormones. Included among the cytokines are OB protein; growth hormones such as human growth hormone, N-methionyl human growth hormone, and bovine growth hormone; parathyroid hormone; thyroxine; insulin; proinsulin; relaxin; prorelaxin; glycoprotein hormones such as follicle stimulating hormone (FSH), thyroid stimulating hormone (TSH), and luteinizing hormone (LH); hepatic growth factor; fibroblast growth factor; prolactin; placental lactogen; tumor necrosis factor-α and -β; mullerian-inhibiting substance; mouse gonadotropin-associated peptide; inhibin; activin; vascular endothelial growth factor; integrin; thrombopoietin (TPO); nerve growth factors such as NGF-β; platelet-growth factor; transforming growth factors (TGFs) such as TGF-α and TGF-β; insulin-like growth factor-I and -II; erythropoietin (EPO); osteoinductive factors; interferons such as interferon-α, -β, and -γ; colony stimulating factors (CSFs) such as macrophage-CSF (M-CSF); granulocyte-macrophage-CSF (GM-CSF); and granulocyte-CSF (G-CSF); interleukins (ILs) such as IL-1, IL-1α, IL-2, IL-3, IL-4, IL-5, IL-6, IL-7, IL-8, IL-9, IL-11, IL-12; and other polypeptide factors including leukemia inhibitory factor (LIF) and kit ligand (KL). As used herein, the term cytokine includes proteins from natural sources or from recombinant cell culture and biologically active equivalents of the native sequence cytokines.

A "lineage-specific cytokine" is one which acts on relatively committed cells in the hematopoietic cascade and gives rise to an expansion in blood cells of a single lineage. Examples of such cytokines include EPO, TPO, and G-CSF.

"Treatment" refers to both therapeutic treatment and prophylactic or preventative measures. Those in need of treatment include those already with the disorder as well as those in which the disorder is to be prevented.

The term "obesity" is used to designate a condition of being overweight associated with excessive bodily fat. The desirable weight for a certain individual depends on a number of factors including sex, height, age, overall built, etc. The same factors will determine when an individual is considered obese. The determination of an optimum body weight for a given individual is well within the skill of an ordinary physician.

"Mammal" for purposes of treatment refers to any animal classified as a mammal, including humans, domestic and farm animals, and zoo, sports, or pet animals, such as dogs, horses, cats, cows, *etc*. Preferably, the mammal is human.

By "solid phase" is meant a non-aqueous matrix to which a reagent of interest (*e.g*.,the WSX receptor or an antibody thereto) can adhere. Examples of solid phases encompassed herein include those formed partially or entirely of glass (*e.g*.,controlled pore glass), polysaccharides (*e.g*.,agarose), polyacrylamides, polystyrene, polyvinyl alcohol and silicones. In certain embodiments, depending on the context, the solid phase can comprise the well of an assay plate; in others it is a purification column (*e.g*.,an affinity chromatography column). This term also includes a discontinuous solid phase of discrete particles, such as those described in U.S. Patent No. 4,275,149.

### II. Modes for Carrying Out the Invention

The present invention is based on the discovery of the WSX receptor. The experiments described herein demonstrate that this molecule is a cytokine receptor which appears to play a role in enhancing proliferation and/or differentiation of hematopoietic cells. In particular, this receptor has been found to be present in enriched human stem cell populations, thus indicating that WSX ligands, such as agonist antibodies, may be used to stimulate proliferation of hematopoietic stem cells/progenitor cells. Other uses for this receptor will be apparent from the following discussion. A description follows as to how WSX receptor or OB proteins may be prepared.

### a. Preparation of WSX Receptor or OB Protein

Techniques suitable for the production of WSX receptor or OB protein are well known in the art and include isolating WSX receptor or OB protein from an endogenous source of the polypeptide, peptide synthesis (using a peptide synthesizer) and recombinant techniques (or any combination of these techniques). The preferred technique for production of WSX receptor or OB protein is a recombinant technique to be described below.

Most of the discussion below pertains to recombinant production of WSX receptor or OB protein by culturing cells transformed with a vector containing WSX receptor or OB protein nucleic acid and recovering the polypeptide from the cell culture. It is further envisioned that the WSX receptor or OB protein of this invention may be produced by homologous recombination, as provided for in WO 91/06667, published 16 May 1991.

Briefly, this method involves transforming primary human cells containing a WSX receptor or OB protein-encoding gene with a construct (*i.e*., vector) comprising an amplifiable gene (such as dihydrofolate reductase (DHFR) or others discussed below) and at least one flanking region of a length of at least about 150 bp that is homologous with a DNA sequence at the locus of the coding region of the WSX receptor or OB protein gene to provide amplification of the WSX receptor or OB protein gene. The amplifiable gene must be at a site that does not interfere with expression of the WSX receptor or OB protein gene. The transformation is conducted such that the construct becomes homologously integrated into the genome of the primary cells to define an amplifiable region.

Primary cells comprising the construct are then selected for by means of the amplifiable gene or other marker present in the construct. The presence of the marker gene establishes the presence and integration of the construct into the host genome. No further selection of the primary cells need be made, since selection will be made in the second host. If desired, the occurrence of the homologous recombination event can be determined by employing PCR and either sequencing the resulting amplified DNA sequences or determining the appropriate length of the PCR fragment when DNA from correct homologous integrants is present and expanding only those cells containing such fragments. Also if desired, the selected cells may be amplified at this point by stressing the cells with the appropriate amplifying agent (such as methotrexate if the amplifiable gene is DHFR), so that multiple copies of the target gene are obtained. Preferably, however, the amplification step is not conducted until after the second transformation described below.

After the selection step, DNA portions of the genome, sufficiently large to include the entire amplifiable region, are isolated from the selected primary cells. Secondary mammalian expression host cells are then transformed with these genomic DNA portions and cloned, and clones are selected that contain the amplifiable region. The amplifiable region is then amplified by means of an amplifying agent if not already amplified in the primary cells. Finally, the secondary expression host cells now comprising multiple copies of the amplifiable region containing WSX receptor or OB protein are grown so as to express the gene and produce the protein.

### i. Isolation of DNA Encoding WSX Receptor or OB Protein

The DNA encoding WSX receptor or OB protein may be obtained from any cDNA library prepared from tissue believed to possess the WSX receptor or OB protein mRNA and to express it at a detectable level. Accordingly, WSX receptor or OB protein DNA can be conveniently obtained from a cDNA library prepared from mammalian fetal liver. The WSX receptor or OB protein-encoding gene may also be obtained from a genomic library or by oligonucleotide synthesis.

Libraries are screened with probes (such as antibodies to the WSX receptor or OB protein, or oligonucleotides of about 20-80 bases) designed to identify the gene of interest or the protein encoded by it. Screening the cDNA or genomic library with the selected probe may be conducted using standard procedures as described in chapters 10-12 of Sambrook *et al., Molecular Cloning: A Laboratory Manual* (New York: Cold Spring Harbor Laboratory Press, 1989). An alternative means to isolate the gene encoding WSX receptor or OB protein is to use PCR methodology as described in section 14 of Sambrook *et al., supra*.

A preferred method of practicing this invention is to use carefully selected oligonucleotide sequences to screen cDNA libraries from various human tissues, preferably human fetal liver. The oligonucleotide sequences selected as probes should be of sufficient length and sufficiently unambiguous that false positives are minimized.

The oligonucleotide must be labeled such that it can be detected upon hybridization to DNA in the library being screened. The preferred method of labeling is to use ³²P-labeled ATP with polynucleotide kinase, as is well known in the art, to radiolabel the oligonucleotide. However, other methods may be used to label the oligonucleotide, including, but not limited to, biotinylation or enzyme labeling.

Amino acid sequence variants of WSX receptor or OB protein are prepared by introducing appropriate nucleotide changes into the WSX receptor or OB protein DNA, or by synthesis of the desired WSX receptor or OB protein. Such variants represent insertions, substitutions, and/or specified deletions of, residues within or at one or both of the ends of the amino acid sequence of a naturally occurring human WSX receptor or OB protein, such as the WSX receptor variants shown in Figs 2A-B or the human OB protein of Zhang *et al., supra*. Preferably, these variants represent insertions and/or substitutions within or at one or both ends of the mature sequence, and/or insertions, substitutions and/or specificed deletions within or at one or both of the ends of the signal sequence of the WSX receptor or OB protein. Any combination of insertion, substitution, and/or specified deletion is made to arrive at the final construct, provided that the final construct possesses the desired biological activity as defined herein. The amino acid changes also may alter post-translational processes of the WSX receptor or OB protein, such as changing the number or position of glycosylation sites, altering the membrane anchoring characteristics, and/or altering the intracellular location of the WSX receptor or OB protein by inserting, deleting, or otherwise affecting the leader sequence of the WSX receptor or OB protein.

Variations in the native sequence as described above can be made using any of the techniques and guidelines for conservative and non-conservative mutations set forth in U.S. Pat. No. 5,364,934. These include oligonucleotide-mediated (site-directed) mutagenesis, alanine scanning, and PCR mutagenesis. See also, for example, Table I therein and the discussion surrounding this table for guidance on selecting amino acids to change, add, or delete.

### ii. Insertion of Nucleic Acid into Replicable Vector

The nucleic acid (*e.g*., cDNA or genomic DNA) encoding the WSX receptor or OB protein is inserted into a replicable vector for further cloning (amplification of the DNA) or for expression. Many vectors are available. The vector components generally include, but are not limited to, one or more of the following: a signal sequence, an origin of replication, one or more marker genes, an enhancer element, a promoter, and a transcription termination sequence.

### (1) Signal sequence component

The WSX receptor or OB proteins of this invention may be produced recombinantly not only directly, but also as a fusion polypeptide with a heterologous polypeptide, which is preferably a signal sequence or other polypeptide having a specific cleavage site at the N-terminus of the mature protein or polypeptide. In general, the signal sequence may be a component of the vector, or it may be a part of the WSX receptor or OB protein DNA that is inserted into the vector. The heterologous signal sequence selected preferably is one that is recognized and processed (*i.e*., cleaved by a signal peptidase) by the host cell. For prokaryotic host cells that do not recognize and process the native WSX receptor or OB protein signal sequence, the signal sequence is substituted by a prokaryotic signal sequence selected, for example, from the group of the alkaline phosphatase, penicillinase, Ipp, or heat-stable enterotoxin II leaders. For yeast secretion the native signal sequence may be substituted by, *e.g*., the yeast invertase leader, α factor leader (including *Saccharomyces* and *Kluyveromyces α*-factor leaders, the latter described in U.S. Pat. No. 5,010,182 issued 23 April 1991), or acid phosphatase leader, the *C. albicans* glucoamylase leader (EP 362,179 published 4 April 1990), or the signal described in WO 90/13646 published 15 November 1990. In mammalian cell expression the native signal sequence (*e.g*., the WSX receptor or OB protein presequence that normally directs secretion of WSX receptor or OB protein from human cells *in vivo*) is satisfactory, although other mammalian signal sequences may be suitable, such as signal sequences from other animal WSX receptors or OB proteins, and signal sequences from secreted polypeptides of the same or related species, as well as viral secretory leaders, for example, the herpes simplex gD signal.

The DNA for such precursor region is ligated in reading frame to DNA encoding the mature WSX receptor or OB protein.

### (2) Origin of replication component

Both expression and cloning vectors contain a nucleic acid sequence that enables the vector to replicate in one or more selected host cells. Generally, in cloning vectors this sequence is one that enables the vector to replicate independently of the host chromosomal DNA, and includes origins of replication or autonomously replicating sequences. Such sequences are well known for a variety of bacteria, yeast, and viruses. The origin of replication from the plasmid pBR322 is suitable for most Gram-negative bacteria, the 2µ plasmid origin is suitable for yeast, and various viral origins (SV40, polyoma, adenovirus, VSV or BPV) are useful for cloning vectors in mammalian cells. Generally, the origin of replication component is not needed for mammalian expression vectors (the SV40 origin may typically be used only because it contains the early promoter).

Most expression vectors are "shuttle" vectors, *i*.*e*., they are capable of replication in at least one class of organisms but can be transfected into another organism for expression. For example, a vector is cloned in *E. coli* and then the same vector is transfected into yeast or mammalian cells for expression even though it is not capable of replicating independently of the host cell chromosome.

DNA may also be amplified by insertion into the host genome. This is readily accomplished using *Bacillus* species as hosts, for example, by including in the vector a DNA sequence that is complementary to a sequence found in *Bacillus* genomic DNA. Transfection of *Bacillus* with this vector results in homologous recombination with the genome and insertion of WSX receptor or OB protein DNA. However, the recovery of genomic DNA encoding WSX receptor or OB protein is more complex than that of an exogenously replicated vector because restriction enzyme digestion is required to excise the WSX receptor or OB protein DNA.

### (3) Selection gene component

Expression and cloning vectors should contain a selection gene, also termed a selectable marker. This gene encodes a protein necessary for the survival or growth of transformed host cells grown in a selective culture medium. Host cells not transformed with the vector containing the selection gene will not survive in the culture medium. Typical selection genes encode proteins that (a) confer resistance to antibiotics or other toxins, *e.g*., ampicillin, neomycin, methotrexate, or tetracycline, (b) complement auxotrophic deficiencies, or (c) supply critical nutrients not available from complex media, *e.g*., the gene encoding D-alanine racemase for *Bacilli*.

One example of a selection scheme utilizes a drug to arrest growth of a host cell. Those cells that are successful transformed with a heterologous gene produce a protein conferring drug resistance and thus survive the selection regimen. Examples of such dominant selection use the drugs neomycin, mycophenolic acid and hygromycin.

Another example of suitable selectable markers for mammalian cells are those that enable the identification of cells competent to take up the WSX receptor or OB protein nucleic acid, such as DHFR or thymidine kinase. The mammalian cell transformants are placed under selection pressure that only the transformants are uniquely adapted to survive by virtue of having taken up the marker. Selection pressure is imposed by culturing the transformants under conditions in which the concentration of selection agent in the medium is successively changed, thereby leading to amplification of both the selection gene and the DNA that encodes WSX receptor or OB protein. Amplification is the process by which genes in greater demand for the production of a protein critical for growth are reiterated in tandem within the chromosomes of successive generations of recombinant cells. Increased quantities of WSX receptor or OB protein are synthesized from the amplified DNA. Other examples of amplifiable genes include metallothionein-I and -II, preferably primate metallothionein genes, adenosine deaminase, ornithine decarboxylase, *etc.*

For example, cells transformed with the DHFR selection gene are first identified by culturing all of the transformants in a culture medium that contains methotrexate (Mtx), a competitive antagonist of DHFR. An appropriate host cell when wild-type DHFR is employed is the Chinese hamster ovary (CHO) cell line deficient in DHFR activity, prepared and propagated as described by Urlaub *et al., Proc. Natl. Acad Sci. USA* 77:4216 (1980). The transformed cells are then exposed to increased levels of methotrexate. This leads to the synthesis of multiple copies of the DHFR gene, and, concomitantly, multiple copies of other DNA comprising the expression vectors, such as the DNA encoding WSX receptor or OB protein. This amplification technique can be used with any otherwise suitable host, *e.g*., ATCC No. CCL61 CHO-K1, notwithstanding the presence of endogenous DHFR if, for example, a mutant DHFR gene that is highly resistant to Mtx is employed (EP 117,060).

Alternatively, host cells (particularly wild-type hosts that contain endogenous DHFR) transformed or co-transformed with DNA sequences encoding WSX receptor or OB protein, wild-type DHFR protein, and another selectable marker such as aminoglycoside 3'-phosphotransferase (APH) can be selected by cell growth in medium containing a selection agent for the selectable marker such as an aminoglycosidic antibiotic, *e.g*., kanamycin, neomycin, or G418. See U.S. Patent No. 4,965,199.

A suitable selection gene for use in yeast is the *trp*1 gene present in the yeast plasmid YRp7 (Stinchcomb *et al., Nature* 282:39 (1979)). The *trp*1 gene provides a selection marker for a mutant strain of yeast lacking the ability to grow in tryptophan, for example, ATCC No. 44076 or PEP4-1. Jones, *Genetics* 85:12 (1977). The presence of the *trp*1 lesion in the yeast host cell genome then provides an effective environment for detecting transformation by growth in the absence of tryptophan. Similarly, *Leu*2-deficient yeast strains (ATCC 20,622 or 38,626) are complemented by known plasmids bearing the *Leu*2 gene.

In addition, vectors derived from the 1.6 µm circular plasmid pKD1 can be used for transformation of *Kluyveromyces* yeasts. Bianchi *et al., Curr. Genet*. 12:185 (1987). More recently, an expression system for large-scale production of recombinant calf chymosin was reported for *K. lactis*. Van den Berg, *Bio*/*Technology* 8:135 (1990). Stable multi-copy expression vectors for secretion of mature recombinant human serum albumin by industrial strains of *Kluyveromyces* have also been disclosed. Fleer *et al*., *Bio*/*Technology* 9:968-975(1991).

### (4) Promoter Component

Expression and cloning vectors usually contain a promoter that is recognized by the host organism and is operably linked to the WSX receptor or OB protein nucleic acid. Promoters are untranslated sequences located upstream (5') to the start codon of a structural gene (generally within about 100 to 1000 bp) that control the transcription and translation of particular nucleic acid sequence, such as the WSX receptor or OB protein nucleic acid sequence, to which they are operably linked. Such promoters typically fall into two classes, inducible and constitutive. Inducible promoters are promoters that initiate increased levels of transcription from DNA under their control in response to some change in culture conditions, *e.g*., the presence or absence of a nutrient or a change in temperature. At this time a large number of promoters recognized by a variety of potential host cells are well known. These promoters are operably linked to WSX receptor or OB protein-encoding DNA by removing the promoter from the source DNA by restriction enzyme digestion and inserting the isolated promoter sequence into the vector. Both the native WSX receptor or OB protein promoter sequence and many heterologous promoters may be used to direct amplification and/or expression of the WSX receptor or OB protein DNA. However, heterologous promoters are preferred, as they generally permit greater transcription and higher yields of WSX receptor or OB protein as compared to the native WSX receptor or OB protein promoter.

Promoters suitable for use with prokaryotic hosts include the β-lactamase and lactose promoter systems (Chang *et al., Nature* 275:615 (1978); Goeddel *et al., Nature* 281:544 (1979)), alkaline phosphatase, a tryptophan (trp) promoter system (Goeddel, *Nucleic Acids Res.* 8:4057 (1980); EP 36,776), and hybrid promoters such as the tac promoter. deBoer *et al., Proc. Natl. Acad Sci. USA* 80:21-25 (1983). However, other known bacterial promoters are suitable. Their nucleotide sequences have been published, thereby enabling a skilled worker operably to ligate them to DNA encoding WSX receptor or OB protein (Siebenlist *et al., Cell* 20:269 (1980)) using linkers or adaptors to supply any required restriction sites. Promoters for use in bacterial systems also will contain a Shine-Dalgamo (S.D.) sequence operably linked to the DNA encoding WSX receptor or OB protein.

Promoter sequences are known for eukaryotes. Virtually all eukaryotic genes have an AT-rich region located approximately 25 to 30 bases upstream from the site where transcription is initiated. Another sequence found 70 to 80 bases upstream from the start of transcription of many genes is a CXCAAT region where X may be any nucleotide. At the 3' end of most eukaryotic genes is an AATAAA sequence that may be the signal for addition of the poly A tail to the 3' end of the coding sequence. All of these sequences are suitably inserted into eukaryotic expression vectors.

Examples of suitable promoting sequences for use with yeast hosts include the promoters for 3-phosphoglycerate kinase (Hitzeman *et al., J. Biol. Chem.* 255:2073 (1980)) or other glycolytic enzymes (Hess *et al., J. Adv. Enzyme Reg.* 7:149 (1968); Holland, *Biochemistry* 17:4900 (1978)), such as enolase, glyceraldehyde-3-phosphate dehydrogenase, hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase.

Other yeast promoters, which are inducible promoters having the additional advantage of transcription controlled by growth conditions, are the promoter regions for alcohol dehydrogenase 2, isocytochrome C, acid phosphatase, degradative enzymes associated with nitrogen metabolism, metallothionein, glyceraldehyde-3-phosphate dehydrogenase, and enzymes responsible for maltose and galactose utilization. Suitable vectors and promoters for use in yeast expression are further described in EP 73,657. Yeast enhancers also are advantageously used with yeast promoters.

WSX receptor or OB protein transcription from vectors in mammalian host cells is controlled, for example, by promoters obtained from the genomes of viruses such as polyoma virus, fowlpox virus (UK 2,211,504 published 5 July 1989), adenovirus (such as Adenovirus 2), bovine papilloma virus, avian sarcoma virus, cytomegalovirus, a retrovirus, hepatitis-B virus and most preferably Simian Virus 40 (SV40), from heterologous mammalian promoters, *e.g.,* the actin promoter or an immunoglobulin promoter, from heat-shock promoters, and from the promoter normally associated with the WSX receptor or OB protein sequence, provided such promoters are compatible with the host cell systems.

The early and late promoters of the SV40 virus are conveniently obtained as an SV40 restriction fragment that also contains the SV40 viral origin of replication. Fiers *et al., Nature* 273:113 (1978); Mulligan *et al., Science* 209:1422-1427 (1980); Pavlakis *et al., Proc. Natl. Acad. Sci. USA* 78:7398-7402 (1981). The immediate early promoter of the human cytomegalovirus is conveniently obtained as a HindIII E restriction fragment. Greenaway *et al., Gene* 18:355-360 (1982). A system for expressing DNA in mammalian hosts using the bovine papilloma virus as a vector is disclosed in U.S. Patent No. 4,419,446. A modification of this system is described in U.S. Patent No. 4,601,978. See also Gray *et al., Nature* 295:503-508 (1982) on expressing cDNA encoding immune interferon in monkey cells; Reyes *et al., Nature* 297:598-601 (1982) on expression of human β-interferon cDNA in mouse cells under the control of a thymidine kinase promoter from herpes simplex virus; Canaani *et al., Proc. Natl. Acad Sci. USA* 79:5166-5170 (1982) on expression of the human interferon β1 gene in cultured mouse and rabbit cells; and Gorman *et al., Proc. Natl. Acad Sci. USA* 79:6777-6781 (1982) on expression of bacterial CAT sequences in CV-1 monkey kidney cells, chicken embryo fibroblasts, Chinese hamster ovary cells, HeLa cells, and mouse NIH-3T3 cells using the Rous sarcoma virus long terminal repeat as a promoter.

### (5) Enhancer element component

Transcription of a DNA encoding the WSX receptor or OB protein of this invention by higher eukaryotes is often increased by inserting an enhancer sequence into the vector. Enhancers are cis-acting elements of DNA, usually about from 10 to 300 bp, that act on a promoter to increase its transcription. Enhancers are relatively orientation and position independent, having been found 5' (Laimins *et al., Proc. Natl. Acad. Sci. USA* 78:993 (1981)) and 3' (Lusky *et al., Mol. Cell Bio.* 3:1108 (1983)) to the transcription unit, within an intron (Banerji *et al., Cell* 33:729 (1983)), as well as within the coding sequence itself. Osbome *et al., Mol. Cell Bio*. 4:1293 (1984). Many enhancer sequences are now known from mammalian genes (globin, elastase, albumin, α-fetoprotein, and insulin). Typically, however, one will use an enhancer from a eukaryotic cell virus. Examples include the SV40 enhancer on the late side of the replication origin (bp 100-270), the cytomegalovirus early promoter enhancer, the polyoma enhancer on the late side of the replication origin, and adenovirus enhancers. See also Yaniv, *Nature* 297:17-18 (1982) on enhancing elements for activation of eukaryotic promoters. The enhancer may be spliced into the vector at a position 5' or 3' to the WSX receptor or OB protein-encoding sequence, but is preferably located at a site 5' from the promoter.

### (6) Transcription termination component

Expression vectors used in eukaryotic host cells (yeast, fungi, insect, plant, animal, human, or nucleated cells from other multicellular organisms) will also contain sequences necessary for the termination of transcription and for stabilizing the mRNA. Such sequences are commonly available from the 5' and, occasionally 3', untranslated regions of eukaryotic or viral DNAs or cDNAs. These regions contain nucleotide segments transcribed as polyadenylated fragments in the untranslated portion of the mRNA encoding WSX receptor or OB protein.

### (7) Construction and analysis of vectors

Construction of suitable vectors containing one or more of the above-listed components employs standard ligation techniques. Isolated plasmids or DNA fragments are cleaved, tailored, and re-ligated in the form desired to generate the plasmids required.

For analysis to confirm correct sequences in plasmids constructed, the ligation mixtures are used to transform *E. coli* K12 strain 294 (ATCC 31,446) and successful transformants selected by ampicillin or tetracycline resistance where appropriate. Plasmids from the transformants are prepared, analyzed by restriction endonuclease digestion, and/or sequenced by the method of Messing *et al., Nucleic Acids Res.* 9:309 (1981) or by the method of Maxam *et al., Methods in Enzymology* 65:499 (1980).

### Transient expression vectors

Particularly useful in the practice of this invention are expression vectors that provide for the transient expression in mammalian cells of DNA encoding WSX receptor or OB protein. In general, transient expression involves the use of an expression vector that is able to replicate efficiently in a host cell, such that the host cell accumulates many copies of the expression vector and, in turn, synthesizes high levels of a desired polypeptide encoded by the expression vector. Sambrook *et al., supra,* pp. 16.17 - 16.22. Transient expression systems, comprising a suitable expression vector and a host cell, allow for the convenient positive identification of polypeptides encoded by cloned DNAs, as well as for the rapid screening of such polypeptides for desired biological or physiological properties. Thus, transient expression systems are particularly useful in the invention for purposes of identifying analogs and variants of WSX receptor or OB protein that are biologically active WSX receptor or OB protein.

### (8) Suitable exemplary vertebrate cell vectors

Other methods, vectors, and host cells suitable for adaptation to the synthesis of WSX receptor or OB protein in recombinant vertebrate cell culture are described in Gething *et al., Nature* 293:620-625 (1981); Mantei *et al., Nature* 281:40-46 (1979); EP 117,060; and EP 117,058. A particularly useful plasmid for mammalian cell culture expression of WSX receptor or OB protein is pRK5 (EP 307,247) or pSV16B. WO 91/08291 published 13 June 1991.

### iii. Selection and Transformation of Host Cells

Suitable host cells for cloning or expressing the DNA in the vectors herein are the prokaryote, yeast, or higher eukaryote cells described above. Suitable prokaryotes for this purpose include eubacteria, such as Gram-negative or Gram-positive organisms, for example, Enterobacteriaceae such as *Escherichia, e.g., E. coli, Enterobacter, Erwinia, Klebsiella, Proteus, Salmonella, e.g., Salmonella typhimurium, Serratia, e.g., Serratia marcescans,* and *Shigella,* as well as *Bacilli* such as *B. subtilis* and *B. licheniformis* (*e.g., B. licheniformis* 41P disclosed in DD 266,710 published 12 April 1989), *Pseudomonas* such as *P. aeruginosa,* and *Streptomyces.* One preferred *E. coli* cloning host is *E. coli* 294 (ATCC 31,446), although other strains such as *E. coli* B*, E. coli* X1776 (ATCC 31,537), and *E. coli* W3110 (ATCC 27,325) are suitable. These examples are illustrative rather than limiting. Strain W3110 is a particularly preferred host or parent host because it is a common host strain for recombinant DNA product fermentations. Preferably, the host cell should secrete minimal amounts of proteolytic enzymes. For example, strain W3110 may be modified to effect a genetic mutation in the genes encoding proteins, with examples of such hosts including *E. coli* W3110 strain 27C7. The complete genotype of 27C7 is *tonA Δ ptr3 phoAΔE15 Δ(argF-lac)169 ompTΔ degP41kan*^{*r*}*.* Strain 27C7 was deposited on 30 October 1991 in the American Type Culture Collection as ATCC No. 55,244. Alternatively, the strain of *E. coli* having mutant periplasmic protease disclosed in U.S. Patent No. 4,946,783 issued 7 August 1990 may be employed. Alternatively still, methods of cloning, *e*.*g*., PCR or other nucleic acid polymerase reactions, are suitable.

In addition to prokaryotes, eukaryotic microbes such as filamentous fungi or yeast are suitable cloning or expression hosts for WSX receptor or OB protein-encoding vectors. *Saccharomyces cerevisiae,* or common baker's yeast, is the most commonly used among lower eukaryotic host microorganisms. However, a number of other genera, species, and strains are commonly available and useful herein, such as *Schizosaccharomyces pombe* (Beach *et al., Nature*, 290:140 (1981); EP 139,383 published 2 May 1985); *Kluyveromyces* hosts (U.S. Patent No. 4,943,529; Fleer *et al., supra*) such as, *e.g., K. lactis* (MW98-8C, CBS683, CBS4574), *K. fragilis* (ATCC 12,424), *K. bulgaricus* (ATCC 16,045), *K. wickeramii* (ATCC 24,178), *K. waltii* (ATCC 56,500), *K. drosophilarum* (ATCC 36,906; Van den Berg *et al., supra*), *K. thermotolerans*, and *K marxianus; yarrowia* (EP 402,226); *Pichia pastoris* (EP 183,070; Sreekrishna *et al., J. Basic Microbiol*. 28:265-278 (1988)); *Candida; Trichoderma reesia* (EP 244,234); *Neurospora crassa* (Case *et al., Proc. Natl. Acad Sci. USA* 76:5259-5263 (1979)); *Schwanniomyces* such as *Schwanniomyces occidentalis* (EP 394,538 published 31 October 1990); and filamentous fungi such as, *e.g., Neurospora, Penicillium, Tolypocladium* (WO 91/00357 published 10 January 1991), and *Aspergillus* hosts such as *A*. *nidulans* (Ballance *et al., Biochem. Biophys. Res. Commun*. 112:284-289 (1983); Tilbum *et al., Gene* 26:205-221 (1983); Yelton *et al., Proc. Natl. Acad Sci. USA* 81:1470-1474 (1984)) and *A. niger*. Kelly *et al., EMBO J*. 4:475-479 (1985).

Suitable host cells for the expression of glycosylated WSX receptor or OB protein are derived from multicellular organisms. Such host cells are capable of complex processing and glycosylation activities. In principle, any higher eukaryotic cell culture is workable, whether from vertebrate or invertebrate culture. Examples of invertebrate cells include plant and insect cells. Numerous baculoviral strains and variants and corresponding permissive insect host cells from hosts such as *Spodoptera frugiperda* (caterpillar), *Aedes aegypti* (mosquito), *Aedes albopictus* (mosquito), *Drosophila melanogaster* (fruitfly), and *Bombyx mori* have been identified. See, *e.g*., Luckow *et al., Bio*/*Technology* 6:47-55 (1988); Miller *et al*., in *Genetic Engineering,* Setlow *et al*., eds., Vol. 8 (Plenum Publishing, 1986), pp. 277-279; and Maeda *et al., Nature* 315:592-594 (1985). A variety of viral strains for transfection are publicly available, *e.g.,* the L-1 variant of *Autographa californica* NPV and the Bm-5 strain of *Bombyx mori* NPV, and such viruses may be used as the virus herein according to the present invention, particularly for transfection of *Spodoptera frugiperda* cells.

Plant cell cultures of cotton, com, potato, soybean, petunia, tomato, and tobacco can be utilized as hosts. Typically, plant cells are transfected by incubation with certain strains of the bacterium *Agrobacterium tumefaciens*, which has been previously manipulated to contain the WSX receptor or OB protein-encoding DNA. During incubation of the plant cell culture with *A. tumefaciens*, the DNA encoding the WSX receptor or OB protein is transferred to the plant cell host such that it is transfected, and will, under appropriate conditions, express the WSX receptor or OB protein-encoding DNA. In addition, regulatory and signal sequences compatible with plant cells are available, such as the nopaline synthase promoter and polyadenylation signal sequences. Depicker *et al., J. Mol. Appl. Gen*. 1:561 (1982). In addition, DNA segments isolated from the upstream region of the T-DNA *780* gene are capable of activating or increasing transcription levels of plant-expressible genes in recombinant DNA-containing plant tissue. EP 321,196 published 21 June 1989.

However, interest has been greatest in vertebrate cells, and propagation of vertebrate cells in culture (tissue culture) has become a routine procedure. See, *e.g., Tissue Culture*, Academic Press, Kruse and Patterson, editors (1973). Examples of useful mammalian host cell lines are monkey kidney CV1 line transformed by SV40 (COS-7, ATCC CRL 1651); human embryonic kidney line (293 or 293 cells subcloned for growth in suspension culture, Graham *et al., J. Gen Virol*. 36:59 (1977)); baby hamster kidney cells (BHK, ATCC CCL 10); Chinese hamster ovary cells/-DHFR (CHO, Urlaub *et al., Proc. Natl. Acad. Sci. USA* 77:4216 (1980)); mouse sertoli cells (TM4, Mather, *Biol. Reprod.* 23:243-251 (1980)); monkey kidney cells (CV1 ATCC CCL 70); African green monkey kidney cells (VERO-76, ATCC CRL-1587); human cervical carcinoma cells (HELA, ATCC CCL 2); canine kidney cells (MDCK, ATCC CCL 34); buffalo rat liver cells (BRL 3A, ATCC CRL 1442); human lung cells (W138, ATCC CCL 75); human liver cells (Hep G2, HB 8065); mouse mammary tumor (MMT 060562, ATCC CCL51); TRI cells (Mather *et al., Annals N.Y. Acad. Sci*. 383:44-68 (1982)); MRC 5 cells; FS4 cells; and a human hepatoma line (Hep G2).

Host cells are transfected and preferably transformed with the above-described expression or cloning vectors for WSX receptor or OB protein production and cultured in conventional nutrient media modified as appropriate for inducing promoters, selecting transformants, or amplifying the genes encoding the desired sequences.

Transfection refers to the taking up of an expression vector by a host cell whether or not any coding sequences are in fact expressed. Numerous methods of transfection are known to the ordinarily skilled artisan, for example, CaPO₄ and electroporation. Successful transfection is generally recognized when any indication of the operation of this vector occurs within the host cell.

Transformation means introducing DNA into an organism so that the DNA is replicable, either as an extrachromosomal element or by chromosomal integrant. Depending on the host cell used, transformation is done using standard techniques appropriate to such cells. The calcium treatment employing calcium chloride, as described in section 1.82 of Sambrook *et al., supra,* or electroporation is generally used for prokaryotes or other cells that contain substantial cell-wall barriers. Infection with *Agrobacterium tumefaciens* is used for transformation of certain plant cells, as described by Shaw *et al., Gene* 23:315 (1983) and WO 89/05859 published 29 June 1989. In addition, plants may be transfected using ultrasound treatment as described in WO 91/00358 published 10 January 1991.

For mammalian cells without such cell walls, the calcium phosphate precipitation method of Graham *et al., Virology* 52:456-457 (1978) is preferred. General aspects of mammalian cell host system transformations have been described in U.S. Pat. No. 4,399,216 issued 16 August 1983. Transformations into yeast are typically carried out according to the method of Van Solingen *et al., J. Bact.* 130:946 (1977) and Hsiao *et al., Proc. Natl. Acad. Sci. USA* 76:3829 (1979). However, other methods for introducing DNA into cells, such as by nuclear microinjection, electroporation, bacterial protoplast fusion with intact cells, or polycations, *e.g*., polybrene, polyornithine, *etc.,* may also be used. For various techniques for transforming mammalian cells, see Keown *et al., Methods in Enzymology* 185:527-537 (1990) and Mansour *et al., Nature* 336:348-352 (1988).

### iv. Culturing the Host Cells

Prokaryotic cells used to produce the WSX receptor or OB protein of this invention are cultured in suitable media as described generally in Sambrook *et al., supra*.

The mammalian host cells used to produce the WSX receptor or OB protein of this invention may be cultured in a variety of media. Commercially available media such as Ham's F10 (Sigma), Minimal Essential Medium ((MEM), Sigma), RPMI-1640 (Sigma), and Dulbecco's Modified Eagle's Medium ((DMEM), Sigma) are suitable for culturing the host cells. In addition, any of the media described in Ham *et al. Meth. Enz*. 58:44 (1979), Barnes *et al., Anal. Biochem*. 102:255 (1980), U.S. Pat. Nos. 4,767,704; 4,657,866; 4,927,762; 4,560,655; or 5,122,469; WO 90/03430; WO 87/00195; or U.S. Patent Re. 30,985 may be used as culture media for the host cells. Any of these media may be supplemented as necessary with hormones and/or other growth factors (such as insulin, transferrin, or epidermal growth factor), salts (such as sodium chloride, calcium, magnesium, and phosphate), buffers (such as HEPES), nucleosides (such as adenosine and thymidine), antibiotics (such as GENTAMYCIN™ drug), trace elements (defined as inorganic compounds usually present at final concentrations in the micromolar range), and glucose or an equivalent energy source. Any other necessary supplements may also be included at appropriate concentrations that would be known to those skilled in the art. The culture conditions, such as temperature, pH, and the like, are those previously used with the host cell selected for expression, and will be apparent to the ordinarily skilled artisan.

In general, principles, protocols, and practical techniques for maximizing the productivity of mammalian cell cultures can be found in *Mammalian Cell Biotechnology: a Practical Approach,* M. Butler, ed. (IRL Press, 1991).

The host cells referred to in this disclosure encompass cells in culture as well as cells that are within a host animal.

### v. Detecting Gene Amplification/Expression

Gene amplification and/or expression may be measured in a sample directly, for example, by conventional Southern blotting, Northern blotting to quantitate the transcription of mRNA (Thomas, *Proc. Natl. Acad. Sci. USA* 77:5201-5205 (1980)), dot blotting (DNA analysis), or *in situ* hybridization, using an appropriately labeled probe, based on the sequences provided herein. Various labels may be employed, most commonly radioisotopes, particularly ³²P. However, other techniques may also be employed, such as using biotin-modified nucleotides for introduction into a polynucleotide. The biotin then serves as the site for binding to avidin or antibodies, which may be labeled with a wide variety of labels, such as radionuclides, fluorescers, enzymes, or the like. Alternatively, antibodies may be employed that can recognize specific duplexes, including DNA duplexes, RNA duplexes, and DNA-RNA hybrid duplexes or DNA-protein duplexes. The antibodies in turn may be labeled and the assay may be carried out where the duplex is bound to a surface, so that upon the formation of duplex on the surface, the presence of antibody bound to the duplex can be detected.

Gene expression, alternatively, may be measured by immunological methods, such as immunohistochemical staining of tissue sections and assay of cell culture or body fluids, to quantitate directly the expression of gene product. With immunohistochemical staining techniques, a cell sample is prepared, typically by dehydration and fixation, followed by reaction with labeled antibodies specific for the gene product coupled, where the labels are usually visually detectable, such as enzymatic labels, fluorescent labels, luminescent labels, and the like. A particularly sensitive staining technique suitable for use in the present invention is described by Hsu *et al., Am. J. Clin. Path.* 75:734-738 (1980).

Antibodies useful for immunohistochemical staining and/or assay of sample fluids may be either monoclonal or polyclonal, and may be prepared as described herein.

### vi. Purification of WSX Receptor or OB Protein

WSX receptor (*e.g.,* WSX receptor ECD) or OB protein preferably is recovered from the culture medium as a secreted polypeptide, although it also may be recovered from host cell lysates. If the WSX receptor is membrane-bound, it can be released from the membrane using a suitable detergent solution (*e*.*g*. Triton-X 100)

When WSX receptor or OB protein is produced in a recombinant cell other than one of human origin, the WSX receptor or OB protein is completely free of proteins or polypeptides of human origin. However, it is necessary to purify WSX receptor or OB protein from recombinant cell proteins or polypeptides to obtain preparations that are substantially homogeneous as to WSX receptor or OB protein. As a first step, the culture medium or lysate is centrifuged to remove particulate cell debris. WSX receptor or OB protein thereafter is purified from contaminant soluble proteins and polypeptides, with the following procedures being exemplary of suitable purification procedures: by fractionation on an ion-exchange column; ethanol precipitation; reverse phase HPLC; chromatography on silica or on a cation-exchange resin such as DEAE; chromatofocusing; SDS-PAGE; ammonium sulfate precipitation; gel filtration using, for example, Sephadex G-75™; and protein A Sepharose™ columns to remove contaminants such as IgG.

WSX receptor or OB protein variants in which residues have been deleted, inserted, or substituted are recovered in the same fashion as native sequence WSX receptor or OB protein, taking account of any substantial changes in properties occasioned by the variation. Immunoaffinity columns such as a rabbit polyclonal anti-WSX receptor or OB protein column can be employed to absorb the WSX receptor or OB protein variant by binding it to at least one remaining immune epitope.

A protease inhibitor such as phenyl methyl sulfonyl fluoride (PMSF) also may be useful to inhibit proteolytic degradation during purification, and antibiotics may be included to prevent the growth of adventitious contaminants.

### vii. Covalent Modifications

Covalent modifications of WSX receptor or OB protein are included within the scope of this invention. Both native sequence WSX receptor or OB protein and amino acid sequence variants of the WSX receptor or OB protein may be covalently modified. One type of covalent modification of the WSX receptor or OB protein is introduced into the molecule by reacting targeted amino acid residues of the WSX receptor or OB protein with an organic derivatizing agent that is capable of reacting with selected side chains or the N- or C-terminal residues of the WSX receptor or OB protein.

Cysteinyl residues most commonly are reacted with α-haloacetates (and corresponding amines), such as chloroacetic acid or chloroacetamide, to give carboxymethyl or carboxyamidomethyl derivatives. Cysteinyl residues also are derivatized by reaction with bromotrifluoroacetone, α-bromo-β-(5-imidozoyl)propionic acid, chloroacetyl phosphate, N-alkylmaleimides, 3-nitro-2-pyridyl disulfide, methyl 2-pyridyl disulfide, p-chloromercuribenzoate, 2-chloromercuri-4-nitrophenol, or chloro-7-nitrobenzo-2-oxa-1,3-diazole.

Histidyl residues are derivatized by reaction with diethylpyrocarbonate at pH 5.5-7.0 because this agent is relatively specific for the histidyl side chain. Para-bromophenacyl bromide also is useful; the reaction is preferably performed in 0.1M sodium cacodylate at pH 6.0.

Lysinyl and amino terminal residues are reacted with succinic or other carboxylic acid anhydrides. Derivatization with these agents has the effect of reversing the charge of the lysinyl residues. Other suitable reagents for derivatizing α-amino-containing residues include imidoesters such as methyl picolinimidate, pyridoxal phosphate, pyridoxal, chloroborohydride, trinitrobenzenesulfonic acid, O-methylisourea, 2,4-pentanedione, and transaminase-catalyzed reaction with glyoxylate.

Arginyl residues are modified by reaction with one or several conventional reagents, among them phenylglyoxal, 2,3-butanedione, 1,2-cyclohexanedione, and ninhydrin. Derivatization of arginine residues requires that the reaction be performed under alkaline conditions because of the high pKₐ of the guanidine functional group. Furthermore, these reagents may react with the groups of lysine as well as with the arginine epsilon-amino group.

The specific modification of tyrosyl residues may be made, with particular interest in introducing spectral labels into tyrosyl residues by reaction with aromatic diazonium compounds or tetranitromethane. Most commonly, N-acetylimidizole and tetranitromethane are used to form O-acetyl tyrosyl species and 3-nitro derivatives, respectively. Tyrosyl residues are iodinated using ¹²⁵I or ¹³¹I to prepare labeled proteins for use in radioimmunoassay, the chloramine T method being suitable.

Carboxyl side groups (aspartyl or glutamyl) are selectively modified by reaction with carbodiimides (R-N=C=N-R'), where R and R' are different alkyl groups, such as 1-cyclohexyl-3-(2-morpholinyl-4-ethyl) carbodiimide or 1-ethyl-3-(4-azonia-4,4-dimethylpentyl) carbodiimide. Furthermore, aspartyl and glutamyl residues are converted to asparaginyl and glutaminyl residues by reaction with ammonium ions.

Derivatization with bifunctional agents is useful for crosslinking WSX receptor or OB protein to a water-insoluble support matrix or surface for use in the method for purifying anti-WSX receptor or OB protein antibodies, and vice-versa. Commonly used crosslinking agents include, *e.g*., 1,1-bis(diazoacetyl)-2-phenylethane, glutaraldehyde, N-hydroxysuccinimide esters, for example, esters with 4-azidosalicylic acid, homobifunctional imidoesters, including disuccinimidyl esters such as 3,3'-dithiobis(succinimidylpropionate), and bifunctional maleimides such as bis-N-maleimido-1,8-octane. Derivatizing agents such as methyl-3-((p-azidophenyl)dithio)propioimidate yield photoactivatable intermediates that are capable of forming crosslinks in the presence of light. Alternatively, reactive water-insoluble matrices such as cyanogen bromide-activated carbohydrates and the reactive substrates described in U.S. Patent Nos. 3,969,287; 3,691,016; 4,195,128; 4,247,642; 4,229,537; and 4,330,440 are employed for protein immobilization.

Glutaminyl and asparaginyl residues are frequently deamidated to the corresponding glutamyl and aspartyl residues, respectively. These residues are deamidated under neutral or basic conditions. The deamidated form of these residues falls within the scope of this invention.

Other modifications include hydroxylation of proline and lysine, phosphorylation of hydroxyl groups of seryl or threonyl residues, methylation of the α-amino groups of lysine, arginine, and histidine side chains (T.E. Creighton, *Proteins: Structure and Molecular Properties*, W.H. Freeman & Co., San Francisco, pp. 79-86 (1983)), acetylation of the N-terminal amine, and amidation of any C-terminal carboxyl group.

Another type of covalent modification of the WSX receptor or OB protein included within the scope of this invention comprises altering the native glycosylation pattern of the polypeptide. By altering is meant deleting one or more carbohydrate moieties found in native WSX receptor or OB protein, and/or adding one or more glycosylation sites that are not present in the native WSX receptor or OB protein.

Glycosylation of polypeptides is typically either N-linked or O-linked. N-linked refers to the attachment of the carbohydrate moiety to the side chain of an asparagine residue. The tripeptide sequences asparagine-X-serine and asparagine-X-threonine, where X is any amino acid except proline, are the recognition sequences for enzymatic attachment of the carbohydrate moiety to the asparagine side chain. Thus, the presence of either of these tripeptide sequences in a polypeptide creates a potential glycosylation site. O-linked glycosylation refers to the attachment of one of the sugars N-aceylgalactosamine, galactose, or xylose to a hydroxylamino acid, most commonly serine or threonine, although 5-hydroxyproline or 5-hydroxylysine may also be used.

Addition of glycosylation sites to the WSX receptor or OB protein is conveniently accomplished by altering the amino acid sequence such that it contains one or more of the above-described tripeptide sequences (for N-linked glycosylation sites). The alteration may also be made by the addition of, or substitution by, one or more serine or threonine residues to the native WSX receptor or OB protein sequence (for O-linked glycosylation sites). For ease, the WSX receptor or OB protein amino acid sequence is preferably altered through changes at the DNA level, particularly by mutating the DNA encoding the WSX receptor or OB protein at preselected bases such that codons are generated that will translate into the desired amino acids. The DNA mutation(s) may be made using methods described above and in U.S. Pat. No. 5,364,934, *supra.*

Another means of increasing the number of carbohydrate moieties on the WSX receptor or OB protein is by chemical or enzymatic coupling of glycosides to the polypeptide. These procedures are advantageous in that they do not require production of the polypeptide in a host cell that has glycosylation capabilities for N- or O-linked glycosylation. Depending on the coupling mode used, the sugar(s) may be attached to (a) arginine and histidine, (b) free carboxyl groups, (c) free sulfhydryl groups such as those of cysteine, (d) free hydroxyl groups such as those of serine, threonine, or hydroxyproline, (e) aromatic residues such as those of phenylalanine, tyrosine, or tryptophan, or (f) the amide group of glutamine. These methods are described in WO 87/05330 published 11 September 1987, and in Aplin *et al., CRC Crit. Rev. Biochem.* 259-306 (1981).

Removal of carbohydrate moieties present on the WSX receptor or OB protein may be accomplished chemically or enzymatically. Chemical deglycosylation requires exposure of the polypeptide to the compound trifluoromethanesulfonic acid, or an equivalent compound. This treatment results in the cleavage of most or all sugars except the linking sugar (N-acetylglucosamine or N-acetylgatactosamine), while leaving the polypeptide intact. Chemical deglycosylation is described by Hakimuddin, *et al., Arch. Biochem. Biophys.* 259:52 (1987) and by Edge *et al., Anal. Biochem.* 118:131 (1981). Enzymatic cleavage of carbohydrate moieties on polypeptides can be achieved by the use of a variety of endo- and exo-glycosidases as described by Thotakura *et al., Meth. Enzymol*. 138:350 (1987).

Glycosylation at potential glycosylation sites may be prevented by the use of the compound tunicamycin as described by Duskin *et al., J. Biol. Chem*. 257:3105 (1982). Tunicamycin blocks the formation of protein-N-glycoside linkages.

Another type of covalent modification of WSX receptor or OB protein comprises linking the WSX receptor or OB protein to one of a variety of nonproteinaceous polymers, *e*.*g*., polyethylene glycol, polypropylene glycol, or polyoxyalkylenes, in the manner set forth in U.S. Patent Nos. 4,640,835; 4,496,689; 4,301,144; 4,670,417; 4,791,192 or 4,179,337.

Since it is often difficult to predict in advance the characteristics of a variant WSX receptor or OB protein, it will be appreciated that some screening of the recovered variant will be needed to select the optimal variant. A change in the immunological character of the WSX receptor or OB protein molecule, such as affinity for a given antibody, is also able to be measured by a competitive-type immunoassay. The WSX receptor variant is assayed for changes in the ability of the protein to induce cell proliferation in the colony assay of Example 8. Other potential modifications of protein or polypeptide properties such as redox or thermal stability, hydrophobicity, susceptibility to proteolytic degradation, or the tendency to aggregate with carriers or into multimers are assayed by methods well known in the art.

### viii. Epitope-Tagged WSX Receptor or OB Protein

This invention encompasses chimeric polypeptides comprising WSX receptor or OB protein fused to a heterologous polypeptide. A chimeric WSX receptor or OB protein is one type of WSX receptor or OB protein variant as defined herein. In one preferred embodiment, the chimeric polypeptide comprises a fusion of the WSX receptor or OB protein with a tag polypeptide which provides an epitope to which an anti-tag antibody can selectively bind. The epitope tag is generally provided at the amino- or carboxyl- terminus of the WSX receptor or OB protein. Such epitope-tagged forms of the WSX receptor or OB protein are desirable as the presence thereof can be detected using a labeled antibody against the tag polypeptide. Also, provision of the epitope tag enables the WSX receptor or OB protein to be readily purified by affinity purification using the anti-tag antibody. Affinity purification techniques and diagnostic assays involving antibodies are described later herein.

Tag polypeptides and their respective antibodies are well known in the art. Examples include the flu HA tag polypeptide and its antibody 12CA5 (Field *et al., Mol. Cell. Biol*. 8:2159-2165 (1988)); the c-myc tag and the 8F9, 3C7, 6E10, G4, B7 and 9E10 antibodies thereto (Evan *et al., Molecular and Cellular Biology* 5:3610-3616 (1985)); and the Herpes Simplex virus glycoprotein D (gD) tag and its antibody. Paborsky *et al., Protein Engineering* 3(6):547-553 (1990). Other tag polypeptides have been disclosed. Examples include the Flag-peptide (Hopp *et al., BioTechnology* 6:1204-1210 (1988)); the KT3 epitope peptide (Martin *et al., Science* 255:192-194 (1992)); an α-tubulin epitope peptide (Skinner *et al., J. Biol. Chem*. 266:15163-15166 (1991)); and the T7 gene 10 protein peptide tag. Lutz-Freyermuth *et al., Proc. Natl. Acad. Sci. USA* 87:6393-6397 (1990). Once the tag polypeptide has been selected, an antibody thereto can be generated using the techniques disclosed herein.

The general methods suitable for the construction and production of epitope-tagged WSX receptor or OB protein are the same as those disclosed hereinabove. WSX receptor or OB protein-tag polypeptide fusions are most conveniently constructed by fusing the cDNA sequence encoding the WSX receptor or OB protein portion in-frame to the tag polypeptide DNA sequence and expressing the resultant DNA fusion construct in appropriate host cells. Ordinarily, when preparing the WSX receptor or OB protein-tag polypeptide chimeras of the present invention, nucleic acid encoding the WSX receptor or OB protein will be fused at its 3' end to nucleic acid encoding the N-terminus of the tag polypeptide, however 5' fusions are also possible.

Epitope-tagged WSX receptor or OB protein can be conveniently purified by affinity chromatography using the anti-tag antibody. The matrix to which the affinity antibody is attached is most often agarose, but other matrices are available (*e.g*. controlled pore glass or poly(styrenedivinyl)benzene). The epitope-tagged WSX receptor or OB protein can be eluted from the affinity column by varying the buffer pH or ionic strength or adding chaotropic agents, for example.

### ix. WSX Receptor or OB Protein Immunoadhesins

Chimeras constructed from a receptor sequence linked to an appropriate immunoglobulin constant domain sequence (immunoadhesins) are known in the art. Immunoadhesins reported in the literature include fusions of the T cell receptor* (Gascoigne *et al., Proc. Natl.Acad. Sci. USA* 84: 2936-2940 (1987)); CD4* (Capon *et al., Nature* 337: 525-531 (1989); Traunecker *et al., Nature* 339: 68-70 (1989); Zettmeissl *et al., DNA Cell Biol. USA* 9: 347-353 (1990); Byrn *et al., Nature* 344: 667-670 (1990)); L-selectin (homing receptor) ((Watson *et al., J. Cell. Biol*. 110:2221-2229 (1990); Watson *et al., Nature* 349: 164-167 (1991)); CD44* (Aruffo *et al*., *Cell* 61: 1303-1313 (1990)); CD28* and B7* (Linsley *et al., J. Exp. Med.* 173: 721-730 (1991)); CTLA-4* (Lisley *et al., J. Exp. Med*. 174: 561-569 (1991)); CD22* (Stamenkovic *et al., Cell* 66:1133-1144 (1991)); TNF receptor (Ashkenazi *et al., Proc. Natl. Acad. Sci. USA* 88: 10535-10539 (1991); Lesslauer *et al., Eur. J. Immunol.* 27: 2883-2886 (1991); Peppel *et al., J. Exp. Med* 174:1483-1489 (1991)); NP receptors (Bennett *et al., J. Biol. Chem*. 266:23060-23067 (1991)); and IgE receptor α* (Ridgway *et al., J. Cell. Biol.* 115:abstr. 1448 (1991)), where the asterisk (*) indicates that the receptor is member of the immunoglobulin superfamily.

The simplest and most straightforward immunoadhesin design combines the binding region(s) of the "adhesin" protein with the hinge and Fc regions of an immunoglobulin heavy chain. Ordinarily, when preparing the WSX receptor or OB-immunoglobulin chimeras of the present invention, nucleic acid encoding OB protein or the extracellular domain of the WSX receptor will be fused C-terminally to nucleic acid encoding the N-terminus of an immunoglobulin constant domain sequence, however N-terminal fusions are also possible. For OB-immunoglobulin chimeras, an OB protein fragment which retains the ability to bind to the WSX receptor may be employed.

Typically, in such fusions the encoded chimeric polypeptide will retain at least functionally active hinge, CH2 and CH3 domains of the constant region of an immunoglobulin heavy chain. Fusions are also made to the C-terminus of the Fc portion of a constant domain, or immediately N-terminal to the CH1 of the heavy chain or the corresponding region of the light chain.

The precise site at which the fusion is made is not critical; particular sites are well known and may be selected in order to optimize the biological activity, secretion or binding characteristics of the WSX receptor or OB-immunoglobulin chimeras.

In some embodiments, the WSX receptor or OB-immunoglobulin chimeras are assembled as monomers, or hetero- or homo-multimers, and particularly as dimers or tetramers, essentially as illustrated in WO 91/08298.

In a preferred embodiment, the OB protein sequence or WSX receptor extracellular domain sequence is fused to the N-terminus of the C-terminal portion of an antibody (in particular the Fc domain), containing the effector functions of an immunoglobulin, *e.g*. immunoglobulin G1 (IgG1). It is possible to fuse the entire heavy chain constant region to the OB protein or WSX receptor extracellular domain sequence. However, more preferably, a sequence beginning in the hinge region just upstream of the papain cleavage site (which defines IgG Fc chemically; residue 216, taking the first residue of heavy chain constant region to be 114, or analogous sites of other immunoglobulins) is used in the fusion. In a particularly preferred embodiment, the OB protein or WSX receptor amino acid sequence is fused to the hinge region, CH2 and CH3, or the CH1, hinge, CH2 and CH3 domains of an IgG1, IgG2, or IgG3 heavy chain. The precise site at which the fusion is made is not critical, and the optimal site can be determined by routine experimentation.

In some embodiments, the WSX receptor or OB-immunoglobulin chimeras are assembled as multimers, and particularly as homo-dimers or -tetramers. Generally, these assembled immunoglobulins will have known unit structures. A basic four chain structural unit is the form in which IgG, IgD, and IgE exist. A four unit is repeated in the higher molecular weight immunoglobulins; IgM generally exists as a pentamer of basic four units held together by disulfide bonds. IgA globulin, and occasionally IgG globulin, may also exist in multimeric form in serum. In the case of multimer, each four unit may be the same or different.

Various exemplary assembled WSX receptor or OB-immunoglobulin chimeras within the scope herein are schematically diagrammed below:
(a) AC_{L}-AC_{L};
(b) AC_{H}-(AC_{H}, AC_{L}-AC_{H}, AC_{L}-V_{H}C_{H}, or V_{L}C_{L}-AC_{H});
(c) AC_{L}-AC_{H}-(AC_{L}-AC_{H}, AC_{L}-V_{H}C_{H}, V_{L}C_{L}-AC_{H}, or V_{L}C_{L}-V_{H}C_{H});
(d) AC_{L}-V_{H}C_{H}-(AC_{H}, or AC_{L}-V_{H}C_{H}, or V_{L}C_{L}-AC_{H});
(e) V_{L}C_{L}-AC_{H}-(AC_{L}-V_{H}C_{H}, or V_{L}C_{L}-AC_{H}); and
(f) (A-Y)ₙ-(V_{L}C_{L}-V_{H}C_{H})₂,
wherein
each A represents identical or different OB protein or WSX receptor amino acid sequences;
V_{L} is an immunoglobulin light chain variable domain;
V_{H} is an immunoglobulin heavy chain variable domain;
C_{L} is an immunoglobulin light chain constant domain;
C_{H} is an immunoglobulin heavy chain constant domain;
n is an integer greater than 1;
Y designates the residue of a covalent cross-linking agent.

In the interests of brevity, the foregoing structures only show key features; they do not indicate joining (J) or other domains of the immunoglobulins, nor are disulfide bonds shown. However, where such domains are required for binding activity, they shall be constructed as being present in the ordinary locations which they occupy in the immunoglobulin molecules.

Alternatively, the OB protein or WSX receptor extracellular domain sequence can be inserted between immunoglobulin heavy chain and light chain sequences such that an immunoglobulin comprising a chimeric heavy chain is obtained. In this embodiment, the OB protein or WSX receptor sequence is fused to the 3' end of an immunoglobulin heavy chain in each arm of an immunoglobulin, either between the hinge and the CH2 domain, or between the CH2 and CH3 domains. Similar constructs have been reported by Hoogenboom *et al., Mol. Immunol*., 28:1027-1037 (1991).

Although the presence of an immunoglobulin light chain is not required in the immunoadhesins of the present invention, an immunoglobulin light chain might be present either covalently associated to an OB protein or WSX receptor-immunoglobulin heavy chain fusion polypeptide, or directly fused to the WSX receptor extracellular domain or OB protein. In the former case, DNA encoding an immunoglobulin light chain is typically coexpressed with the DNA encoding the OB protein or WSX receptor-immunoglobulin heavy chain fusion protein. Upon secretion, the hybrid heavy chain and the light chain will be covalently associated to provide an immunoglobulin-like structure comprising two disulfide-linked immunoglobulin heavy chain-light chain pairs. Methods suitable for the preparation of such structures are, for example, disclosed in U.S. Patent No. 4,816,567 issued 28 March 1989.

In a preferred embodiment, the immunoglobulin sequences used in the construction of the immunoadhesins of the present invention are from an IgG immunoglobulin heavy chain constant domain. For human immunoadhesins, the use of human IgG1 and IgG3 immunoglobulin sequences is preferred. A major advantage of using IgG1 is that IgG1 immunoadhesins can be purified efficiently on immobilized protein A. In contrast, purification of IgG3 requires protein G, a significantly less versatile medium. However, other structural and functional properties of immunoglobulins should be considered when choosing the Ig fusion partner for a particular immunoadhesin construction. For example, the IgG3 hinge is longer and more flexible, so it can accommodate larger adhesin domains that may not fold or function properly when fused to IgG1. Another consideration may be valency; IgG immunoadhesins are bivalent homodimers, whereas Ig subtypes like IgA and IgM may give rise to dimeric or pentameric structures, respectively, of the basic Ig homodimer unit. For immunoadhesins designed for *in vivo* application, the pharmacokinetic properties and the effector functions specified by the Fc region are important as well. Although IgG1, IgG2 and IgG4 all have *in vivo* half-lives of 21 days, their relative potencies at activating the complement system are different. IgG4 does not activate complement, and IgG2 is significantly weaker at complement activation than IgG1. Moreover, unlike IgG1, IgG2 does not bind to Fc receptors on mononuclear cells or neutrophils. While IgG3 is optimal for complement activation, its *in vivo* half-life is approximately one third of the other IgG isotypes. Another important consideration for immunoadhesins designed to be used as human therapeutics is the number of allotypic variants of the particular isotype. In general, IgG isotypes with fewer serologically-defined allotypes are preferred. For example, IgG1 has only four serologically-defined allotypic sites, two of which (G1m and 2) are located in the Fc region; and one of these sites G1m1, is non-immunogenic. In contrast, there are 12 serologically-defined allotypes in IgG3, all of which are in the Fc region; only three of these sites (G3m5, 11 and 21) have one allotype which is nonimmunogenic. Thus, the potential immunogenicity of a γ3 immunoadhesin is greater than that of a γ1 immunoadhesin.

With respect to the parental immunoglobulin, a useful joining point is just upstream of the cysteines of the hinge that form the disulfide bonds between the two heavy chains. In a frequently used design, the codon for the C-terminal residue of the WSX receptor or OB protein part of the molecule is placed directly upstream of the codons for the sequence DKTHTCPPCP (SEQ ID NO:44) of the IgG1 hinge region.

The general methods suitable for the construction and expression of immunoadhesins are the same as those disclosed hereinabove with regard to WSX receptor and OB protein. Immunoadhesins are most conveniently constructed by fusing the cDNA sequence encoding the WSX receptor or OB protein portion in-frame to an Ig cDNA sequence. However, fusion to genomic Ig fragments can also be used (see, *e*.*g*., Gascoigne *et al*., *Proc. Natl. Acad. Sci. USA,* 84:2936-2940 (1987); Aruffo *et al., Cell* 61:1303-1313 (1990); Stamenkovic *et al., Cell* 66:1133-1144 (1991)). The latter type of fusion requires the presence of Ig regulatory sequences for expression. cDNAs encoding IgG heavy-chain constant regions can be isolated based on published sequence from cDNA libraries derived from spleen or peripheral blood lymphocytes, by hybridization or by polymerase chain reaction (PCR) techniques. The cDNAs encoding the WSX receptor or OB protein and Ig parts of the immunoadhesin are inserted in tandem into a plasmid vector that directs efficient expression in the chosen host cells. For expression in mammalian cells, pRK5-based vectors (Schall *et al., Cell* 61:361-370 (1990)) and CDM8-based vectors (Seed, *Nature* 329:840 (1989)) can be used. The exact junction can be created by removing the extra sequences between the designed junction codons using oligonucleotide-directed deletional mutagenesis (Zoller *et al., Nucleic Acids Res.* 10:6487 (1982); Capon *et al., Nature* 337:525-531 (1989)). Synthetic oligonucleotides can be used, in which each half is complementary to the sequence on either side of the desired junction; ideally, these are 36 to 48-mers. Alternatively, PCR techniques can be used to join the two parts of the molecule in-frame with an appropriate vector.

The choice of host cell line for the expression of the immunoadhesin depends mainly on the expression vector. Another consideration is the amount of protein that is required. Milligram quantities often can be produced by transient transfections. For example, the adenovirus ElA-transformed 293 human embryonic kidney cell line can be transfected transiently with pRK5-based vectors by a modification of the calcium phosphate method to allow efficient immunoadhesin expression. CDM8-based vectors can be used to transfect COS cells by the DEAE-dextran method (Aruffo *et al., Cell* 61:1303-1313 (1990); Zettmeissl *et al., DNA Cell Biol. US* 9:347-353 (1990)). If larger amounts of protein are desired, the immunoadhesin can be expressed after stable transfection of a host cell line. For example, a pRK5-based vector can be introduced into Chinese hamster ovary (CHO) cells in the presence of an additional plasmid encoding dihydrofolate reductase (DHFR) and conferring resistance to G418. Clones resistant to G418 can be selected in culture; these clones are grown in the presence of increasing levels of DHFR inhibitor methotrexate; clones are selected, in which the number of gene copies encoding the DHFR and immunoadhesin sequences is co-amplified. If the immunoadhesin contains a hydrophobic leader sequence at its N-terminus, it is likely to be processed and secreted by the transfected cells. The expression of immunoadhesins with more complex structures may require uniquely suited host cells; for example, components such as light chain or J chain may be provided by certain myeloma or hybridoma cell hosts (Gascoigne *et al*., 1987, *supra*, Martin *et al., J. Virol*. 67:3561-3568 (1993)).

Immunoadhesins can be conveniently purified by affinity chromatography. The suitability of protein A as an affinity ligand depends on the species and isotype of the immunoglobulin Fc domain that is used in the chimera. Protein A can be used to purify immunoadhesins that are based on human γ1, γ2, or γ4 heavy chains (Lindmark *et al., J. Immunol. Meth*. 62:1-13 (1983)). Protein G is recommended for all mouse isotypes and for human γ3 (Guss *et al., EMBO J*. 5:1567-1575 (1986)). The matrix to which the affinity ligand is attached is most often agarose, but other matrices are available. Mechanically stable matrices such as controlled pore glass or poly(styrenedivinyl)benzene allow for faster flow rates and shorter processing times than can be achieved with agarose. The conditions for binding an immunoadhesin to the protein A or G affinity column are dictated entirely by the characteristics of the Fc domain; that is, its species and isotype. Generally, when the proper ligand is chosen, efficient binding occurs directly from unconditioned culture fluid. One distinguishing feature of immunoadhesins is that, for human γ1 molecules, the binding capacity for protein A is somewhat diminished relative to an antibody of the same Fc type. Bound immunoadhesin can be efficiently eluted either at acidic pH (at or above 3.0), or in a neutral pH buffer containing a mildly chaotropic salt. This affinity chromatography step can result in an immunoadhesin preparation that is >95% pure.

Other methods known in the art can be used in place of, or in addition to, affinity chromatography on protein A or G to purify immunoadhesins. Immunoadhesins behave similarly to antibodies in thiophilic gel chromatography (Hutchens *et al., Anal. Biochem.* 159:217-226 (1986)) and immobilized metal chelate chromatography (Al-Mashikhi *et al., J. Dairy Sci.* 71:1756-1763 (1988)). In contrast to antibodies, however, their behavior on ion exchange columns is dictated not only by their isoelectric points, but also by a charge dipole that may exist in the molecules due to their chimeric nature.

If desired, the immunoadhesins can be made bispecific. Thus, the immunoadhesins of the present invention may combine a WSX receptor extracellular domain and a domain, such as the extracellular domain, of another cytokine receptor subunit. Exemplary cytokine receptors from which such bispecific immunoadhesin molecules can be made include TPO (or *mpl* ligand), EPO, G-CSF, IL-4, IL-7, GH, PRL, IL-3, GM-CSF, IL-5, IL-6, LIF, OSM,CNTF and IL-2 receptors. Alternatively, an OB protein domain may be combined with another cytokine, such as those exemplified herein, in the generation of a bispecific immunoadhesin. For bispecific molecules, trimeric molecules, composed of a chimeric antibody heavy chain in one arm and a chimeric antibody heavy chain-light chain pair in the other arm of their antibody-like structure are advantageous, due to ease of purification. In contrast to antibody-producing quadromas traditionally used for the production of bispecific immunoadhesins, which produce a mixture of ten tetramers, cells transfected with nucleic acid encoding the three chains of a trimeric immunoadhesin structure produce a mixture of only three molecules, and purification of the desired product from this mixture is correspondingly easier.

### x. Long Half-Life Derivatives of OB Protein

Prefered OB protein functional derivatives for use in the methods of the present invention include OB-immunoglobulin chimeras (immunoadhesins) and other longer half-life molecules. Techniques for generating OB protein immunoadhesins have been described above. The prefered OB immunoadhesin is made according to the techniques described in Example 11 below.

Other derivatives of the OB proteins, which possess a longer half-life than the native molecules comprise the OB protein or an OB-immunoglobulin chimera covalently bonded to a nonproteinaceous polymer. The nonproteinaceous polymer ordinarily is a hydrophilic synthetic polymer, *i*.*e*., a polymer not otherwise found in nature. However, polymers which exist in nature and are produced by recombinant or *in vitro* methods are useful, as are polymers which are isolated from native sources. Hydrophilic polyvinyl polymers fall within the scope of this invention, *e*.*g*. polyvinylalcohol and polyvinylpyrrolidone. Particularly useful are polyalkylene ethers such as polyethylene glycol (PEG); polyelkylenes such as polyoxyethylene, polyoxypropylene, and block copolymers of polyoxyethylene and polyoxypropylene (Pluronics™); polymethacrylates; carbomers; branched or unbranched polysaccharides which comprise the saccharide monomers D-mannose, D- and L-galactose, fucose, fructose, D-xylose, L-arabinose, D-glucuronic acid, sialic acid, D-galacturonic acid, D-mannuronic acid (*e*.*g*. polymannuronic acid, or alginic acid), D-glucosamine, D-galactosamine, D-glucose and neuraminic acid including homopolysaccharides and heteropolysaccharides such as lactose, amylopectin, starch, hydroxyethyl starch, amylose, dextrane sulfate, dextran, dextrins, glycogen, or the polysaccharide subunit of acid mucopolysaccharides, *e*.*g*. hyaluronic acid; polymers of sugar alcohols such as polysorbitol and polymannitol; heparin or heparon. The polymer prior to cross-linking need not be, but preferably is, water soluble, but the final conjugate must be water soluble. In addition, the polymer should not be highly immunogenic in the conjugate form, nor should it possess viscosity that is incompatible with intravenous infusion or injection if it is intended to be administered by such routes.

Preferably the polymer contains only a single group which is reactive. This helps to avoid cross-linking of protein molecules. However, it is within the scope herein to optimize reaction conditions to reduce cross-linking, or to purify the reaction products through gel filtration or chromatographic sieves to recover substantially homogenous derivatives.

The molecular weight of the polymer may desirably range from about 100 to 500,000, and preferably is from about 1,000 to 20,000. The molecular weight chosen will depend upon the nature of the polymer and the degree of substitution. In general, the greater the hydrophilicity of the polymer and the greater the degree of substitution, the lower the molecular weight that can be employed. Optimal molecular weights will be determined by routine experimentation.

The polymer generally is covalently linked to the OB protein or to the OB-immunoglobulin chimera though a multifunctional crosslinking agent which reacts with the polymer and one or more amino acid or sugar residues of the OB protein or OB-immunoglobulin chimera to be linked. However, it is within the scope of the invention to directly crosslink the polymer by reacting a derivatized polymer with the hybrid, or via versa.

The covalent crosslinking site on the OB protein or OB-immunoglobulin chimera includes the N-terminal amino group and epsilon amino groups found on lysine residues, as well as other amino, imino, carboxyl, sulfhydryl, hydroxyl or other hydrophilic groups. The polymer may be covalently bonded directly to the hybrid without the use of a multifunctional (ordinarily bifunctional) crosslinking agent. Covalent binding to amino groups is accomplished by known chemistries based upon cyanuric chloride, carbonyl diimidazole, aldehyde reactive groups (PEG alkoxide plus diethyl acetal of bromoacetaldehyde; PEG plus DMSO and acetic anhydride, or PEG chloride plus the phenoxide of 4-hydroxybenzaldehyde, succinimidyl active esters, activated dithiocarbonate PEG, 2,4,5-trichlorophenylcloroformate or P-nitrophenylcloroformate activated PEG). Carboxyl groups are derivatized by coupling PEG-amine using carbodiimide.

Polymers are conjugated to oligosaccharide groups by oxidation using chemicals, *e.g*. metaperiodate, or enzymes, *e.g*. glucose or galactose oxidase (either of which produces the aldehyde derivative of the carbohydrate), followed by reaction with hydrazide or amino derivatized polymers, in the same fashion as is described by Heitzmann *et al., P.N.A.S.* 71:3537-41 (1974) or Bayer *et al., Methods in Enzymology* 62:310 (1979), for the labeling of oligosaccharides with biotin or avidin. Further, other chemical or enzymatic methods which have been used heretofore to link oligosaccharides are particularly advantageous because, in general, there are fewer substitutions than amino acid sites for derivatization, and the oligosaccharide products thus will be more homogenous. The oligosaccharide substituents also are optionally modified by enzyme digestion to remove sugars, *e*.*g*. by neuraminidase digestion, prior to polymer derivatization.

The polymer will bear a group which is directly reactive with an amino acid side chain, or the N- or C-terminus of the polypeptide linked, or which is reactive with the multifunctional cross-linking agent, In general, polymers bearing such reactive groups are known for the preparation of immobilized proteins. In order to use such chemistries here, one should employ a water soluble polymer otherwise derivatized in the same fashion as insoluble polymers heretofore employed for protein immobilization. Cyanogen bromide activation is a particularly useful procedure to employ in crosslinking polysaccharides.

"Water soluble" in reference to the starting polymer means that the polymer or its reactive intermediate used for conjugation is sufficiently water soluble to participate in a derivatization reaction.

"Water soluble" in reference to the polymer conjugate means that the conjugate is soluble in physiological fluids such as blood.

The degree of substitution with such a polymer will vary depending upon the number of reactive sites on the protein, whether all or a fragment of the protein is used, whether the protein is a fusion with a heterologous protein (*e*.*g*. an OB-immunoglobulin chimera), the molecular weight, hydrophilicity and other characteristics of the polymer, and the particular protein derivatization sites chosen. In general, the conjugate contains about from 1 to 10 polymer molecules, while any heterologous sequence may be substituted with an essentially unlimited number of polymer molecules so long as the desired activity is not significantly adversely affected. The optimal degree of cross-linking is easily determined by an experimental matrix in which the time, temperature and other reaction conditions are varied to change the degree of substitution, after which the ability of the conjugates to function in the desired fashion is determined.

The polymer, *e.g.* PEG, is cross-linked by a wide variety of methods known *per se* for the covalent modification of proteins with nonproteinaceous polymers such as PEG. Certain of these methods, however, are not preferred for the purposes herein. Cyanuronic chloride chemistry leads to many side reactions, including protein cross-linking. In addition, it may be particularly likely to lead to inactivation of proteins containing sulfhydryl groups. Carbonyl diimidazole chemistry (Beauchamp *et al., Anal Biochem*. 131:25-33 (1983)) requires high pH (>8.5), which can inactivate proteins. Moreover, since the "activated PEG" intermediate can react with water, a very large molar excess of "activated PEG" over protein is required. The high concentrations of PEG required for the carbonyl diimidazole chemistry also led to problems in purification, as both gel filtration chromatography and hydrophilic interaction chromatography are adversely affected. In addition, the high concentrations of "activated PEG" may precipitate protein, a problem that *per se* has been noted previously (Davis, U.S. Patent No. 4,179,337). On the other hand, aldehyde chemistry (Royer, U.S. Patent No. 4,002,531) is more efficient since it requires only a 40-fold molar excess of PEG and a 1-2 hr incubation. However, the manganese dioxide suggested by Royer for preparation of the PEG aldehyde is problematic "because of the pronounced tendency of PEG to form complexes with metal-based oxidizing agents" (Harris *et al., J. Polym. Sci. Polym. Chem. Ed*. 22:341-52 (1984)). The use of a Moffatt oxidation, utilizing DMSO and acetic anhydride, obviates this problem. In addition, the sodium borohydride suggested by Royer must be used at high pH and has a significant tendency to reduce disulfide bonds. In contrast, sodium cyanoborohydride, which is effective at neutral pH and has very little tendency to reduce disulfide bonds is preferred.

Functionalized PEG polymers to modify the OB protein or OB-immunoglobulin chimeras of the present invention are available from Shearwater Polymers, Inc. (Huntsville, AL). Such commercially available PEG derivatives include, but are not limited to, amino-PEG, PEG amino acid esters, PEG-hydrazide, PEG-thiol, PEG-succinate, carboxymethylated PEG, PEG-propionic acid, PEG amino acids, PEG succinimidyl succinate, PEG succinimidyl propionate, succinimidyl ester of carboxymethylated PEG, succinimidyl carbonate of PEG, succinimidyl esters of amino acid PEGs, PEG-oxycarbonylimidazole, PEG-nitrophenyl carbonate, PEG tresylate, PEG-glycidyl ether, PEG-aldehyde, PEG vinylsulfone, PEG-maleimide, PEG-orthopyridyl-disulfide, heterofunctional PEGs, PEG vinyl derivatives, PEG silanes, and PEG phospholides. The reaction conditions for coupling these PEG derivatives will vary depending on the protein, the desired degree of PEGylation, and the PEG derivative utilized. Some factors involved in the choice of PEG derivatives include: the desired point of attachment (lysine or cysteine), hydrolytic stability and reactivity of the derivatives, stability, toxicity and antigenicity of the linkage, suitability for analysis, etc. Specific instructions for the use of any particular derivative are available from the manufacturer.

The long half-life conjugates of this invention are separated from the unreacted starting materials by gel filtration. Heterologous species of the conjugates are purified from one another in the same fashion. The polymer also may be water-insoluble, as a hydrophilic gel.

The conjugates may also be purified by ion-exchange chromatography. The chemistry of many of the electrophilically activated PEG's results in a reduction of amino group charge of the PEGylated product. Thus, high resolution ion exchange chromatography can be used to separate the free and conjugated proteins, and to resolve species with different levels of PEGylation. In fact, the resolution of different species (*e.g*. containing one or two PEG residues) is also possible due to the difference in the ionic properties of the unreacted amino acids.

### C. Non-Therapeutic Uses for the WSX Receptor

WSX receptor nucleic acid is useful for the preparation of WSX receptor polypeptide by recombinant techniques exemplified herein which can then be used for production of anti-WSX receptor antibodies having various utilities described below.

The WSX receptor (polypeptide or nucleic acid) can be used to induce proliferation and/or differentiation of cells *in vitro*. In particular, it is contemplated that this molecule may be used to induce proliferation of stem cell/progenitor cell populations (*e.g*. CD34+ cell populations obtained as described in Example 8 below). These cells which are to be grown *ex vivo* may simultaneously be exposed to other known growth factors or cytokines, such as those described herein. This results in proliferation and/or differentiation of the cells having the WSX receptor.

In yet another aspect of the invention, the WSX receptor may be used for affinity purification of WSX ligand. Briefly, this technique involves: (a) contacting a source of WSX ligand with an immobilized WSX receptor under conditions whereby the WSX ligand to be purified is selectively adsorbed onto the immobilized receptor; (b) washing the immobilized WSX receptor and its support to remove non-adsorbed material; and (c) eluting the WSX ligand molecules from the immobilized WSX receptor to which they are adsorbed with an elution buffer. In a particularly preferred embodiment of affinity purification, WSX receptor is covalently attaching to an inert and porous matrix (*e.g*., agarose reacted with cyanogen bromide). Especially preferred is a WSX receptor immunoadhesin immobilized on a protein A column. A solution containing WSX ligand is then passed through the chromatographic material. The WSX ligand adsorbs to the column and is subsequently released by changing the elution conditions (*e.g*. by changing pH or ionic strength).

The WSX receptor may be used for competitive screening of potential agonists for binding to the WSX receptor. Such agonists may constitute potential therapeutics for treating conditions characterized by insufficient WSX receptor activation.

The preferred technique for identifying molecules which bind to the WSX receptor utilizes a chimeric receptor (*e*.*g*., epitope tagged WSX receptor or WSX receptor immunoadhesin) attached to a solid phase, such as the well of an assay plate. Binding of molecules which are optionally labelled (*e*.*g*., radiolabelled) to the immobilized receptor can be evaluated.

To identify WSX receptor agonists the thymidine incorporation assay can be used.

The WSX receptor polypeptides are also useful as molecular weight markers. To use a WSX receptor polypeptide as a molecular weight marker, gel filtration chromatography or SDS-PAGE, for example, will be used to separate protein(s) for which it is desired to determine their molecular weight(s) in substantially the normal way. The WSX receptor and other molecular weight markers will be used as standards to provide a range of molecular weights. For example, phosphorylase b (mw = 97,400), bovine serum albumin (mw = 68,000), ovalbumin (mw = 46,000), WSX receptor (mw = 44,800), trypsin inhibitor (mw = 20,100), and lysozyme (mw = 14,400) can be used as mw markers. The other molecular weight markers mentioned here can be purchased commercially from Amersham Corporation, Arlington Heights, IL. The molecular weight markers are generally labeled to facilitate detection thereof. For example, the markers may be biotinylated and following separation can be incubated with streptavidin-horseradish peroxidase so that the various markers can be detected by light detection.

The purified WSX receptor, and the nucleic acid encoding it, may also be sold as reagents for mechanism studies of WSX receptor and its ligands, to study the role of the WSX receptor and WSX ligand in normal growth and development, as well as abnormal growth and development, *e*.*g*., in malignancies.

WSX receptor variants are useful as standards or controls in assays for the WSX receptor for example ELISA, RIA, or RRA, provided that they are recognized by the analytical system employed, *e*.*g*., an anti-WSX receptor antibody.

### D. WSX Receptor Antibody Preparation

### 1. Polyclonal antibodies

Polyclonal antibodies are generally raised in animals by multiple subcutaneous (sc) or intraperitoneal (ip) injections of the relevant antigen and an adjuvant. In that the preferred epitope is in the ECD of the WSX receptor, it is desirable to use WSX receptor ECD or a molecule comprising the ECD (*e*.*g*., WSX receptor immunoadhesin) as the antigen for generation of polyclonal and monoclonal antibodies. It may be useful to conjugate the relevant antigen to a protein that is immunogenic in the species to be immunized, *e*.*g*., keyhole limpet hemocyanin, serum albumin, bovine thyroglobulin, or soybean trypsin inhibitor using a bifunctional or derivatizing agent, for example, maleimidobenzoyl sulfosuccinimide ester (conjugation through cysteine residues), N-hydroxysuccinimide (through lysine residues), glutaraldehyde, succinic anhydride, SOCl₂, or R¹N=C=NR, where R and R¹ are different alkyl groups.

Animals are immunized against the antigen, immunogenic conjugates, or derivatives by combining 1 mg or 1 µg of the peptide or conjugate (for rabbits or mice, respectively) with 3 volumes of Freund's complete adjuvant and injecting the solution intradermally at multiple sites. One month later the animals are boosted with 1/5 to 1/10 the original amount of peptide or conjugate in Freund's complete adjuvant by subcutaneous injection at multiple sites. Seven to 14 days later the animals are bled and the serum is assayed for antibody titer. Animals are boosted until the titer plateaus. Preferably, the animal is boosted with the conjugate of the same antigen, but conjugated to a different protein and/or through a different cross-linking reagent. Conjugates also can be made in recombinant cell culture as protein fusions. Also, aggregating agents such as alum are suitably used to enhance the immune response.

### 2. Monoclonal antibodies

Monoclonal antibodies are obtained from a population of substantially homogeneous antibodies, *i.e*., the individual antibodies comprising the population are identical except for possible naturally occurring mutations that may be present in minor amounts. Thus, the modifier "monoclonal" indicates the character of the antibody as not being a mixture of discrete antibodies.

For example, the monoclonal antibodies may be made using the hybridoma method first described by Kohler *et al., Nature* 256:495 (1975), or may be made by recombinant DNA methods (Cabilly *et al., supra*).

In the hybridoma method, a mouse or other appropriate host animal, such as a hamster, is immunized as hereinabove described to elicit lymphocytes that produce or are capable of producing antibodies that will specifically bind to the protein used for immunization. Alternatively, lymphocytes may be immunized *in vitro*. Lymphocytes then are fused with myeloma cells using a suitable fusing agent, such as polyethylene glycol, to form a hybridoma cell (Goding, *Monoclonal Antibodies: Principles and Practice,* pp.59-103 (Academic Press, 1986)).

The hybridoma cells thus prepared are seeded and grown in a suitable culture medium that preferably contains one or more substances that inhibit the growth or survival of the unfused, parental myeloma cells. For example, if the parental myeloma cells lack the enzyme hypoxanthine guanine phosphoribosyl transferase (HGPRT or HPRT), the culture medium for the hybridomas typically will include hypoxanthine, aminopterin, and thymidine (HAT medium), which substances prevent the growth of HGPRT-deficient cells.

Preferred myeloma cells are those that fuse efficiently, support stable high-level production of antibody by the selected antibody-producing cells, and are sensitive to a medium such as HAT medium. Among these, preferred myeloma cell lines are murine myeloma lines, such as those derived from MOPC-21 and MPC-11 mouse tumors available from the Salk Institute Cell Distribution Center, San Diego, California USA, and SP-2 cells available from the American Type Culture Collection, Rockville, Maryland USA. Human myeloma and mouse-human heteromyeloma cell lines also have been described for the production of human monoclonal antibodies (Kozbor, *J. Immunol*. 133:3001 (1984); Brodeur *et al., Monoclonal Antibody Production Techniques and Applications*, pp. 51-63 (Marcel Dekker, Inc., New York, 1987)).

Culture medium in which hybridoma cells are growing is assayed for production of monoclonal antibodies directed against the antigen. Preferably, the binding specificity of monoclonal antibodies produced by hybridoma cells is determined by immunoprecipitation or by an *in vitro* binding assay, such as radioimmunoassay (RIA) or enzyme-linked immunoabsorbent assay (ELISA).

The binding affinity of the monoclonal antibody can, for example, be determined by the Scatchard analysis of Munson *et al., Anal. Biochem*. 107:220 (1980).

After hybridoma cells are identified that produce antibodies of the desired specificity, affinity, and/or activity, the clones may be subcloned by limiting dilution procedures and grown by standard methods (Goding, *supra*). Suitable culture media for this purpose include, for example, D-MEM or RPMI-1640 medium. In addition, the hybridoma cells may be grown *in vivo* as ascites tumors in an animal.

The monoclonal antibodies secreted by the subclones are suitably separated from the culture medium, ascites fluid, or serum by conventional immunoglobulin purification procedures such as, for example, protein A-Sepharose, hydroxylapatite chromatography, gel electrophoresis, dialysis, or affinity chromatography.

DNA encoding the monoclonal antibodies is readily isolated and sequenced using conventional procedures (*e.g*., by using oligonucleotide probes that are capable of binding specifically to genes encoding the heavy and light chains of murine antibodies). The hybridoma cells serve as a preferred source of such DNA. Once isolated, the DNA may be placed into expression vectors, which are then transfected into host cells such as *E. coli* cells, simian COS cells, Chinese hamster ovary (CHO) cells, or myeloma cells that do not otherwise produce immunoglobulin protein, to obtain the synthesis of monoclonal antibodies in the recombinant host cells. Review articles on recombinant expression in bacteria of DNA encoding the antibody include Skerra *et al., Curr. Opinion in Immunol*. 5:256-262 (1993) and Plückthun, *Immunol. Revs.* 130:151-188 (1992).

In a further embodiment, antibodies or antibody fragments can be isolated from antibody phage libraries generated using the techniques described in McCafferty *et al., Nature* 348:552-554 (1990). Clackson *et al., Nature* 352:624-628 (1991) and Marks *et al., J. Mol. Biol*. 222:581-597 (1991) describe the isolation of murine and human antibodies, respectively, using phage libraries. Subsequent publications describe the production of high affinity (nM range) human antibodies by chain shuffling (Mark *et al., Bio*/*Technology* 10:779-783 (1992)), as well as combinatorial infection and *in vivo* recombination as a strategy for constructing very large phage libraries (Waterhouse *et al., Nuc. Acids. Res*. 21:2265-2266 (1993)). Thus, these techniques are viable alternatives to traditional monoclonal antibody hybridoma techniques for isolation of monoclonal antibodies.

The DNA also may be modified, for example, by substituting the coding sequence for human heavy-and light-chain constant domains in place of the homologous murine sequences (Cabilly *et al., supra*; Morrison, *et al., Proc. Nat. Acad. Sci. USA* 81:6851 (1984)), or by covalently joining to the immunoglobulin coding sequence all or part of the coding sequence for a non-immunoglobulin polypeptide.

Typically such non-immunoglobulin polypeptides are substituted for the constant domains of an antibody, or they are substituted for the variable domains of one antigen-combining site of an antibody to create a chimeric bivalent antibody comprising one antigen-combining site having specificity for an antigen and another antigen-combining site having specificity for a different antigen.

Chimeric or hybrid antibodies also may be prepared *in vitro* using known methods in synthetic protein chemistry, including those involving crosslinking agents. For example, immunotoxins may be constructed using a disulfide-exchange reaction or by forming a thioether bond. Examples of suitable reagents for this purpose include iminothiolate and methyl-4-mercaptobutyrimidate.

### 3. Humanized and human antibodies

Methods for humanizing non-human antibodies are well known in the art. Generally, a humanized antibody has one or more amino acid residues introduced into it from a source which is non-human. These non-human amino acid residues are often referred to as "import" residues, which are typically taken from an "import" variable domain. Humanization can be essentially performed following the method of Winter and co-workers (Jones *et al., Nature* 321:522-525 (1986); Riechmann *et al., Nature* 332:323-327 (1988); Verhoeyen *et al., Science* 239:1534-1536 (1988)), by substituting rodent CDRs or CDR sequences for the corresponding sequences of a human antibody. Accordingly, such "humanized" antibodies are chimeric antibodies (Cabilly *et al., supra*), wherein substantially less than an intact human variable domain has been substituted by the corresponding sequence from a non-human species. In practice, humanized antibodies are typically human antibodies in which some CDR residues and possibly some FR residues are substituted by residues from analogous sites in rodent antibodies.

The choice of human variable domains, both light and heavy, to be used in making the humanized antibodies is very important to reduce antigenicity. According to the so-called "best-fit" method, the sequence of the variable domain of a rodent antibody is screened against the entire library of known human variable-domain sequences. The human sequence which is closest to that of the rodent is then accepted as the human framework (FR) for the humanized antibody (Sims *et al., J. Immunol*. 151:2296 (1993); Chothia *et al., J. Mol. Biol*. 196:901 (1987)). Another method uses a particular framework derived from the consensus sequence of all human antibodies of a particular subgroup of light or heavy chains. The same framework may be used for several different humanized antibodies (Carter *et al., Proc. Natl. Acad Sci. USA* 89:4285 (1992); Presta *et al., J. Immnol*. 151:2623 (1993)).

It is further important that antibodies be humanized with retention of high affinity for the antigen and other favorable biological properties. To achieve this goal, according to a preferred method, humanized antibodies are prepared by a process of analysis of the parental sequences and various conceptual humanized products using three-dimensional models of the parental and humanized sequences. Three-dimensional immunoglobulin models are commonly available and are familiar to those skilled in the art. Computer programs are available which illustrate and display probable three-dimensional conformational structures of selected candidate immunoglobulin sequences. Inspection of these displays permits analysis of the likely role of the residues in the functioning of the candidate immunoglobulin sequence, *i.e*., the analysis of residues that influence the ability of the candidate immunoglobulin to bind its antigen. In this way, FR residues can be selected and combined from the consensus and import sequences so that the desired antibody characteristic, such as increased affinity for the target antigen(s), is achieved. In general, the CDR residues are directly and most substantially involved in influencing antigen binding.

Alternatively, it is now possible to produce transgenic animals (*e.g*., mice) that are capable, upon immuniration, of producing a full repertoire of human antibodies in the absence of endogenous immunoglobulin production. For example, it has been described that the homozygous deletion of the antibody heavy-chain joining region (J_{H}) gene in chimeric and germ-line mutant mice results in complete inhibition of endogenous antibody production. Transfer of the human germ-line immunoglobulin gene array in such germ-line mutant mice will result in the production of human antibodies upon antigen challenge. See, *e*.*g*., Jakobovits *et al., Proc. Natl. Acad. Sci. USA* 90:2551 (1993); Jakobovits *et al., Nature* 362:255-258 (1993); Bruggermann *et al., Year in Immuno*. 7:33 (1993). Human antibodies can also be produced in phage- display libraries (Hoogenboom *et al., J. Mol. Biol*. 227:381 (1991); Marks *et al., J. Mol. Biol*.222:581 (1991)).

### 4. Bispecific antibodies

Bispecific antibodies (BsAbs) are antibodies that have binding specificities for at least two different antigens. BsAbs can be used as tumor targeting or imaging agents and can be used to target enzymes or toxins to a cell possessing the WSX receptor. Such antibodies can be derived from full length antibodies or antibody fragments (*e*.*g*. F(ab')₂ bispecific antibodies). In accordance with the present invention, the BsAb may possess one arm which binds the WSX receptor and another arm which binds to a cytokine or another cytokine receptor (or a subunit thereof) such as the receptors for TPO, EPO, G-CSF, IL-4, IL-7, GH, PRL; the a or β subunits of the IL-3, GM-CSF, IL-5, IL-6, LIF, OSM and CNTF receptors; or the α, β or γ subunits of the IL-2 receptor complex. For example, the BsAb may bind both WSX receptor and gp130.

Methods for making bispecific antibodies are known in the art. Traditional production of full length bispecific antibodies is based on the coexpression of two immunoglobulin heavy chain-light chain pairs, where the two chains have different specificities (Milistein *et al., Nature* 305:537-539 (1983)). Because of the random assortment of immunoglobulin heavy and light chains, these hybridomas (quadromas) produce a potential mixture of 10 different antibody molecules, of which only one has the correct bispecific structure. Purification of the correct molecule, which is usually done by affinity chromatography steps, is rather cumbersome, and the product yields are low. Similar procedures are disclosed in WO 93/08829, published 13 May 1993, and in Traunecker *et al., EMBO J.* 10:3655-3659 (1991).

According to a different and more preferred approach, antibody variable domains with the desired binding specificities (antibody-antigen combining sites) are fused to immunoglobulin constant domain sequences. The fusion preferably is with an immunoglobulin heavy chain constant domain, comprising at least part of the hinge, CH2, and CH3 regions. It is preferred to have the first heavy-chain constant region (CH1) containing the site necessary for light chain binding, present in at least one of the fusions. DNAs encoding the immunoglobulin heavy chain fusions and, if desired, the immunoglobulin light chain, are inserted into separate expression vectors, and are co-transfected into a suitable host organism. This provides for great flexibility in adjusting the mutual proportions of the three polypeptide fragments in embodiments when unequal ratios of the three polypeptide chains used in the construction provide the optimum yields. It is, however, possible to insert the coding sequences for two or all three polypeptide chains in one expression vector when the expression of at least two polypeptide chains in equal ratios results in high yields or when the ratios are of no particular significance.

In a preferred embodiment of this approach, the bispecific antibodies are composed of a hybrid immunoglobulin heavy chain with a first binding specificity in one arm, and a hybrid immunoglobulin heavy chain-light chain pair (providing a second binding specificity) in the other arm. It was found that this asymmetric structure facilitates the separation of the desired bispecific compound from unwanted immunoglobulin chain combinations, as the presence of an immunoglobulin light chain in only one half of the bispecific molecule provides for a facile way of separation. This approach is disclosed in WO 94/04690 published March 3, 1994. For further details of generating bispecific antibodies see, for example, Suresh *et al., Methods in Enzymology* 121:210 (1986).

Bispecific antibodies include cross-linked or "heteroconjugate" antibodies. For example, one of the antibodies in the heteroconjugate can be coupled to avidin, the other to biotin. Such antibodies have, for example, been proposed to target immune system cells to unwanted cells (US Patent No. 4,676,980), and for treatment of HIV infection (WO 91/00360, WO 92/200373, and EP 03089). Heteroconjugate antibodies may be made using any convenient cross-linking methods. Suitable cross-linking agents are well known in the art, and are disclosed in US Patent No. 4,676,980, along with a number of cross-linking techniques.

Techniques for generating bispecific antibodies from antibody fragments have also been described in the literature. The following techniques can also be used for the production of bivalent antibody fragments which are not necessarily bispecific. According to these techniques, Fab'-SH fragments can be recovered from *E. coli,* which can be chemically coupled to form bivalent antibodies. Shalaby *et al., J. Exp. Med*. 175:217-225 (1992) describe the production of a fully humanized BsAb F(ab')₂ molecule. Each Fab' fragment was separately secreted from *E. coli* and subjected to directed chemical coupling *in vitro* to form the BsAb. The BsAb thus formed was able to bind to cells overexpressing the HER2 receptor and normal human T cells, as well as trigger the lytic activity of human cytotoxic lymphocytes against human breast tumor targets. See also Rodrigues *et al., Int. J. Cancers* (Suppl.) 7:45-50 (1992).

Various techniques for making and isolating bivalent antibody fragments directly from recombinant cell culture have also been described. For example, bivalent heterodimers have been produced using leucine zippers. Kostelny *et al., J. Immunol*. 148(5):1547-1553 (1992). The leucine zipper peptides from the Fos and Jun proteins were linked to the Fab' portions of two different antibodies by gene fusion. The antibody homodimers were reduced at the hinge region to form monomers and then re-oxidized to form the antibody heterodimers. The "diabody" technology described by Hollinger *et al., Proc. Natl. Acad. Sci. USA* 90:6444-6448 (1993) has provided an alternative mechanism for making BsAb fragments. The fragments comprise a heavy-chain variable domain (V_{H}) connected to a light-chain variable domain (V_{L}) by a linker which is too short to allow pairing between the two domains on the same chain. Accordingly, the V_{H} and V_{L} domains of one fragment are forced to pair with the complementary V_{L} and V_{H} domains of another fragment, thereby forming two antigen-binding sites. Another strategy for making BsAb fragments by the use of single-chain Fv (sFv) dimers has also been reported. See Gruber *et al., J. Immunol*. 152:5368 (1994).

### 5. Antibody Screening

It may be desirable to select antibodies with a strong binding affinity for the WSX receptor. Antibody affinities may be determined by saturation binding; enzyme-linked immunoabsorbent assay (ELISA); and competition assays (*e.g*. RIA's), for example. The antibody with a strong binding affinity may bind the WSX receptor with a binding affinity (K_{d}) value of no more than about 1 x 10⁻⁷ M, preferably no more than about 1 x 10⁻⁸ M and most preferably no more than about 1 x 10⁻⁹ M (*e.g*. to about 1 x 10⁻¹²M).

In another embodiment, one may screen for an antibody which binds a WSX receptor epitope of interest. For example, an antibody which binds to the epitope bound by antibody 2D7, IG4, 1E11 or 1C11 (see Example 13) or antibody clone #3, #4 or #17 (see Example 14) can be identified. To screen for antibodies which bind to the epitope on WSX receptor bound by an antibody of interest (*e*.*g*., those which block binding of any one of the above antibodies to WSX receptor), a routine cross-blocking assay such as that described in *Antibodies, A Laboratory Manual*, Cold Spring Harbor Laboratory, Ed Harlow and David Lane (1988), can be performed. Alternatively, epitope mapping, *e.g.* as described in Champe *et al., J. Biol. Chem.* 270:1388-1394 (1995), can be performed to determine whether the antibody binds an epitope of interest.

In one particularly preferred embodiment of the invention, agonist antibodies are selected. Various methods for selecting agonist antibodies are available. In one embodiment, one evaluates the agonistic properties of the antibody upon binding to a chimeric receptor comprising the WSX receptor extracellular domain in an assay called the kinase receptor activation enzyme linked immunoadsorbent assay (KIRA ELISA) described in WO95/14930 (expressly incorporated herein by reference).

To perform the KIRA ELISA, a chimeric receptor comprising the extracellular domain of the WSX receptor and the transmembrane and intracellular domain of Rse receptor (Mark *et al., Journal of Biological Chemistry* 269(14):10720-10728 (1994)) with a carboxyl-terminal herpes simplex virus glycoprotein D (gD) tag is produced and dp12.CHO cells are transformed therewith as described in Example 4 of WO95/14930.

The WSX/Rse.gD transformed dp12.CHO cells are seeded (3x10⁴ per well) in the wells of a flat-bottom-96 well culture plate in 100µl media and cultured overnight at 37°C in 5% CO₂. The following morning the well supernatants are removed and various concentrations of the antibody are added to separate wells. The cells are stimulated at 37°C for 30 min., the well supernatants are decanted. To lyse the cells and solubilize the chimeric receptors, 100 µl of lysis buffer is added to each well. The plate is then agitated gently on a plate shaker (Bellco Instruments, Vineland, NJ) for 60 min. at room temperature.

While the cells are being solubilized, an ELISA microtiter plate (Nunc Maxisorp, Inter Med, Denmark) coated overnight at 4°C with the 5B6 monoclonal anti-gD antibody (5.0 µg/ml in 50 mM carbonate buffer, pH 9.6, 100 µl/well) is decanted and blocked with 150 µl/well of Block Buffer for 60 min. at room temperature. After 60 minutes, the anti-gD 5B6 coated plate is washed 6 times with wash buffer (PBS containing 0.05 % TWEEN 20™ and 0.01 % thimerosal).

The lysate containing solubilized WSX/Rse.gD from the cell-culture microtiter well is transferred (85µl/well) to anti-gD 5B6 coated and blocked ELISA well and is incubated for 2 h at room temperature. The unbound WSX/Rse.gD is removed by washing with wash buffer and 100 µl of biotinylated 4G10 (anti-phosphotyrosine) diluted 1:18000 in dilution buffer (PBS containing 0.5 % BSA, 0.05 % Tween-20, 5 mM EDTA, and 0.01 % thimerosal), *i.e*. 56 ng/ml is added to each well. After incubation for 2 h at room temperature the plate is washed and HRPO-conjugated streptavidin (Zymed Laboratories, S. San Francisco, CA) is added to each well. The plate is incubated for 30 minutes at room temperature with gentle agitation. The free avidin-conjugate is washed away and 100 µl freshly prepared substrate solution (tetramethyl benzidine (TMB); 2-component substrate kit; Kirkegaard and Perry, Gaithersburg, MD) is added to each well. The reaction is allowed to proceed for 10 minutes, after which the color development is stopped by the addition of 100µl/wax 1.0 MH₃PO₄. The absorbance at 450 nm is read with a reference wavelength of 650 nm (ABS_{450/650}), using a *vmax* plate reader (Molecular Devices, Palo Alto, CA) controlled with a Macintosh Centris 650 (Apple Computers, Cupertino, CA) and DeltaSofk software (BioMetallics, Inc, Princeton, NJ).

Those antibodies which have an IC50 in the KIRA ELISA of about 0.5µg/ml or less (*e.g*. from about 0.5µg/ml to about 0.001 µg/ml), preferably about 0.2µg/ml or less and most preferably about 0.1µg/ml or less are preferred agonists.

In another embodiment, one screens for antibodies which activate downstream signaling molecules for OB protein. For example, the ability of the antibody to activate Signal Transducers and Activators of Transcription (STATs) can be assessed. The agonist antibody of interest may stimulate formation of STAT-1 and STAT-3 complexes, for example. To screen for such antibodies, the assay described in Rosenblum *et al. Endocrinology* 137(11):5178-5181 (1996) may be performed.

Alternatively, an antibody which stimulates proliferation and/or differentiation of hematopoietic cells can be selected. For example, the hematopoiesis assays of Example 10 below can be performed. For example, murine fetal liver flASK stem cells may be isolated from the midgestational fetal liver as described in Zeigler *et al., Blood* 84:2422-2430 (1994) and studied in stem cell suspension culture or methylcellulose assays. For the stem cell suspension cultures, twenty thousand of the fLASK cells are seeded in individual wells in a 12 well format in DMEM 4.5/F12 media supplemented with 10% heat inactivated fetal calf serum (Hyclone, Logan, UT) and L-glutamine. Growth factors are added at the following concentrations: kit ligand (KL) at 25 ng/mL. interleukin-3 (IL-3) at 25 ng/mL, interleukin-6 (IL-6) at 50 ng/mL, G-CSF at 100 ng/mL, GM-CSF at 100 ng/mL, EPO at 2U/mL, interleukin-7 (IL-7) at 100 ng/mL (all growth factors from R and D Systems, Minneapolis, MN). The agonist antibody is then added and the ability of the antibody to expand the flASK cells grown in suspension culture is assessed. Methylcellulose assays are performed as previously described (Zeiger *et al., supra*). Briefly, methylcellulose colony assays are performed using "complete" methylcellulose or pre-B methylcellulose medium (Stem Cell Technologies, Vancouver, British Columbia, Canada) with the addition of 25 ng/mL KL (R and D Systems, Minneapolis, MN). Cytospin analyses of the resultant colonies are performed as previously described in Zeigler *et al*. The ability of the agonist antibody to augment myeloid, lymphoid and erythroid colony formation is assessed. Also, the effect of the agonist antibody on the murine bone marrow stem cell population; Lin^{lo}Sca⁺ may be evaluated.

One may select an agonist antibody which induces a statistically significant decrease in body weight and/or fat-depot weight and/or food intake in an obese mammal (*e.g.* in an *ob*/*ob* mouse). Methods for screening for such molecules are described in Levin *et al. Proc. Natl. Acad. Sci. USA* 93:1726-1730 (1996), for example. Preferred agonist antibodies are those which exert adipose-reducing effects in an obese mammal, such as the *ob*/*ob* mouse, which are in excess of those induced by reductions in food intake.

The antibody of interest herein may have the hypervariable region residues of one of the antibodies in Examples 13 and 14. Also, the invention encompasses "affinity matured" forms of these antibodies in which hypervariable region residues of these antibodies have been modified. Such affinity matured antibodies will preferably have a biological activity which is the same as or better than that of the original antibody. The affinity matured antibody may have from about 1-10, *e.g*. 5-10 deletions, insertions or substitutions (but preferably substitutions) in the hypervariable regions thereof. One useful procedure for generating affinity matured antibodies is called "alanine scanning mutagenesis" (Cunningham and Wells *Science* 244:1081-1085 (1989)). Here, one or more of the hypervariable region residue(s) are replaced by alanine or polyalanine residue(s) to affect the interaction of the amino acids with the WSX receptor. Those hypervariable region residue(s) demonstrating functional sensitivity to substitution are then refined by introducing further or other mutations at or for the sites of substitution. The ala-mutants produced this way are screened for their biological activity as described herein. Another procedure is affinity maturation using phage display (Hawkins *et al. J. Mol. Biol*. 254:889-896 (1992) and Lowman *et al. Biochemistry* 30(45):10832-10837 (1991)). Briefly, several hypervariable region sites (*e.g.* 6-7 sites) are mutated to generate all possible amino substitutions at each site. The antibody mutants thus generated are displayed in a monovalent fashion from filamentous phage particles as fusions to the gene III product of M13 packaged within each particle. The phage-displayed mutants are then screened for their biological activity (*e.g.* binding affinity).

### 6. Antibody Modifications

It may be desirable to tailor the antibody for various applications. Exemplary antibody modifications are described here.

In certain embodiments of the invention, it may be desirable to use an antibody fragment, rather than an intact antibody. In this case, it may be desirable to modify the antibody fragment in order to increase its serum half-life. This may be achieved, for example, by incorporation of a salvage receptor binding epitope into the antibody fragment. See WO96/32478 published October 17, 1996. Alternatively, the antibody may be conjugated to a nonproteinaceous polymer, such as those described above for the production of long half-life derivatives of OB protein.

Where the antibody is to be used to treat cancer for example, various modifications of the antibody (*e.g*. of a neutralizing antibody) which enhance the effectiveness of the antibody for treating cancer are contemplated herein. For example, it may be desirable to modify the antibody of the invention with respect to effector function. For example cysteine residue(s) may be introduced in the Fc region, thereby allowing interchain disulfide bond formation in this region. The homodimeric antibody thus generated may have improved internalization capability and/or increased complement-mediated cell killing and antibody-dependent cellular cytotoxicity (ADCC). See Caron *et al., J. Exp Med*. 176:1191-1195 (1992) and Shopes, B. *J. Immunol*. 148:2918-2922 (1992). Homodimeric antibodies with enhanced anti-tumor activity may also be prepared using heterobifunctional cross-linkers as described in Wolff *et al. Cancer Research* 53:2560-2565 (1993). Alternatively, an antibody can be engineered which has dual Fc regions and may thereby have enhanced complement lysis and ADCC capabilities. See Stevenson *et al. Anti-Cancer Drug Design* 3:219-230 (1989).

The invention also pertains to immunoconjugates comprising the antibody described herein conjugated to a cytotoxic agent such as a chemotherapeutic agent, toxin (*e*.*g*. an enzymatically active toxin of bacterial, fungal, plant or animal origin, or fragments thereof), or a radioactive isotope (*i*.*e*., a radioconjugate).

Chemotherapeutic agents useful in the generation of such immunoconjugates have been described above. Enzymatically active toxins and fragments thereof which can be used include diphtheria A chain, nonbinding active fragments of diphtheria toxin, exotoxin A chain (from *Pseudomonas aeruginosa*), ricin A chain, abrin A chain, modeccin A chain, alpha-sarcin, *Aleurites fordii* proteins, dianthin proteins, *Phytolaca americana* proteins (PAPI, PAPII, and PAP-S), momordica charantia inhibitor, curcin, crotin, sapaonaria officinalis inhibitor, gelonin, mitogellin, restrictocin, phenomycin, enomycin and the tricothecenes. A variety of radionuclides are available for the production of radioconjugate antibodies. Examples include ²¹²Bi, ¹³¹I, ¹³¹In, ⁹⁰Y and ¹⁸⁶Re.

Conjugates of the antibody and cytotoxic agent are made using a variety of bifunctional protein coupling agents such as N-succinimidyl-3-(2-pyridyldithiol) propionate (SPDP), iminothiolane (IT), bifunctional derivatives of imidoesters (such as dimethyl adipimidate HCL), active esters (such as disuccinimidyl suberate), aldehydes (such as glutareldehyde), bis-azido compounds (such as bis (p-azidobenzoyl) hexanediamine), bis-diazonium derivatives (such as bis-(p-diazoniumbenzoyl)-ethylenediamine), diisocyanates (such as tolyene 2,6-diisocyanate), and bis-active fluorine compounds (such as 1,5-difluoro-2,4-dinitrobenzene). For example, a ricin immunotoxin can be prepared as described in Vitetta *et al. Science* 238: 1098 (1987). Carbon-14-labeled 1-isothiocyanatobenzyl-3-methyldiethylene triaminepentaacetic acid (MX-DTPA) is an exemplary chelating agent for conjugation of radionucleotide to the antibody. See WO94/11026.

In another embodiment, the antibody may be conjugated to a "receptor" (such streptavidin) for utilization in tumor pretargeting wherein the antibody-receptor conjugate is administered to the patient, followed by removal of unbound conjugate from the circulation using a clearing agent and then administration of a "ligand" (*e.g.* avidin) which is conjugated to a cytotoxic agent (*e.g.* a radionucleotide).

The antibody may also be formulated as an immunoliposome. Liposomes containing the antibody are prepared by methods known in the art, such as described in Epstein *et al., Proc. Natl. Acad Sci. USA*, 82:3688 (1985); Hwang *et al., Proc. Natl Acad. Sci. USA*, 77:4030 (1980); and U.S. Pat. Nos. 4,485,045 and 4,544,545. Liposomes with enhanced circulation time are disclosed in U.S. Patent No. 5,013,556.

Particularly useful liposomes can be generated by the reverse phase evaporation method with a lipid composition comprising phosphatidylcholine, cholesterol and PEG-derivatized phosphatidylethanolamine (PEG-PE). Liposomes are extruded through filters of defined pore size to yield liposomes with the desired diameter. Fab' fragments of the antibody of the present invention can be conjugated to the liposomes as described in Martin *et al. J. Biol. Chem.* 257: 286-288 (1982) via a disulfide interchange reaction. A chemotherapeutic agent (such as Doxorubicin) is optionally contained within the liposome. See Gabizon *et al. J. National Cancer Inst*. 81(19)1484 (1989).

The antibody of the present invention may also be used in ADEPT by conjugating the antibody to a prodrug-activating enzyme which converts a prodrug (*e.g*. a peptidyl chemotherapeutic agent, see WO81/01145) to an active anti-cancer drug. See, for example, WO 88/07378 and U.S. Patent No. 4,975,278.

The enzyme component of the immunoconjugate useful for ADEPT includes any enzyme capable of acting on a prodrug in such a way so as to covert it into its more active, cytotoxic form.

Enzymes that are useful in the method of this invention include, but are not limited to, alkaline phosphatase useful for converting phosphate-containing prodrugs into free drugs; arylsulfatase useful for converting sulfate-containing prodrugs into free drugs; cytosine deaminase useful for converting non-toxic 5-fluorocytosine into the anti-cancer drug, 5-fluorouracil; proteases, such as serratia protease, thermolysin, subtilisin, carboxypeptidases and cathepsins (such as cathepsins B and L), that are useful for converting peptide-containing prodrugs into free drugs; D-alanylcarboxypeptidases, useful for converting prodrugs that contain D-amino acid substituents; carbohydrate-cleaving enzymes such as β-galactosidase and neuraminidase useful for converting glycosylated prodrugs into free drugs; β-lactamase useful for converting drugs derivatized with β-lactams into free drugs; and penicillin amidases, such as penicillin V amidase or penicillin G amidase, useful for converting drugs derivatized at their amine nitrogens with phenoxyacetyl or phenylacetyl groups, respectively, into free drugs. Alternatively, antibodies with enzymatic activity, also known in the art as "abzymes", can be used to convert the prodrugs of the invention into free active drugs (see, *e*.*g*., Massey, *Nature* 328: 457-458 (1987)). Antibody-abzyme conjugates can be prepared as described herein for delivery of the abzyme to a tumor cell population.

The enzymes of this invention can be covalently bound to the antibody mutant by techniques well known in the art such as the use of the heterobifunctional crosslinking reagents discussed above. Alternatively, fusion proteins comprising at least the antigen binding region of an antibody of the invention linked to at least a functionally active portion of an enzyme of the invention can be constructed using recombinant DNA techniques well known in the art (see, *e*.*g*., Neuberger *et al., Nature*, 312: 604-608 (1984)).

In other embodiments, the antibody can be covalently modified, with exemplary such modifications described above.

### E. Therapeutic Uses for WSX Receptor Ligands and Antibodies

In one embodiment, the WSX ligands (such as agonist WSX receptor antibodies) of the present invention can be used to enhance repopulation of mature blood cell lineages in mammals having undergone chemo- or radiation therapy or bone marrow transplantation therapy. Generally, the ligands will act via an enhancement of the proliferation and/or differentiation (but especially proliferation) of primitive hematopoietic cells. The ligands may similarly be useful for treating diseases characterized by a decrease in blood cells. Examples of these diseases include: anemia (including macrocytic and aplastic anemia); thrombocytopenia; hypoplasia; immune (autoimmune) thrombocytopenic purpura (ITP); and HIV induced ITP. Also, the ligands may be used to treat a patient having suffered a hemorrhage. WSX ligands may also be used to treat metabolic disorders such as obesity and diabetes mellitus, or to promote kidney, liver or lung growth and/or repair (*e.g*., in renal failure).

The WSX receptor ligands and antibodies may be administered alone or in concert with one or more cytokines. Furthermore, as an alternative to adminstration of the WSX ligand protein, gene therapy techniques are also contemplated herein.

In gene therapy applications, genes are introduced into cells in order to achieve *in vivo* synthesis of a therapeutically effective genetic product, for example for replacement of a defective gene. "Gene therapy" includes both conventional gene therapy where a lasting effect is achieved by a single treatment, and the administration of gene therapeutic agents, which involves the one time or repeated administration of a therapeutically effective DNA or mRNA. Antisense RNAs and DNAs can be used as therapeutic agents for blocking the expression of certain genes *in vivo.* It has already been shown that short antisense oligonucleotides can be imported into cells where they act as inhibitors, despite their low intracellular concentrations caused by their restricted uptake by the cell membrane. (Zamecnik *et al., Proc. Natl. Acad. Sci. USA*, 83:4143-4146 (1986)). The oligonucleotides can be modified to enhance their uptake, *e.g*., by substituting their negatively charged phosphodiester groups by uncharged groups.

There are a variety of techniques available for introducing nucleic acids into viable cells. The techniques vary depending upon whether the nucleic acid is transferred into cultured cells *in vitro,* or *in vivo* in the cells of the intended host. Techniques suitable for the transfer of nucleic acid into mammalian cells *in vitro* include the use of liposomes, electroporation, microinjection, cell fusion, DEAE-dextran, the calcium phosphate precipitation method, etc. The currently preferred *in vivo* gene transfer techniques include transfection with viral (typically retroviral) vectors and viral coat protein-liposome mediated transfection (Dzau *et al., Trends in Biotechnology* 11:205-210(1993)). In some situations it is desirable to provide the nucleic acid source with an agent that targets the target cells, such as an antibody specific for a cell surface membrane protein or the target cell, a ligand for a receptor on the target cell, etc. Where liposomes are employed, proteins which bind to a cell surface membrane protein associated with endocytosis may be used for targeting and/or to facilitate uptake, *e.g*. capsid proteins or fragments thereof tropic for a particular cell type, antibodies for proteins which undergo internalization in cycling, and proteins that target intracellular localization and enhance intracellular half-life. The technique of receptor-mediated endocytosis is described, for example, by Wu *et al., J. Biol. Chem.* 262:4429-4432 (1987); and Wagner *et al., Proc. Natl. Acad. Sci. USA* 87:3410-3414 (1990). For review of the currently known gene marking and gene therapy protocols see Anderson *et al., Science* 256:808-813 (1992).

For therapeutic applications, the WSX receptor ligands and antibodies of the invention are administered to a mammal, preferably a human, in a physiologically acceptable dosage form, including those that may be administered to a human intravenously as a bolus or by continuous infusion over a period of time, by intramuscular, intraperitoneal, intra-cerobrospinal, subcutaneous, intra-articular, intrasynovial, intrathecal, oral, topical, or inhalation routes. The WSX receptor ligands and antibodies also are suitably administered by intratumoral, peritumoral, intralesional, or perilesional routes or to the lymph, to exert local as well as systemic therapeutic effects.

Such dosage forms encompass physiologically acceptable carriers that are inherently non-toxic and non-therapeutic. Examples of such carriers include ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts, or electrolytes such as protamine sulfate, disodium hydrogen phosphate, potassium hydrogen phosphate, sodium chloride, zinc salts, colloidal silica, magnesium trisilicate, polyvinyl pyrrolidone, cellulose-based substances, and PEG. Carriers for topical or gel-based forms of WSX receptor antibodies include polysaccharides such as sodium carboxymethylcellulose or methylcellulose, polyvinylpyrrolidone, polyacrylates, polyoxyethylene-polyoxypropylene-block polymers, PEG, and wood wax alcohols. For all administrations, conventional depot forms are suitably used. Such forms include, for example, microcapsules, nano-capsules, liposomes, plasters, inhalation forms, nose sprays, sublingual tablets, and sustained-release preparations. The WSX receptor ligand or antibody will typically be formulated in such vehicles at a concentration of about 0.1 mg/ml to 100 mg/ml.

Suitable examples of sustained-release preparations include semipermeable matrices of solid hydrophobic polymers containing the WSX receptor ligand or antibody, which matrices are in the form of shaped articles, *e.g*. films, or microcapsules. Examples of sustained-release matrices include polyesters, hydrogels (for example, poly(2-hydroxyethyl-methacrylate) as described by Langer *et al*., J. Biomed. Mater. Res, 15:167-277 (1981) and Langer, Chem. Tech 12:98-105(1982), or poly(vinylalcohol), polylactides (U.S. Pat. No. 3,773,919), copolymers of L-glutamic acid and γ ethyl-L-glutamate (Sidman *et al*., Biopolymers 22:547-556 (1983)), non-degradable ethylene-vinyl acetate (Langer *et al., supra*), degradable lactic acid-glycolic acid copolymers such as the Lupron Depot™ (injectable microspheres composed of lactic acid-glycolic acid copolymer and leuprolide acetate), and poly-D-(-)-3-hydroxybutyric acid. While polymers such as ethylene-vinyl acetate and lactic acid-glycolic acid enable release of molecules for over 100 days, certain hydrogels release proteins for shorter time periods. When encapsulated WSX receptor antibodies remain in the body for a long time, they may denature or aggregate as a result of exposure to moisture at 37°C, resulting in a loss of biological activity and possible changes in immunogenicity. Rational strategies can be devised for stabilization depending on the mechanism involved. For example, if the aggregation mechanism is discovered to be intermolecular S-S bond formation through thio-disulfide interchange, stabilization may be achieved by modifying sulfhydryl residues, lyophilizing from acidic solutions, controlling moisture content, using appropriate additives, and developing specific polymer matrix compositions.

Sustained-release WSX receptor ligand or antibody compositions also include liposomally entrapped antibodies. Liposomes containing the WSX receptor ligand or antibody are prepared by methods known in the art, such as described in Epstein *et al., Proc. Natl. Acad. Sci. USA* 82:3688 (1985); Hwang *et al., Proc. Natl. Acad. Sci. USA* 77:4030 (1980); and U.S. Pat. Nos. 4,485,045 and 4,544,545. Ordinarily, the liposomes are the small (about 200-800 Angstroms) unilamelar type in which the lipid content is greater than about 30 mol.% cholesterol, the selected proportion being adjusted for the optimal WSX receptor ligand or antibody therapy. Liposomes with enhanced circulation time are disclosed in U.S. Patent No. 5,013,556.

For the prevention or treatment of disease, the appropriate dosage of WSX receptor ligand or antibody will depend on the type of disease to be treated, as defmed above, the severity and course of the disease, whether the antibodies are administered for preventive or therapeutic purposes, previous therapy, the patient's clinical history and response to the WSX receptor ligand or antibody, and the discretion of the attending physician. The WSX receptor ligand or antibody is suitably administered to the patient at one time or over a series of treatments.

Depending on the type and severity of the disease, about 1 µg/kg to 15 mg/kg of WSX receptor ligand or antibody is an initial candidate dosage for administration to the patient, whether, for example, by one or more separate administrations, or by continuous infusion. A typical daily dosage might range from about 1 µg/kg to 100 µg/kg (*e.g*. 1-50 µg/kg) or more, depending on the factors mentioned above. For example, the dose may be the same as that for other cytokines such as G-CSF, GM-CSF and EPO. For repeated administrations over several days or longer, depending on the condition, the treatment is sustained until a desired suppression of disease symptoms occurs. However, other dosage regimens may be useful. The progress of this therapy is easily monitored by conventional techniques and assays.

When one or more cytokines are co-administered with the WSX receptor ligand, lesser doses of the WSX ligand may be employed. Suitable doses of a cytokine are from about 1µg/kg to about 15mg/kg of cytokine. A typical daily dosage of the cytokine might range from about 1 µg/kg to 100 µg/kg (*e*.*g*. 1-50 µg/kg) or more. For example, the dose may be the same as that for other cytokines such as G-CSF, GM-CSF and EPO. The cytokine(s) may be administered prior to, simultaneously with, or following administration of the WSX ligand. The cytokine(s) and WSX ligand may be combined to form a pharmaceutically composition for simultaneous administration to the mammal. In certain embodiments, the amounts of WSX ligand and cytokine are such that a synergistic repopulation of blood cells (or synergistic increase in proliferation and/or differentiation of hematopoietic cells) occurs in the mammal upon administration of the WSX ligand and cytokine thereto. In other words, the coordinated action of the two or more agents (*i*.*e*. the WSX ligand and cytokine(s)) with respect to repopulation of blood cells (or proliferation/differentiation of hematopoietic cells) is greater than the sum of the individual effects of these molecules.

### F. Articles of Manufacture

An article of manufacture containing materials useful for the treatment of the conditions described above is also provided. The article of manufacture comprises a container and a label. Suitable containers include, for example, bottles, vials, syringes, and test tubes. The containers may be formed from a variety of materials such as glass or plastic. The container holds a composition which is effective for treating the condition and may have a sterile access port (for example the container may be an intravenous solution bag or a vial having a stopper pierceable by a hypodermic injection needle). The active agent in the composition is the WSX ligand. The label on, or associated with, the container indicates that the composition is used for treating the condition of choice. The article of manufacture may further comprise a second container holding a cytokine for co-administration with the WSX ligand. Further container(s) may be provided with the article of manufacture which may hold, for example, a pharmaceutically-acceptable buffer, such as phosphate-buffered saline, Ringer's solution or dextrose solution. The article of manufacture may further include other materials desirable from a commercial and user standpoint, including other buffers, diluents, filters, needles, syringes, and package inserts with instructions for use.

### G. Non-Therapeutic Uses for WSX Receptor Ligands and Antibodies

WSX receptor ligands and antibodies may be used for detection of and/or enrichment of hematopoietic stem cell/progenitor cell populations in a similar manner to that in which CD34 antibodies are presently used. For stem cell enrichment, the WSX receptor antibodies may be utilized in the techniques known in the art such as immune panning, flow cytometry or immunomagnetic beads.

In accordance with one *in vitro* application of the WSX ligands, cells comprising the WSX receptor are provided and placed in a cell culture medium. Examples of such WSX-receptor-containing cells include hematopoietic progenitor cells, such as CD34+ cells.

Suitable tissue culture media are well known to persons skilled in the art and include, but are not limited to, Minimal Essential Medium (MEM), RPMI-1640, and Dulbecco's Modified Eagle's Medium (DMEM). These tissue culture medias are commercially available from Sigma Chemical Company (St. Louis, MO) and GIBCO (Grand Island, NY). The cells are then cultured in the cell culture medium under conditions sufficient for the cells to remain viable and grow in the presence of an effective amount of WSX ligand and, optionally, further cytokines and growth factors. The cells can be cultured in a variety of ways, including culturing in a clot, agar, or liquid culture.

The cells are cultured at a physiologically acceptable temperature such as 37°C, for example, in the presence of an effective amount of WSX ligand. The amount of WSX ligand may vary, but preferably is in the range of about 10 ng/ml to about 1mg/ml. The WSX ligand can of course be added to the culture at a dose determined empirically by those in the art without undue experimentation. The concentration of WSX ligand in the culture will depend on various factors, such as the conditions under which the cells and WSX ligand are cultured. The specific temperature and duration of incubation, as well as other culture conditions, can be varied depending on such factors as, *e*.*g*., the concentration of the WSX ligand, and the type of cells and medium.

It is contemplated that using WSX ligand to enhance cell proliferation and/or differentiation *in vitro* will be useful in a variety of ways. For instance, hematopoietic cells cultured *in vitro* in the presence of WSX ligand can be infused into a mammal suffering from reduced levels of the cells. Also, the cultured hematopoietic cells may be used for gene transfer for gene therapy applications. Stable *in vitro* cultures can be also used for isolating cell-specific factors and for expression of endogenous or recombinantly introduced proteins in the cell. WSX ligand may also be used to enhance cell survival, proliferation and/or differentiation of cells which support the growth and/or differentiation of other cells in cell culture.

The WSX receptor antibodies disclosed herein are also useful as affinity purification agents. In this process, the antibodies against WSX receptor are immobilized on a suitable support, such a Sephadex resin or filter paper, using methods well known in the art. The immobilized antibody then is contacted with a sample containing the WSX receptor to be purified, and thereafter the support is washed with a suitable solvent that will remove substantially all the material in the sample except the WSX receptor, which is bound to the immobilized antibody. Finally, the support is washed with another suitable solvent, such as glycine buffer, pH 5.0, that will release the WSX receptor from the antibody.

WSX receptor antibodies may also be useful in diagnostic assays for WSX receptor, *e*.*g*., detecting its expression in specific cells, tissues, or serum. For diagnostic applications, antibodies typically will be labeled with a detectable moiety. The detectable moiety can be any one which is capable of producing, either directly or indirectly, a detectable signal. For example, the detectable moiety may be a radioisotope, such as ³H, ¹⁴C, ³²P, ³⁵S, or ¹²⁵I; a fluorescent or chemiluminescent compound, such as fluorescein isothiocyanate, rhodamine, or luciferin; radioactive isotopic labels, such as, *e*.*g*., ¹²⁵I, ³²P, ¹⁴C, or ³H; or an enzyme, such as alkaline phosphatase, beta-galactosidase, or horseradish peroxidase.

Any method known in the art for separately conjugating the polypeptide variant to the detectable moiety may be employed, including those methods described by Hunter *et al., Nature* 144:945 (1962); David *et al*., *Biochemistry* 13:1014 (1974); Pain *et al., J. Immunol. Meth* 40:219 (1981); and Nygren, *J. Histochem. and Cytochem.* 30:407 (1982).

The antibodies of disclosed herein may be employed in any known assay method, such as competitive binding assays, direct and indirect sandwich assays, and immunoprecipitation assays. Zola, *Monoclonal Antibodies: A Manual of Techniques*, pp.147-158 (CRC Press, Inc., 1987).

Competitive binding assays rely on the ability of a labeled standard to compete with the test sample analyte for binding with a limited amount of antibody. The amount of WSX receptor in the test sample is inversely proportional to the amount of standard that becomes bound to the antibodies. To facilitate determining the amount of standard that becomes bound, the antibodies generally are insolubilized before or after the competition, so that the standard and analyte that are bound to the antibodies may conveniently be separated from the standard and analyte which remain unbound.

Sandwich assays involve the use of two antibodies, each capable of binding to a different immunogenic portion, or epitope, of the protein to be detected. In a sandwich assay, the test sample analyte is bound by a first antibody which is immobilized on a solid support, and thereafter a second antibody binds to the analyte, thus forming an insoluble three-part complex. See, *e*.*g*., US Pat No. 4,376,110. The second antibody may itself be labeled with a detectable moiety (direct sandwich assays) or may be measured using an anti-immunoglobulin antibody that is labeled with a detectable moiety (indirect sandwich assay). For example, one type of sandwich assay is an ELISA assay, in which case the detectable moiety is an enzyme.

### H. Deposit of Materials

The following biological materials have been deposited with the American Type Culture Collection, 12301 Parklawn Drive, Rockville, MD, USA (ATCC):

| **Deposit Designation** | **ATCC No.** | **Deposit Date** |
|---|---|---|
| Baf3/WSX E63x7 sort | ATCC CRL 12015 | Jan 10, 1996 |
| (Baf3 cells expressing human WSX receptor variant 13.2) | | |
| | | |
| 2D7 hybridoma cell line | | |
| | | |
| 1G4 hybridoma cell line | ATCC HB-1224 | 3 Dec 11, 1996 |
| | | |
| 1E11 hybridoma cell line | | |
| | | |
| 1C11 hybridoma cell line | | |

These deposits were made under the provisions of the Budapest Treaty on the International Recognition of the Deposit of Microorganisms for the Purpose of Patent Procedure and the Regulations thereunder (Budapest Treaty). This assures maintenance of a viable culture for 30 years from the date of deposit. Each of the deposited cultures will be made available by ATCC under the terms of the Budapest Treaty, and subject to an agreement between Genentech, Inc. and ATCC, which assures (a) that access to the culture will be available during pendency of the patent application to one determined by the Commissioner to be entitled thereto under 37 CFR §1.14 and 35 USC §122, and (b) that all restrictions on the availability to the public of the culture so deposited will be irrevocably removed upon the granting of the patent.

The assignee of the present application has agreed that if any of the cultures on deposit should die or be lost or destroyed when cultivated under suitable conditions, it will be promptly replaced on notification with a viable specimen of the same culture. Availability of the deposited cell lines is not to be construed as a license to practice the invention in contravention of the rights granted under the authority of any government in accordance with its patent laws.

The foregoing written specification is considered to be sufficient to enable one skilled in the art to practice the invention. The present invention is not to be limited in scope by any culture deposited, since the deposited embodiment is intended as an illustration of one aspect of the invention and any culture that is functionally equivalent is within the scope of this invention. The deposit of material herein does not constitute an admission that the written description herein contained is inadequate to enable the practice of any aspect of the invention, including the best mode thereof, nor is it to be construed as limiting the scope of the claims to the specific illustration that it represents. Indeed, various modifications of the invention in addition to those shown and described herein will become apparent to those skilled in the art from the foregoing description and fall within the scope of the appended claims.

### III. Experimental

Below are examples of specific embodiments for carrying out the present invention. The examples are offered for illustrative purposes only, and are not intended to limit the scope of the present invention in any way.

### EXAMPLE 1

### Cloning of Human WSX Receptor

An oligonucleotide probe designated WSX.6 #1 was synthesized based upon the T73849 EST sequence. The WSX.6#1 probe was a 51mer having the following sequence:

The radiolabeled WSX.6 #1 probe was used to probe 1.2 x 10⁶ clones from a random and oligo dT primed λgt10 fetal liver library (Clontech, Palo Alto, CA). Following hybridization at42°C overnight, the filters were washed at 50°C in 0.5 x SSC and 0.1% NaDodSO₄ (SDS). From the initial screen, 10 clones were selected and upon subsequent screening 5 individual plaque pure clones were isolated. Of these 5 individual clones, four clones designated 1, 5, 6 and 9 were subcloned into pBSSK⁻ (Stratagene) following EcoRI digestion. Sequence analysis revealed clone 5 and clone 9 contained the putative initiation methionine and signal peptide. Clone 6 (designated 6.4) contained the most 3' end sequence and subsequently was used for further screening.

To obtain the full length gene, clone 6.4 (fragment Nsi-Hind III) was radiolabeled and used to screen 1.2 x 10⁶ clones from a λgt 10 library constructed from a hepatoma Hep3B cell line. This screen resulted in 24 positive clones. Following PCR analysis of the clones using λgt10 primers (F and R), the four longest clones 12.1, 13.2, 22.3, and 24.3 were isolated. These clones were subcloned into pBSSK⁻ using the EcoRI site, and following examination by restriction enzyme digest, clones 12.1 and 13.2 were submitted for sequencing. DNA sequencing was performed with the Taq dye deoxynucleotide terminator cycle sequencing kit on an automated Applied Biosystems DNA sequencer.

The assembled contiguous sequence from all the isolated clones encoded a consensus amino terminus for the newly identified polypeptide designated the WSX receptor. However, sequence analysis revealed that at least three naturally occurring variants of the WSX receptor exist which have different cytoplasmic regions. These variants appear to be differentially spliced at the lysine residue at position 891. Clone 6.4 stops 5 amino acids after Lys 891. Clone 12.1 is different from 13.2 and 6.4 following Lys 891 and encodes a putative box 2 region which is distinct from that encoded by clone 13.2. Clone 13.2 contains a potential box 1 region and following Lys 891 encodes putative box 2 and box 3 motifs. See, Baumann *et al., Mol. Cell. Biol*. 14(1):138-146 (1994).

The full length WSX gene based on the clone 13.2 cytoplasmic region putatively encodes an 1165 amino acid transmembrane protein. The 841 amino acid extracellular domain (ECD) contains two WSXWS domains. The ECD is followed by a 24 amino acid transmembrane domain and a 300 amino acid cytoplasmic region.

### EXAMPLE 2

### WSX Receptor Immunoadhesin

Using polymerase chain amplification, a WSX receptor immunoadhesin was created by engineering an in-frame fusion of the WSX receptor gene extracellular domain (WSX.ECD) with human CH2CH3(Fc)IgG (Bennett *et al., J.Biol. Chem*. 266(34):23060-23067 (1991)) at the C terminus of the ECD and cloned into pBSSK⁻ (Stratagene). For expression, the WSX-Fc was excised with Clal and BstEII and ligated into the pRK5.HuIF.grbhIgG Genenase I vector (Beck *et al*., *Molecular Immunology* 31(17):1335-1344 (1994)), to create the plasmid pRK5.WSX-IgG Genenase I. This plasmid was transiently transfected into 293 cells using standard calcium phosphate transfection techniques. The transfected cells were cultured at 37°C in 5% CO₂ in DMEM F12 50:50 supplemented with 10% FBS, 100mM HEPES (pH 7.2) and 1mM glutamine. The WSX receptor immunoadhesin was purified using a ProSepA™ protein A column.

### EXAMPLE 3

### Antibody Production

In order to raise antibodies against the WSX receptor, the WSX receptor immunoadhesin of Example 2 was used to inoculate rabbits to raise polyclonal antibodies and mice to raise monoclonal antibodies using conventional technology.

### EXAMPLE 4

### Generation of a Cell Line Expressing WSX Receptor

The nucleic acid encoding full length WSX receptor variant 13.2 was inserted in the pRKtkNeo plasmid (Holmes *et al., Science* 253:1278-1280 (1991)). 100 µgs of the pRKtkNeo.WSX plasmid thus generated was linearized, ethanol precipitated and resuspended in 100 µL of RPMI 1640. 7 x 10⁶ Baf3 cells (5 x 10⁵/ml) were suspended in 900 µL of RPMI and added to the linearized plasmid. Following electroporation at 325V, 1180 µF using a BRL electroporation apparatus, the cells were plated into 15 mls of RPMI 1640 containing 5% WEHI3B conditioned media and 15% serum. 48 hours later cells were selected in 2mg/ml G418.

To obtain the Baf3/WSX cell line expressing WSX receptor variant 13.2, the G418 selected clones were analyzed by FACS using the rabbit polyclonal antisera raised against the WSX-Fc chimeric protein as described above. The highest expressing clone (designated E6) was sorted by FACS to maintain a population with a high level of WSX receptor expression.

### EXAMPLE 5

### Role of WSX Receptor in Cellular Proliferation

The proliferative potentials of WSX receptor variants 13.2 and 12.1 were tested by constructing human growth hormone receptor-WSX receptor (GH-WSX) fusions encoding chimeric proteins consisting of the GH receptor extracellular and transmembrane domains and the WSX receptor variant 13.2 or 12.1 intracellular domains. These chimeric gene fusions were transfected into the IL-3 dependent cell line Baf3. The ability of the GH-WSX transfected Baf3 cells to respond to exogenous growth hormone (GH) was tested in a thymidine incorporation assay. As can be seen in Figs. 6 and 8, the GH-WSX receptor variant 13.2 chimera was capable of increasing thymidine uptake in the transfected Baf3 cells, thus indicating the proliferative potential of the WSX receptor variant 13.2. However, WSX receptor variant 12.1 was unable to transmit a proliferative signal in this experiment (Fig. 8).

### Materials and Methods

Recombinant PCR was used to generate the chimeric receptors containing the extracellular and transmembrane domains of the hGH receptor and the cytoplasmic domain of either WSX receptor variant 12.1 or variant 13.2. In short, the cytoplasmic domain of either variant 12.1 or 13.2 beginning with Arg at amino acid 866 and extending down to amino acid 958 or amino acid 1165 respectively, was fused in frame, by sequential PCR, to the hGH receptor extracellular and transmembrane domain beginning with Met at amino acid 18 and extending down to Arg at amino acid 274. The GH-WSX chimera was constructed by first using PCR to generate the extracellular and transmembrane domain of the human GH receptor. The 3' end primer used for this PCR contained 20 nucleotides at the 5' end of the primer corresponding to the first 20 nucleotides of the WSX cytoplasmic domain. The 3' end of the chimera was generated using PCR where the 5' end primer contained the last 19 nucleotides of the human GH receptor transmembrane domain. To generate the full length chimera, the 5' end of the human GH receptor product was combined with the 3' end WSX receptor cytoplasmic PCR product and subsequently amplified to create a fusion of the two products.

This chimeric fusion was digested with ClaI and XbaI and ligated to pRKtkNeo (Holmes *et al., Science* 253:1278-1280 (1991)) to create the chimeric expression vector. The IL-3 dependent cell line Baf3 was then electroporated with this hGH/WSX chimeric expression vector.

Briefly, 100µg of the pRKtkNeo/GH.WSX plasmid was linearized, ethanol precipitated and resuspended in 100 µL of RPMI 1640. 7 x 10⁶ Baf3 cells (5 x 105/ml)were suspended in 900 µL of RPMI and added to the linearized plasmid. Following electroporation at 325V, 1180 µF using a BRL electroporation apparatus, the cells were plated into 15 mls of RPMI 1640 containing 5% wehi conditioned media and 15% serum. 48 hours later, cells were selected in 2mg/ml G418.

To obtain the Baf3/GH.WSX cell lines, the G418 selected cells were FACS sorted using an anti-human GH mAb (3B7) at 1µg/ml. The top 10% expressing cells were selected and expanded.

### EXAMPLE 6

### Expression Analysis of the WSX Receptor

The expression profile of the WSX receptor was initially examined by Northern analysis. Northern blots of human fetal or adult tissue mRNA were obtained from Clontech (Palo Alto, California). A transcript of approximately 6 kb was detected in human fetal lung, liver and kidney. In the adult, low level expression was detected in a variety of tissues including liver, placenta, lung skeletal muscle, kidney, ovary, prostate and small intestine.

PCR analysis of human cord blood identified transcripts in CD34⁺ subfraction. By PCR analysis, all three variants of the WSX receptor were present in CD34⁺ cells. The CD34⁻ subfraction appeared negative by this same PCR analysis.

By PCR analysis, both the 6.4 variant and 13.2 variant were evident in the AA4⁺Sca⁺Kit⁺ (flASK) cell population isolated from the mid-gestation fetal liver as described in Zeigler *et al., Blood* 84:2422-2430 (1994). No clones containing the 12.1 variant cytoplasmic tail have been isolated from murine tissues.

Human B cells isolated from peripheral blood using anti-CD19/20 antibodies were also positive for short form (6.4 variant) and long from (13.2 variant) receptor mRNA expression.

The WSX receptor appears to be expressed on both progenitor and more mature hematopoietic cells.

### EXAMPLE 7

### Cloning of Murine WSX Receptor

The human WSX receptor was used as a probe to isolate murine WSX receptor. The pRKtkNeo.WSX plasmid of Example 4 was digested using Sspl. This Sspl fragment (1624 bps) was isolated, and radiolabelled, and used to screen a murine liver λgt10 library (Clontech). This resulted in 4 positive clones which were isolated and sequenced after sub-cloning into pBSSK⁻ via EcoRI digestion. The resultant clones, designated 1, 2, 3, 4 showed homology to the extracellular domain of the human WSX receptor; the contiguous sequences resulting from these clones extended from the initiation methionine to tryptophan at position 783. The overall similarity of human WSX receptor and murine WSX receptor is 73 % over this region of the respective extracellular domains (see Figs. 4A-B).

### EXAMPLE 8

### The Role of WSX Receptor in Hematopoietic Cell Proliferation

The presence of the WSX receptor in the enriched human stem cell population CD34⁺ from cord blood is indicative of a potential role for this receptor in stem cell/progenitor cell proliferation. The proliferation of CD34⁺ human blood cells in methylcellulose media (Stem Cell Technologies) was determined in the presence or absence of WSX receptor antisense oligonucleotides. These experiments were also repeated in the murine hematopoietic system using AA4⁺ Sca⁺ Kit⁺ stem cells from the murine fetal liver. In both instances, the antisense oligonucleotides statistically significantly inhibited colony formation from the hematopoietic progenitor cells. See Table 1 below. The anti-proliferative effects were most pronounced using the -20 antisense and the +85 antisense oligonucleotide constructs. This inhibition was not lineage specific to any particular myeloid lineage that resulted from the progenitor expansion. The principal effect of the antisense oligonucleotides was a reduction of overall colony numbers. The size of the individual colonies was also reduced.

Antisense oligonucleotide experiments using both human and murine stem cells demonstrated an inhibition of myeloid colony formation. Although, the reduction in myelopoiesis observed in these assays could be prevented by the additional inclusion of G-CSF and GM-CSF in the culture medium. These data serve to illustrate the redundancy of cytokine action in the myelopoietic compartment.

**TABLE 1**

| **EXPERIMENT** | **OLIGO** | **AVG. COLONY #** | **% INHIBITION** |
|---|---|---|---|
| Human Cord Blood (KL) | (-20)AS | 32 | |
| | (-20)S | 100 | 70 |
| | (-20)SCR | 114 | |
| | (+85)AS | 80 | |
| | (+85)S | 123 | 38 |
| | (+85)SCR | 138 | |
| | Control | 158 | |
| Human Cord Blood | (-20)AS | 78 | |
| (IL-3, IL-6, KL) | (-20)S | 188 | 54 |
| | (-20)SCR | 151 | |
| | (+85)AS | 167 | |
| | (+85)S | 195 | 18 |
| | (+85)SCR | 213 | |
| | Control | 266 | |
| Human Cord Blood (KL) | (-20)AS | 42 | |
| | (-20)S | 146 | 69 |
| | (-20)SCR | 121 | |
| | (+85)AS | 123 | |
| | (+85)S | 162 | 23 |
| | (+85)SCR | 156 | |
| | Control | 145 | |
| Murine Fetal Liver (KL) | (+84)AS | 33 | |
| | (+84)S | 86 | 54 |
| | (+84)SCR | 57 | |
| | (-20)AS | 27 | |
| | (-20)S | 126 | 71 |
| | (-20)SCR | 60 | |
| | (-99)AS | 109 | |
| | (-99)S | 93 | 0 |
| | (-99)SCR | 109 | |
| | Control | 121 | |
| Murine Fetal Liver (KL) | (-213)AS | 51 | |
| | (-213)S | 60 | 10 |
| | (-213)SCR | 53 | |
| | (+211)AS | 58 | |
| | (+211)S | 54 | 3 |
| | (+211)SCR | 66 | |
| | Control | 59 | |

### Materials and Methods

*Human stem cells:* Human umbilical cord blood was collected in PBS/Heparin (1000µ/ml). The mononuclear fraction was separated using a dextran gradient and any remaining red blood cells lysed in 20 mM NH₄ Cl. CD34⁺ cells were isolated using CD34⁺ immunomagnetic beads (Miltenyi, CA). These isolated CD34⁺ cells were found to be 90-97% CD34⁺ by FACS analysis.

*Murine stem cells*: Midgestation fetal liver were harvested and positively selected for the AA4⁻ antigen by immune panning. The AA4⁻ positive fraction was then further enriched for stem cell content by FACS isolation of the AA4⁺ Sca⁺ Kit⁺ fraction.

*Antisense experiments:* Oligodeoxynucleotides were synthesized against regions of the human or murine WSX receptors. For each oligonucleotide chosen, antisense (AS), sense (S) and scrambled (SCR) versions were synthesized (see Fig. 7). + or - indicates position relative the initiation methionine of the WSX receptor. CD34⁺ or AA4⁺ Sca⁺ Kit⁺ cells were incubated at a concentration of 10³/ml in 50:50 DMEM/F12 media supplemented with 10% FBS, L-glutamine, and GIBCO^{TM} lipid concentrate containing either sense, antisense or scrambled oligonucleotides at a concentration of 70 µg/ml. After 16 hours, a second aliquot of the respective oligonucleotide was added (35 µg/ml) and the cells incubated for a further 6 hours.

*Colony assays:* 5000 cells from each of the above conditions were aliquoted into 5 ml of methylcellulose (Stem Cell Technologies) containing kit ligand (KL) (25 ng/ml), interleukin-3 (IL-3) (25 ng/ml) and interleukin-6 (IL-6) (50 ng/ml). The methylcellulose cultures were then incubated at 37° C for 14 days and the resultant colonies counted and phenotyped. All assays were performed in triplicate.

### EXAMPLE 9

### WSX Receptor Variant 13.2 is a Receptor for OB Protein

The WSX receptor variant 13.2 has essentially the same amino acid sequence as the recently cloned leptin (OB) receptor. See Tartaglia *et al., Cell* 83:1263-1271 (1995). OB protein was able to stimulate thymidine incorporation in Baf3 cells transfected with WSX receptor variant 13.2 as described in Example 4 (See Fig. 9).

OB protein expression in hematopoietic cells was studied. Oligonucleotide primers designed specifically against the OB protein illustrated the presence of this ligand in fetal liver and fetal brain as well as in two fetal liver stromal cell lines, designated 10-6 and 7-4. Both of these immortalized stromal cell lines have been demonstrated to support both myeloid and lymphoid proliferation of stem cell populations (Zeigler *et al*., *Blood* 84:2422-2430 (1994)).

### EXAMPLE 10

### Role of OB Protein in Hematopoiesis

To examine the hematopoietic activity of OB protein, a variety of *in vitro* assays were performed.

Murine fetal liver flASK stem cells were isolated from the midgestational fetal liver as described in Zeigler *et al., Blood* 84:2422-2430 (1994) and studied in stem cell suspension culture or methylcellulose assays.

For the stem cell suspension cultures, twenty thousand of the fLASK cells were seeded in individual wells in a 12 well format in DMEM 4.5/F12 media supplemented with 10% heat inactivated fetal calf serum (Hyclone, Logan, UT) and L-glutamine. Growth factors were added at the following concentrations: kit ligand (KL) at 25 ng/mL, interleukin-3 (IL-3) at 25 ng/mL, interleukin-6 (IL-6) at 50 ng/mL, G-CSF at 100 ng/mL, GM-CSF at 100 ng/mL, EPO at 2U/mL, interleukin-7 (IL-7) at 100 ng/mL (all growth factors from R and D Systems, Minneapolis, MN). OB protein was added at 100 ng/mL unless indicated otherwise. Recombinant OB protein was produced as described in Levin *et al., Proc. Natl. Acad. Sci. (USA)* 93:1726-1730 (1996).

In keeping with its ability to transduce a proliferative signal in Baf3 cells (see previous Example), OB protein dramatically stimulated the expansion of flASK cells grown in suspension culture in the presence of kit ligand (Fig. 10A). The addition of OB protein alone to these suspension cultures was unable to effect survival of the hematopoietic stem cells (HSCs). When a variety of hematopoietic growth factors in suspension culture assays were tested, the main synergy of OB protein appeared to be with KL, GM-CSF and IL-3 (Table 2). No preferential expansion of any particular lineage was observed from cytospin analysis of the resultant cultures.

**TABLE 2**

| **Factor** | **KL** | **KL+OB protein** | **OB protein** |
|---|---|---|---|
| N/A | 128+/-9 | 192+/-13 | |
| G-CSF | 131+/-3 | 177+/-8 | 30+/-5 |
| GM-CSF | 148+/-4 | 165+/-6 | 134+/-10 |
| IL-3 | 189+/-7 | 187+/-4 | 144+/- |
| IL-6 | 112+/-4 | 198+/-5 | 32+/-3 |
| EPO | 121+/-3 | 177+/-8 | 30+/-6 |
| IL-3 & IL-6 | 112+/-12 | 198+/-7 | 32+/-7 |

flASK stem cells were isolated. Twenty thousand cells were plated in suspension culture with the relevant growth factor combination. Cells were harvested and counted after 7 days. Cell numbers are presented x 10³. Assays were performed in triplicate and repeated in two independent experiments.

Methylcellulose assays were performed as previously described (Zeiger *et al*., *supra*). Briefly, methylcellulose colony assays were performed using "complete" methylcellulose or pre-B methylcellulose medium (Stem Cell Technologies, Vancouver, British Columbia, Canada) with the addition of 25 ng/mL KL (R and D Systems, Minneapolis, MN). Cytospin analyses of the resultant colonies were performed as previously described in Zeigler *et al*.

When these methylcellulose assays were employed, OB protein augmented myeloid colony formation and dramatically increased lymphoid and erythroid colony formation (Figs. 10B and 10C) which demonstrates that OB protein can act on very early cells of the hematopoietic lineage. Importantly, the hematopoietic activity of OB protein was not confined to fetal liver stem cells, the murine bone marrow stem cell population; Lin^{lo}Sca⁺ also proliferated in response to OB protein (KL: 5 fold expansion, KL and OB protein: 10 fold expansion).

Further hematopoietic analysis of the role of the WSX receptor was carried out by examining hematopoietic defects in the *db*/*db* mouse.

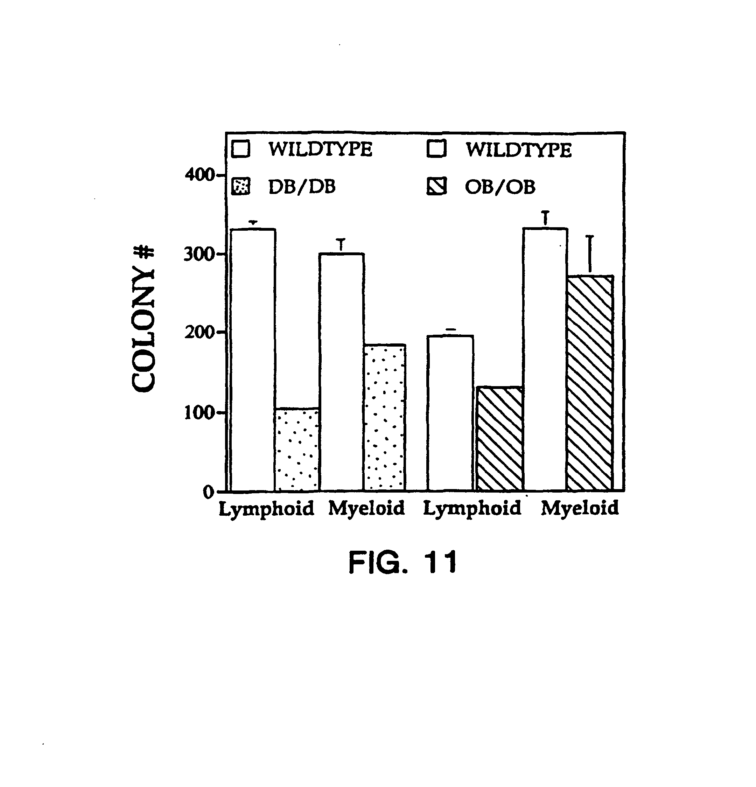

These defects were assessed by measuring the proliferative potential of *db*/*db* homozygous mutant marrow. Under conditions favoring either myeloid (Humphries *et al*., *Proc. Natl. Acad. Sci. (USA)* 78:3629-3633 (1981)) or lymphoid (McNiece *et al., J. Immunol*. 146:3785-90 (1991)) expansion, the colony forming potential of the *db*/*db* marrow was significantly reduced when compared to the wild-type control marrow (Fig. 11). This was particularly evident when the comparison was made under pre-B methylcellulose conditions where KL and IL-7 are used to drive lymphopoiesis (McNiece *et al., supra*). Corresponding analysis of the complementary mouse mutation *ob*/*ob*, which is deficient in the production of OB protein (Zhang *et al., Nature* 372:425-431 (1994)), also indicated that the lymphoproliferative capacity is compromised in the absence of a functional OB protein signalling pathway (Fig. 11). However, this reduction was less than the reduction observed using *db*/*db* marrow.

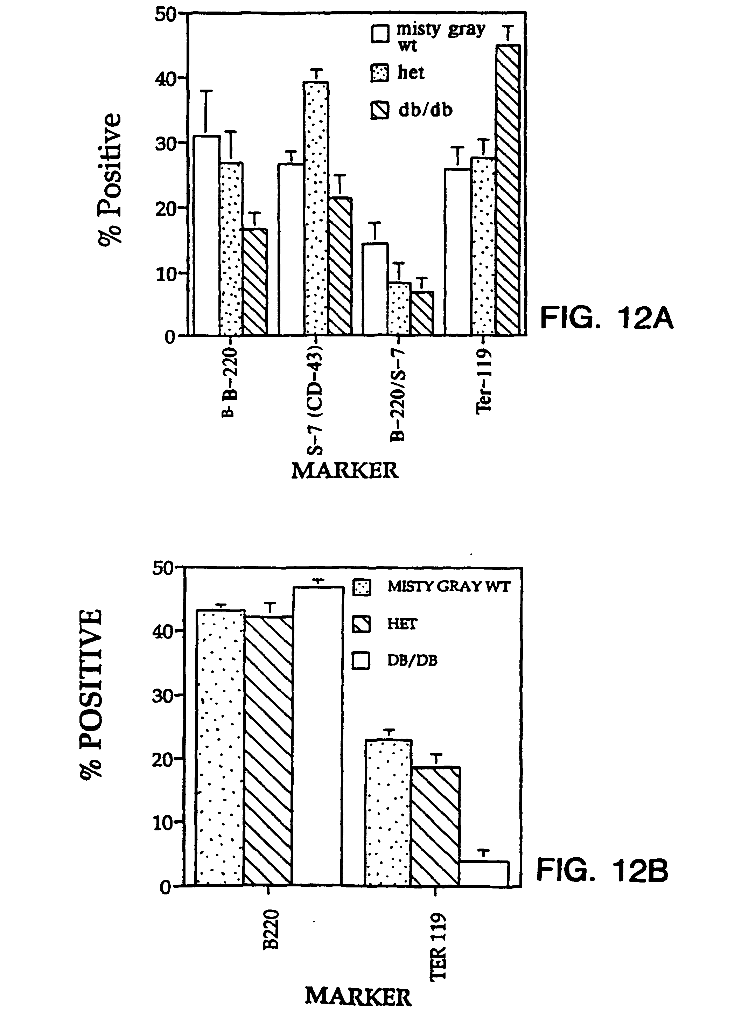

Analysis of the cellular profile of the *db*/*db* and wild-type marrow revealed significant differences between the two. Overall cellularity of the *db*/*db* marrow was unchanged. However, when various B cell populations in the *db*/*db* marrow were examined, both decreased levels of B220⁺ and B220⁺/CD43⁺ cells were found. B220⁺ cells represent all B cell lineages while CD43 is considered to be expressed preferentially on the earliest cells of the B cell hierarchy (Hardy *et al., J. Exp. Med*. 173:1213-25 (1991)). No differences were observed between the CD4/CD8 staining profiles of the two groups. The TER119 (a red cell lineage marker) population was increased in the *db*/*db* marrow (Fig. 12A).

Comparison of the spleens from the two groups revealed a significant decrease in both tissue weight and cellularity of the *db*/*db* mice compared to the homozygote misty gray controls (0.063 ±0.009 g vs. 0.037±0.006 g and 1.10x10⁷±1x10⁴ vs. 4.3x10⁶±10³ cells > p0.05). This decreased cellularity in the *db* spleen was reflected in a marked reduction in TER119 staining (Fig. 12B). This result appears to confirm the synergy demonstrated between OB protein and EPO and points to a role for OB protein in the regulation of erythropoiesis.

Examination of the hematopoietic compartment of the *db*/*db* mouse *in vivo* demonstrated a significant reduction in peripheral blood lymphocytes when compared to heterozygote or wild-type controls. *Db*/*db* mice fail to regulate blood glucose levels and become diabetic at approximately 6-8 weeks of age; therefore, peripheral blood counts as the animals matured were followed.

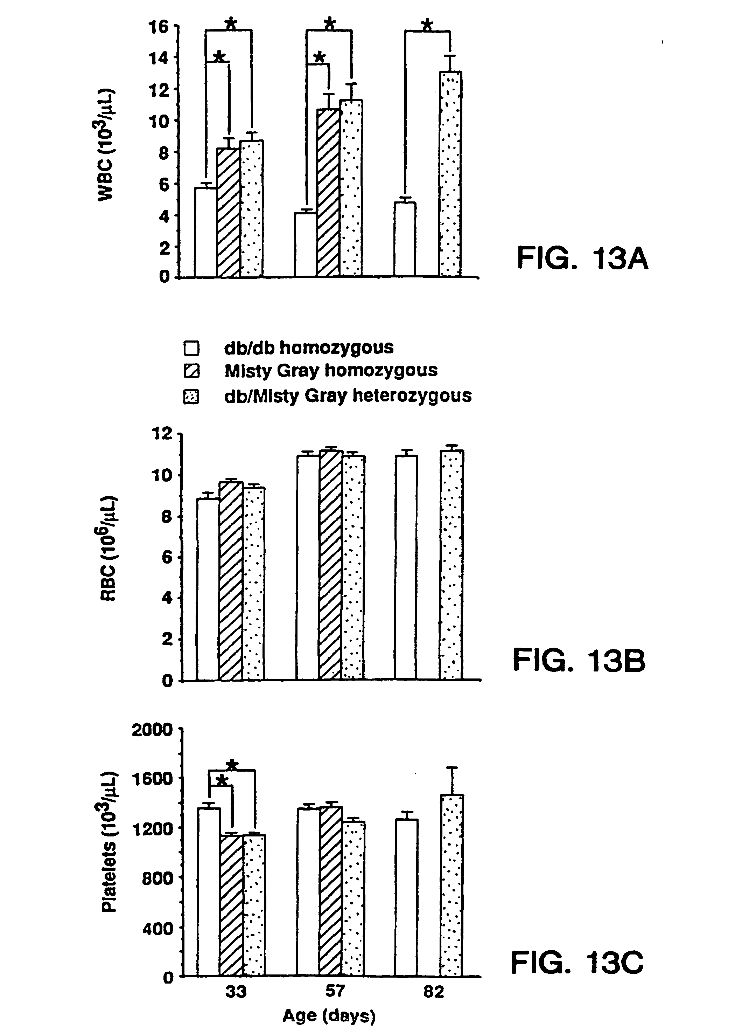

For procurement of blood samples, prior to the experiment and at time points throughout the study, 40 µL of blood was taken from the orbital sinus and immediately diluted into 10 mL of diluent to prevent clotting. The complete blood count from each blood sample was measured on a Serrono Baker system 9018 blood analyzer within 60 min. of collection. Only half the animals in each dose group were bled on any given day, thus, each animal was bled on alternate time points. Blood glucose levels were measured in orbital sinus blood samples using One Touch glucose meters and test strips (Johnson and Johnson). The results of this experiment are shown in Figs. 13A-C.

This analysis demonstrated that peripheral blood lymphocytes are significantly reduced at all time points compared to control animals and that the peripheral lymphocyte population of the *db*/*db* mouse does not change significantly with age. FACS analysis revealed that the decreased lymphocyte population represented a decrease in both B220⁺ cells and CD4/CD8 cells. Both erythrocyte and platelets are at wild-type levels throughout all time periods examined. The peripheral blood lymphocyte levels in *ob*/*ob* homozygous mutant mice were unchanged from wild-type controls.

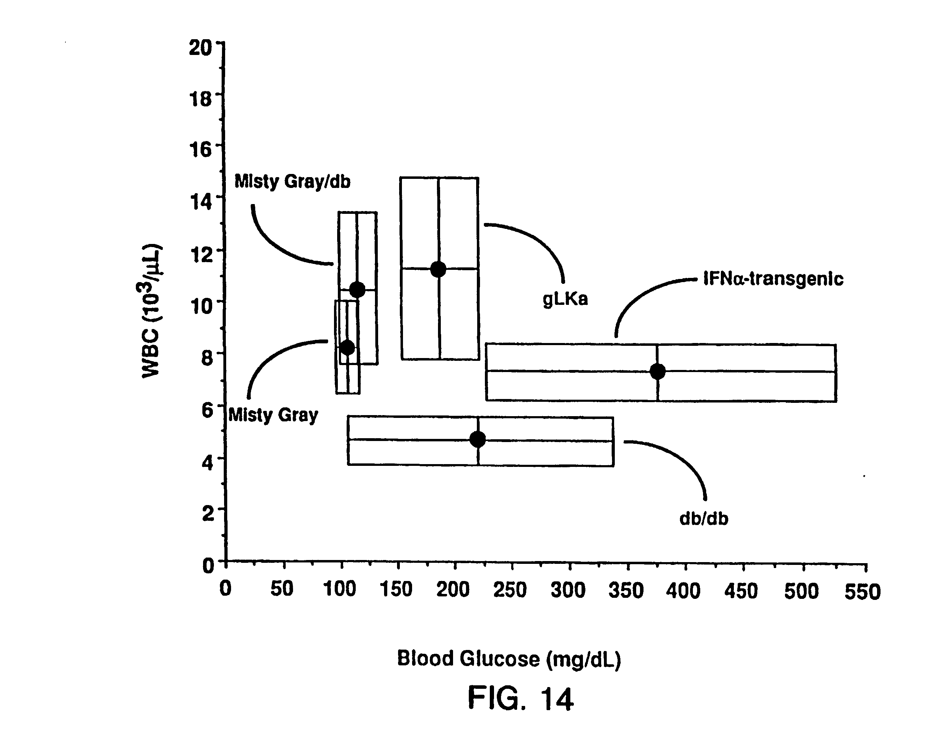

Hematopoietic analysis of the *db*/*db* mouse can be complicated by the onset of diabetes. Therefore, the impact of high glucose levels on lymphopoiesis was examined by comparing the peripheral blood profiles and blood glucose levels in two other diabetic models, the glucokinase knockout heterozygote mouse (Grupe *et al., Cell* 83:69-78 (1995)) and the IFN-α transgenic mouse (Stewart *et al., Science* 260:1942-6 (1993)). Comparison of peripheral lymphocytes and blood glucose in *db*/*db* mice, their appropriate controls and the high glucose models illustrated no relationship between blood-glucose and lymphocyte counts (Fig. 14). These results suggest therefore that the lymphoid defects observed in the *db*/*db* mouse are directly attributed to the hematopoietic function of the OB protein signalling pathway.

To test the capacity of the *db*/*db* hematopoietic compartment to respond to challenge, the *db*/*db* mice and controls were subjected to sub-lethal irradiation C57BLKS/J *db*/*db,* C57BLKS/Jm⁺/db, and C57BLKS/J ⁺m/⁺m mice were subjected to sub-lethal whole body irradiation (750 cGy, 190 cGy/min) as a single dose from a ¹³⁷Cs source. Ten animals were used per experimental group. The kinetics of hematopoietic recovery were then followed by monitoring the peripheral blood during the recovery phase. This experiment illustrated the inability of the *db*/*db* hematopoietic system to fully recover the lymphopoietic compartment of the peripheral blood 35 days post-irradiation. Platelet levels in these mice followed the same recovery kinetics as controls, however the reduction in erythrocytes lagged behind controls by 7-10 days. This finding may reflect the increased TER 119 population found in the marrow of the *db*/*db* mice (Fig. 12A).

### Materials and Methods

Bone marrow, spleens and peripheral blood was harvested from the diabetic mouse strains: C57BLKS/J *db*/*db* (mutant), C57BLKS/J m+/db (lean heterozygote control littermate), C57BLKS/J+m/+m (lean homozygote misty gray coat control littermate) and the obese mouse strains: *C57BL*/*6J-ob*/*ob* (mutant) and the C57BL/6J-ob/+(lean littermate control). All strains from the Jackson Laboratory, Bar Harbor, ME. A minimum of five animals were used per experimental group. Femurs were flushed with Hank's balanced salt solution (HBSS) plus 2% FCS and a single cell suspension was made of the bone marrow cells. Spleens were harvested and the splenic capsule was ruptured and filtered through a nylon mesh. Peripheral blood was collected through the retro-orbital sinus in phosphate buffered saline (PBS) with 10U/mL heparin and Immol EDTA and processed as previously described. The bone marrow, splenocytes and peripheral blood were then stained with the monoclonal antibodies against the following antigens: B220/CD45R (Pan B cell) FITC antimouse, TER-119/erythroid cell R-PE antimouse, CD4 (L3T4), FITC antimouse, CD8 (Ly 3.2), FITC antimouse, and slgM (Igh-6b), FITC antimouse (All monoclonals from Pharmigen, San Diego, CA). The appropriate isotype controls were included in each experiment. For methylcellulose assays, the bone marrow from five animals per group was pooled and 100,000 cell aliquots from each group used for each assay point.

### EXAMPLE 11

### Expression of OB-immunoadhesin

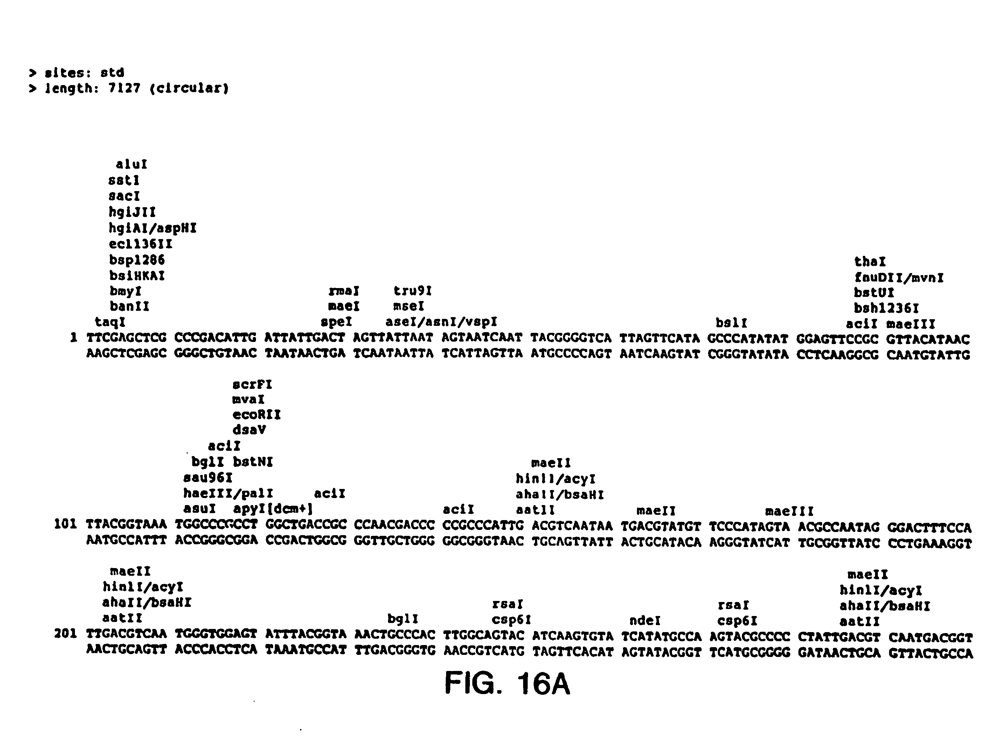

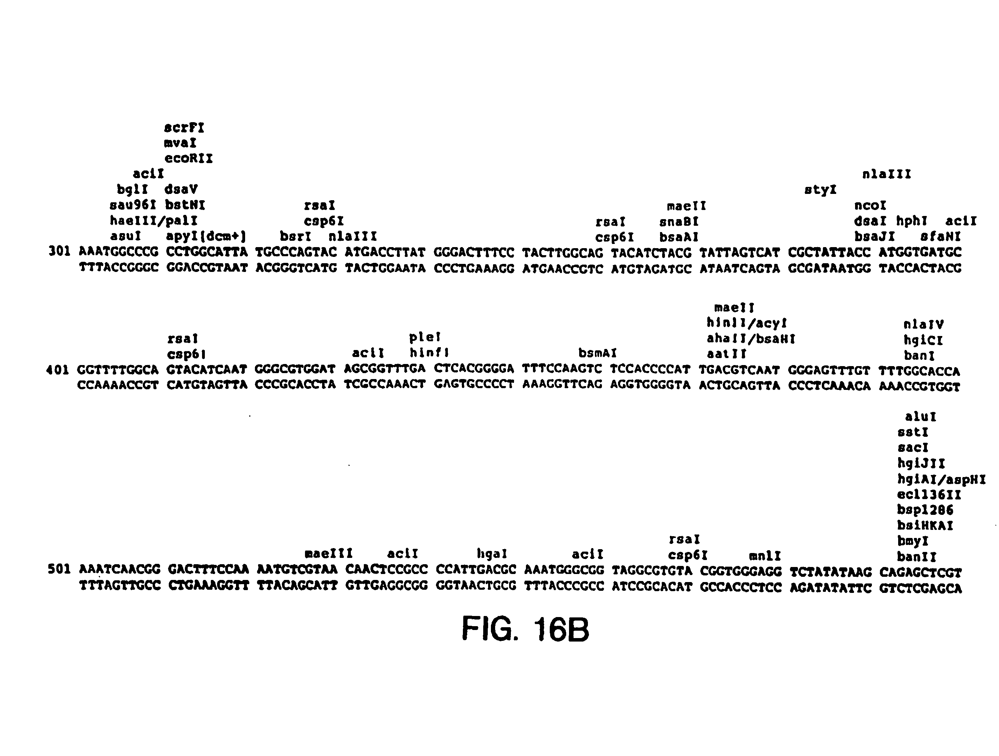

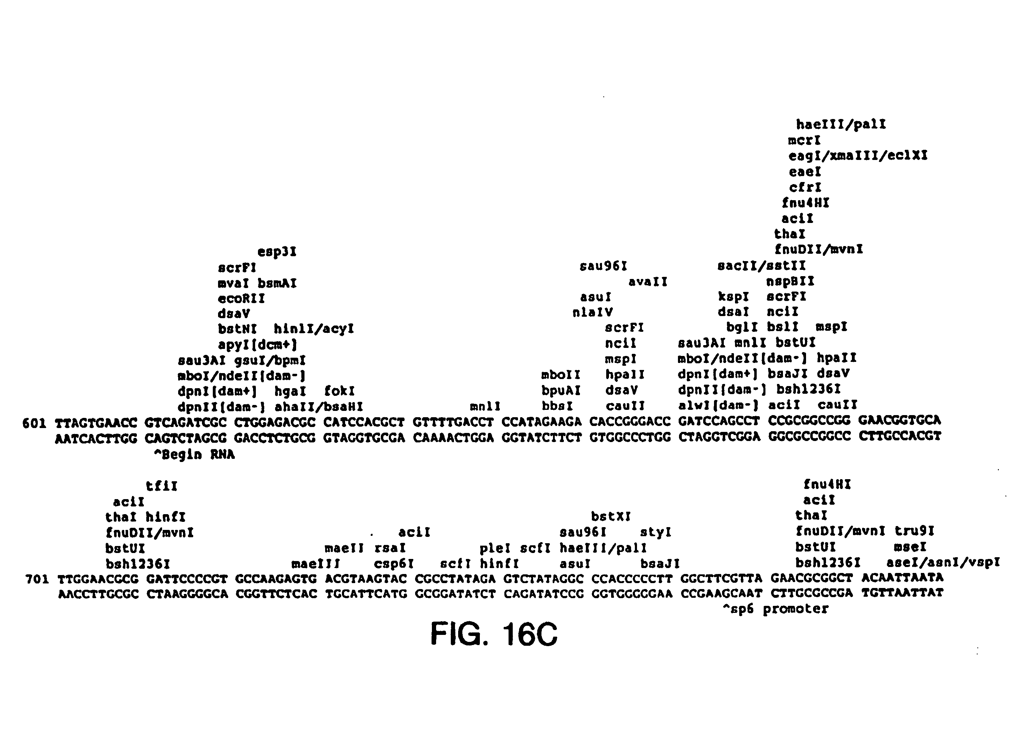

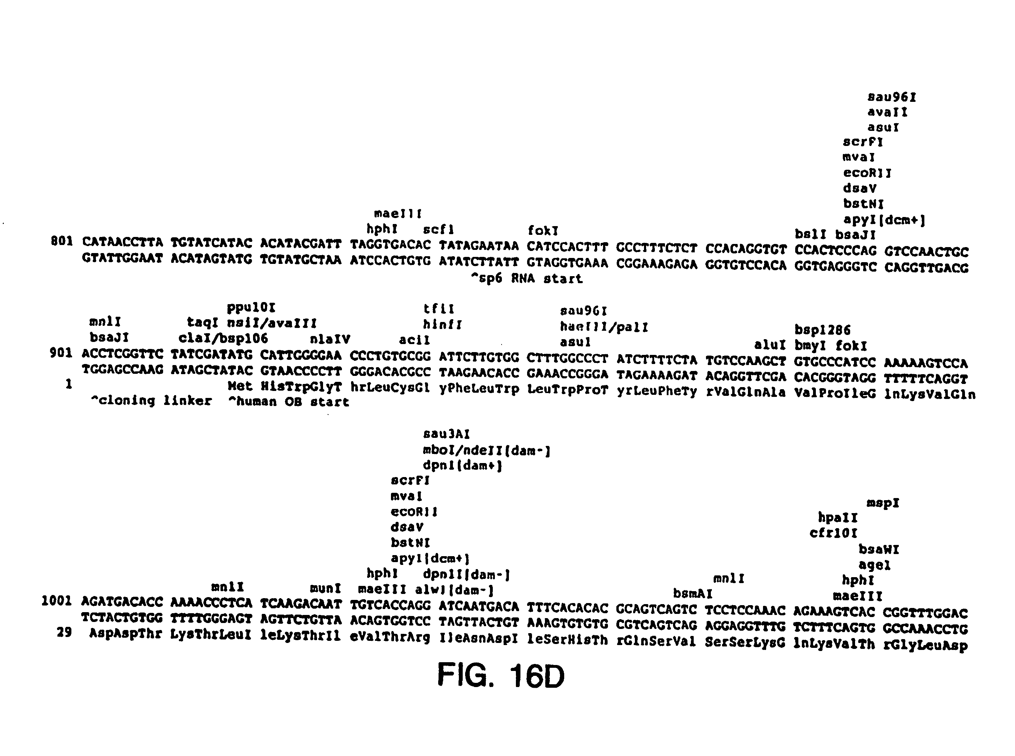

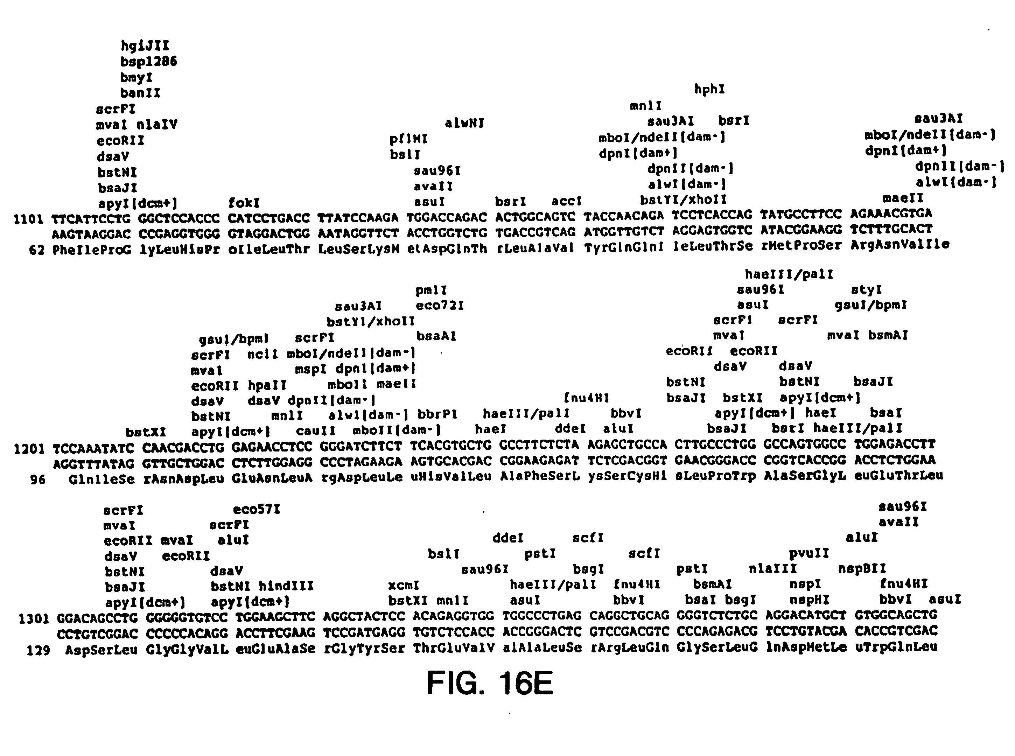

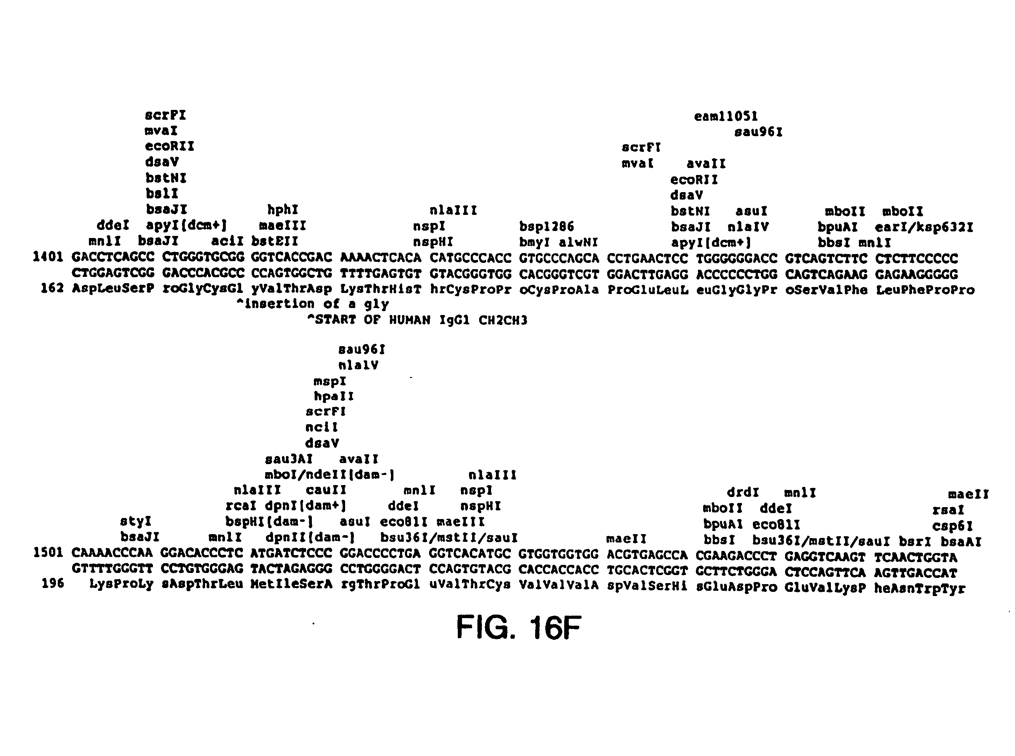

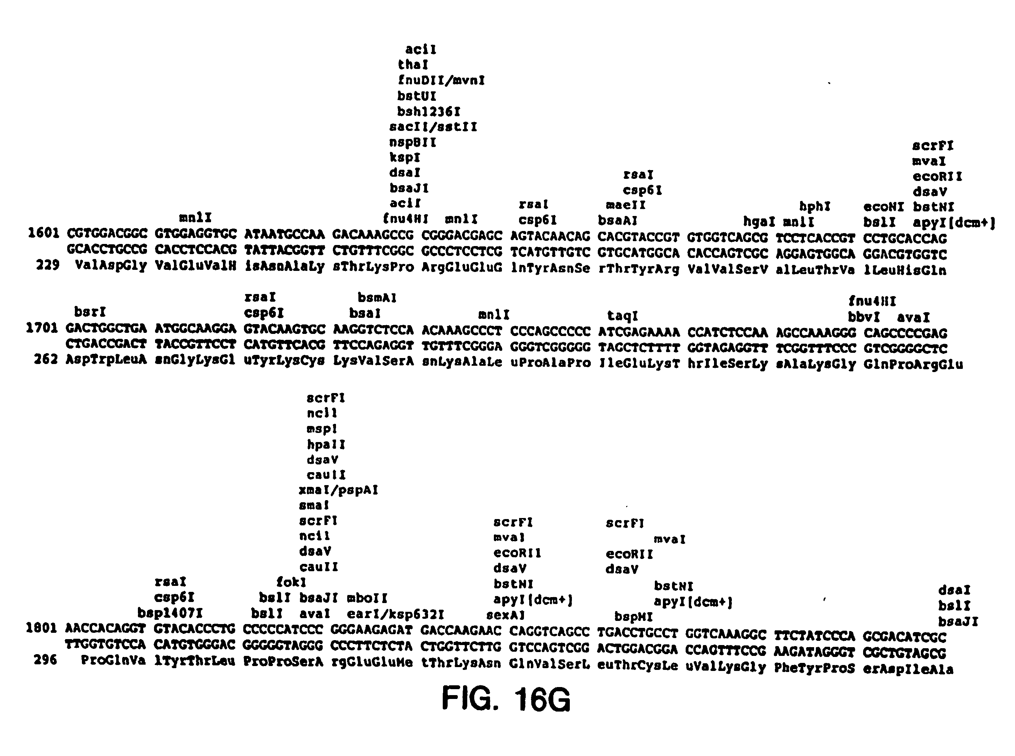

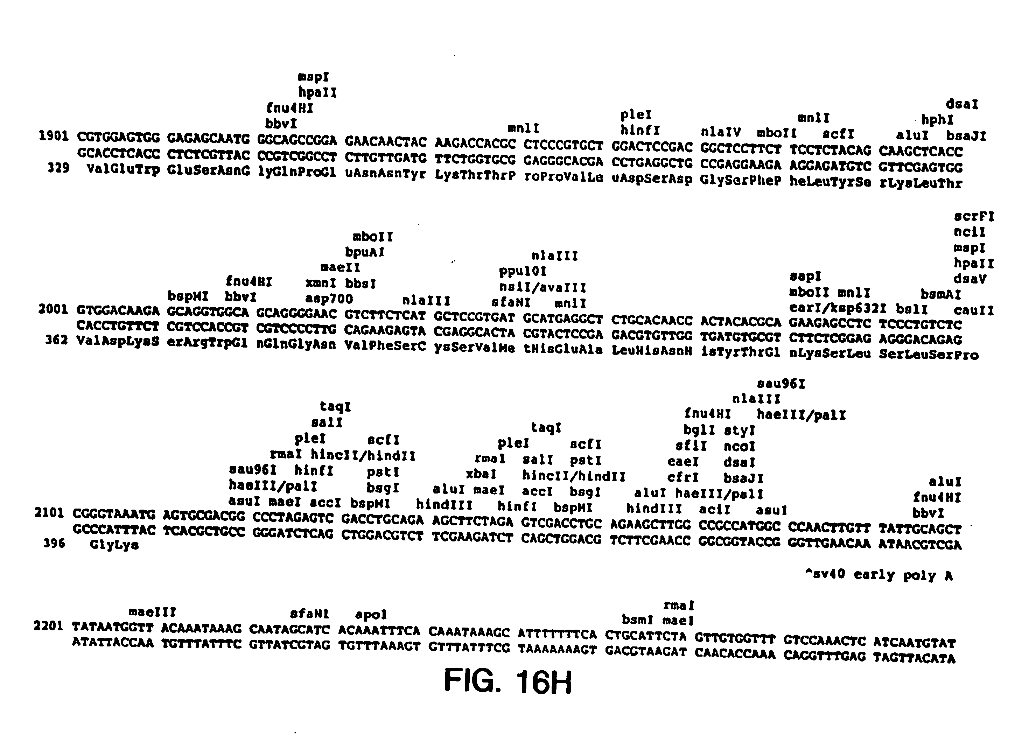

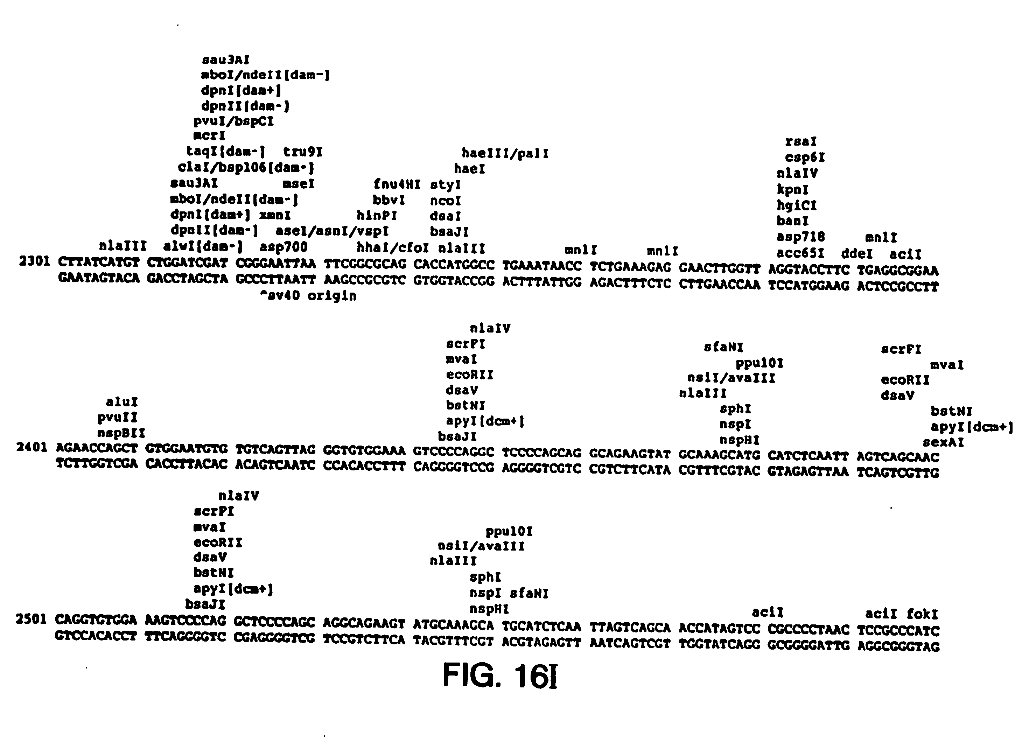

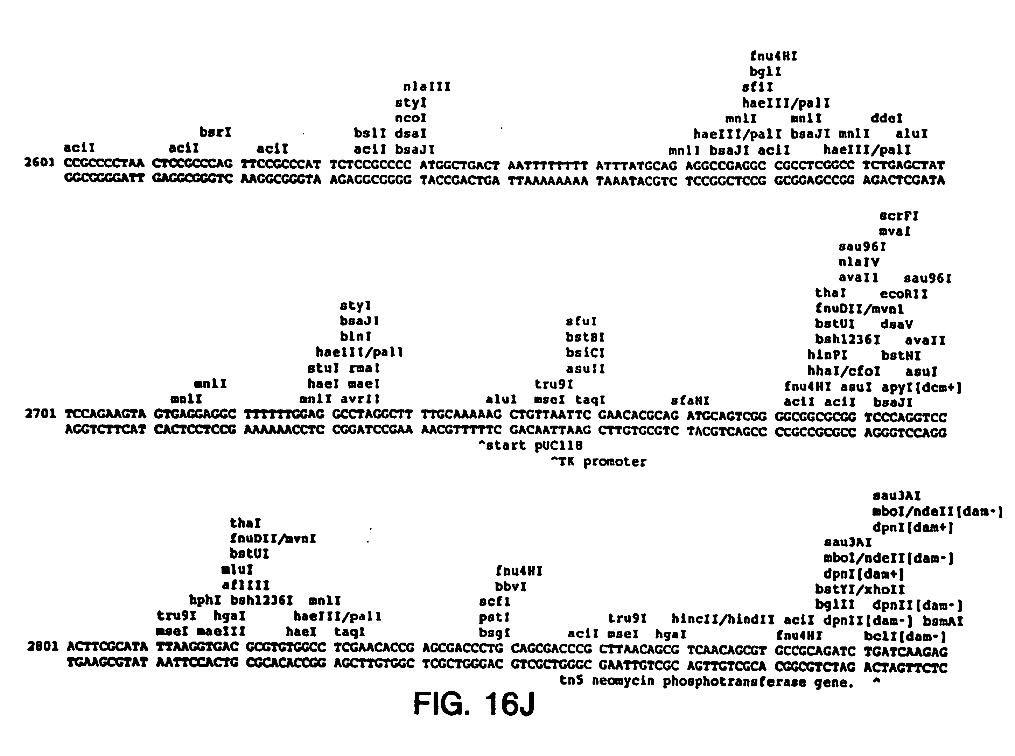

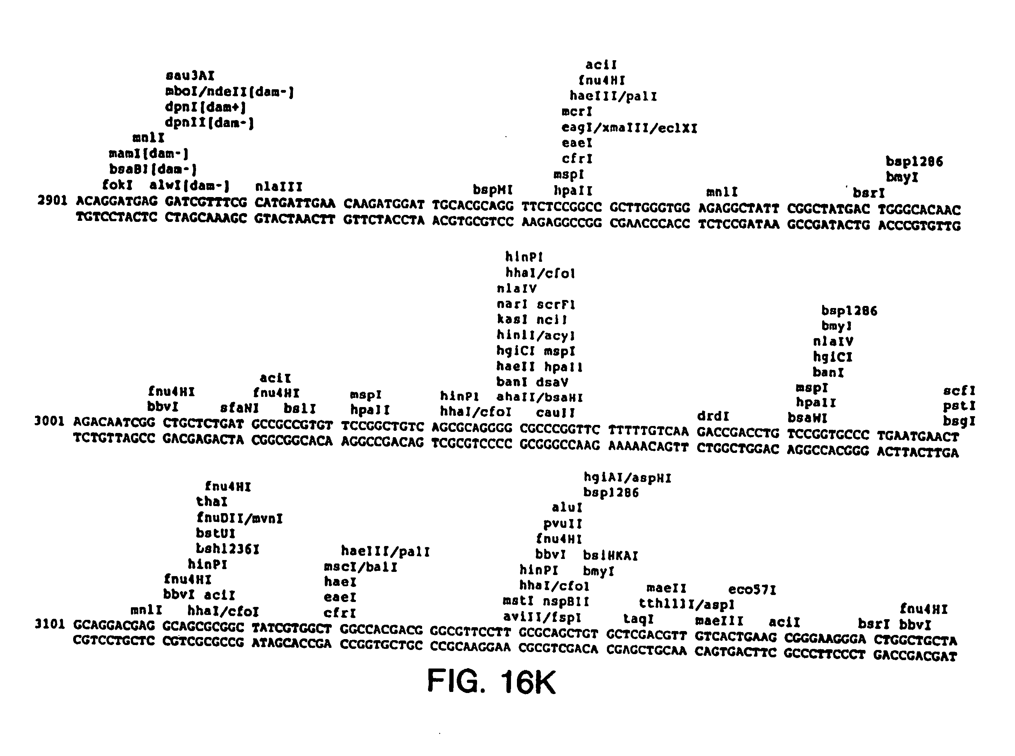

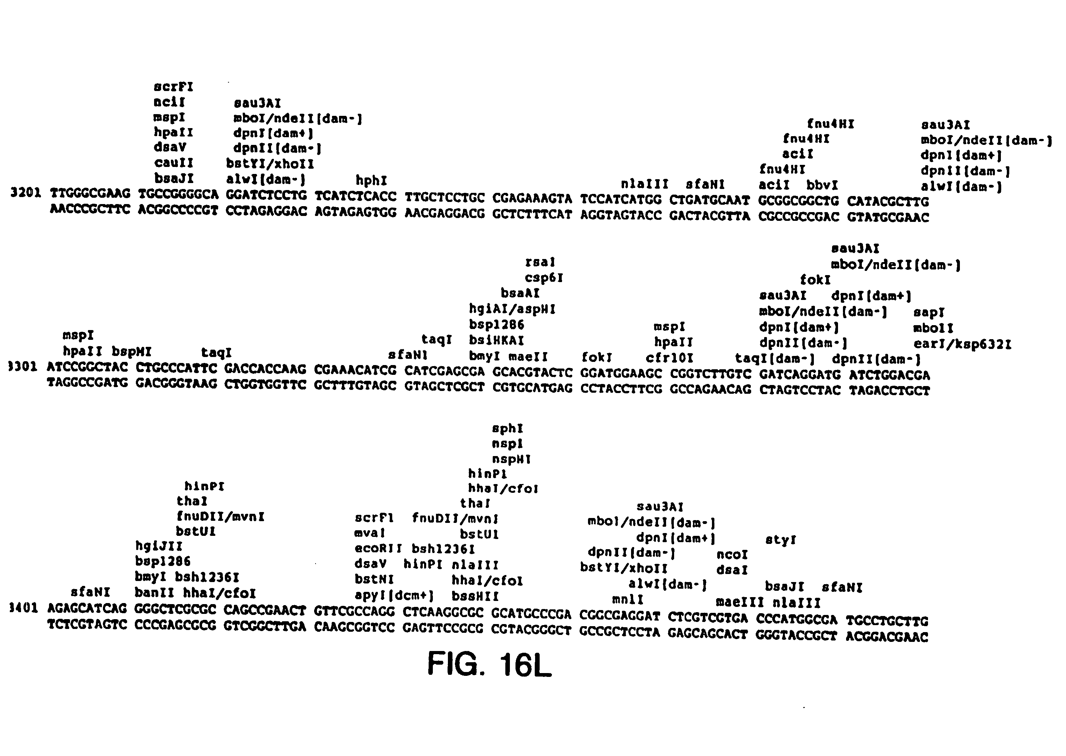

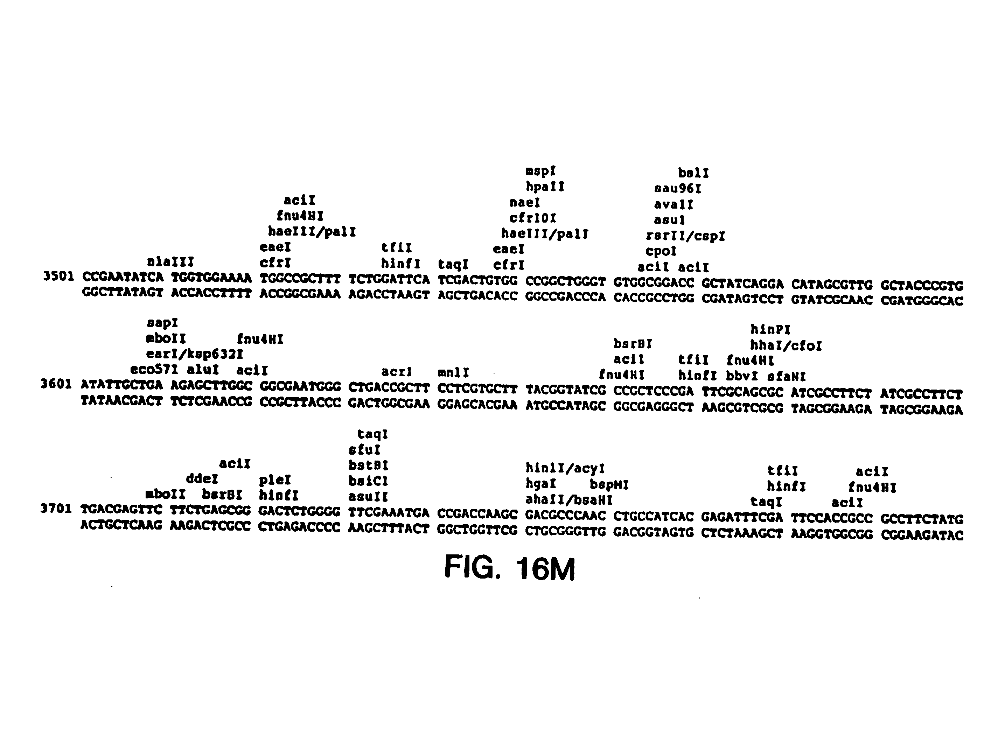

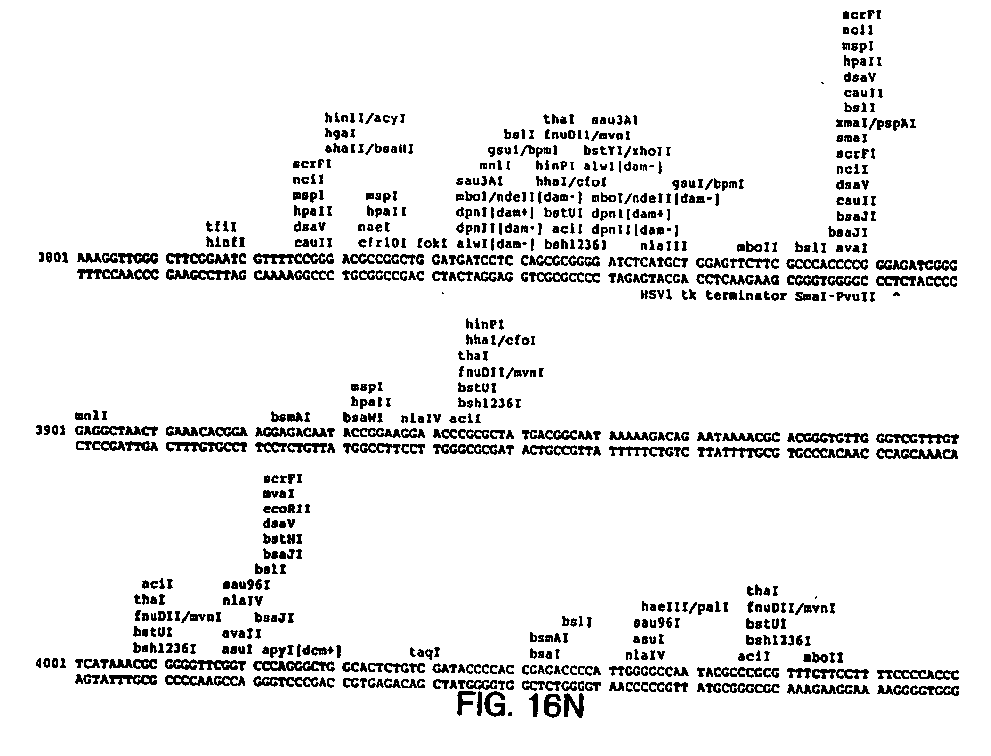

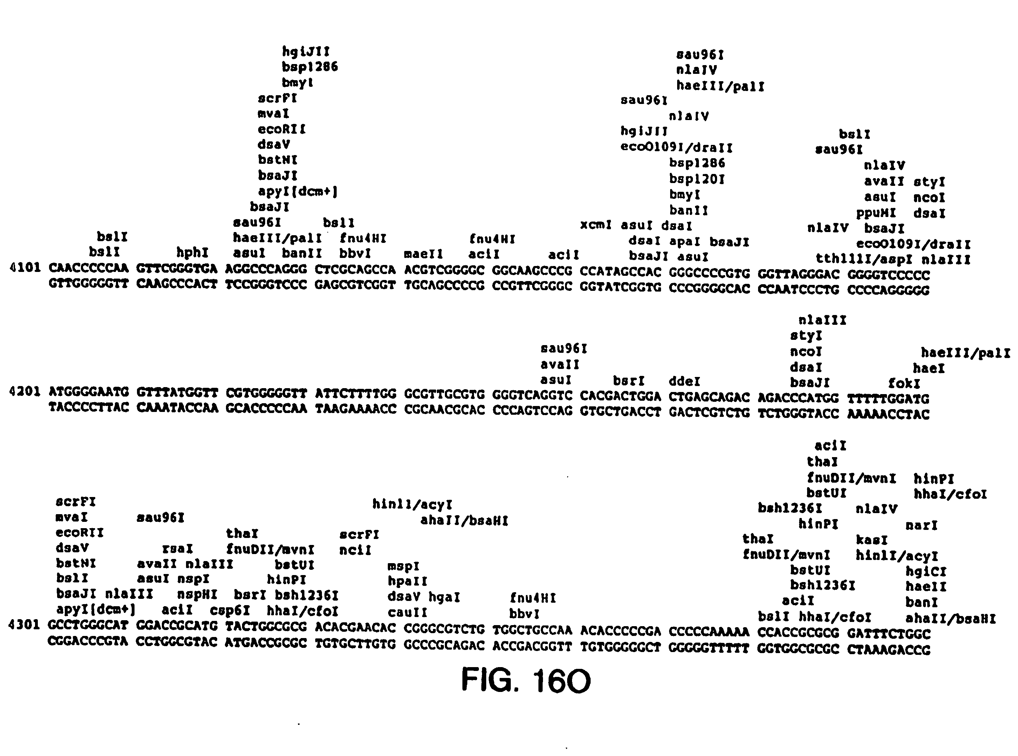

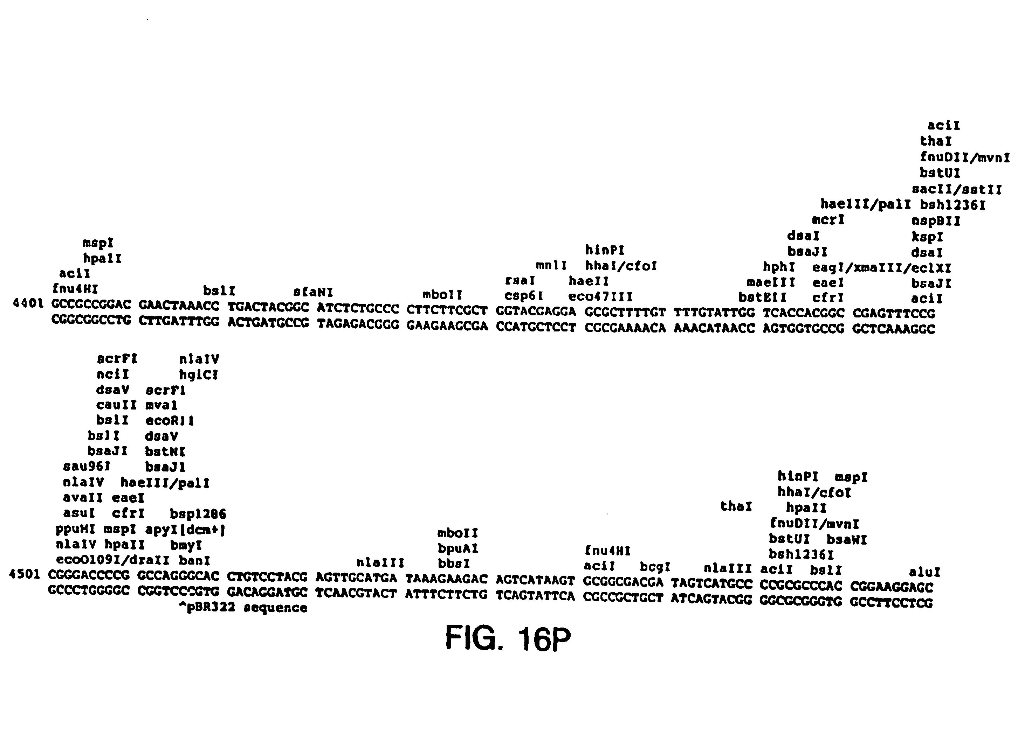

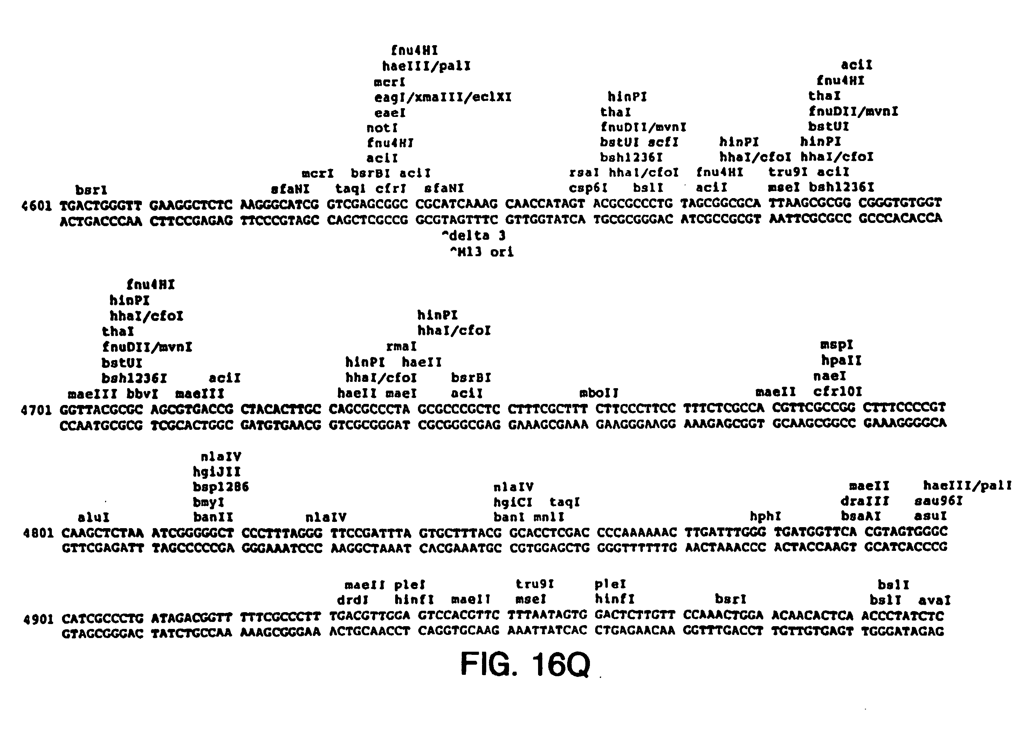

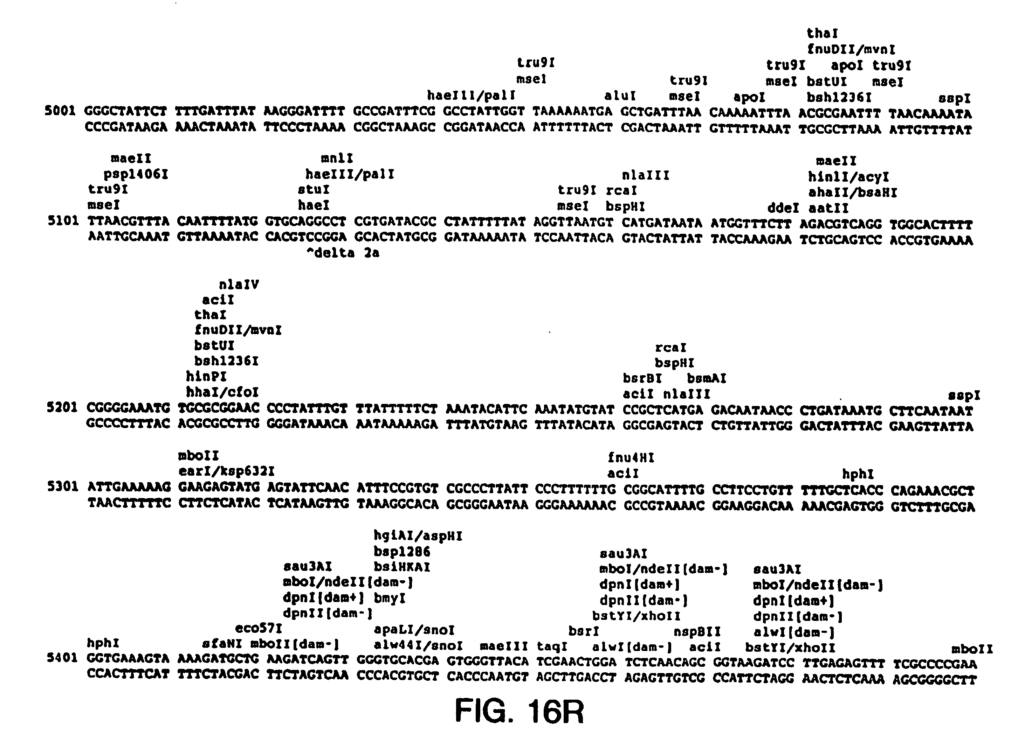

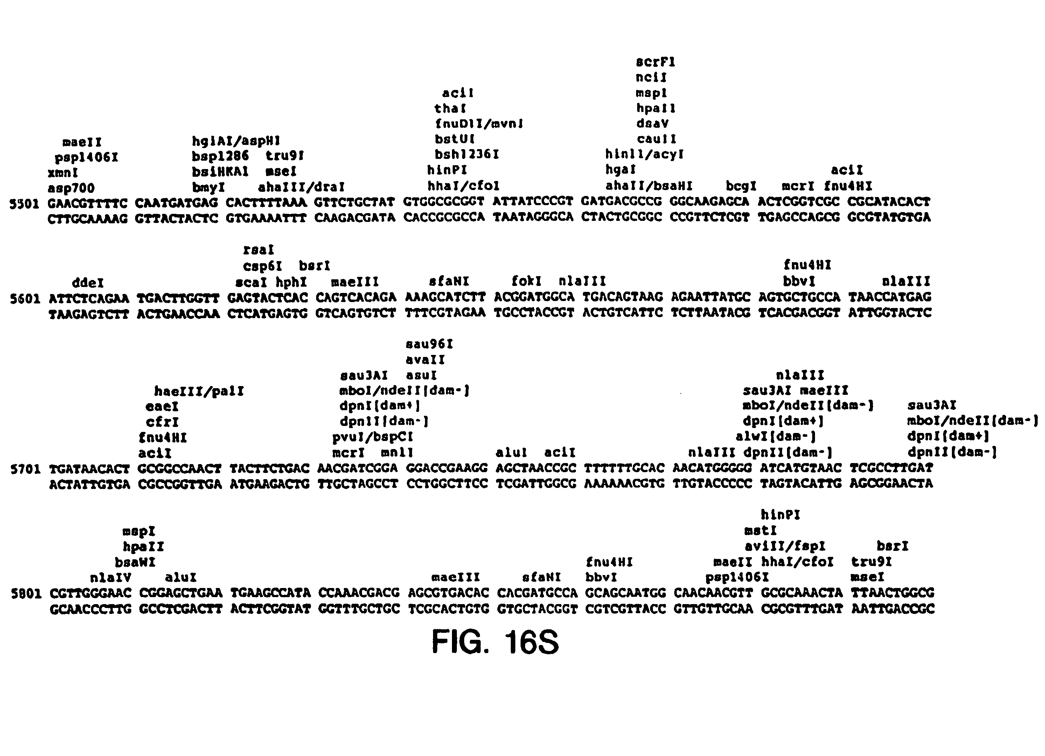

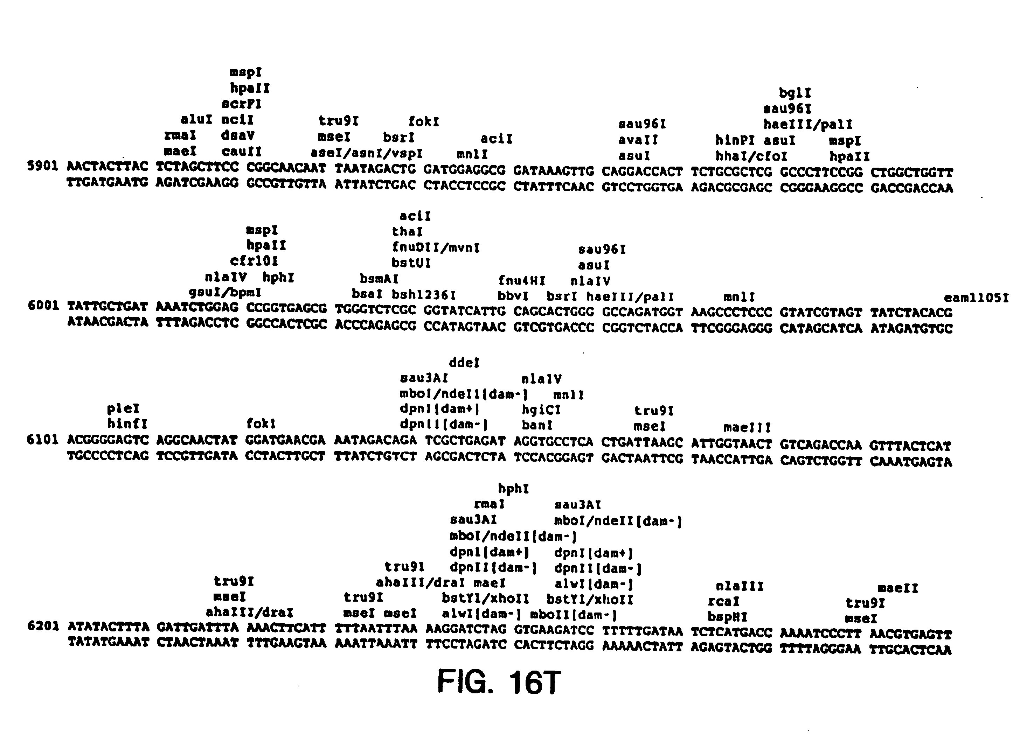

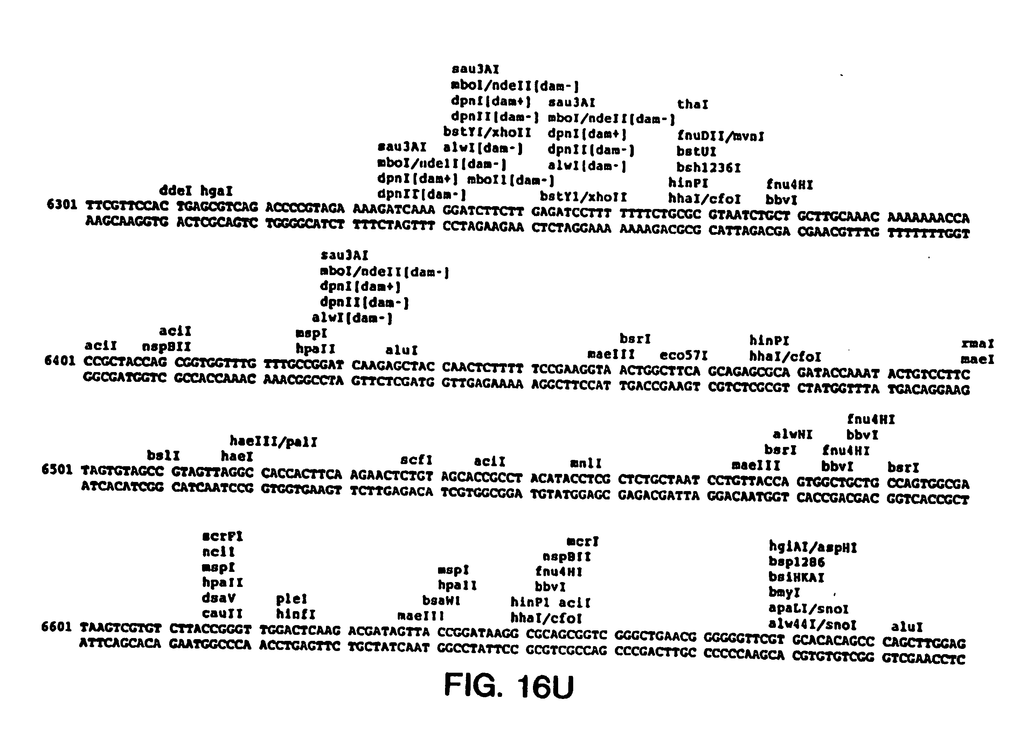

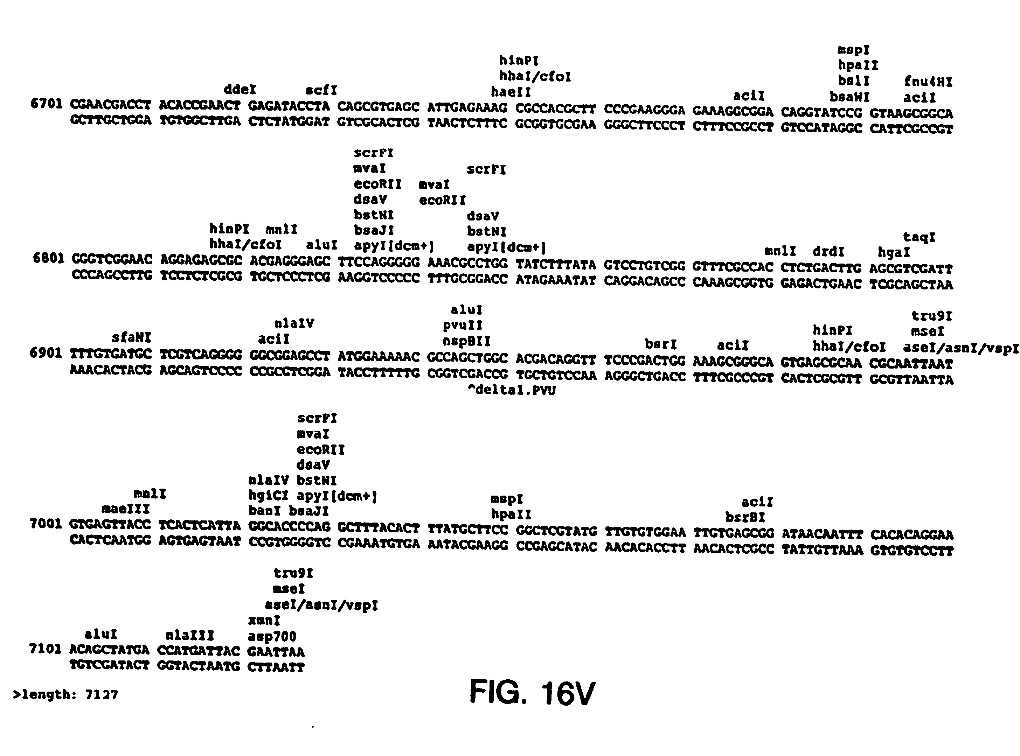

Using protein engineering techniques, the human OB protein was expressed as a fusion with the hinge, CH2 and CH3 domains of IgG1. DNA constructs encoding the chimera of the human OB protein and IgG1 Fc domains were made with the Fc region clones of human IgG1. Human OB cDNA was obtained by PCR from human fat cell dscDNA (Clontech Buick-Clone cDNA product). The source of the IgG1 cDNA was the plasmid pBSSK-CH2CH3. The chimera contained the coding sequence of the full length OB protein (amino acids 1-167 in Figure 16) and human IgG1 sequences beginning at aspartic acid 216 (taking amino acid 114 as the first residue of the heavy chain constant region (Kabat *et al., Sequences of Proteins of Immunological Interest* 4th ed. (1987)), which is the first residue of the IgG1 hinge after the cysteine residue involved in heavy-light chain bonding, and ending with residues 441 to include the CH2 and CH3 Fc domains of IgG1. There was an insert of codons for three amino acids (GlyValThr) between the OB protein and IgG1 coding sequences. If necessary, this short linker sequence can easily be deleted, for example by site directed deletion mutagenesis, to create an exact junction between the coding sequences of the OB protein and the IgG1 hinge region. The coding sequence of the OB-IgG1 immunoadhesin was subcloned into the pRK5-based vector pRK5tk-neo which contains a neomycine selectable marker, for transient expression in 293 cells using the calcium phosphate technique (Suva *et al., Science* 237:893-896 (1987)). 293 cells were cultured in HAM's: Low Glucose DMEM medium (50:50), containing 10% FBS and 2 mM L-Gln. For purification of OB-IgG1 chimeras, cells were changed to serum free production medium PS24 the day after transfection and media collected after three days. The culture media was filtered.

The filtered 293 cell supernatant (400 ml) containing recombinant human OB-IgG1 was made 1 mM in phenylmethylsulfonyl fluoride and 2 µg/ml in aprotinin. This material was loaded at 4°C onto a 1 x 4.5 cm Protein A agarose column (Pierce catalog # 20365) equilibrated in 100 mM HEPES pH 8. The flow rate was 75 ml/h. Once the sample was loaded, the column was washed with equilibration buffer until the A₂₈₀ reached baseline. The OB-IgG1 protein was eluted with 3.5 M MgCl₂ + 2% glycerol (unbuffered) at a flow rate of 15 ml/h. The eluate was collected with occasional mixing into 10 ml of 100 mM HEPES pH 8 to reduce the MgCl₂ concentration by approximately one-half and to raise the pH. The eluted protein was then dialyzed into phosphate buffered saline, concentrated, sterile filtered and stored either at 4°C or frozen at -70 °C. The OB-IgG1 immunoadhesin prepared by this method is estimated by SDS-PAGE to be greater than 90% pure.

### EXAMPLE 12

### Preparation of PEG-OB

The PEG derivatives of the human OB protein were prepared by reaction of hOB protein purified by reverse phase chromatography with a succinimidyl derivative of PEG propionic acid (SPA-PEG) having a nominal molecular weight of 10 kD, which had been obtained from Shearwater Polymers, Inc. (Huntsville, AL). After purification of the hOB protein by reverse phase chromatography, an approximately 1-2 mg/ml solution of the protein in 0.1% trifluoroacetic acid and approximately 40% acetonitrile, was diluted with 1/3 to 1/2 volume of 0.2 M borate buffer and the pH adjusted to 8.5 with NaOH. SPA-PEG was added to the reaction mixture to make 1:1 and 1:2 molar ratios of protein to SPA-PEG and the mixture was allowed to incubate at room temperature for one hour. After reaction and purification by gel electrophoresis or ion exchange chromatography, the samples were extensively dialyzed against phosphate-buffered saline and sterilized by filtration through a 0.22 micron filter. Samples were stored at 4°C. Under these conditions, the PEG-hOB resulting from the 1:1 molar ratio protein to SPA-PEG reaction consisted primarily of molecules with one 10 kD PEG attached with minor amounts of the 2 PEG-containing species. The PEG-hOB from the 1:2 molar reaction consisted of approximately equal amounts of 2 and 3 PEGs attached to hOB, as determined by SDS gel electrophoresis. In both reactions, small amounts of unreacted protein were also detected. This unreacted protein can be efficiently removed by the gel filtration or ion exchange steps as needed. The PEG derivatives of the human OB protein can also be prepared essentially following the aldehyde chemistry described in EP 372,752 published June 13, 1990.

### EXAMPLE 13

### Murine Agonist Antibodies

Mice were immunized five times with 20µg of the WSX receptor immunoadhesin (see Example 2 above) resuspended in MPL-TDM (monophosphoryl lipid A/trehalose dicorynomycolate; Rabi, Immunochemical Research Inc.) into each foot pad. Three days after the last immunization, popliteal lymphoid cells were fused with mouse myeloma cells, X63-Ag8.8.653 cells, using 50% polyethylene glycol as described (Laskov *et al. Cell. Immunol*. 55:251 (1980)).

The initial screening of hybridoma culture supernatants was done using a capture ELISA. For the capture ELISA, microtiter plates (Maxisorb; Nunc, Kamstrup, Denmark) were coated with 50µl/well of 2µg/ml of goat antibodies specific to the Fc portion of human IgG (Goat anti-hIgG-Fc; Cappel), in PBS, overnight at 4°C and blocked with 2x BSA for 1 hr at room temperature. Then, 50µl/well of 2µg/ml of WSX receptor immunoadhesin was added to each well for 1 hr. The remaining anti-Fc binding sites were blocked with PBS containing 3% human serum and 10µg/ml of CD4-IgG for 1 hr. Plates were incubated with 50µl/well of 2µg/ml of anti-WSX receptor monoclonal antibody (or hybridoma culture supernatant) for 1 hr. Plates were then incubated with 50µl/well of HRP-goat anti-mouse IgG. The bound enzyme was detected by the addition of the substrate (OPD) and the plates were read at 490nM with an ELISA plate reader. Between each step, plates were washed in wash buffer (PBS containing 0.05% TWEEN 20^{TM}).

Agonist antibodies were screened for using the KIRA ELISA described in WO95/14930. A chimeric receptor comprising the extracellular domain of the WSX receptor and the transmembrane and intracellular domain of Rse receptor (Mark *et al*., *Journal of Biological Chemistry* 269(14):10720-10728 (1994)) with a carboxyl-terminal herpes simplex virus glycoprotein D (gD) tag was produced and dp12.CHO cells were transformed therewith as described in Example 4 of WO95/14930.

The WSX/Rse.gD transformed dp12.CHO cells were seeded (3x10⁴ per well) in the wells of a flat-bottom-96 well culture plate in 100µl media and cultured overnight at 37°C in 5% CO₂. The following morning the well supernatants were removed and various concentrations of purified mAb were then added to separate wells. The cells were stimulated at 37° C for 30 min. and the well supernatants were decanted. To lyse the cells and solubilize the chimeric receptors, 100 µl of lysis buffer was added to each well. The plate was then agitated gently on a plate shaker (Bellco Instruments, Vineland, NJ) for 60 min. at room temperature.

While the cells were being solubilized, an ELISA microtiter plate (Nunc Maxisorp, Inter Med, Denmark) coated overnight at 4°C with the 5B6 monoclonal anti-gD antibody (5.0 µg/ml in 50 mM carbonate buffer, pH 9.6, 100 µl/well) was decanted and blocked with 150 µl/well of Block Buffer containing 2% BSA for 60 min. at room temperature. After 60 minutes, the anti-gD 5B6 coated plate was washed 6 times with wash buffer (PBS containing 0.05 % TWEEN 20^{TM} and 0.01 % thimerosal).

The lysate containing solubilized WSX/Rse.gD from the cell-culture microtiter well was transferred (85µl/well) to anti-gD 5B6 coated and blocked ELISA well and was incubated for 2 h at room temperature. The unbound WSX/Rse.gD was removed by washing with wash buffer and 100 µl of biotinylated 4G10 (anti-phosphotyrosine) diluted 1:18000 in dilution buffer (PBS containing 0.5 % BSA, 0.05 % Tween-20, 5 mM EDTA, and 0.01 % thimerosal), *i.e*. 56 ng/ml was added to each well. After incubation for 2 h at room temperature the plate was washed and HRPO-conjugated streptavidin (Zymed Laboratories, S. San Francisco, CA) was added to each well. The plate was incubated for 30 minutes at room temperature with gentle agitation. The free avidin-conjugate was washed away and 100 µl freshly prepared substrate solution (tetramethyl benzidine (TMB); 2-component substrate kit; Kirkegaard and Perry, Gaithersburg, MD) was added to each well. The reaction was allowed to proceed for 10 minutes, after which the color development was stopped by the addition of 100µl/well 1.0 M H₃PO₄. The absorbance at 450 nm was read with a reference wavelength of 650 nm (ABS_{450/650}), using a vmax plate reader (Molecular Devices, Palo Alto, CA) controlled with a Macintosh Centris 650 (Apple Computers, Cupertino, CA) and DeltaSoft software (BioMetallics, Inc, Princeton, NJ).

Four of the 25 anti-WSX receptor monoclonal antibodies activated the chimeric WSX/Rse receptor in the KIRA ELISA. The antibodies were designated: 2D7, 1G4, 1E11 and 1C11.

To determine whether the four agonist anti-WSX receptor mAbs recognized the same or different epitopes, a competitive binding ELISA was performed as described in *Kim et al. J. Immunol. Method* 156:9-17 (1992) using biotinylated mAbs (Bio-mAb). Bio-mAb were prepared using N-hydroxyl succinimide as described in *Antibodies, A Laboratory Manual* Cold Spring Harbor Laboratory, Eds. Harlow E. and D. Lane, p. 341 (1988). Microtiter wells were coated with 50µl of Goat anti-hlgG-Fc and kept overnight at 4°C, blocked with 2% BSA for 1 hr, and incubated with 25 µl/well of human WSX receptor immunoadhesin (1µg/ml) for 1 hr at room temperature. After washing, a mixture of a predetermined optimal concentration of Bio-mAb bound and a thousand-fold excess of unlabeled mAb was added into each well. Following 1hr incubation at room temperature, plates were washed and the amount of Bio-mAb was detected by the addition of HRP-streptavidin. After washing the plates, the bound enzyme was detected by the addition of the substrate o-phenylenediamine dihydrochloride (OPD), and the plates were read at 490nm with an ELISA plate reader.

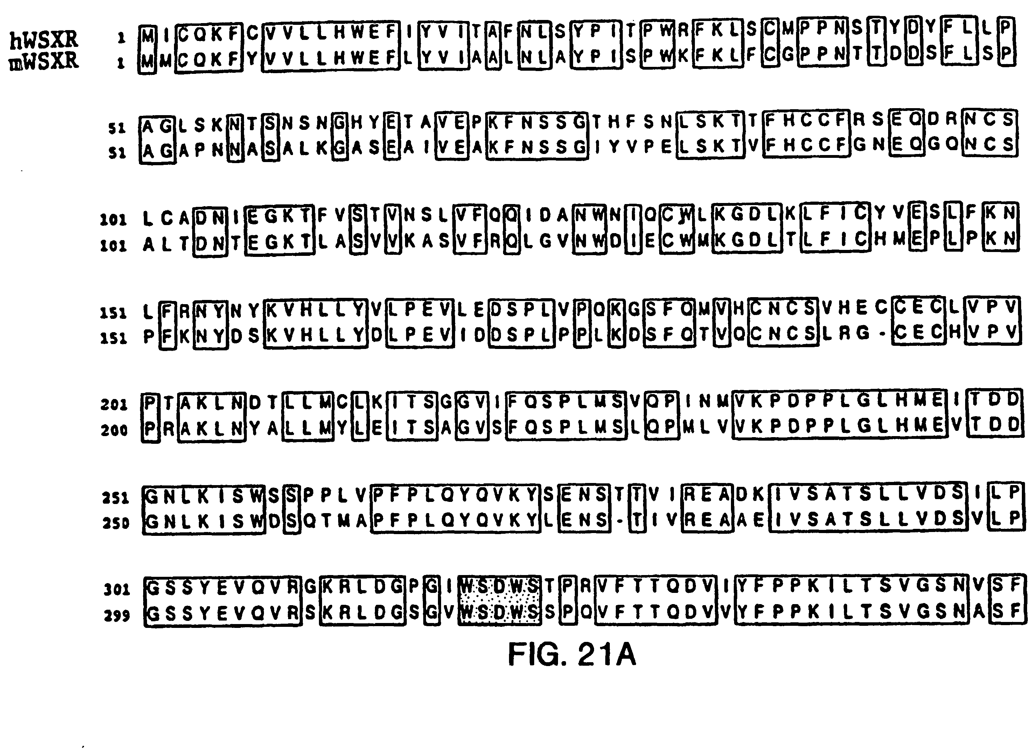

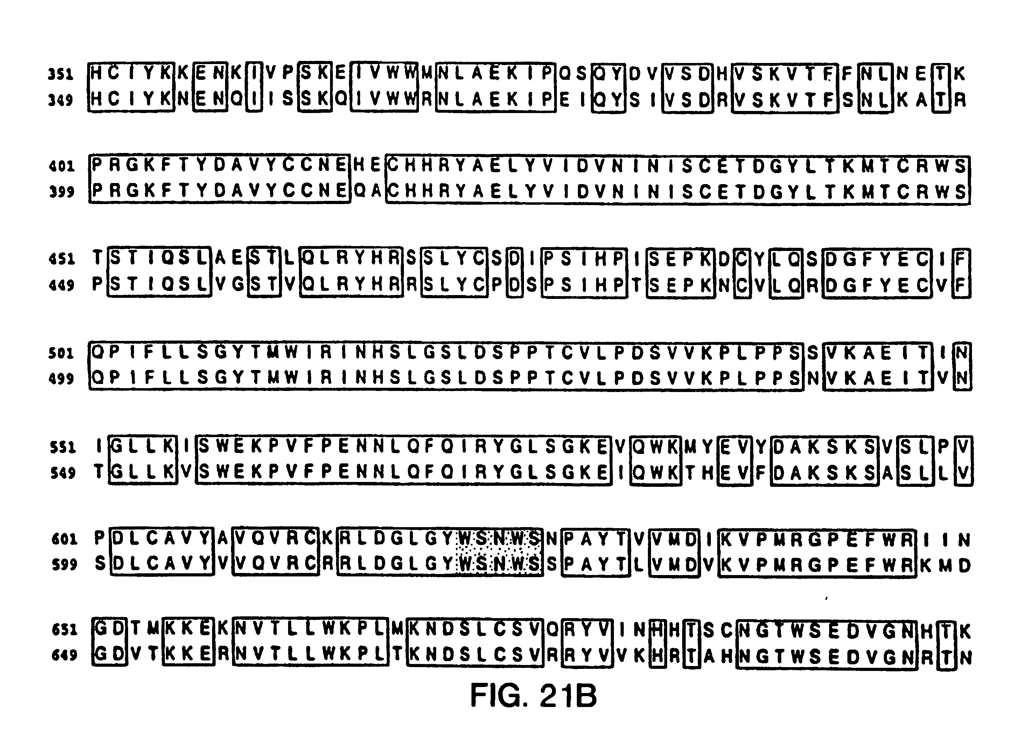

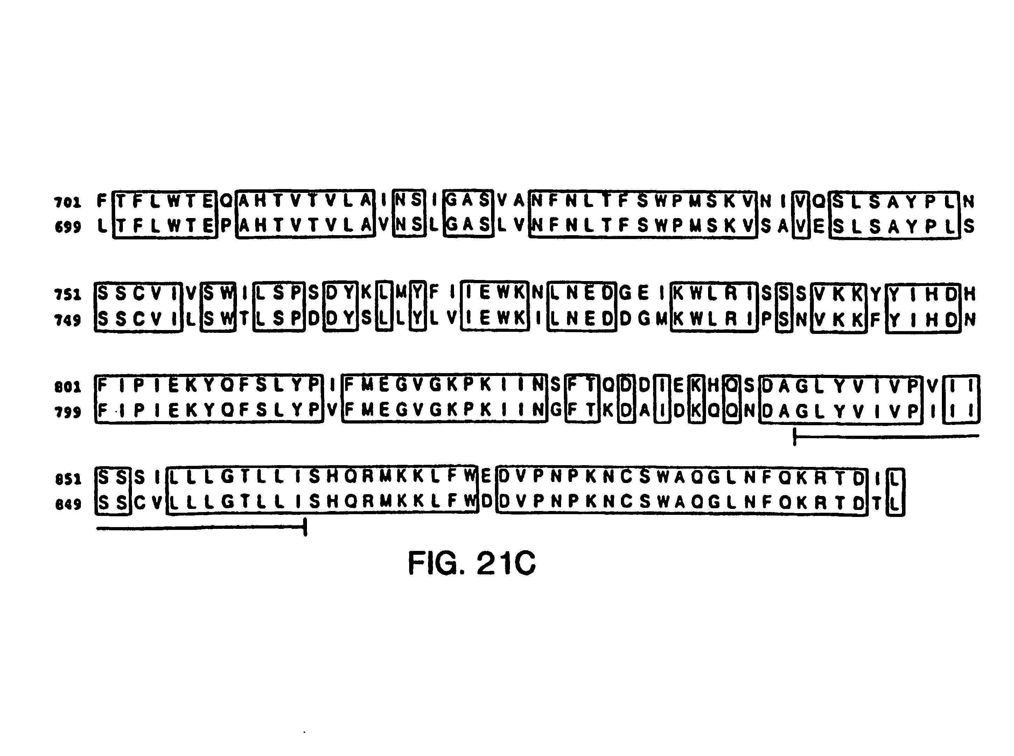

The ability of the mAbs to recognize murine WSX receptor was determined in a capture ELISA. Murine WSX receptor (Fig. 21) fused to a gD tag (see above) was captured by an anti-gD (5B6) coated ELISA plate. After washing, various concentrations of biotinylated mAbs were added into each well. Biotinylated mAbs bound to murine WSX receptor-gD were detected using HRP-streptavidin as described above.

To determine whether the antibodies bound membrane-bound receptor, FACS analysis was performed using 293 cells transfected with WSX receptor. 10⁵ WSX receptor-transfected 293 cells were resuspended in 100µl of PBS plus 1% fetal calf serum (FSC) and incubated with 2D7 or 1G4 hybridoma cell supernatant for 30 min on ice. After washing, cells were incubated with 100µl of FITC-goat anti-mouse IgG for 30 min at 4°C. Cells were washed twice and resuspended in 150µl of PBS plus 1% FCS and analyzed by FACscan (Becton Dickinson, Mountain View, CA). The antibodies 2D7 and IG4 bound to membrane WSX receptor according to the FACS analysis.

The properties of agonist antibodies 2D7 and 1G4 are summarized in the following table.

**TABLE 2**

| mAb | Isotype | epilope^{a} | hWSXR^{b} | mWSXR^{b} | Agonist^{c} |
|---|---|---|---|---|---|
| 2D7 | IgG1 | A | +++ | ++ | + |
| IG4 | IgG1 | B | +++ | + | + |

| | | | | | |
|---|---|---|---|---|---|
| ^{a} These mAbs are shown to recognize different epitopes by competitive binding ELISA. | | | | | |
| ^{b} These results are determined by ELISA (hWSXR is human WSX receptor and mWSXR is murine WSX receptor). | | | | | |
| ^{c} The agonistic activities were determined by KIRA ELISA. | | | | | |

### EXAMPLE 14

### Human Agonist Antibodies

Single-chain Fv (scFv) fragments binding to the human WSX receptor (hWSXR) were isolated from a large human scFv library (Vaughan *et al. Nature Biotechnology* 14:309-314 (1996)) using antigen coated on immunotubes or biotinylated antigen in conjunction with streptavidin-coated magnetic beads (Griffiths *et al*. *EMBO J.* 13:3245-3260 (1994); and Vaughan *et al.* (1996)). Briefly, immunotubes coated overnight with 10µg/ml human WSX receptor immunoadhesin (see Example 2 above) in phosphate buffered saline (PBS) were used for three rounds of panning. The humanized antibody, huMAb4D5-8 (Carter *et al. Proc. Natl. Acad. Sci. USA* 89:4285-4289 (1992)) was used to counter-select for antibodies binding to the Fc of the immunoadhesin. This was done by using 1mg/ml huMAb4D5-8 in solution for the panning steps. In addition, human WSX receptor extracellular domain (cleaved from the WSX receptor immunoadhesin with Genenase (Carter *et al. Proteins: Structure, Function and Genetics* 6:240-248 (1989)) was biotinylated and used for three rounds of panning. Individual phage following two or three rounds of panning were characterized by antigen-binding ELISA (Tables 3 and 4).

**TABLE 3**

| **Panning with human WSX receptor immunoadhesin-coated immunotubes** | | | | |
|---|---|---|---|---|
| Phage ELISA | | | # clones characterized | # BstNI fingerprints |
| Round | hWSXR | Fc | | |
| 2 | 74/96 | 0/96 | 74 | 11^{a} |
| 3 | 191/192 | 1/192 | 58 | 8^{a} |

| | | | | |
|---|---|---|---|---|
| ^{a} Total of 11 different clones identified. | | | | |

**TABLE 4**

| **Panning with biotinylated human WSX receptor** | | | | |
|---|---|---|---|---|
| Phage ELISA | | | # clones characterized | # BstNI fingerprints |
| Round | hWSXR | Fc | | |
| 2 | 8/96 | 0/96 | 8 | 4^{a} |
| 3 | 49/192 | 1 / 192 | 49 | 4^{a} |

| | | | | |
|---|---|---|---|---|
| ^{a} Total of 7 different clones identified. | | | | |

Clones binding to human WSX receptor were further characterized by BstNI fingerprinting of a PCR fragment encoding the scFv. A total of 18 clones were identified: 11 from the panning using immunotubes and 7 from the panning using biotinylated antigen (there was no overlap between these groups). The DNA for all 18 clones was sequenced.

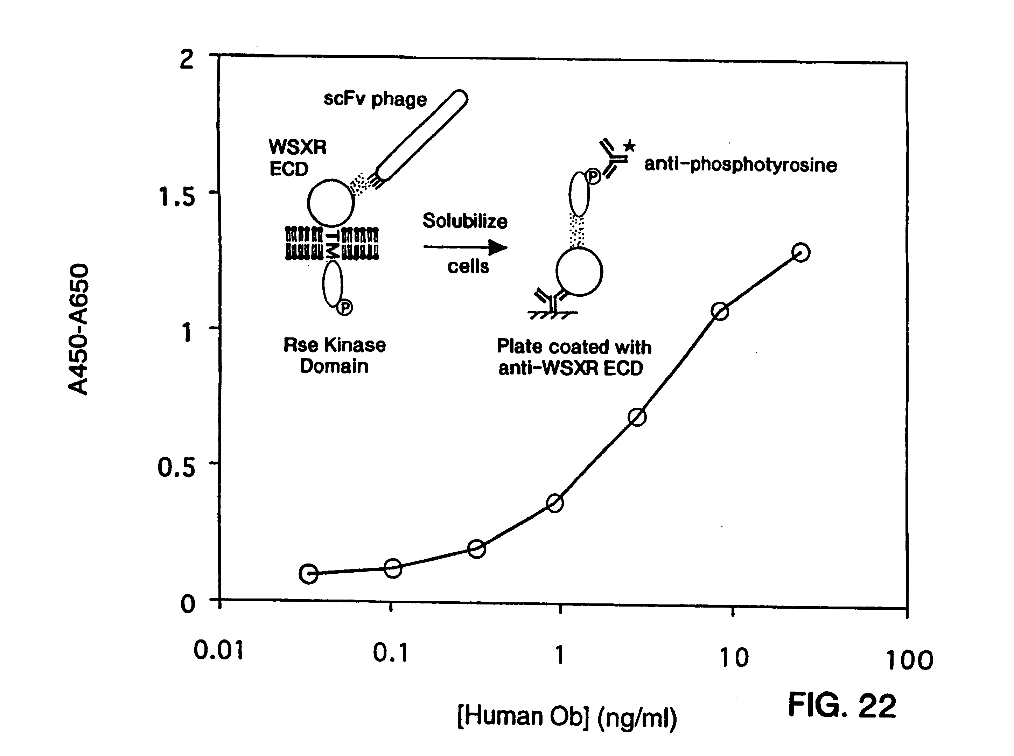

Anti-huWSXR clones obtained as described above were analyzed for agonist activity in a KIRA-ELISA assay (see above and Fig. 22) firstly as scFv phage and then as scFv. The scFv phage were PEG-precipitated (Carter *et al., Mutagenesis: A Practical Approach,* McPherson, M. ed. IRL Press, Oxford, UK, Chapter 1, pp 1-25 (1991)) and resuspended in PBS prior to screening. To prepare the scFv, DNA from the clones was transformed into 33D3 cells (a non-suppressor strain for expression of soluble protein). The cells were plated onto 2YT/2%glucose/50µg per ml of carbenicillin and incubated at 37°C overnight. A 5 ml culture (2YTG: 2YT, 2% glucose, 50µg/ml carbenicillin) was innorulated and grown at 30°C overnight. The next morning, the 5ml culture was diluted into 500ml 2YTG media and grown at 30°C until OD550 = 0.3. Then, the media was changed from 2YTG into 2YT/50µg/ml carbenicillin/2mM IPTG and grown at 30°C for 4-5 hrs for scFv production. The culture was harvested and the cell pellet was frozen at -20°C. For purification, the cell pellet was resuspended in 10ml shockate buffer (50mM TrisHCI pH8.5, 20% sucrose, 1mM EDTA) and agitated at 4°C for 1hr. The debris was spun down and supernatant was taken to be purified on Ni NTA Superose (Qiagen) column. MgCl₂ was added to the supernatant to 5mM and loaded onto 0.5ml Ni NTA Superose packed into a disposable columnn. The column was then washed with 2x5ml wash buffer 1 (50mM sodium phosphate, 300mM NaCl, 25mM imidazole pH 8.0) followed by 2x5ml wash 2 buffer (50mM sodium phosphate, 300mM NaCl, 50mM imidazole pH 8.0). The scFv was then eluted with 2.5ml elution buffer (50mM sodium phosphate, 300mM NaCl, 250mM imidazole, pH8.0). The eluted pool was buffer exchanged into PBS with a NAP5 column (Pharmacia) and stored at 4°C.

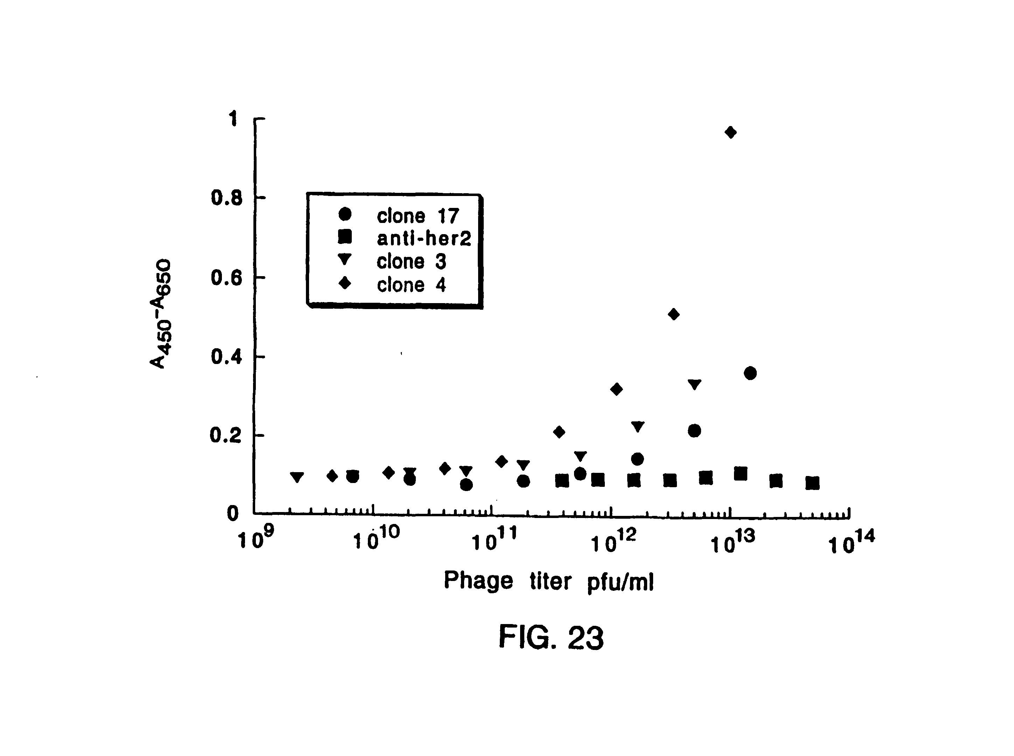

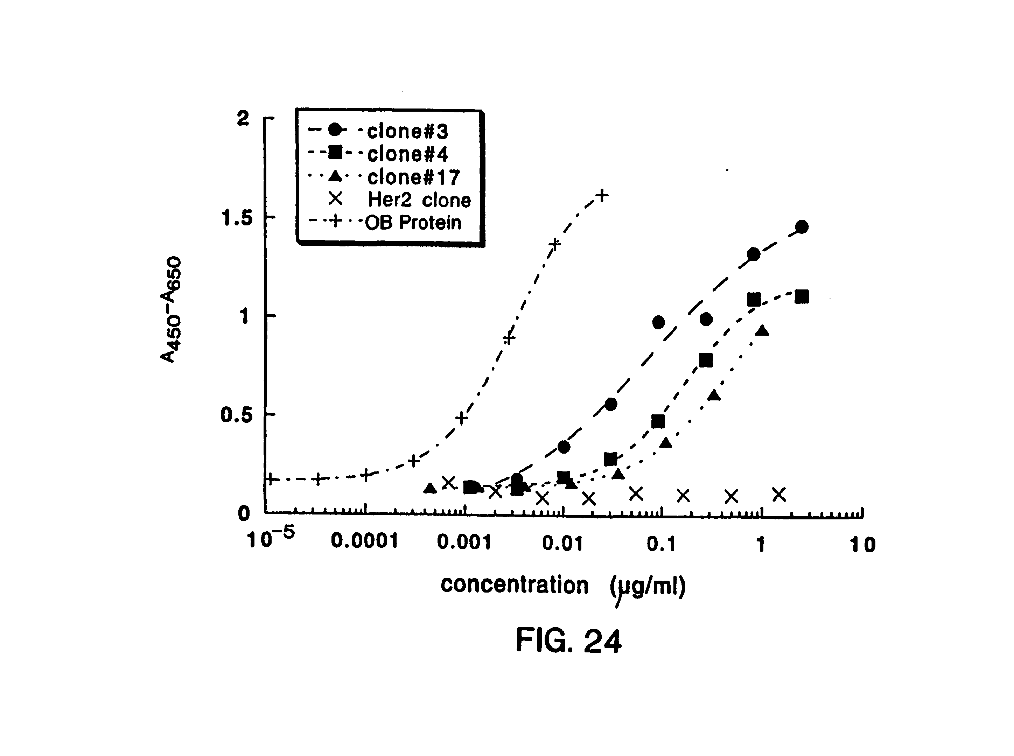

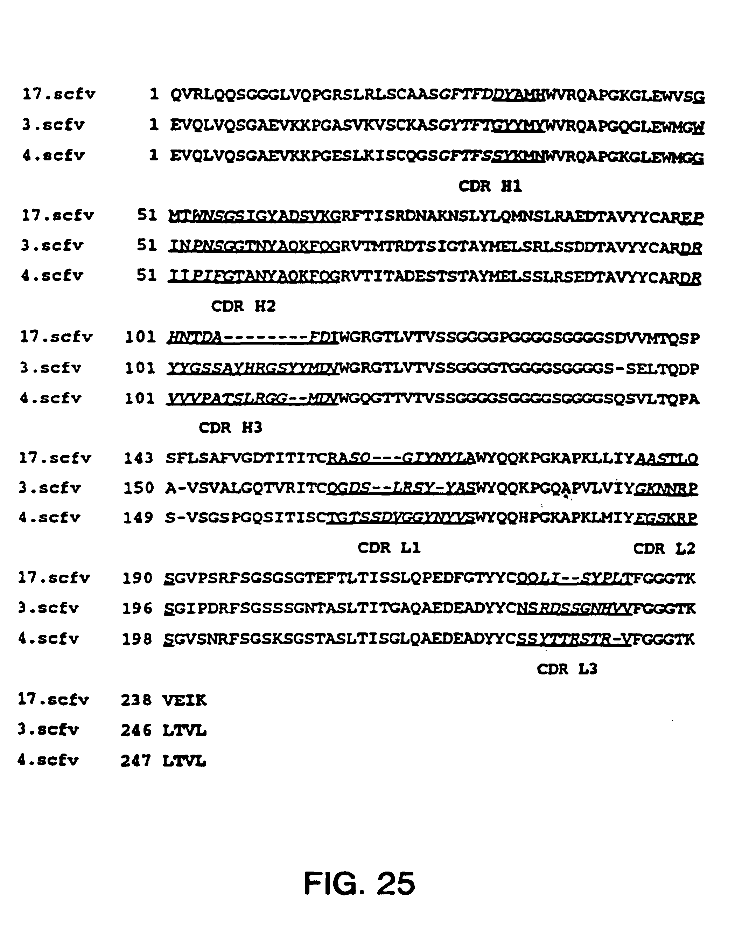

Clones #3, #4 and #17 were found to have agonist activity as phage and as scFv (see Figs. 23 and 24). The sequences of these agonist clones are shown in Fig. 25. The activity of the antibodies as F(ab')₂ in the KIRA ELISA was assessed, with clone #4 and clone # 17 showing enhanced activity as F(ab')₂. The ability of the antibodies to bind murine WSX receptor in a capture ELISA (see Example 13) was assessed. Clone #4 and clone # 17 bound murine WSX receptor in this assay.

## Claims

1. A method for enhancing the *in vitro* proliferation or differentiation of a cell comprising the OB receptor comprising exposing the cell to an amount of OB receptor ligand wherein the ligand is selected from OB protein or an OB agonist antibody which is effective for enhancing proliferation or differentiation of the cell.

2. The method of claim 1 wherein the OB receptor is the OB receptor variant 13.2 having an amino acid sequence according to SEQ ID NO:2.

3. The method of claim 1 or claim 2 wherein the cell is a hematopoietic progenitor cell.

4. The method of claim 3 wherein the cell is a CD34+ cell.

5. The method of any one of claims 1 to 4 which enhances proliferation of the cell.

6. The method of any one of claims 1 to 4 which enhances differentiation of the cell.

7. The method of any one of claims 1 to 6, wherein the OB receptor ligand is OB protein.

8. The method of any one of claims 1 to 6 wherein the OB receptor ligand is an anti-OB receptor agonist antibody.

9. The method of any one of claims 1 to 8 which enhances proliferation or differentiation of myeloid blood cell lineages.

10. The method of any one of claims 1 to 8 which enhances proliferation or differentiation of erythroid blood cell lineages.

11. The method of any one of claims 1 to 10 further comprising exposing the cell to a further cytokine.

12. Use of an anti-OB receptor agonist antibody in the preparation of a physiologically acceptable dosage form for therapeutic and\or prophylactic treatment of a mammal having a disorder **characterized by** a decrease in blood cells,
which treatment causes enhancement of the proliferation and/or differentiation of primitive hematopoietic cells to enhance repopulation of mature blood cell lineages.

13. The use of claim 12 wherein the mammal is human.

14. The use of claim 12 or claim 13 wherein the decreased blood cell levels are caused by chemo- or radiation therapy or bone marrow transplantation therapy.

15. The use of claim 12 or claim 13 wherein the disorder is a disease selected from: anemia; thrombocytopenia; hypoplasia; immune (autoimmune) thrombocytopenic purpura (ITP); HIV induced ITP.

16. A method for identifying a molecule which activates the OB receptor comprising exposing the OB receptor to a molecule suspected of being capable of activating the OB receptor and measuring activation of the OB receptor, wherein said method is used to screen for an agonist antibody having the property of inducing proliferation and/or differentiation of a cell which expresses the OB receptor at its cell surface

17. A method for identifying a molecule which activates the OB receptor comprising exposing the OB receptor to a molecule suspected of being capable of activating the OB receptor and measuring activation of the OB receptor, wherein said activation is determined by its ability to induce proliferation of CD34+ human blood cells in methylcellulose media.

## Patentansprüche

1. Verfahren zur Steigerung der In-vitro-Proliferation oder -Differenzierung einer Zelle, die den OB-Rezeptor umfasst, umfassend das Aussetzen der Zelle gegenüber einer Menge von OB-Rezeptorliganden, worin der Ligand aus OB-Protein oder einem OB-Agonistenantikörper ausgewählt ist, die zur Steigerung von Proliferation oder Differenzierung der Zelle wirksam ist.

2. Verfahren nach Anspruch 1, worin der OB-Rezeptor die OB-Rezeptorvariante 13.2 mit einer Aminosäuresequenz gemäß Seq.-ID Nr. 2 ist.

3. Verfahren nach Anspruch 1 oder Anspruch 2, worin die Zelle eine blutbildende Vorläuferzelle ist.

4. Verfahren nach Anspruch 3, worin die Zelle eine CD34+-Zelle ist.

5. Verfahren nach einem der Ansprüche 1 bis 4, das Proliferation der Zelle steigert.

6. Verfahren nach einem der Ansprüche 1 bis 4, das Differenzierung der Zelle steigert.

7. Verfahren nach einem der Ansprüche 1 bis 6, worin der OB-Rezeptorligand OB-Protein ist.

8. Verfahren nach einem der Ansprüche 1 bis 6, worin der OB-Rezeptorligand ein Anti-OB-Rezeptor-Agonistenantikörper ist.

9. Verfahren nach einem der Ansprüche 1 bis 8, das Proliferation oder Differenzierung von Knochenmarkblutzellstammbäumen steigert.

10. Verfahren nach einem der Ansprüche 1 bis 8, das Proliferation oder Differenzierung von Erythrozytenblutzellstammbäumen steigert.

11. Verfahren nach einem der Ansprüche 1 bis 10, weiters umfassend das Aussetzen der Zelle gegenüber einem weiteren Cytokin.

12. Verwendung eines Anti-OB-Rezeptor-Agonistenantikörpers bei der Herstellung einer physiologisch annehmbaren Dosierungsform für therapeutische und/oder prophylaktische Behandlung eines Säugetiers mit einer Störung, die durch einen Rückgang an Blutzellen charakterisiert ist, wobei diese Behandlung die Steigerung der Proliferation und/oder Differenzierung von primitiven blutbildenden Zellen verursacht, um die Repopulation von reifen Blutzellstammbäumen zu fördern.

13. Verwendung nach Anspruch 12, worin das Säugetier ein Mensch ist.

14. Verwendung nach Anspruch 12 oder Anspruch 13, worin die rückläufigen Blutzellkonzentrationen durch Chemo- oder Bestrahlungstherapie oder durch Knochenmarktransplantationstherapie verursacht werden.

15. Verwendung nach Anspruch 12 oder Anspruch 13, worin die Störung eine aus: Anämie, Thrombozytopenie, Hypoplasie, Immun- (Autoimmun-) thrombozytopenischer Purpura (ITP) und HIV-induzierter ITP ausgewählte Erkrankung ist.

16. Verfahren zur Identifikation eines Moleküls, das den OB-Rezeptor aktiviert, umfassend das Aussetzen des OB-Rezeptors gegenüber einem Molekül, von dem angenommen wird, dass es in der Lage ist, den OB-Rezeptor zu aktivieren, und das Messen von Aktivierung des OB-Rezeptors, worin das Verfahren verwendet wird, um auf einen Agonistenantikörper mit der Eigenschaft, Proliferation und/oder Differenzierung einer Zelle, die den OB-Rezeptor an ihrer Oberfläche exprimiert, zu induzieren, zu screenen.

17. Verfahren zur Identifikation eines Moleküls, das den OB-Rezeptor aktiviert, umfassend das Aussetzen des OB-Rezeptors gegenüber einem Molekül, von dern angenommen wird, das es in der Lage ist, den OB-Rezeptor zu aktivieren, und das Messen von Aktivierung des OB-Rezeptors, worin die Aktivierung durch seine Fähigkeit bestimmt wird, Proliferation von menschlichen CD34+-Blutzellen in Methylcellulose-Medium zu induzieren.

## Revendications

1. Procédé pour intensifier la prolifération ou la différenciation in vitro d'une cellule comprenant le récepteur OB, comprenant l'exposition de la cellule à une quantité de ligand de récepteur OB, le ligand étant choisi parmi une protéine OB ou un anticorps agoniste de OB, efficace pour intensifier la prolifération ou la différenciation de la cellule.

2. Procédé selon la revendication 1, dans lequel le récepteur OB est le variant de récepteur OB 13.2 ayant une séquence d'acides aminés selon SEQ ID N° 2.

3. Procédé selon la revendication 1 ou la revendication 2, dans lequel la cellule est une cellule progénitrice hématopoïétique.

4. Procédé selon la revendication 3, dans lequel la cellule est une cellule CD34+.

5. Procédé selon l'une quelconque des revendications 1 à 4, qui intensifie la prolifération de la cellule.

6. Procédé selon l'une quelconque des revendications 1 à 4, qui intensifie la différenciation de la cellule.

7. Procédé selon l'une quelconque des revendications 1 à 6, dans lequel le ligand de récepteur OB est une protéine OB.

8. Procédé selon l'une quelconque des revendications 1 à 6, dans lequel le ligand de récepteur OB est un anticorps agoniste anti-récepteur OB.

9. Procédé selon l'une quelconque des revendications 1 à 8, qui intensifie la prolifération ou la différenciation des lignées de cellules sanguines myéloïdes.

10. Procédé selon l'une quelconque des revendications 1 à 8, qui intensifie la prolifération ou la différenciation des lignées de cellules sanguines érythrocytaires.

11. Procédé selon l'une quelconque des revendications 1 à 10, comprenant de plus l'exposition de la cellule à une autre cytokine.

12. Utilisation d'un anticorps agoniste anti-récepteur OB dans la préparation d'une forme posologique physiologiquement acceptable pour le traitement thérapeutique et/ou prophylactique d'un mammifère atteint d'un trouble **caractérisé par** une diminution des cellules sanguines, lequel traitement provoque une intensification de la prolifération et/ou de la différenciation des cellules hématopoiétiques primitives pour intensifier le repeuplement des lignées de cellules sanguines matures.

13. Utilisation selon la revendication 12, dans laquelle le mammifère est un être humain.

14. Utilisation selon la revendication 12 ou la revendication 13, dans laquelle la diminution des taux de cellules sanguines est provoquée par chimio- ou radiothérapie ou par thérapie de greffe de moelle osseuse.

15. Utilisation selon la revendication 12 ou la revendication 13, dans laquelle le trouble est une maladie choisie parmi : l'anémie ; la thrombocytopénie ; l'hypoplasie ; le purpura thrombocytopénique immun (autoimmun) (ITP) ; l'ITP induit par VIH.

16. Procédé pour identifier une molécule qui active le récepteur OB, comprenant l'exposition du récepteur OB à une molécule soupçonnée d'être capable d'activer le récepteur OB et la mesure de l'activation du récepteur OB, ledit procédé étant utilisé pour rechercher un anticorps agoniste ayant la faculté d'induire la prolifération et/ou la différenciation d'une cellule qui exprime le récepteur OB à sa surface cellulaire.

17. Procédé pour identifier une molécule qui active le récepteur OB, comprenant l'exposition du récepteur OB à une molécule soupçonnée d'être capable d'activer le récepteur OB et la mesure de l'activation du récepteur OB, ladite activation étant déterminée par sa faculté d'induire la prolifération de cellules sanguines humaines CD34+ dans un milieu de méthylcellulose.
